(19) Europäisches Patentamt
European Patent Office
Office européen des brevets

(11) EP 1 636 386 B1

(12) **EUROPEAN PATENT SPECIFICATION**

(45) Date of publication and mention of the grant of the patent:
**05.08.2015 Bulletin 2015/32**

(21) Application number: **04777046.6**

(22) Date of filing: **23.06.2004**

(51) Int Cl.:
*C12Q 1/68* (2006.01) *C07H 21/00* (2006.01)

(86) International application number:
**PCT/US2004/020336**

(87) International publication number:
**WO 2005/001141 (06.01.2005 Gazette 2005/01)**

(54) **METHODS AND NUCLEIC ACIDS FOR ANALYSES OF COLORECTAL CELL PROLIFERATIVE DISORDERS**

VERFAHREN UND NUKLEINSÄUREN FÜR ANALYSEN VON STÖRUNGEN DER PROLIFERATION VON KOLOREKTALZELLEN

METHODES ET ACIDES NUCLEIQUES POUR L'ANALYSE DE TROUBLES DE LA PROLIFERATION DE CELLULES COLORECTALES

(84) Designated Contracting States:
**AT BE BG CH CY CZ DE DK EE ES FI FR GB GR HU IE IT LI LU MC NL PL PT RO SE SI SK TR**

(30) Priority: **23.06.2003 US 603138**
**23.06.2003 US 602494**
**27.02.2004 EP 04090072**
**06.05.2004 EP 04090175**
**03.10.2003 US 679062**

(43) Date of publication of application:
**22.03.2006 Bulletin 2006/12**

(60) Divisional application:
**10174462.1 / 2 354 248**
**10174640.2 / 2 354 249**
**10174701.2 / 2 354 250**
**10174719.4 / 2 345 745**

(73) Proprietor: **Epigenomics AG**
**10829 Berlin (DE)**

(72) Inventors:
- **LOFTON-DAY, Cathy**
  **Brier, WA 98036 (US)**
- **MODEL, Fabian**
  **Seattle, WA 98133 (US)**
- **SLEDZIEWSKI, Andrew**
  **Shoreline, WA 98177 (US)**
- **RUJAN, Tamas**
  **D-13189 Berlin (DE)**
- **LEWIN, Jörn**
  **D-10787 Berlin (DE)**
- **DISTLER, Juergen**
  **D-10825 Berlin (DE)**

(74) Representative: **Zwicker, Jörk et al**
**ZSP Patentanwälte**
**Partnerschaftsgesellschaft**
**Radlkoferstrasse 2**
**81373 München (DE)**

(56) References cited:
**WO-A-02/24056** **WO-A-98/56952**
**WO-A-02/077272** **WO-A-02/081749**
**WO-A-03/014388**

- **CROSS S H ET AL: "PURIFICATION OF CPG ISLANDS USING A METHYLATED DNA BINDING COLUMN" NATURE GENETICS, NEW YORK, NY, US, vol. 6, no. 3, 1 March 1994 (1994-03-01), pages 236-244, XP000578157 ISSN: 1061-4036 -& DATABASE EMBL [Online] 18 October 1995 (1995-10-18), "H.sapiens CpG island DNA genomic Mse1 fragment, clone 40c10, forward read cpg40c10.ft1k ." XP002434963 retrieved from EBI accession no. EMBL:Z58446 Database accession no. Z58446 -& DATABASE EMBL [Online] 18 October 1995 (1995-10-18), "H.sapiens CpG island DNA genomic Mse1 fragment, clone 40c10, reverse read cpg40c10.rt1a ." XP002434964 retrieved from EBI accession no. EMBL:Z58447 Database accession no. Z58447**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- YAN P S ET AL: "CpG island arrays: an application toward deciphering epigenetic signatures of breast cancer" CLINICAL CANCER RESEARCH, THE AMERICAN ASSOCIATION FOR CANCER RESEARCH, US, vol. 6, no. 4, April 2000 (2000-04), pages 1432-1438, XP002245955 ISSN: 1078-0432
- EBERT ET AL: "Aristaless-like Homeobox-4 Gene Methylation Is a Potential Marker for Colorectal Adenocarcinomas" GASTROENTEROLOGY, ELSEVIER, PHILADELPHIA, PA, US, vol. 131, no. 5, 11 November 2006 (2006-11-11), pages 1418-1430, XP005725528 ISSN: 0016-5085
- DATABASE EMBL [Online] 15 May 2003 (2003-05-15), "Homo sapiens SLIT and NTRK-like family, member 1, mRNA (cDNA clone MGC: 51091 IMAGE:4816570), complete cds." XP002328022 retrieved from EBI accession no. EM_HUM:BC051738 Database accession no. BC051738
- DATABASE EMBL [Online] 17 September 2001 (2001-09-17), "Homo sapiens mRNA for KIAA1910 protein, partial cds." XP002328023 retrieved from EBI accession no. EM_HUM:AB067497 Database accession no. AB067497
- TOYOTA M ET AL: "CpG island methylator phenotype in colorectal cancer" PROCEEDINGS OF THE NATIONAL ACADEMY OF SCIENCES OF USA, NATIONAL ACADEMY OF SCIENCE. WASHINGTON, US, vol. 96, July 1999 (1999-07), pages 8681-8686, XP002307651 ISSN: 0027-8424
- VAN RIJNSOEVER M ET AL: "Characterisation of colorectal cancers showing hypermethylation at multiple CpG islands." GUT, vol. 51, no. 6, December 2002 (2002-12), pages 797-802, XP002328020 ISSN: 0017-5749
- RASHID ASIF ET AL: "CpG island methylation in colorectal adenomas" AMERICAN JOURNAL OF PATHOLOGY, vol. 159, no. 3, September 2001 (2001-09), pages 1129-1135, XP002328021 ISSN: 0002-9440

SEQUENCE LISTING

<110> Epigenomics AG

<120> Methods and nucleic acids for the analysis of colorectal cell proliferative disorders

<130> 536-26PCTEP

<140> US/10/562,383A
<141> 2005-12-23

<150> PCT/US04/20336
<151> 2004-06-23

<150> US 10/679,062
<151> 2003-10-03

<150> US 10/603,138
<151> 2003-06-23

<150> US 10/602,494
<151> 2003-06-23

<150> EP 04090175.3
<151> 2004-05-06

<150> EP 04090072.2
<151> 2004-02-27

<160> 286

<170> PatentIn version 3.5

<210> 1
<211> 2280
<212> DNA
<213> Homo Sapiens

<400> 1

accagcacag aattgctacc ataggatcgc agcctaagca ctagagtgac attaattggt 60
catgcttaac tgcctcaaaa tcatttttta aataattaca ctgatactat aatagaaatc 120
atgggtactt attttacatt cagatggaag gcattattgg atatgtatta aaaaaaagac 180
cccctgaaaa aaataaaata aaataaaaca tcaccatcaa aataaaagaa cccaaaacaa 240
cccctaaaaa cttccctcaa caaaatacat tgttaactca taaaatggac tgatgactag 300
ccatgcaaat gtcctaaata aaacctttac atttttttca cagttaactt atgctctgaa 360
ctgcctaccg atcacaaata atggcgaaat ggcactttct gattatactg tattttgttt 420

atagaaagtt tgatacgatg gaacttatca ggtaagaggg tgggtgctgt gaacgagatg 480
ccgtctccag tcgcgggggc gggcagagtc cctggagcgc gtggattcca tgcgagccat 540
gcagcacttt ttgtttttg tcagaagtca aagttactta tttacaatac attcatgcct 600
tcgtgcaact gcccatccct gcgtagccaa cagggagcca tcacggggct aatccacagg 660
ggaaaaatag atatctatct ctctatatag atgtggatat atgtatatat gtatagaacc 720
gcggcatcca accccacagg ccccggggcc gagggcgagg gcactgtcag ttcttccagc 780
agggtcgtgc tgggctttct gtcaaaaggg gctctcagca aagagcgagc tggctgcgct 840
ctcccagctc tccacagtct gctctttgtt tcaaggaggg agctaagtaa ggggtcaggc 900
cctttcggtt gtgcgagctc acagttattt atctacttat gcccatccag gctgatggcg 960
cggggatttg ggtacacgcc cctccagccc ccggggtgcc tgcggtgggg aaggatgtat 1020
cgccttccct ctgccctccc ctattggggt tggggtctta gtctgagagc gagtgagagc 1080
cacagtcata cactctgtgg gccccatctg cgttgtaagg cccattgtgc cagtaggaag 1140
agtcacagac tgtctgtagg gaattaatct cggacgcgga ggagttggca tctcgtctct 1200
tggaccgctt tcggttcctc aggataaaca cgagcatgcc caccacggtg aaggcggagg 1260
tgacaaacac cagcagcagt cccgggacca acaccgagat ggacaccctg ctggtgtcta 1320
ggtaggagtt ggagtgcgtc ccggtctccg ccaacccagt gctgttttta ctgtgcgaag 1380
ttaacgtggg cgagatccta gcgtacagct gagggcagat ctcgtcattg gagaggagca 1440
tgaaatcctt tctaaagaag ttcaccggcg tctcacactt gaggtcgctc atcagcactt 1500
cggaacccaa gcgttctgcc cactgcttga aaggcacaat tgtgcaggag cactcccagg 1560
ggtttccgtg gaggtctatc tggatgatgg aggttaactg gtccagcacc cctgccaccg 1620
ggaggtacat gaagtaattg ttgtgcaggc tgagtttaga gagcgagacc ccagcgaaca 1680
cgtccacagg cagggacctc agcaggttgt tgttgagaat gaggatcctc agtttgggca 1740
tggcattgaa agtgcccggg aggatgagct ggatagcgtt gtactccacg ttcaggtact 1800
ctaggttttg cagccccgcg aatttctccc gggacagcgt gtccaggtaa ttgctatcca 1860
tgtatagcca cctgaggtcc aaaaggttct tgaaagtgtt gttctctaca gtagcgatgt 1920
tattgttgcc cagatccaac agaatgaggt tcttgtaatc cacaaagtgc gattttcgga 1980
tgctgtggat cttgttatct cgtaggaaaa gctcctgcac gttagagagc ttgggcttca 2040

aatcagccaa gctgctcacg ttcctgttgt tgcagttcat ctttaaaccc gaccctggga 2100

tgtggtcgca gctgcagccc ccagggcagg gtaaactgtt agctaagggt ttgttcctgg 2160

agctacccgt cgctatcgct gctgtgggtc tgattttgat ctgccagttg cctgggatct 2220

ttgtacctcc gtttggagca gaccctggtg tggcatgatc ctcttgccca tttgtcttga 2280

<210> 2
<211> 2477
<212> DNA
<213> Homo Sapiens

<400> 2

tggcacagac atggccctta aagccaaaaa ccatgatgga gtgaaggaca gctgtgctca 60

gacctgcgtc ctgtcctctg gaggctgctg tggttctggg gatgagtcgg aggtgggcag 120

gcagcggtct ccggggcctg cagcagggag gccaccacct gcacagcaga acacagcacc 180

attccacagg gagcatcccg gggcgggcag caactctgga ggcacccccg ggaaggagcc 240

aggcatgggg gaggcggagg cgaccccaga agcagaggag ccgctgggga gccacggcct 300

tctgggtgtg gagctgtgga ggatgagtgc cacccctccc ttccgggcct tctactctgt 360

agggatccct acgtccagga ccatttgatt gtcaaggtct cggcaccctc gtcaggtcag 420

ctgggggcca aggcccctaa ctccgaggac tggggccgcc aacatgggca gcagcacagg 480

aggccgagag gaggtccaca agggctgcac ttccttcctg gctgcagtcc accccccaga 540

gcagggcggg cgtggagcca ctctcaccag tattgttggc cttgagggcc tggtgggcag 600

tgccgcctgg tgtgggggac agctcctgag caggctgccg cattcctggc actctggtct 660

ccctgggatt ctcaggtggc ccacgctcgg cagtgctcgc cttctgggct ctcagggctc 720

gtttctgttg cagctcctgc agtgggagca tcgctgagca ctgtccctgg gagaggacga 780

ccccaggctc cacagccaag ctgccctgag gctaagtgtc ccgggggccca gggagcccca 840

gcagcagccc cgcacggtca ggcctgggcc acggtgctgc tcagtggtcc ctgcgcggtc 900

agcacctgaa tggctgctcg cctggcttgg cgtgccttct cctcccgggc cttcctcctg 960

gctgcctctt tctgctggtg caggtccaag agggtcccct cgcctgaccg ctgccgctgc 1020

tcctgccaaa gaagccggtg ccatgtggcg tttacatctg gaagacggaa tccagaccca 1080

gaccagaggt ttccccacaa cgccctggag tcccctagag ttcgtggaat ctcagggcca 1140

gcagggaaag tgaaacccca ccaggacctt gaacctgcca tttctctgct ctacagagga 1200

tgagccctac gtcgcaggtg gccaacagtt ctggcgtggg ggagacagct ctgcccccac 1260

agaacgtcct cctgagactc tccagcctgg gatggaccca gcaggcgttt cctgacaccc 1320

aggccctgag gaccgccagt gaggaccacc ctgactgtcc gcggggaccc agggtcaaga 1380

aggatgctgc acccagagac ggggcccacg agcaatccta ggctttgatt ccacctgtcc 1440

cagaaactca aacccctggg attctgggcc agaccccccg ggggagcatg gagtgactga 1500

gacgcccaca ctgacctccc gcttggcctg aagcaagtgc tccaggcggg cagctcctgc 1560

tccgcgccgc tgcacctggt gccggtacca gcgctggatg gtgacagtgg cctggttcac 1620

ctggtggata aacctgcccg gagagcagga ctcagggcca agacaggacc agcgccccgg 1680

caggacccag ctggctgctg gtggaaggtc gagccgggga agagagaagc cggcggctgc 1740

actcaagtag cccttttcac aaggcaggac cagcccacct ggaccatttt cacatggcag 1800

gaccagccgc ctggaccagc tccactaaaa cccaaaacct tcttcctggg aaggtgccca 1860

ggggagagga aacgcctacc cacgcaggcc tgtgtggcct tatttacaat tgccaggaag 1920

tgggaagagt tcaaatgccc atgacctggc tacagcgtga atactggatg gcgggaccct 1980

acccacaggc aacaggcccg ggactcaggc cagtgcagac cacaggccgt ggctccacag 2040

agagggtggt cggaacaggc agggctatga gggaacagac tggtggctgc tggcatctgg 2100

gcggggaagg agctcgaggg aagcctggtg gtgctgggcc gtttccttat cttggtctgc 2160

tgtgtccaca gacagcacta tacaccctgg tggaatctca tggaactgca cacttacaat 2220

gggcaccttc tattgtatac aaattatact aagtaaaact gattaagcaa aaaaaaaaat 2280

gcttcacctt ggctctcggg accatgtgtg gattctctca gcaaaggcac taacagagaa 2340

cccagaacgt gtgagcccta gctcgggagc agtctgcccc gggcagaact ggggggcttc 2400

aaggggttgc gggcttctgg tccccttcc ctcgtgccac ccaccgcgtc tacaggcctg 2460

gtctccctag ccctaac 2477

<210> 3
<211> 3685
<212> DNA
<213> Homo Sapiens

<400> 3

caagcctcct tccccggcgc acaggcacgc agccacaggc caagctgcga cgcgagctcc 60

```
gcgcgcggga tctccgcaaa aggtccgccc gatgcgttgg gcgggaatcg agcccgggtc      120
aactgcttgg aaggcaacta tgctcaccac tataccacca acgccgcacg gcgcgggcag      180
ccccgccgcg ccggcccggg gctcccacca gcgcgccgcc gacgcccggg gcaggccggc      240
cccgacgccc ggtccgtccg cccgcccgca gctccgcgct gccgcggcct ctccaaaggc      300
cgccccgcgc cccaccggtg caaggccagc gcggctgacc cgttacgccc gcctgcctct      360
gggggcgctc tcgctcactc gccgcctcgg gcccccagag cccttcccca acaggtgccc      420
gggggggaaa cggccggcgc cggcagggtc cccactccta cgcttcccac cgggcgcagg      480
gccccgtgtg tcgcagaaag cctcttccga caaccacttt gagccggcag ctcgttgggc      540
gccgtgcagg gccagaggag gcctcccggc tgccgctacc aagccgcgga cgagaagaaa      600
agacaggagg ccaaggaggc ccagaaggcc aaagggccag gcaggccagg cccgagatgg      660
caccctgcga ctagctggag ggcggaggaa ggagaaggag ggcccacagg ctgggtccga      720
ggcggcggcc caaaaagcac ggctgcctcc ccgtcgggga atcgaacccc ggtctcccgc      780
gtgacaggcg gggatactca ccactatact aacgaggacg acggcgacgg tcgccgggac      840
gccagacccc actccgaccg cggacgccta gccctgcctt gatcccctcc cccgacggca      900
ggggccgggc gcgtgctcgc cttccacccg ccgcccgccg cccgccaccc gccacacgcc      960
acccgtcacc tgccacccgc cccccgccac cgttggcacg acctaccccg acacccaaca     1020
aagcaccctg cgatcccgct gggacccgga gccggagccg gaccccgaca ggtaccggag     1080
cggcgtggaa cctccccgcg cgccctgcct gctgtctcca acgcggggat cgcgccgggg     1140
caggaggagg cgcgggcgaa acagtcaggc cgctgctcct aggacgcggt gggcgcacgc     1200
cctgcggggt tcggcgagcg gaggcgcggg ggctggggcg tgcgccggcg gcggccggcc     1260
cgacgcggac cctttgggtc cggggtgggg acgcgggggc gtccacgcca acgccagccg     1320
gctccgttca cttggcgccc gctccccccg cgcggtccgt cggttgcgca ccgaacccag     1380
acaggcgccg gccaagggcg caggcgttcg cgccgggtcc cagccatgcc agcggcgaag     1440
cgccccggcg cgccgtcagg atggccgagc ggtctaaggc gctgcgttca ggtcgcagtc     1500
tcccctggag gcgtgggttc gaatcccact cctgacaagc cgaccttttg gcccgcccgc     1560
cggagggcaa cgcccatggc aaccctggag cacctttggc cgcttcctgc cttgagccct     1620
tgcccgctct ccagactcca gccccttga agcaagcctc caaaacgccg ccgcttctca      1680
```

ggcacgtccg ttcttcctgc ccacccgccg gctgtcgcag aaacagccca ggaccatgcg 1740
ccagcgcccg cgaccctcta ccaattgccc ttcggacaga cgccctcccc accacctcac 1800
acgccctctt ccctggcccc acacacagcg agcgaccgcg accaccttcc acgctcttcc 1860
ctgcctatct cctccgcccg ccttctcctc actcgcccaa acagacacag cccagattct 1920
tcccctattc ctccttttcc ctccttcctc ccaccggcct ccgcccaccg cccaccgcct 1980
tgaatcgccg ctgcgctgcc cagaggcgtc ctggcctgaa cagcccgccc ggtttcaccc 2040
tccaacttct gaccgctgag cagcagcgag cgactcgctc gtggagccgc acacacgtct 2100
cccaccagag gcacgccatc caacatcctg tcctttcctc cgacccctcg gaccccggcc 2160
gcgcattcca ttctgccgac accctagcca ggtcgccgat cccacctcgc tacctgtgct 2220
cccttcccgc taacacctgc ctgccggccc acctgcagcc cggacgcctg ccggccagag 2280
gcagcgggaa ccctgcacac agccgggcag gcgagtccaa acccggaaag acagcccaag 2340
aggaatcacg agcggaagcc ctagatcccc gtcacccgcc cacaaacgcc tggccccgcc 2400
gggaccagct ctgcgccaca gcgcatcccc acgcgggaag ccgcggcctg ggccgtccca 2460
gccacaccca gcgcgccttc tccagggtca gccagctgcg gctctgccga agcgctcctc 2520
cgctcctttc tcgcgctcca gcctccctac cagcccaggg ggccggaccc caagtgcgag 2580
ccggtggcgt gggtcagagc gcaggagcga ggcgcccacg gacctggtct gcgtttctga 2640
gccgcacgcc acggctgcga gacccgttcc ccatcgccgc ccccgctcgc tgacacaccc 2700
atcccgcctc tcacctgctg gtgacacaag tgagaaggct ggccccacgg tggtgaaaaa 2760
aaaaaacacc acacgaaaga aagaaagaaa gaaagaaaga aagaaagaaa gaaagaaaga 2820
aagaaagaaa gacagaaaga aacaacaaaa acaaaacaca aaaactctgg gtctgtgccg 2880
gggatccgcg ctcagcaagg cccgccacag caaatctgcc cacacgggca ttcgggcgcg 2940
ggccacggcc ggtccttccc ctggagaccc cggcgggcag tctctcgacc ctgggcggca 3000
gagaaagcgc aagatgggac gagtcggcct ctctccctcc gctctccctc cgcgccccgc 3060
ctcaggtccc tcgacgtgac gagagcctcc ccttctgctc gccccatcgg gccagcctct 3120
cgtggacgct gcaataggac ggaggcccac ggcaggcggt gaccagtgaa cggcggctgg 3180
tggcgagttc cgctgtgcca gcttccgttg gcgtttgcca tcggtgcatg ggtggttcag 3240
tggtagaatt ctcgcctgcc acgcgggagg cccgggttcg attcccggcc catgcagcac 3300

gccctcccat tttggtgctg cagcagcacc aaggcgtagc tgcgctcgcc tctgccgcct 3360

ccttacactc ggggcgcgcg agcgagtccg gcaccggctg cgctcccacg cgcgacggcc 3420

ctctgccctt tcttccgtgc ctctctcgac tgacttaggg atgagcctac cccccgcacc 3480

cacacacctt ggtgacaaca acccctccag acacgagagc gcgccagaca ccagaacttg 3540

gcagcctcct ggtcctgttt ctcttcattg ccctgccacc gcctctgccc gacgcatttc 3600

acttcacgga acaccgccag gcaccacggg cttgcagcca ctcgcaccac cccttctctt 3660

cacatttcac cgcctcgcct ctctc 3685

<210> 4
<211> 2407
<212> DNA
<213> Homo Sapiens

<400> 4

taaggtctgg gtattctcag gcagcaggga caaggtgggc ttttttcctg gttgctaaac 60

ccacgtcaaa gtcgagctca gggactggag ctcaagaaac ccaccgccca ttctccagtc 120

cgaccgggga cctgcatgca cctctgccgt gctgccctga gtcctccaat cctccacact 180

cttcctctgt tatgtacacg tctccaccca ggcctgcaaa agtcccagct tcctccaggg 240

gcagggaccc gcacgccggc ccagggcttg gcacgcgggg atgctgaaac agggccaggc 300

ctggtttcca gccgatcgtc agagtcccaa ggcccagcaa ccttcctcac aaaggcctcg 360

ttaagaggcg aggaaacaag agccgggaga ggggcgcgga acggcgggcg ggacgaacga 420

ccagctccgc gcctccggcc agctgcgtcg agccaggggc accgcggctg ttgtgcggct 480

ggaaatctag gaatgggaag gttcggggcc tgctcggctc cggaggcagc tggcgggtcg 540

tccctggcgg cgttggagcg gtcagtggca gccgggcacg ggcgaccggg tcgcccgggt 600

cgccctcaga ccgtgactcc cgaaaaacct tgcgggcggg gcgcgcccgc gccgtctctt 660

gccggaaggt gcgagttagt gcgctcgatt gtgggcgggg gcggaaagag gcgcgtttta 720

aagtggtaac agatggtttt cttatccaat aggattaaaa aatttgtcct tacccggccg 780

accgcggaag tagagtaggc gggcggccaa tggggacatg atgggggcg gagccgaggc 840

ctccgaagcg gaagtgggtt gctgttgagg cggcggcatc tttctcgagg agctctcctg 900

ggcggctgaa gaaggagctt cttctccgga gtgcgccggc ggtggcgcct gcggacctaa 960

ctagctccag gttaggccga gctttgcggg aaagcagcgg taagtcaggg ccttgcagat 1020

gcgaggttta ggcagcttcg cggcctacag aggcctcggc ccgcgcctct tgggggagcc 1080
gcgctgcgcg gcttgaccca gccgaggctt tgcagcccgg gacctcgagc cagctctggt 1140
cgctcgcact gccgtccgcg cgggcgcacc gagcccggct tggcgcgggc aacagaagtt 1200
aggaggtctg cgtctgggtc tcggctcacc ctgggggccc gcggccatgg ggcttagttc 1260
ctagcctagg aagggaaact gagactctgg gaggggcagg aacgccccca aggtcacttg 1320
gaaagtcggg caggatgtgc tgttaggggg aagacccggg cagggttttt gttccccgct 1380
gacgacgcct cctttgtgtg ttcgcgccgc cgccccgcca tcgtggggcc tgcgagtttg 1440
ccggggtgcg tgggccgcgt ggcggggcct tttgtaggtc gggaggatct gagtacgggt 1500
gcgggcctga ccgtgggggc gccgaggtcg cagtctaaaa cttagtaggg cctcgatttc 1560
cgggcgcgct tccgggcccc ggctggtggt tggtggaacg tgcgactgtg aggcttgcgg 1620
cccagccctg caccgctcgg gcccttcacc gctctggcgc gcctatagac aggtgtatga 1680
agattctcac gacccgaaac agagttgcta gtaaacaccg cttttccgcc tttgatccat 1740
cggggaagag ggaaaaggat agagcttggg caagccgttt tggtagggat ttcagctttt 1800
gtctttcact tgtcagttcc catagacgtt cacaaactta ataatcttcg ttctgtttct 1860
gcaccaagtt cttgagccag acgtagggtc tcagctctgg agcctggctt agactgtcca 1920
actgactggg gagactgagg tccagaaaag tgaagtggtc tgcccaaggt cacatagcca 1980
gctatttggc agcagatgag gttaagtcct acctgcaaga tttgggtttt gaattcattg 2040
accaggagtt ttgggaccac tgtcaataaa agagacattg aagggaatct tttgttactt 2100
tcttggtgat ttgcttttta atggacaagg acatattggg ttcagtttta tctgtgagtt 2160
tgaggtgaaa tagaggcatt cgagtagcaa gatatattgc tggcttttgt attgcctgaa 2220
tttgagcttc caaaaatctt actttaacac atcgtttatt gatcttttct tgaattacta 2280
cctttgtaag gaccttttgt aaacattgtt tttctaatct tcatgaaatc ttaatgccat 2340
acgtaaacta tttcttttta tataatgtat gcacatctgt gctttgtaca taaaatgagt 2400
aagattt 2407

<210> 5
<211> 2229
<212> DNA
<213> Homo Sapiens

<400> 5

```
tctttcctcg gcgctggctg gtgcgggttg gggtcaggtg gagaagccgc tctttgttaa      60
ggtgacagaa cgtgctgggg gtgggggccg gggccagggc cggtgcaact agggggccgc     120
tgccctttcc tggacacagt ggaagcttct tccgcatcac caaatttttg tcatcctttc     180
tgagggacct gcttccaggc agcacgcaag ttgttgtccc gggtttactc cgcacccctc     240
tactgggtga ggaaggagca tcttgaatgg agatgggggt gtccccggtt tatacatctg     300
cagagaagag gtgtgccggg ctgcacctct ggaggccgcg gtaactgata ttagagaaga     360
ccccggttgc agctgggaag gctcactggc tggaaagagg tgcctcctcc ttccagcaaa     420
gggccctgtt tggaagggct gcttctcacc tgtctagtgg caccacagga cggtcggctt     480
ccactcgaat tcccccggac ggtatcatca catagccggg tcctcgcagt gttggtttcc     540
caatccgatg actgtcacct cggtgaggac ctgtgctgat ggccggagaa ccctgcgctg     600
cgggcgcaca tggccaggtg gcgcctggca ggcgacgtcc gggtgcagga cggcgctctt     660
accgccccac cccaaaccgt tgcctgggcc taggtccttc ggcttcctga acaggggttt     720
ggggggctaa ggacgctgag gctccggggg caggaagttc tctctggtta agcgttctct     780
cttctctccg gcatacactc ccctacccac ccacctcgcc taccctcggg gcgagaggct     840
caccaaggca gggcgcgccc cccccatgaa tcatcccaag gcctctgagc cgcgggggct     900
ccgggcaact atccccctcc tctcctggcc tcaggcaccc cagtccaggg gtctgcagag     960
aagcccgaag cccggacaaa cgcgccggac gtcaacaacc tctcatccct ggcagcagca    1020
aaggccaata tatttccatt tcttatttca gtttgccacc aaaacaaagc tgcgcgcggc    1080
tgagggcagg aaggcgctga gaccgagaag aagggacgtc ccggagaaag tgcgcccagc    1140
tgatcttaga aaccagagtc ctccgggact tcgccgagat tttctgtagg gcgttttaat    1200
ctgttttcct actgcgtgcc ggcgtcgcag cgcgtgcggc tcagggcttg gtgactccgg    1260
cttagcccgg cggtcgcggc gaggttcctg gcgcagccgc ttggaacttc gcattagaat    1320
cgggaccgcg caaatgccct ggctgaagtg tcaccctatt caagaaacac tgctgtcagg    1380
aacaaaatgg ggtccccggt gctccgaagt atcttctgaa attttcttaa aacaacttac    1440
aaaaaatgtt tttgctttaa cgttttacaa cgtttaagga aacatgtaaa tggtctgttt    1500
ctttatcgag atggtcgtcc taactaacag tgtacacata cataacaatt cttccaactt    1560
```

tcctcctcag agctaagcac ttcactatat gtaaattata ataaagaaaa gattgtgcaa 1620

gatcatgcaa gtcgattgac ttaaaatatt gagttttaat ccaggccctc tgtttttcta 1680

tttaacaact tttgtgtttg gaccagactg gtgaagcagg ctatggaaat taacaaagta 1740

aaaaattaaa agcatcttcc ttcgccatcc ctccctccaa aattaaacaa cagtcgcccc 1800

ttcctgagca ggcttcagtc ccaggctcga gttttcctgc gatcacccca cagtcaccca 1860

cagcagctgt tgctgcttct gtcgggtttt cgtttctgcc ttctttgggt cgtctcttgt 1920

atacaaaaca caccccagtt ctctaactaa attcaaatac gaccccggca gaatttacac 1980

atttcgtggt gcatggattg tgtcggtgca ggggaaataa ataccctctg gtatttaacc 2040

actgagtcta attcgaaaaa tcgggactgg gcccctaggc ggcaccccag gggctccaac 2100

ctggcccgcg cctccccaga ccttggcgct gagagcgctg cttttgcggg tgggtggacg 2160

gagaggtaac aatctgcttt caacaaaaac ctgtcgccac cgaatcgaaa gcgaaaggga 2220

agggagaag 2229

<210> 6
<211> 6887
<212> DNA
<213> Homo Sapiens

<400> 6

cgccgcccgt gggccgccgg ccggccgcag ctcgctggcg acgagggcac tacagttgct 60

ctgaccgcgt agattatgca tgtcccggcc tcgggaattt accatgcatt agaatacatt 120

agcgcctgca ttttaaaagg ctaaactatt ggctcccagc tagggactct cggtaagtgg 180

cttgttagtg acgagtgttt gtctatactg gcacatagcg gagtcttttg ctcccggctt 240

actcgcctcc aggaaagctt tggggtgagg cgaaggcgat tgaagcaatg ccccttcccc 300

cagatcgcag ctgctcaggg gggacacagc acggcatctt tcaccgaatc tctctcgctc 360

gctcgcactc cagcctccct ctccccagcg accccccac cttctcctcc ctccctctcc 420

ttgacgtttg attccagtag caaaggaggt aaaaaaggca ccgagccgtc agccaaacct 480

gaaaagtgcg gccccgcccc ctccacagcc actggtagct tcccgtggaa ggcccgcctc 540

ccggggcagc tgcggcctcg gagtggttgc gcttggcgcc cgtcggcgt ggccccgccc 600

caggtccggg agggtaggtt ggctgccccg gcgagcggca gagcccttct ggacagctcc 660

cgctcaccca aacagaagac gtcggcgccg gagcgggctc ggacatggcg aggctgcgag 720

ccggcccgag cggcggggcc cggtgatccc tccctccctc cccgtcccct cccctctccc 780

gcacgcacgc cccgtccgcc cccaccccgc ccccaccccg ggcgagcccg cccgcagccc 840

ggggcgcaca cccgcacgcg cactcctctc cactcactcc cgcgcccgcc cccactcccg 900

cagccgagcc ccgccacgcg cgccttgccc gcccgccggc cgcccccgcc gcccccgccg 960

cccccgggcc ctgatggact gaatgaaggc tgcctacacc gcctatcgat gcctcaccaa 1020

agacctagaa ggctgcgcca tgaacccgga gctgacaatg gaaagtctgg gcactttgca 1080

cgggccggcc ggcggcggca gtggcggggg cggcggcggg ggcggcgggg gcggcggcgg 1140

gggcccgggc catgagcagg agctgctggc cagccccagc ccccaccacg cgggccgcgg 1200

cgccgctggc tcgctgcggg gccctccgcc gcctccaacc gcgcaccagg agctgggcac 1260

ggcggcagcg gcggcagcgg cggcgtcgcg ctcggccatg gtcaccagca tggcctcgat 1320

cctggacggc ggcgactacc ggcccgagct ctccatcccg ctgcaccacg ccatgagcat 1380

gtcctgcgac tcgtctccgc ctggcatggg catgagcaac acctacacca cgctgacacc 1440

gctccagccg ctgccaccca tctccaccgt gtctgacaag ttccaccacc ctcacccgca 1500

ccaccatccg caccaccacc accaccacca ccaccagcgc ctgtccggca acgtcagcgg 1560

cagcttcacc ctcatgcgcg acgagcgcgg gctcccggcc atgaacaacc tctacagtcc 1620

ctacaaggag atgcccggca tgagccagag cctgtccccg ctggccgcca cgccgctggg 1680

caacgggcta ggcggcctcc acaacgcgca gcagagtctg cccaactacg gtccgccggg 1740

ccacgacaaa atgctcagcc ccaacttcga cgcgcaccac actgccatgc tgacccgcgg 1800

tgagcaacac ctgtcccgcg gcctgggcac ccccacctgcg gccatgatgt cgcacctgaa 1860

cggcctgcac cacccgggcc acactcagtc tcacgggccg gtgctggcac ccagtcgcga 1920

gcggccaccc tcgtcctcat cgggctcgca ggtggccacg tcgggccagc tggaagaaat 1980

caacaccaaa gaggtggccc agcgcatcac agcggagctg aagcgctaca gtatccccca 2040

ggcgatcttt gcgcagaggg tgctgtgccg gtctcagggg actctctccg acctgctccg 2100

gaatccaaaa ccgtggagta aactcaaatc tggcagggag accttccgca ggatgtggaa 2160

gtggcttcag gagcccgagt tccagcgcat gtccgcctta cgcctggcag gtaaggccgg 2220

ggctagccag gggccaggct gctgggaaga gggctccggg tccggtgctt gtggcccaag 2280

tctgcgcgcc gagtcacttc tcttgattct ttccttctct ttcctataca cgtcctcttt 2340

cttctcgttt ttatttcttc ttccattttc tctttctctt ccgctcttcc cctactttcc 2400
cttctccctt ttctttttct ttcttactct ctccttgtcc ctgagctttc attgaccgac 2460
ccccccccat ttcattcgcc ctcccctcaa tgtgccaacc tttgccctat ttccgatctt 2520
cccaggtact gggaggcggg atgggggtgt gcgttttcct ctaggagccc tgtctttcca 2580
agacccacag aaaccaggac ctgcccttat tcaaaacccc atgcacttca agtctctttt 2640
agacaacaca tttcaatttt ccgggctgac tagtctccct gtgcagaggc agttgagagg 2700
ctttgctctg cagagggaaa agagctctct actctcccac ccaccatata ggcaaactta 2760
tttggtcatt ggctgaaggc acagccttgc ccccgcgggg aaccggcggc caggatacaa 2820
cagcgctcct ggagcccatc tctggccttg gcgttggcgc agggactttc tgaccgggct 2880
tgaggggctc gggccagctc caatgtcact acctacagcg agggcagggt gtaaggttga 2940
gaaggtcaca ttcaccgctt tgggaggacg tgggagaaga gactgaggtg gaaagcgctt 3000
tgccttgctc accggccgtc cttgccccgg tcccagcgtt tgctgggatt tgccaggatt 3060
tgccggggct ccgggagacc ctgagcactc gcaggaagag gtgctgagaa attaaaaatt 3120
caggttagtt aatgcatccc tgccgccggc tgcaggctcc gcctttgcat taagcgggcg 3180
ctgattgtgc gcgcctggcg accgcgggga ggactggcgg cccgcgggag gggacgggta 3240
gaggcgcggg ttacattgtt ctggagccgg ctcggctctt tgtgcctcct ctagcggcca 3300
agctgcgagg tacagccctc tattgttcta ggagcacaga aacctcctgt gtgggcggcg 3360
ggtgcgcgag ctagagggaa agatgcagta gttactgcga ctggcacgca gttgcgcgct 3420
tttgtgcgca cggaccccgc gcggtgtgcg tggcgactgc gctgccccta ggagcaagcc 3480
acgggcccag aggggcaaaa tgtccaggtc ccccgctggg aaggacacac tataccctat 3540
ggcaagccag ggtgggcgac ttcccatgga tcgggtggag gggggtatct ttcaggatcg 3600
gcgggcggtc taggggaaca attcgtggtg gcgatgattt gcatagcgcg ggtcttggga 3660
tgcgcgcggt tccgagccag cctcgcacag ctcgcttccg gagctgcgag ctcaggtttc 3720
cacccccgat cccccgggct ttcctcgcac cgctgagccc agcttgtggg gtgcactcga 3780
ccaacgcccg acagggctgg ggaatgtgac aggcagcagg ttcacccggg cttggggagg 3840
gggagtttcc gctttgacag cattttcctt tgccgtctgc tggtggattc ctattcccag 3900
tcggtaatcg ccccgcagtg ttgatctaag aaggtaaaga aaactaggtt tccctgcaaa 3960

gagcctcccc caaatcggcg gactccggat actttgagtg gatttagaaa tttatgtaat 4020
ctttctcctt tagtttattt ttcatcctct cctacagttt tctctgattt gctgttggtt 4080
cggggcaaga taaagcagcc agtagagagc gataataata gcggcgggaa atgaactgga 4140
gactggctga cagttcttaa cattttgtca tagatccccc cgaatgtccc aggctgtctc 4200
tggtgggttt tagtacccgc cggcttcttg ggcaccgggg accagaagga acttggcagc 4260
tggtcttagg ggtacagtta aaggcaggat gacagctatt ctcctgctca tctcagagcg 4320
ctgccgcccc ctcatgccgg tcgcgcaaag aacacagctt ttaaaaaaca cgtgccttct 4380
gcccatatag gtctgaaagt gatgaggaaa gtaatgcttc gcctattagc gagtttcagc 4440
ttttaaaatg atcccaagcg ttgctgagat gagaaagcgt ggcatcccgg gggtcctcag 4500
ccccacccgc gcccatggtg caagtctgca gggacaggcc cgggacagca ctgcccacgc 4560
tgctagattt tccgcagagg atcgctgaag ctgccttcgt gggagacaga atgcctcctc 4620
cagcgagtgg aaaaggcctg ctgaggaccc cgctttgctc gagcattcaa atgtgtgtct 4680
gttttattac cctgggttga aaagggacaa gagctttagc ctttttatct ggccatttta 4740
tcagcaacta caagtgtgtt gagtggttat tattacatag gaggcttttc agtttggggt 4800
cagtagatca gtctcttcag acactgatgc agaagctggg actggtaagt aggtattatg 4860
tgctcggagc gctaggggac aggagcaaat ggagaagaaa agcggaggct ttctccgccc 4920
ggagtatcga tcggaatccc cgccggtacg ccgcagaggg ccctcgccgt tgggccccgg 4980
gggtttaaca agcccagccg ctccgcaggc ggctcggccg gactctcaga ccggtgcctg 5040
gaagacaccg tccctgcccc cctcccgcca aacctgcctc ttctctttct ctcataggtt 5100
ataggttccc tttctctctc attttggccc cgcccccggg tcctgccaaa cagccaagca 5160
ggccggggtt tagggggctc agaatgaaga ggtctgattt ggccagcgcc ggcaaagctc 5220
acccttaggc gaggtcacaa cagaggcagg tccttcctgc ccagcctgcc ggtgtagtca 5280
cagccaaggg tggcacttga aaggaaaagg gagaaaactt cggagaaatt tagattgccc 5340
caacgttaga tttcagagaa attgactcca aatgcacgga ttcgttcgga aagggcggct 5400
aagtggcagg tggttgcaac cccgcccggt cgggccttcg cagaggttcc ccaagaccag 5460
cccttgcagg gcggttttca gcaacctgac aagaggcggc caagacaaat ttctgcgggt 5520
tcgagcacac actctcgggc gttgggcccc agagacctct aaaccaagca caaacaagaa 5580

gggagtgaga gaacccaggc tagaacttgc acgggcatcc cactgaggaa aagcgaggcc 5640

tcggtggcag gcatgttttc ttccgacgcc cgaaaatcga gccgagcgcc cgactacatt 5700

tactgcagag gtttccgcct ccagtgagcc cggatccccc agcggcctgc ccggagctgg 5760

tctccagtcc ccgccgtagt ccgacgcacg gccctctcct ggcagcaagc tcccagcggc 5820

cagtctgaag ccaattctgt tcaggcggcc gagggccctt agccaaccca ccatgatgtc 5880

gcctgggcca cctgatgccc gcagcggcgg gacacggccc gggcagtgcg cagtggctcc 5940

tgctaggggc accgcgtgcg tgcttgtctc ccgctgcgcc ggggacgtcc ttgggtgaca 6000

cgggccgctg ggcacctccc aagccgagga aacggacccc cttcgcagag tctcgcgccc 6060

acccccccaac ctcccacctc gtttctcgct gctagggctc ccgactcagc ccacctctcc 6120

tggcggttta gttagggatc agagctggag aggctgaacg caacccgtgc cagtacggaa 6180

cagacgatat gtttgcctgc tagctgcttg gatgaataat tgaaaagttc gctgcagtct 6240

gtgcttcgtc aagtcccggg tgccgggaga acaccttccc aacacgcatc agggtgggcg 6300

ggagcgggca gaggaggcgg gacccgaggg aggagagtga acccgagcag gagaagcagc 6360

ccaggcagcc aggcgccctc gatgcgagag gctgggcatt tatttttatt ccaggctttc 6420

cactgtgtgg ttatgtcact ttctcaaaca aatgtgtata tggagggaga tcgatgctga 6480

taatgtttag aagattaaaa gagcattaat gctggcaaca ataacgtaaa cgtgtggacc 6540

cagatttcat tgatctggaa cttgatccgg cgcgtttcca gtaagcccga cggcgcgctc 6600

ttcccagcag agcgctcacc agcgccacgg ccccgcggtt ttccagcggt gccgcttcgc 6660

cagctctgcg cgggttctcc cgtctgaccg cagctcctcc cccgcgaggc cccagcccgc 6720

cttacttccc cgaggttttc tcctcctctc gcggggctct ctgccctctg caccccctcc 6780

cccgacctct gcaccacccg cccctgtgcg cacacaccgc tacttgcgct tccggcgatc 6840

cgcctgggcg gctgggtccg cgaagccaat gcgctgaacg gtgcccg 6887

<210> 7
<211> 3952
<212> DNA
<213> Homo Sapiens

<400> 7

ggccgcagcc ggagaggaac aggaaccgca gtggggacgc cctggtcccc gggcccacag 60

catccgggac ggacgcgcag tagcgcgggc cgggaactgg gtaccagggc gggatgggtg 120
agaggctcta agggacaagg cagggagaag cgcagcgggg tgcggggaac cgcacgccct 180
ccctttgcct ctgcttccca ccccgaggcg gcagggcggg cgggcgcggt tccgggggtg 240
ggcgggctgg gcggggcgga ggcggggccg cagcactggc ttcacccagc ctctcccgcc 300
cgcagccaga gcgagccgag ccgcggccag ctccggcggg caggggggc gctggagcgc 360
agcgcagcgc agccccatca gtccgcaaag cggaccgagc tggaagtcga gcgctgccgc 420
gggaggcggg cgatgggggc aggtgccacc ggccgcgcca tggacgggcc gcgcctgctg 480
ctgttgctgc ttctgggggt gagtgttagc cggagggggc ccgctccctt tcccgggatc 540
agaactccga gaagagccgg gcgccgccac caaggaaaca gaacagagca ttggggtccc 600
agatactgag ggtgggtggt gggaaaggac ctctgatgcc gggaccacga aggagggtct 660
agggttcccg gagcgcagag gcgactctcc agggtggaga tgagggcaag accggagcac 720
ggatgccggt cctcaggtac cgcagggggc ggtgggggag ctgggagggg tctttcaaga 780
gggggcatgg ggctctccga tgcccaggtt cttcggaaga ggacactcga atgccgggat 840
cccgaaggga ctttcccctc agcatctcgg tctctggaga gtcgtgggac agaaccaagg 900
cgagagaagg agggggaact ggacggggac tagagatgag aggggcgagc tgggctgggg 960
cggggagccc gggacgacgg gatggaacaa tggaggaggt cggagggact ggaggcagag 1020
gggacccggc aaggacaatg gggaccggcc agacaatggg ggggaggggc gtcggaggga 1080
gaggttgggg agcgggagag aggttgagaa gtgggttagg gagggcagaa gaggggcgac 1140
ggcgagggcg gcagagagat ggaagcagaa ggaaaggaga cagagcgggg cgcggaccct 1200
gaagtggtcg gggtcccgcc tgcccgctga gggacagtga gacgagaaga gagagcagag 1260
gcttggacat ttccagcgcc tgtccccgcg ctcctgctcg cggcctctcc cagtcccgga 1320
attggggctg cggatctaag gctgggaggg gagtgcccac ttcgccgggg cgaacccgct 1380
cccggggttc ccccacggcc ggcgggctcc tccgacgcgc gtgttctcac ggcttgaagc 1440
agcgcagcgg atcgcgaggg acggagcggg tgtgccccta cccgggctgg cggtggctac 1500
gtctcggggt gcagagcgcc ggggcgcact aggcggggac ccttcctggc cgcgcgcagc 1560
tctggctacg caggcgggag gcggcaggca gggggcgctg cggggcgagg caagtgcagt 1620
ccgccgctgc ctgcctgcga gtctggggct gcgcgccgct ccccacctag ctgcgcgacc 1680

cgagcgccgc gcccaccgcg ctccgtgggc tcccgcgcct cgaagggtcg ggatccggtc 1740
tgagcctctc ctggctctcg gtgcaagccg ctgggcacct aacgagacgg gggcgcccgg 1800
acgcctcgga ttctgtagcg cggtatcccg ggccaccgac tcggcaaagg tcggcgggtc 1860
ctccgggtgg tccgacgaga ttgtcagatg tggcctgctt tatggccatg aaccgaatgc 1920
cctggaaggt ctggcaacgg aggaggcgtc ttcactctcc ccgccagcat ctttcatcag 1980
agcactgcgc cccaaattcc ggctttacag atacggagac tgaggctctg agagtttaaa 2040
cgacttggcc aagatctcac agcaagttgg gccagagtcg tggctagact ccccgacctg 2100
attcccagct cggtactccc ccacctttc agggggcagc cgctgtccca gctcggggtc 2160
acggctgcgt ttcccagcag ggcggggggg gttgacactg tccccagagt ccgtcggcgg 2220
tagagcctgc gggtgaggtg ggcagagatt ctgtccgggt gcgggcccca gcccgaaagc 2280
tcagccccag ttccctggag ctgtcaggac ctgtggctgc gtccggggat ttgggagcga 2340
ccgcgggcac gtctgctgac taacgccgct ggttagagac gctgctcaga cgcgggtggg 2400
cgagcgtaga ccaggagagc ggggaggagg gaccggatcc cagaggggca acgacttggg 2460
ctccgggtct gggcaagagc gagggtcccc gcgcgaagac ccaaggaagg aggggagctg 2520
ggcgtggagg acctgggcgc ttgggaaggt gtaccccgac aggaacctgc cgtctgcagc 2580
ccctgcggct cccgcctccg cctcggcgcg cggctgctcg gtctgggatg gctgtgcgcg 2640
gtccgagttt cttcctgggc cgcggtgaga gagagagggc tgaaaccaga gcgcgtcccg 2700
gcgggtcggc tggcgggccg cgccggtaat ggaggcactt tgtcattcag acgtctgtaa 2760
ccagagccgc cgggctggct aatgcgccta atagggatgg aacgagggca gcaaatgggc 2820
gtgcgtgagc ggccgggctg agctgggtgg atggtggatg gggaggcagc tccgcggggg 2880
acgggcccct accgcactgt tccagcccag tctggctcaa gcgcctcgct tcttccctgg 2940
gggaccgcgt ggggaggcgg gaataatcga tgtgctgagt tcctactaag tgccttgcac 3000
ttccctggta gtttctcttg aattatttaa tccttataag gtcctagcaa ggtgaattta 3060
gttattccca cttcgcagtt gaggaaacag aaccttctag actttcaggc ccgagaccag 3120
gctcccccag tgagtcagtg gtggcgtctt tgttggaacc caggatgctt ggcctgggat 3180
gttctctttc ctacatatgc caggctccct cccgccccct accttctaga tcatggaagg 3240
agtcgtggct ctgagagatt aaatcgtgcc ctatgccttt ggccccacac atttagatct 3300

gagccccttc tccacacgca gaggccctgc cccctctcaa gtctctatgc tacattatct 3360

tgggtaatta gctcctttcc tcccaactcc tcccctatcc aaagggagtg aggggtatgt 3420

gccagtgcct cccctgggtt tgcatcgacc tcaaaaggcc ctccaggtgt ttgaaaatct 3480

ctcccatccc atggcggtga ccacggcccc attcacttaa gagctctttg ctgaagctgt 3540

tggaatggcc agggctgatt atactgaaac attcgtatgg tttcaaatac tttcaactgt 3600

ttcgctgttt ctctcttttc tctaattaaa aaaaatgaaa attcttaaaa gtcaattcat 3660

ctaaggatcc ttccacctta gttccttgta agccttgaaa tggccaaaac aaaaagatta 3720

caagcgttct taaaaatgag atgaaatcat tctgtcaaat gtgaaatgac taagtgtaac 3780

acatacaaat ttcagggaaa tttatctgtc atccctataa attaatcctt ttggatctga 3840

ttttggagca attagcctga atgttagtca cccctcccta cttggctaaa ctctcccctc 3900

ctgcctgggg tctccttaag atgagatgct tctttgataa ctaacattta tt 3952

<210> 8
<211> 2820
<212> DNA
<213> Homo Sapiens

<400> 8

cctaggggac aatacccagg catgttaacg gagtttaaaa tgccaaggaa attacaccac 60

aattctgccc agtatactac aggctgtcaa accgaaatgc tatgccagct aggagtgcag 120

caactcccat cctctggccc tatttaatta ggaagcttca gcagagcgaa gcctgccaag 180

cgttcgccgt cagaatctga aggaacccga gcgagcaaga agagtgcctg acccactcca 240

cagaagcctg tccagaaatg gaggagtcag cgcccactga agtcggttcc gccctcggct 300

cgcctacatg gagcctgacc agcctcagtc atgcccactc cggcctggga gacccgcaaa 360

gtgttctttt tctcaactcc cctgtactac cttgaagctt agggaagcaa agagaggggc 420

atatctggac tgcaaaacca atgtcttttg ccgcctagga gagaagggaa tgagagagag 480

agagagatag atagatagag agagagagag agagagagag agagagagag agagagagag 540

agaaattcta ttgaaaccca gctcctctag aatctgtgtg acctggtctt caacgggaga 600

ccagtgcgac ctcatggcac ctttgccagg aatcagcgat tcccctgcag tcaccatttg 660

atttattgct ttctcgctca ttctttctca taaagttatt tcttcctcat cctagtaaga 720

ctttttttctt taatgatgac aaagcttctg tttcagtgtt tcccctagga ttggtgctct 780

ttcaaaacag tgaacccaga aaaccatccc gtttaatatt tctcaaaatc ctcgcagctc 840
caatgtaagc gcaagcatgc aaaggtttcc tgctacacct gcactttctg cccatcccag 900
aaccacccct cacccccggg cctgcaacag ttccccttgt ttctctggat agaggtgggt 960
ggtattaggg gtctagggca gtaggaggtg aggggctgag gaggccgcta gggtaggctg 1020
gtctgtgctg gatacgcgtg ttcttctgcg gagttaaagg gtcggggacg gggttctgg 1080
acttaccaga gcaattccag ccggtgggcg tttggcagtc acttaaggag gtagggaaag 1140
cagcgagctt caccgggcgg gctacgatga gtagcatgac gggcagcagc agcagccagc 1200
aaaagccctc gcaaagtgtc cagctgctgc actgccgcgg ggactcccac agcaccatga 1260
ctagttcgtg caactctgca gcagcaaacg gcttccgagg aacacaggat cgcgggggcc 1320
gggcagcggg ctactgagca tcccgcggac ggcggcagca gaggcggcgg cggtggcagt 1380
ggcacccggc ggggaagcag cagccaaacc cgcgcatgat ctcgagagtt tcagcaacat 1440
ccagggactg ggctcagccc cggagcgaga gggtcgtccg ctgagaagct gcgccggaga 1500
cgcgggaagc tgctgccata aggagggagc tctgggaagc cggaggacag gaggagacgg 1560
gagtccaggg gcagacgagt ggagcccgag gaggcagggt ggagggagag tcaaggcgcc 1620
ccgcagcccg gcagccgcct ctcgagctct gccgcccgca tccctctggc gtttgggaag 1680
cagcaggtcc tcagcccgcc cggggtcacg tgggaagagg cagtcgggct ctgattggtg 1740
gagcaggatg caggtcccgg gagggagggg tcgacgagga ggtgcaagga tgcaaggagg 1800
aggcggccgc ggaagccaca gatgggctcg ctcgccaggc gctggcccga gtggggctag 1860
gcggggatgg ctcaaatgag aagctcgggc ttcagggtgg gctacccgca cactcatata 1920
ccattcgcct cactctccgc tccaggacgc cccctaccga aggcggggtc cggactagcg 1980
cccctcttcc gcgcgtgacc ccgggccgcg agtgcgggcc gcggctgggt ggcgtctctc 2040
cgagctggag atggtggggg cggaggtgtc agaggagcag cagcagcagg gcagagaggg 2100
gcgagtcggc gcgggagagg gcgtcctgct ggcgaccggc gctccagcgt gcgggagcgc 2160
gccgcctagg ctgtaggggg atgcaggctg ggaatgtcgc ggcggagagg ccagggacgt 2220
ttctctaggg atttacagga aagagggtga gaggcgatgg tgttagaacc gctcttgccg 2280
acctggaagc aacagcagca tctcccacaa gagcgtgcaa ccccaaggct gctcgccgag 2340
gcagctcagc catcccggca ggcgctctcc ttccttctct cttctcccct ctctcctccc 2400

aggccccccg cagctccgac ccagcccaag cgttcgcagg tttgaatccc tctcctcatc 2460
acccgctcct ctccagcccg tagcctatta gtgtgtccac ctgggaggtg cggtcagatg 2520
tgtttggaag gtcagattgg tcgggacaag tggtctgaga gaaagagaaa ggctcctctg 2580
catacgccgc gggtgggttg ccgggagcat cggccgggca gcggcgtccg ggaaggggag 2640
agcgggctcc atttgttggc ccaggcagtg accctgcgtt ccttactcgg gtctttgccg 2700
gatggccggt gacctggggc gacgagagaa ggtctaactc ggcaggagtc tctggctctg 2760
cgcgtttctt tcattctctc cagcgggaag ggcaaacggc atagcgggac ccgccttccg 2820

<210> 9
<211> 2265
<212> DNA
<213> Homo Sapiens

<400> 9

cacccccacc cagcgtgctc agggcacccg caccgtgcta gcttgtggcg gctctcaaac 60
taatcctgac tgagtttttg aggcagcctt ctattcccac tcgcaccaac ccagcacgct 120
taccaaaatt cccaagtaac cttctctttg ttacgcaaac gccaggagct gcttgattct 180
ttcttctctc cacccctatc ccctttccag cctagccggc ttcctggccg ggcaagctgg 240
gaattcagcg actgaagggc cttggaaggt gccggagggg agagacgcta gttcgaacct 300
ccacagggct ctcctactaa aaatccctaa aatgctggcc cgagaaactc tctttgttgg 360
agggtctgag tcctactccc ttctgccgcg tgcgccccca ctgccgtgaa ccgctgtgac 420
ccccaatcta ccaccccatc tcgcgccccc agcgtcctag tcgccctgta tctgcccagc 480
cttccccctg tccatgtacc tggctggttg gtggcgcttg ggacgcggtt gtaggcgccg 540
ccgtactggt ggtaggagct agcgtagcta tagtcggcat aagctttggc tgggtagctc 600
ccggcggagc cgttcacgcc gtgatactga tactggtagg ggttgagagc tttgccatag 660
gaagccgagg taggagagca gtagccgtgc ggggctcccc ccgtagggct gtagtagtca 720
gaatcggtag ctgaagactc gggcaaagtt ggcgattcct gagacggatg gtgcatagct 780
gcggacgtct ggaacggagc ttggaagtcg ccggatcgga tgctggggac ccttctgtca 840
aacactcctg tcatcgctca cgggcggcgg cagcggctgt ccttgctgtt gtggcggcgg 900
cagctgccct agttggctgt ggggctgctc tggtctaagc agacatggct gtgggagcga 960

gggaggagga ggaagaggag gaggagagaa ggaggaggcg gcggccgcgg cgaggaggag 1020
actgggagtc gtgaagtctc tgtctccggc cggctgactg ctggctgagg cgcagcacag 1080
ccttggttaa atccttaatt gcgcgcttac gcacagcggg gtggatctgg ttctattggc 1140
cagggcctcg ggagcaacag taacacccta actcgtccaa ctagttttag cacaacaaag 1200
cattgcttaa aaagggggtg gggggtactg agggggtgtg tgtgtttgtg tgtgtgcgcg 1260
cccgcgcgct tgtgtgtgtt tttgcttgtt gtggagtggg gaggggtctt agtctatctg 1320
tttttagtaa attccaaaga catcgcagaa gccacagcac aaggctctgt gatgtctggg 1380
gacaatgtgt tccaatcaga agcctcaaaa gtgcacatgt tttgggaatg aggtgggcga 1440
ccatctttcc ttgcttccca ctaccccgcc cccgtgggct gcaccaaagg cgagtgtaga 1500
attaaacact gatactgaaa tagggagtca aggtgcaggg agtggggagc ggggggatgt 1560
aggatggagg caagaaggaa gggaaacagc attttttttt tttttagtgg agtgttggga 1620
agcaagttgt aacttacatg ttttgaaaat ttgctctttg gggaaagagg caacttcctt 1680
tgcaggagat gagtatctcc cggacgcggg ctggacagga atgcccaaga aggcacaatg 1740
tccattaagc cggtgaatgg cagggagagt tcctgcagcc caaagtcctt ataatgactt 1800
ttaaaaatta ctgtcttaaa cacatataac ttaaggaagc ccaaatcctc gttgctggct 1860
tgcaaaaaga gccaaacagc tccacttctg tttaatattt tcatctattt tcaagacatt 1920
ggaataaagg ctctttaact atgttcagtg gccaaagact cccgcaaagg tgaatggatc 1980
catgtataac ctgccatacc tccaggaaag aatgggaaag tctcggaagt ggggaaggag 2040
acaaggatca aagcctttgc aaagtcagat gatgctcatt gatttccccc tcctggctcc 2100
atgaatgtgg cccagtctct ccatttccca gacccgggtt tctgtaccct ctcccctttta 2160
tttcctgctg caaagacatc ctacttagct cgtatgcaag ggaagaaagg attcaagaat 2220
cattcagttg atacacgaga tctaaacttt gctcttcagg aggtg 2265

<210> 10
<211> 5907
<212> DNA
<213> Homo Sapiens

<400> 10

gccgtcggag cagggcccca gcgcaccgcg agcctctgct tcctccgcct tgcagcgccg 60
cgagcccttc tttcctccgc gcctggcccc gtctgcgccc ctctcggcgt gggcattcgc 120

agacccgcta ggaggctgac gagaggtgtc ctcatagagt cctgccttct tccagacccc 180
caaacgaaaa gaaagagaaa agccaacctt tcgcctccat tctgcacgca tttggagagc 240
ggctggtggc gagcgatccg cacaatcgct tccctcgcgg cgtccctgga cgggcggaga 300
gaacccgggt gactgagcct gaggctgagc ttccttggga gcgatccatg ccagccactt 360
ctaccttcaa gggggactcg cggccgggtt tacgcgcgaa ggatgggtgc cagggaaact 420
gacttataga cgacccgctc cgcgagagac tgacaactca gtcctgcggg ctttcggggc 480
gcccagacca gtccttagcc agaggatctg gggctgcaca gctcccggac cctggagggg 540
gtggtccggg ggtgggagac tgcgagcatc agcccacgcc ggccccctcc caccctgcgg 600
cggcgaggtg ggaagggcca ggatgcgagc ttagcacctc ccctagaaat gcagcacttc 660
gcccccccac ctcccctgcc ctcggcgctc cctctttcac gcgctccctc ccgcctcctt 720
cccaagggcg cccttcttct gcccccagct cacgtctgaa tccctcggcg ccccctttct 780
cttctcctag ccccttcctc acgtcccctg cctccgggta tttcctctct ccaatccccc 840
acccccgcac cgcctgattc cgaggggcgg gagcgcattg ggctgcgcac gggtgggggc 900
gccgcgccag cttcgcgtag ctgctctgac gccgctgccg ccgccgccgc cgccgccgcc 960
ctccgcagcc cagctcgcgc cccgcggcag ctccgcagtg cactagccac caccgccgcc 1020
gccgccgctc cgccagacct gctgccagct tgcccggtcc agccctgaga gagcctcgaa 1080
cgccagctgc gagggtcatg agccagagag ccccggggcg ccgcgcggag agcaagcgga 1140
gatagcgact ttgcgccccc cagccctcgc cttcttgcat cgcgttcccc gcatcctcgg 1200
gtccttctgt cctttccgct gtccccaccg ccgccatggc caccttgctc cgcagcaagc 1260
tgtccaacgt ggccacgtcc gtgtccaaca agtcccaggc caagatgagc ggcatgttcg 1320
ccaggatggg ttttcaggcg gccacggatg aggaggcggt gggcttcgcg cattgcgacg 1380
acctcgactt tgagcaccgc cagggcctgc agatggacat cctgaaagcc gagggagagc 1440
cctgcgggga cgagggcgct gaagcgcccg tcgagggaga catccattat cagcgaggca 1500
gcggagctcc tctgccgccc tccggctcca aggaccaggt gggaggtggt ggcgaattcg 1560
ggggccacga caagcccaaa atcacggcgt gggaggcagg ctggaacgtg accaacgcca 1620
tccaggtaag cgcgggattc ccagttctgc ctgtcctccc ccctcccagc tcagcgtgcc 1680
gggctctgcc cccgacagtc gcccggtgat ctcggcctgg agacccccctc ctgtacccag 1740

gaatctccct ttctccatcc ctcccagccc tgcgcgggga cctacgcccc caggcggtgt 1800
tttccgccct aacccacgct ccctcccaac ggcaccagct gcaagaccgc taggctgaag 1860
ttcggtctga gacacctgtc cggagacact gcaaaagtga aggaaatggg gggagggagc 1920
aggaagcgat gagaaagaaa gaaaatcagg attggagggc acggtttggt cttggactct 1980
ggaacggatt cacagctgca tttttgggag gaaagaagaa ggggaaatcg ctgaggtcgg 2040
agtctcctcc ccccgcacac acgcatagac acgcacacgt atataggcag gcacaccaca 2100
tgcccacagc ccccttccc acgaacaaag gcgcaggcac aggcgcacac gacatgtatc 2160
tatatataga ccacagtact atccccaaat gcacctgcgt acacccctac agctccacaa 2220
acaaaagcat atacctgcac ataccaaaca cacgaatatt cgtttgaaca tatgcacaca 2280
cacatgcaca gatgcatgca gacaggaacc agcctgctcc cttgggtgcg gccctgttgg 2340
agacatacag gtatacacac acgaaagcac acacatgctc ccacacaaac acatttagtc 2400
cgcaataatc cagcattacc tgtgcacaga ctcgcccctc tcgcccgaaa tcccggctgc 2460
accgcaaaga ggcggccagc tcctccccgt gtgaccctgg cgaagccctc tccagaggct 2520
aagaatggcc cctgctctct aggccctctc cccttattcc taacccccca ctccccacca 2580
ggcggcgcag gactcctaca tagggagcag gtttggagtc ttggcaacga cctccgagct 2640
agcaggggct ggcaggccgc agctttctgg gccgaaggag acttcagctc taggcaaccc 2700
cggtccctta cagcttggcc ccgcggcgtc cctggagcgc actatcactg gggccacgga 2760
aggcaggttt tctgggagca gaggcctccc aggggttgtt ccatgtatcg gggtaagcag 2820
gactccaccg ggccccgaac accagatggc ccgacccaag gcgctttccg ctccggggcc 2880
cggcgagacg gccttgctgg atcacggacc cggggctgct cccgccttcg ctgtgccccg 2940
cgacttcccg gggcgtcttt caaggctccg tttgataggc cccaagggag gccagggcag 3000
gaggctggag gctcgtaggc ctccgggacc ctggatttcg ttagctctta gtccccgtgt 3060
ggattctaaa ccctacagcc ctgtccgcct gaccctgaag cttctggttc tagactcttg 3120
ctcggttcgg ctttctggcc tccctggcgc cttcccgcca agctcctcct cctgcttgcc 3180
tccccagcct ccacttagtc cccttcctta ggtctcgccc tcgcctcact ctaactccag 3240
ccttgtccta agactctcaa tttccagccc tgcttcctcc tccccgcgcg ctgctcccca 3300
agcgtcttca cattgtctct gttcttgcct cccaggggct ttgccccacc ttcagcccct 3360

ctccctctct accctatttc cttttcgcct cacaactgca gcctttaacg tttcctcctc 3420
tggtctgagc ctgagacgcc cgagttttcc tctttagctc ccgtgcccca ttccagcccc 3480
accacaaatc ccgtgcccac tctttccact ggcccaggcc cagctcacta cctttcttag 3540
agcccctttc cgactacgct cccttctgtc ccttctcatc tcctccccca actcctcatt 3600
ctgttcctgt ccccagaaac catcccgaag tatctccctt ccctcctgtg gcttctccct 3660
attctcagcc ctctcccacc ctagccctct cgctatcacc agccattagc gcacctttcc 3720
ttctcagatc cgtccataca tccctccctc tccatctctg gctcgacacc cgggctcact 3780
attagttccc ctacatccac cccgcagcct gctcttaacc tctcctcccc ggcggctcag 3840
acccaattct cagtgtcctt agcgcccccct tcgggccaca gcgttaagcc acgccccccg 3900
ggcccctcat ccgttgccaa gttcgctgag cgtccgcgtc tggttgcctc tccgccccac 3960
agggcatgtt cgtgctgggc ctaccctacg ccatcctgca cggcggctac ctggggttgt 4020
ttctcatcat cttcgccgcc gttgtgtgct gctacaccgg caagatcctc atcgcgtgcc 4080
tgtacgagga gaatgaagac ggcgaggtgg tgcgcgtgcg ggactcgtac gtggccatag 4140
ccaacgcctg ctgcgccccg cgcttcccaa cgctgggcgg ccgagtggtg aacgtagcgc 4200
agatcatcga gctggtgatg acgtgcatcc tgtacgtggt ggtgagtggc aacctcatgt 4260
acaacagctt cccggggctg cccgtgtcgc agaagtcctg gtccattatc gccacggccg 4320
tgctgctgcc ttgcgccttc cttaagaacc tcaaggccgt gtccaagttc agtctgctgt 4380
gcactctggc ccacttcgtc atcaatatcc tggtcatagc ctactgtcta tcgcgggcgc 4440
gcgactgggc ctgggagaag gtcaagttct acatcgacgt caagaagttc cccatctcca 4500
ttggcatcat cgtgttcagc tacacgtctc agatcttcct gccttcgctg gagggcaata 4560
tgcagcagcc cagcgagttc cactgcatga tgaactggac gcacatcgca gcctgcgtgc 4620
tcaagggcct cttcgcgctc gtcgcctacc tcacctgggc cgacgagacc aaggaggtca 4680
tcacggataa cctgcccggc tccatccgcg ccgtggtcaa catctttctg gtggccaagg 4740
cgctgttgtc ctatcctctg ccattctttg ccgctgtcga ggtgctggag aagtcgctct 4800
tccaggaagg cagccgcgcc tttttcccgg cctgctacag cggcgacggg cgcctgaagt 4860
cctgggggct gacgctgcgc tgcgcgctcg tcgtcttcac gctgctcatg gccatttatg 4920
tgccgcactt cgcgctgctc atgggcctca ccggcagcct cacgggcgcc ggcctctgtt 4980

tcttgctgcc cagcctcttt cacctgcgcc tgctctggcg caagctgctg tggcaccaag 5040

tcttcttcga cgtcgccatc ttcgtcatcg gcggcatctg cagcgtgtcc ggcttcgtgc 5100

actccctcga gggcctcatc gaagcctacc gaaccaacgc ggaggactag ggcgcaaggg 5160

cgagcccccg ccgcgcttct gcgctctctc ccttctcccc tcaccccgcc cccaccagcc 5220

cagtgcgccc tgccgccgcg ttgggaggcc aagctttaaa catctctggt tcctagtttc 5280

tgattattcg ggatgggggg ggatgggagg ggacagggat tcacgatcca tcgcgtctgc 5340

gtttctgttg tcctttcttt tccacaacac cctggttttg gggggaggcg gggtgcattt 5400

gcgggcaggg ttctctgtcc ttccaagtgg ggccccgaca ctttggttcc agtcatcgag 5460

ggggttggga agggagggag agggggcgca gctcgcaggc gtggcaactt gaccttgggg 5520

gaatatttca catccatcca gagctcggaa tctacagcgt ccagccattt ccagcaagag 5580

cgcttcccat tccggagacg tttcaaccct gcagcgggaa aggctgactg ggaaatccat 5640

tttgggtggg caatttcctt caacgaagcc ggaaggcgag aagccgcggc ggggccagct 5700

tgcctgccgg ttttcaggaa tctaaactct catcttgtgc aatttatcag gtgtggaact 5760

gttctactgt gcgtgtggtg tgctcgtggt gaataagatg aaatgtatat cagaaaaaaa 5820

tctatctcta atttagagtg cggtacataa ttatatccgc aaataaagaa gagacaaagg 5880

cttgcgcggc ccggtgtcgg gtttgtg 5907

<210> 11
<211> 3049
<212> DNA
<213> Homo Sapiens

<400> 11

tactcggtgt agctggtcca gatcttgtcc tcgctctggt cggtgctgct ggcaaaggcg 60

caggtgcccg tggagctgca cgccaccatg tggaagcccg actcggacag cttgtcgaag 120

gcctgctcca ggaagttgaa cttgaggtaa tagcgcgagg tgtagcgctc cgggggacgg 180

tcggggtccc ggctttcgtt cagggtgtcc ccaaacacct ccttggccag cgacgtcttt 240

ccgcaaacgg tgatgcgcgc cactcgccgg aacttggcgt ccgcctgcgc gtcccgcccg 300

atggtgtagg agccgcggta gccgatggtg atgtagcccg agcgccggct gccgtccagc 360

gactgggacg gcgtgagcag cgggcccgcc gcgcccccgg acggactgcg gctagccagc 420

tccagcgtgg gcgacggcgc cccggcagag gcgccctcct gctgttcggg ctccgagtag 480
ccaagcggca gcagctcgtc acccagcgag ccctccttgt gcaccccgcg ccgcgagggc 540
ggcggccccg ggccgggctg ctggggcgcc ccgaggcggc gcacgagctc tggcagctcg 600
aagtactcgg cctcgcgctg cagccggctg cgctcgggga agtagtcggg cagcacgagc 660
tgcaagtccc gcaggtaatc caggatgtag cggaagagga agccgtcccg gtccagaaag 720
aagcggcctt tgctgtcccg ggccagctcc tgcggctgct gctgcgtgaa catgcgccag 780
agcagcgagt cgggcaccga caccaccgtg cagcgccggg tcacgtacac ctggcccccc 840
acgttcagct ccacgatgtc ggggaagagc ggtggctccg cggaggacga cgaggagcca 900
ctgccgcccc cgccgccgcc cccgttgggt aatccacgtg tgctgtccgc cagagccatg 960
acagagaggt ggccgggccg ggacagtggc aggaagccgc gctgcactca ggagctgcaa 1020
ccgccttccc cggagccccg gaacccggac gctcgctcag ccctgcgccc cgccgccgcc 1080
gccgccgcca ccgccgccac cgccaccgcc gccacctcct agagccgcgc ggagccgagc 1140
ggtgcgagcg cgccgctgtg cgccccettg agttccagtg cgctccgccc gccttgcccg 1200
cgcgctccag gcgagtgccg ggtcgcggcc cgcgcgctgc ttaagtagag ccgcctgctc 1260
tggtgccgtg gcgcgcagga ggcgtgggcg gcggcggagc ctcgcttacc gggcgagggg 1320
aggtggggag cggggactcg atttgcatct taaacgcgga cgccacgcgg ggctgctggc 1380
tccaccccgg ctcgcgagcg ctgggggcgc ccggactccc acccgctccc cacccaccag 1440
ccccgccccg tggtcccgcc caactttctc cctcttccga agaggacccg cccgagactt 1500
ggcggcttcg gaagacagct ccttggaggg acagtcaccg cgccccctcc cctctcgccc 1560
cagcccgcac ccaaggactc tggaaggagg acgtgaactt tcggaagcct gtcgccccgg 1620
gagtggaaat gcgtggccgc ctccggccct cccgcctgcg gaactgggcg cttccagcag 1680
tgcagctgcg gcctgccatc gttggttgtc gtgtcactga aagtagagag acgcgaaaaa 1740
cctggagaga cgaaagagcc gccccagtcc gagcgagcca gattttgttc aagttgcctt 1800
gcagccagcc tggaaaataa ataaataaac ctagaaacaa aaagcaaacc tcagtgtccc 1860
gcagtaggat gctttacgat agtttatttt ctggctgcag agacaaaatg agatgaagct 1920
tcaaatctgc tcttaattgc aattccctct tcccgcaggg ccttcgaggc acttcacttc 1980
gaagtgaagg ggtaagactc cggaacgttc ggtctccggg tcccattcag ccttcgttga 2040

atcgcgccct tagactcaca acagttgcag atctaacgta atttttaaag ccacctttgg 2100
cgcctgcatg tttatccttc caaaagtcca ggcgcccctt ctctcccgca tgcctcggtc 2160
actcttcggc cctgtccaag ttcccgtcag tctcagaaag ggatatttat atatctgaag 2220
gcccttgtct ggatcccatc tctcaactcc tgctgcctac acgtttgctt ttccccctcg 2280
gactgcagcc cacagaaccg ccccctcccc ctggaagctt tccctggctt cccaggtggg 2340
tggggcctct ggatggctcc ctacacactg tctgcgctgc tcccagcacg ttctgaaatg 2400
tcatatttat gagtgattat ctgatcactg atttgggtat gaaaataagt aagagctcct 2460
gaatgctgca aaaagagaag agtgcagagg agggtgtcaa gaaatgattg ggtcactgaa 2520
tgtttcttgt gtacctgcta ttatttgcag ggcaccaggg agtgaggaat gcaaaggcga 2580
ataatctcca aagcgctttc agtgtagccc aataagcaaa gggtcagggg tctgcgcagt 2640
tatcacatca gtttgaagta tagaccacac atcaggataa atcaggcaag atctttgttc 2700
ttgataaaat ttatccttct gtgtaactac ccagtgagct tcacagtctc ctcacagata 2760
aatgtcattt ttattttgaa atgtttatat ctgctcaaca attaccgcgc tcatgtcagt 2820
gtgctgaaat gagatcaacc cccaaaccag ttatcttaat tgcaagtaca gattttgata 2880
ctgaatgtat ttcaacattc tttttttgtt gttgttctgt tctttgggat aaataataga 2940
taacacaaaa cttccaagtc ccagtgcagg atttcgaggt ggttgtgcta ctaggcgtta 3000
tattagtgtc agagacctga caaaaccagc ctcttttcta caaatccaa 3049

<210> 12
<211> 4721
<212> DNA
<213> Homo Sapiens

<400> 12

acgtgaagcc cagcacctcc ccaagccttt gcctacacac aaaaagccac gttctagagg 60
ctctatttct aacttaagat aagcaggata ttttaaaaat catctactca ccagttggtt 120
tgagaaaatc catcgggtat ctggagtaag gagggggggg agactctgaa attaaaaaag 180
caagagaaaa taaaggccca gccatgagaa gagagataga aacttatgat aacagaggca 240
ttcttttagc ccaactgttt gtcttagctg tgggggttgg aggcagggcc cgaggcggtg 300
attgccttga tatttagctg tcagataaac aaaagaggcc gcctggcgcc agcctcggcg 360
ctccgctcct ccacgtctgc ggccgcagcc gccgcgctcg gctggcccgg ctggaaacca 420

ccggctcggc cggagccgcg cacttcacag ttcacagggc ggggggcgac catgccccct 480
ccccgtcctc ggactccaga cccagctccc agcaacctcg gctccccgcc ccggcccgcc 540
ccccaggcgt gcaattcacc tctgcccccg ccggtccgct cggcactcgc gcgggggaca 600
cggggctgcc cattcctagc gcactcggga gatgcatccc aagcgtgctg gggaacgtgg 660
gggatctgcg ggcgcccggg gtgaaccctc acatcacact ctgtgtacag agacgtagag 720
taaaggaagg aatggagaaa gctgagagga taaaaggaaa ggagtttcag ggtctcagcc 780
gagaagccca ggccccagcg cagtaagagg cgacaacgtc actgaggcgc cggggcccca 840
ctaatctaca gacttatctg tgcggaagga aggaaaaagt aggggacacc caacttctcc 900
tgcctgtttg ttcctctgca ccctggggct ggcagagttg aaaggagaca gatacccagt 960
tcggccttcc atccaactct ctttgcccca aaactccaaa ggtgcgcggc cacgacagta 1020
tctgggccac tggtgttcga cgtgattctc cgtatttact aaaggagtaa atacggagac 1080
cttccagcct ttatagcacg cctctcccag ccttccttgg cgggcctgga ccaaatccaa 1140
ctcggccaca cacctcaacg tctttccggg tgcggggagg cgtgaccccc ccacctccct 1200
ctgcaccccc ttgttgatcc ggaaatgaag gcgtctaatt tttaaccgca gactgcagga 1260
gcagtgcccg tagacccctt cggggcaaca caggagaaaa cctggaaggg aacccccagg 1320
gaatgccctt tgagtttccc cttgtccctt ctctactttc agcccccagt ctcttcccag 1380
gagggtatgc acactctccc aggagggtat gcacactctc ccaggagggt atgcacactc 1440
cccctggagg gatggctgca caaacgcact gccctagggg cttttcttcc agcactggcg 1500
ctccggaaaa tgttgggggt agggggacaa cttctcacct agttcgcaga gtcggctaag 1560
gtgatggggg ttgcagcaca ccagctcggg gttgatcttc ccgtaagatt cacagcaaca 1620
cagcctcttg acttccgagg aatgcctgag atccggccac ctgaacactt tgcacagcag 1680
gagggggagc gagtaggacg agggcggctg cgcaggctgc gcgccggcgg gcgcccccgg 1740
gcccagcctg cagtccaggc ggccgggcag caggaggcac gcggtgcgcg tcccgccgcg 1800
ggactccacg gcctggagca gcagctccag ctgccgctcc ttcagtttct tgagcaccga 1860
gtgcgtgagc gccttcagat ccgcctcggc gcccccggcc gcgccggcgc ccgcggctgg 1920
cgggtgggga tggtggtgac ctttggcacc tcgcaccgcc ttgcccaggc agcatccagc 1980
cctgcccggg ccgccgccac cggccccatg cgctcggctg tccgtcgccc cttctccccg 2040

cagctcgcct cctcctccac ctccccctgc gccctcctcc tcgtcctcgc cgccgggcgc 2100
acggctcctc cagagacgcc ggacgagcgc agatcgtttg gtcctgaaca tgcggggcga 2160
ggaggcgagg agaaaagtcg tttgcctgct aaggagcgaa catgacctcc gcacaccatg 2220
aagaagtcgg gcgccgagtt ggggcagcag gcgcaggcga cagcagcagc agcaggggcc 2280
cgggcaggag cggcggcggc ccgaggggcg ctccgtggca tgcgccagtc tcccggaggc 2340
cggggcgcgc gcgggggccc gggggcgccc gccggggatc gggggcctgc gctccggctg 2400
ccccaccccg cgcggcccgc gccctgcgcg gctctccggc cccggcgcgc cccggaggaa 2460
cccggccgcc gcttccctgg ggacggccga gcctgccccc gtcggcgcct ccccaaaaag 2520
aggccccccc gcagtggctc ccgaatgtcg ggctcgccag cctcggcttc ctacatggaa 2580
ggtccgcggg ggcaaaaaac gaaaggcgtt cggctgggct gttggaagaa ggaaaaagcc 2640
tctttccccc ttgctaagca acttaatttg ggggtgggga gaagcaggca attaaaaaaa 2700
aaaaagcaag cgatttattt ttttcctcta tatccttagt aaccggatct cctcgaattc 2760
cgcgcacacg aagactcagg ggaggggggcc gagtggactt caccccgcat gagacgtctg 2820
gcaaaataag aaggctctcg caaaacctaa caaccaaata tgcaaagccc caaatgaaaa 2880
ccaccacctc ctcgaacctc agaggtctgg gggcgtccgg ctggaactgg ggtttaaaaa 2940
aagaaaatgt ttacaaagta taacaagatg tttgatgggt ggaaaaatgt atccacgagt 3000
tacatccccc cgtttccttg caaagccccg ctggtcttcc tctccttttc ttctgccaaa 3060
aaaaaaaaaa aaaatcgtgt atttttttaa tccacagaaa gctttggcta gaccgcttca 3120
atcctgcgca tctgggtggt ttaggggagt ctctggtctt tccccctgcg ctcctggggg 3180
cccaggtcct cggcggggac ttcctcgagg ctggcgcggg cgcaggggca gaagatgctg 3240
cggcggcggc tgagcccggc ggggctgaca gcgcgggggga gggtggcgcg gcggcggcgg 3300
agggccccag acgggtcgcg cgttctcgcc ccccccgggc acaagctgct tgctagtgca 3360
ggggccgccg atgtcccttc ccctggccgc ggctggccgc cgaggctccc cgcatgggct 3420
gctcgcctcg acccagctgc ggcggcagga ggccccggtg tcctctcggc gcctcctcct 3480
ccgagactct cctcgtcgcg cccgggagcc tccttgtccc cggtccgccc tctcctggcg 3540
ctccggtcct tctgccgccg ccaggggctc gccgcgccgc actcaggggc tcaggggccg 3600
ggcgctcggc ggctcggcgc cgacggactg gctctgtctc gggcagctct ctcccgcgcg 3660

gcgagcggac cgagcacggc gcccggctgg ctcggctggc gcggctcggg gacaggatct 3720
tccgtgcgcc gagcagcaag cgagtgtgcc cggggctcac cgcctcccgc aaggcctccc 3780
gcccccgccc ccttccctcc cccttcctcc cccttccctc ccccgccccc agccgccgca 3840
gccgcgccgc ctcctccccg cccccccgca cccccccctcc ggccctctgc tcggctggtt 3900
ccactgcgca gtggcgcgcc cggctccggc ctcgtcacgt ggccgtctag acaccctgtc 3960
gctttaaaaa aaaaaaaaaa gcgattgtgt ttcgcaaaca acagatcggg tttctaaaag 4020
ctatttctcc ccccaacccc ccgccaccgc caccccctcc cgggtctgta gagggggtac 4080
cgatggaggg gagagagata ggtggggggc agagaagctc ccagaatgga ttgagccccg 4140
gccggagcca tggagaaatt ggaaaagcag ggagcaccga gcgggcttcg ccgcgagttt 4200
tggagctgag cgagcgggtc ggtggcccga tttcgacccg gctgggtttc gcggtggcca 4260
tctcgcgcgc gctcgcccta gcgcttcatt cattggtttt gttttaaagg ccctggcggt 4320
ggatcccttg gccgcgcccg agggcaaggg gaggagagcg ctgtctcggt ttaaaagaca 4380
tttataccgg actggaccga ggccctggga agtgtgcgct gaggggaaca gccgccgagg 4440
gcggggaggc ggcgtgaata tgacctcagc ggcggccgcg cgctccctcc cgccctctca 4500
gctccgggct ccggtttcta ggactgcctg gagaagtgtg tcttgtgcac agctctggaa 4560
tgcatttggc cggctgacga gctgtgaggg gcagcatccc ggcgggagaa ggggagcggg 4620
ggtgggggct cgcctgcgcg ccgcgggcag gtttcctccc gggcccggaa gacctccgcc 4680
acccgccacc ctgcctcccg gcgcgggaag gttacccagc g 4721

<210> 13
<211> 2146
<212> DNA
<213> Homo Sapiens

<400> 13

aggtttgtgc acccgggtag tccctggctg ctgctgccag gctgcctcag ccggtcgctg 60
ctgccgcggc gactggcgac aagctaccag ccacctacga tggcccaagg aggcgaagaa 120
gagcaagcga tcaggacacc aaaacggtta tcggaggcag gttcccagca caccagccgg 180
ccgagggccg agccccgcgg gcggcagcaa gttttgggag ctggaggtaa ccgaattaaa 240
aggcgcctta gaaactccgc ttcgggactt tgctcagcag ggctccgggt tggagggcgc 300

cgaggcctgg cggacgggac agtgggaaga gagaaaggtg ctaaggggac ccaagatctg 360
ggatccagaa caagaggggg tggggaacaa ctctaccaag ccaaacagat ctatttccct 420
tgcctccatg ttggaaaaat tcaggttcca tatggctcct cgggagggag ggagggagac 480
ggtggctgcc ggcttccccc cagggtctcg ctggagaagg gagatgagcc ccccgcaaga 540
accccacctt gccaagcaca ccccagggag agccaggaga gtaacaatag ctggggaagc 600
cccgagagag gggataggcc atcttccagg acttaaaaaa attaatggga gaaagaaaaa 660
taacaagaaa ggaagacaga gaaaggttga gactttctta gcagccgcgg agaaaattca 720
gccctggatc tggctgctga aagaaaagag agagaggaga aaagaggaga gaagcaggaa 780
gacagagggg agagagagag ggagagagag agagagagag agagggagag agagggaggg 840
agggagggag ggagagagag agtcgctgcg tggaaggaag cttaaagctt gtgaactctt 900
cttgaaccga gattggagtc atatgggcca taaatcattg agacatactc tccgccattc 960
acaaactgat agcctatttc agtccagctt accttagcca ccgacgaggg gagaacaggc 1020
agacataata tatattcaca tcgagcccca gagcgagcgg caggcgacaa atctcccctc 1080
cttgaaggca aaggaaaaaa agaccactgt ttaaagctgc gtcgcccccc gcccccccgc 1140
ccccgccccg caaagccacc ccggctgggg agcccgaggg gcagaccggc cagaggagcc 1200
gcgcggcgtc cgcttcaatt cactcggctt aggagcaggg gtgcgcagga gggagggggt 1260
gggggagcgg gaaaaaaaat agaagaccga aaggtgccgg gcggctcagc tcgccagaat 1320
ccagctccgg gctccagcag ggctaagccg ccacagtgtg gttgccctat aagactggta 1380
cgccctactc tccgtaacaa tggcctctgc ttttgcacag ggaaaaaaaa ccacacagac 1440
acacacacac actcactcac acacaagctc acaccccccc acctctcccc ctttagcaag 1500
cttagatgcc tgataaggag ggaaggtgat ggtggtgatg gggaaggggg gaaattttaa 1560
aatacccgcc tccccaaaaa aagagtaaga agaaaagaag gagggttttt tttttttttcc 1620
ttaaagaaca ggtaaaaatt taaatctaga ttggagggac agggaggaag aggtgagcaa 1680
aggcaaggaa gggcggtgaa gttcacagct ccccccatac cccactagtg aactattatg 1740
ctaatatgag aagtagtatt tggaggctga gtcaaaggca ggcctggccc gggggtgagg 1800
gtgccgggtg cccctctgca ctcattcgca attggaacac caggactttt ttagaaagta 1860
gttctgggtc tccaaccctc acactctggc tccctccttc tgagtctcca gccccggctt 1920

cttccctcca ctacaccctc tctattcctc ccaaatctcc ctccagtctc caggcccagt 1980

ctctaaccaa gggagaggga aagaccagaa cttaaaaaaa aaacaaaaaa aaaacacccc 2040

tccccctttt tctttttgag aacagacaac aagcggtgag tgatgatttt aaaaactctg 2100

aacatcttgc agggggagga gggttgagaa aagggtgaca aaagga 2146

<210> 14
<211> 2427
<212> DNA
<213> Homo Sapiens

<400> 14

tttcctgggg tctgggtggg gagccctttc cagtaataaa catgagaggg acagagaatg 60

ccagagagct tcctgttggg actgggatgg ctggagggca tcacagcact ggtttcgaac 120

actgaacgga gaactgccat gcaacaagaa cctgggcaca tcagacccct gcaggcaccc 180

tcgggaccca cagatcgcac actcgatggg cgctcgcagg acgtgaatgg catctcggta 240

acgccaagct ccaccccaga gccacagggt gcctttcacc aggctacgga gcctcctggg 300

ccacactgct cagggacatt ctgcagggga ggaaattgct cttttttttt tttcatgatt 360

cttgtatgaa ttctaattaa gaaacattac attcagaatt atagcactca cagtgatagt 420

tttcagaaga ctgtaacagc ttctttgcta cttaaggggt acagacggtg cagctgctta 480

cctgaatgac ttcttaaatg agtgactacc accactctaa ttttcctagt tgtagctctg 540

ttacaggtta ctgaccacaa gtgttggcaa agaaactgtt cttttaagca ctttggcgct 600

ccctggagtg acggccattc ccagcagcta aactgtatca tactcccact ttaactctgt 660

gactgtttca gtatttttaa aaatacaaat cgactctcca ggaaaaaaca aaaaaacgaa 720

aaaacttctc acttccttca aatccctggt ccaggctcac ctgacctcct gcaagctgcc 780

cttcagggca ggtcccagcc ctacctgctg cgtctgcttc tccaccccac tccagctgag 840

caggcagagc ctgtccttgg aaacggcccg gccacctggg agggaacaaa caggcactgc 900

ccagacccgg agctgcaggc agggctcagc aggaccccag gcgggcacac aggggctcat 960

ctggagggac ctgaggggca caggctacag tcgggagagc cccgggcagg cacacagggg 1020

ctcatctgga gggacctgag gggcacaggc tacagtcagg agagcacaga caagaggccc 1080

ccgaggccac cagggccgac ttttacttta tcctaaggat cagggacgca aaaggatttt 1140

aacaaagacg gccacaccac ctgctgcaga atgagtataa atgcccttct tagccttcga 1200

ggccggcact ggcctcgctg acccctctaa tcctgccacc tgtgcagcag gcggcaacag 1260

ggacaggcag acgacacagc tggggccaga ggacgacgca gagagaccca catcagagac 1320

cctcggggag tgacagagcc cccgccccaa ccccagtgag tgggacaggc tgagtgggga 1380

ctgagcacgt ctagggccag agtgggaggg ccagccgtgc cggcttccaa agaggacccc 1440

gggaaacaaa caccctccca ctcggcaagg acaaatcaag caactgccga gacaggtgtc 1500

cgcgtcccaa cccaaggctg cccagacaga caagcgctca ccgccgcact gccctaacag 1560

ctgctgagac acattcctgc agccgcctca gctagagcca gggaggccct gggagaatgg 1620

ccttgcctgc tctcagcctc ctctcatctt caggagcctt ttcccccctt taccctaact 1680

gacatctccg ggcccctcat ttgctcagag tctgctcagt ggggcaccca acggtggaca 1740

ggtgagagag gtgaggggct gggctgtcac cgcggccaag ggagccatgg ccctccagcc 1800

tgaacggctt ctgcccgaaa tcaggacaca aacacagcat catacacagc agaaacagcc 1860

cagaaaggcc ccagcccaga tggcactgga ataaacagtt ttccccaagt cactttaaaa 1920

tgaaagtttt gggctaccta cttctaaaga ggacttcagg acagttccag gagttcctcc 1980

atgacacacc ccatggagaa tgcttggctg ctgcgtttac tcaatgtagc cattgaacag 2040

aaactcaaga gcgttgctct gccagcctga aaccttcttc atgcttccag ccatcagggt 2100

ttcagccagg tcagaaacaa cagaggcagt aggctcttcc agaacagcag tgtgaggctc 2160

tccacagacc ctctccagca acatcactgc cagtggtgaa cattacaaac agcacccact 2220

taaagtccct aacgtgtctt agggacacac ggcgaatgga gaaacgctta tccaagaaaa 2280

ccacctaagt ggcagcagga acagcaactt cgtgacactt gggccacaac ctgcttcccc 2340

atccccatgt gacaaaagct ctactcccag caggcaaccg agaagacagg ctccctctcc 2400

cccagtgcca ctgcagcagt ggcgggc 2427

<210> 15
<211> 3015
<212> DNA
<213> Homo Sapiens

<400> 15

tctccttctt ctcctggcag aactgctcga tggccttgat catgcccagg cccatctcga 60

tgcagcagta ggcatggtcg gcccggggct cgggacagcc cgccacgcag tagtaacagt 120

ctcccagggt gctgattttc tcacacttgg tctcctcaca caggcggtcg aagcgaccga 180
acagatcgtt caggagaccc accagggcgt gggcagactt gttggcactc atcttggtga 240
agcccacgat atctgcaaat aaaatactga cttcttcgat ctgctgcatc ttaaaagggc 300
ggaaggctat aggagctttt tggatggaag actttttctt cctgttcttg gggctcgagg 360
tggcatgcct cttgacagaa ttctcactct cctcatctcc ctgcttcatt aagtcatcgg 420
ctatgattct tggcatcacg gaatgaatca tcctctcttt gagggctttt tccacttcca 480
ggtccttccc gtgcataatg gattgcccca ccttgaggaa ggtgctcctg gacctcacct 540
gggacatgac gaacaggtgg accccgatgg cgtggatgca gccgtggagc agccccctgc 600
tcagcagctc ccagtgcagg gccccggctc cgggcgaggg gaagcaggct tcatcccgga 660
aatggtagcc aaaggtctcg aaaaggacag agtaggccac ccccagacac aaactcaggt 720
acaaaggtaa gtgcatgacg gtatagagca aaaagagcac ttcgatgcac atggagaagc 780
tccccacttg agataagcaa gtatctgtgg gccggctgtg gccgtaaggt tggagctgtc 840
gccgcgtcct gagacaggcg tcaagacctg gaactgcgca gccagggtca gggcgaacac 900
cagcagggtg agagccagcg aggtccacgc gtaatgccgg gcgtacagct tggtgaaggt 960
aaacagaaag aagcccacac acaccaggag gaagcacagc gcgggggcga ccatgacgat 1020
cagtctggat ctcatgtgga ccgcaaaata gatgctccac agaaggcagg cgaagccgat 1080
gtagaagagc gcataccgga accggcgctg ggtctgcggg aagcagcgct ccaggcaggc 1140
ctcctccagg ttcaccgagt cgaacttggg gtcccaccag cggctggagg ccctctcgaa 1200
cagctggggc agcttcttct gcctgcgcag ccggcctccg ccgcccactc gccgggggac 1260
gcccccggag tccccagagc tgctgcagct agaggagatg ctgtatttgc agtgcttggg 1320
gtggctgttg gaggacagct gcttggggtt gatcttgacg cgcacgctgt tgctgtcccc 1380
gctggagtcg cagctcacct cggtgctgtg gtgatgcagc agctgctggt ggggtgggga 1440
agccatgttg tcgagtcccg gggcctgccc cggccggggt caccagtacc tgccagcaaa 1500
acggggagag ttagcggcgc tcccacctag gcatgcacgc ctagaggccc gggacctgct 1560
cctgtcctaa ggggcggctc cagcacgcga cctggacagg caccatctgt tcctgtggtt 1620
cccggctcag cggtgctccc accgccccca ccgcccccac cttcgaggcg cacgagaacc 1680
gctcgggacg gacctagaac gcccgggggt ccccgccgcg tggccgccgt ggctccggga 1740

ccgctttgct cgctcgcctt ccgcgcctct cgccccgagg gtggcctccg cgccgcgcgg 1800
cttctcctcc tcgcgcgctc gcctcctcca gctgcggctc cggagggaag ttcagacctt 1860
gagcgctccc agccccgcga gccgcctgcg cacaaacaac tccgctggcg gcgcggagcc 1920
ctccatcctg caggagccgc gctccgatgc gtcaaaggcg gcgcgcggcc ggccccgggc 1980
ccggaccccg acccggagca gcgagcttcg gcgggcgccc ccggctcgcg ctccccggcc 2040
gcccccgcg ctccgggccg gccctgcccg cggcggcggg cgctgggggt gggggcgccc 2100
cgggctgcga gtgcgcggag cccgtcggcg cggcccgtct agtggggcct gcttcccccc 2160
gagtgcgccg ccgctcccgc cgcggccgca gctgtcccgg gaccgagcgc gtggaaccac 2220
ggacgcgggc ccctcgccgg gcggcggtgc agacgctgcc gcagagccgg gctcccgcga 2280
cgccggccgg gacgcccgcc cgcccgcgcc cacgccgggg gccgcctccc cgagctagag 2340
atgcggccgc cgccgcgccc gccgccgtgt cccccgcggc cgctgcctca tgtcggaaga 2400
gctgccgccg ccaccatctc cggcccctgc cccgcccgct gtcactgaca gacgcgcgcc 2460
cgcgccgccc ctcgcccagc cgcagcgcgc acgcgcggcc cgcgctcgcc gccaccgcct 2520
cctggccccg gacacgcgca cgggccgcgc gccgccccgc cttggtgcgc gcgctgggct 2580
ggggctgcgg ggcggggcgg ggcggggcgg ggcgcgcgct cggctccgcc ccccgccccg 2640
ggcggcgccg cttctaggcg gcgggctgtg gccgctgtcc gcgctccggg cgctgtgggc 2700
ccgccctccc tcggcgtccc agaggccgcg cgccgcccgg ggtcccgacc ctgcggagag 2760
acctgaaggc gcctgccgtg gaagaagccg ctggccgcaa cgtttgggaa atcaaaatgt 2820
tgccttccca ctcggcacgc ctttgggata aatcgaagtt tccaaccgtt ggatttccct 2880
cccgggaggc tccccttgct ctgtaagcaa tgaatgcggg ccccggggca ctcggcctgc 2940
cctggacctg ctcacggggc gaccttcacg ggcctagcct ccaaagctgg gcccaccgac 3000
gaccacgttc ccccg 3015

<210> 16
<211> 3093
<212> DNA
<213> Homo Sapiens

<400> 16

gtcactacat ccggcagcgg ggtggcccct agctcctgct gcccccccgc cctttctccc 60
cgcccgcccc cggagctcag ccgatttctg aggctccaac tctacccact ccctccccgg 120

gccgccgccg ccgcgccttc ccccattctt actccctcga ggagagccac aggttgcaaa 180
tccaaccaac ctcgcaatct atttttgcaa aatcactcac aaagatctcc ctttcgcgcc 240
cgcgcccgct cctcccgcgc cgggtcccct cagccacggc cacaaagtgc ccttctctcc 300
tcctgagtct tgcacataag gaacgcgggc tggggctctg ttcgtctttc tcctcgccca 360
aggtaaggac ctcgggaatc tgaagcctgg cgtccactac gctcaggccc gcagttccct 420
ttttacagag cttgcaccat gggaaaaaat aaaataaaat ttaggaaagg gaggcaacag 480
ccattgggag ccaacacaga gtcacgcagc gcccaaaata caaacaccgc agcggccaga 540
aatcccgcca cctttctcgt tctcccaggc tgtcctgtcg aggttccctg agtcccccg 600
cacactgaaa ggcatcgcag gtgcagtgcg cacccctttc ccacccaccc caagaagccc 660
tgtcccgcca tcagtctctc tcctcgggat gagcagggag agcgcgcgga ggttcccgac 720
tccctcgact acaaccaaga aagaataatt ttcaaagtgt tcaacatccc cgcccccaag 780
ctccccaaaa cacaggggca gggaacacca aaacactcgg ctctcattag gaagatcacg 840
gctctgaaag gaaatagtag acacgatact tcatctcatc tggatttatg accaaaaaaa 900
caaaaacaaa aacccaaaga gttcgcttgc attttttcct tccaaatctc ggttcggctc 960
gaaggcaggg aatctaaaag accgaggccg atggaagaga gccagcgggg cgagcgagcg 1020
ggcagcctcc ctttttgcct cccggagtta cccagaagga caggggaagg gaaggaagaa 1080
gaggcgagga aaaagaggag ggagggaagc ggaggccagg agcgacggag caaggaaagc 1140
agtttgcaag cgagaaaaga gggaaaaaac acagccgcac gaatccagag agatcacaag 1200
ccgtacgcaa gcagcagcag aaagagcgag agcgcgagcg cgcgtcctct ccgcgtctgg 1260
ggccagacag cccccagact agcccgaatc accccccaag cactgtctcg tcctctctgc 1320
tccggccgcc ccctaattcc cctccttcct ctcctccacc tcctttccaa aaaccaaaac 1380
aacacaaggg agggtggcaa aagcctcccc aaaccggccg attcactcaa agacaacaat 1440
aataataata aatacataac aatctatatc ctatggtggg agagacgtgg gactaatctt 1500
cggcatttat tttaacacct gacagctaga ataaataaat atatacattt atatcaatag 1560
atacacatag aaaacttgga gccaaagcat ttggcaagag cggaaaaaaa aagaattaaa 1620
aggtaaaata atgatcatga gcagcggcgg cggcagcggc accagcggca acagcggcgg 1680
cggcggcagt agcagcagca gcggcggcag caacagcaat aatcacctgg tgtccggcct 1740

ttcctagaaa cttcttgcat caccacttct aagaacccca gttctaagaa tcaacagagc 1800
tcaattctcg gaatttgagc ttcggacttt accactgcta cgtggcaggg gaggacttgg 1860
tgtcagctct ccgagatttt tactgcccct ggccaaccaa aagccctcaa agccacaaga 1920
tttttcact ggccggcata tttcgaggtc ctcataagca gagcgtctcg gatttggagg 1980
ttccggttcg aggctcgagg ggcctgaagg tggctctccc tccccgggcc caagacgatg 2040
gtatggcctg ctccgccacc atcacgtggg ctcctcctct gtgacgtcgg cgccttcgct 2100
gtagcaaagc tcggcctctg gaattctgag aactaatttg ctattcggtg acataagagg 2160
gggagtgcgc tttgctttcc cggggtctgg ggctaattcc ttctttctta cccataaact 2220
cagcagatcg agctaaatgc acaaaaggga gcgagaggtt tgaaccactg ggaaaagtat 2280
gttatatata tagtagggtt agagaggcga gtaagagaaa aataaaataa aataaacatc 2340
acagctcttt ccaactagaa tattaggcac cacgagaaaa atatttgcca agcagttttc 2400
ggtgggttca tttgctttat ttttatttag gacaggggtt tttgctgttg ttctgggttt 2460
ttttctttct ggtgtggtgg cttgggattt ttggtttctg tattttgatg gtttatggat 2520
ttttgcttct gatttttgc cttttgcaag tttgtggtgt tacgtaaatc acaggatcgg 2580
catcggttgg atttttttgt acgtgccttt tctttcccta tctaatccct caagcgtttt 2640
aaagatgtat tatttcaata ctaatactat tgaaagaagc ttaaattttt ggccatatgt 2700
aacaatccca gcccccactt tttttttttt tttttccttt ggtgcaattt tctttttccc 2760
ccttggactt ttgctgaagt gtgtctctcc tgcacttcag agaaatgttc aaaggatttg 2820
ttttggtttg gtttgtttct ttccaggaca gcaagtggtg ggtttaatct gttattgttg 2880
actcttggga aatttcttgt tgcaagaaac gtgtgtgtgg gggggagggt gggggtggcg 2940
gggtggtatg tgtgtgtttt ctacaaaatt ctgtgagcca aatacctgtt tgtgttttgt 3000
tttctcttaa ggtcttgaga tttttgtttt cgaggctcgt ttcaaggtcg ttgtaaaaaa 3060
atctcttcag tctgtgttta agagatcagc cgg 3093

<210> 17
<211> 2436
<212> DNA
<213> Homo Sapiens

<400> 17

```
agttctggat gacaaggact aattgcccag ggcgttggcc gagaggaata ggaagagcgc      60
cccgtggagc agggcgggcg agaagagccg ggcggccgcg gggtcctcgc gcatgctggg     120
caggaagagg aagagctgca gcacgaaggt caccagggag atgcacaaga agtaggcggg     180
ggccaccacg ttgagcagcc gcagggccag catcctcgat tacattcctg cggggcccgg     240
ggtgagaaga ggactgactg actacggcgt ccagggggcg ccctcgcccg ctcctccgtg     300
ccgcgcgtct ggggcactgc acgcgactcc acgcccccaa ccccctgtcc cgcggatttc     360
ccctccccag ttccctaacc ccggatcagg caggccagag acccgagccc gcagcggcct     420
cgggtctagg ccccacctgg gggtgagtgt tgcctggggt tttgagcgag ctcggcgcct     480
tggcgcggca gagtctggca caggagggag ggactcgttt gcagtcttta ggagttgtga     540
gggtgggggg caccggggga taggactcct cccctctgtg gatgtggaaa caagcccagg     600
tttgggattt ctggttcaga cctccctagg aggagaaagg gaaggatttg gggtcggggg     660
caggagaatc agcggcactt gacctgctgg aaatcctgct tcacactccc ctttctccgt     720
ccctgcgtcc ccacccacac acacatcttg ggcagcactc agggacctca ccaggttacc     780
caaggctctt tggaggtatg tgtgcgtgag ggaccacgat gtgtgtgtgt gtgtccttgt     840
ctgtgactct gtacgtgtaa tctgtgtgcg cttgagtatc tgtatttgtg tatgtatata     900
tctgcgtgta cagacacgca gcctctgcta tctagcttta aaaaaatcaa ttgaaacaat     960
cgatacactc acactcgctc tcgggcaccg ggtgaggtgg gggcgcaacc agaatacctg    1020
tgtcagcggg tcctggccag cacatctctc caacgctggc tgcccgggct atttctttgc    1080
gaagagcgtt ttcatttgag gcgaaattaa aatcccccct tcgcgcccgc ctcccgccct    1140
cctgctgaga gaaacccaaa caaccctcat ggcgccgaaa tcctttccat gccgacaagc    1200
ccgccctggg cgcacgagtg gatgcttggc cagcctttcc tgggatcgac cccgccgccg    1260
tgctcagccc tcaccacgtc cccatcccac cccacgcctc acagccgggg ttcctggcca    1320
gcttcggaag ccaccgagaa ataggattcc gtgcgcccga gagaactttt ccaggggcta    1380
aggaatcggc cagccggagg cgcgagaaaa gttctgggaa ggcggttgca cctaggatgg    1440
gtggatgcca cggggcctcc gtccaggctg tcccgtccgc acgggtcgac tggtcacctt    1500
ggaatcccct ttgcaggtcc cagcgccccc cgggaacccg cagcctccgc ggagagcgtg    1560
ggcctctccc taccgctggg gcgcagcgca gtgcacgcct gagggtggtc gccgggggct    1620
```

gggcacgccc ccagtcctgc gccgccgggg gctgcggcgg tgctgcccac cccagagagc 1680

cctcggcctg gggctccggc gaagcaagtg ccttcccggc gccggtcgcc agggggcgc 1740

gggagcagcc agatgcgccg cagcgctggg aaggcggcga aggacagggg ctaggggagt 1800

gaggggcgct cggcaggcag cctcagccct ggccctgcgc gggagaaggg acagcagaga 1860

ccgcccgtgg ggccccgggg tgtagggagc tgtccgttca gccctggcgc ccgcctcgcc 1920

cgcggcagag ggcggcacag ccggagcctt ggaaagaccg gcagcgccgg cagccgcggg 1980

cttctcggcc actgcctccc ggacgcacgg gaagccgccc cccgcgccgc cgccgccgca 2040

ctgccgccgt cgcagagggg tgaggaaatc aactcaccga gctctggtcg ccgacaagag 2100

gagccccgga cgccggctct cgccctgccc gaggctgcaa agttgtggac tcggcccggc 2160

tggctcgcag cctgcgcttc gcttcgggaa ctgggcaagt agcggggatg tggggaggag 2220

ggagcgggca gctgctggct tcccacttgg gcgccagcga gtaagggcca ggaagcggcg 2280

gggatggcag ctgggcaccc cccagccccg ccacccctcc ctccgctcct gggggcggtg 2340

ctctggccgc cagtctcagc atcgtggact tgccccctcc tcccctctcg attcccccttg 2400

agcgggctgg ggcgccctgc ctggtttcag cgcccg 2436

<210> 18
<211> 17219
<212> DNA
<213> Homo Sapiens

<400> 18

ttcactgcac accatccatc ctttaggctg ctagcgtggc cctcagtttc cttcaagcca 60

aagacacatt atttccctgt gaataagtgt gtgaatgtgg ttgtgtggcc cactcttctg 120

tacccaagtc ggtctcactc actcggcagg aaagtcttcg tcaggcattg aggagtgttt 180

gtcattcagc ctgtgtatct aagcatggat gtatgcatat gaatgctttt tgtgtttcca 240

agtgaccctc ttcacacctt taaatgggct attgtctctg gaacgcactt acagaaattc 300

tgccccagtt tccagctcaa caaagtctgg gatttttctc tcagaatatc gctatacatc 360

tctgtatttg catacccatg tttacattcc ccttggccag ttctttatct cttatgtatc 420

tcttgttcca tttgggcaac ggagcccaag tgtcttgatt tgtctcagca catgtcattc 480

aagcgcacat cttaatctga gcattcgtac tacttccatt ctttttgctt tgcctataat 540

gtggctcttt cccaggccag accaatatgg gtttgcagtt taaatgtaaa cagaggatcc 600

cttaagatca caaactctta aagaaaggaa gaggagggag gaggagactg gaatgttagt 660

aatgagttat ttatgtatag ggtaacccat ccgcccattt caagctgaag aaagaactag 720

ataaatgtct gcagaggtgt attaaaattt tagaccaatt taatattagc catatatata 780

cacacacaaa cgatggatct cacaacacat cagtgtccag ctcctctttg gaggctgtct 840

ccttgcacat atctaactcc tcacaagttc ctggttaaaa gtatgtctat ttatatagag 900

acaactcaaa gaccgctttg ctctcttcag tgcatacctg tacatgctgg cggggcattt 960

caggctcagg tccttggtct gcagcagtca tagtcagagg ccccacccga ctacagctat 1020

gtgcagacct tcccgtcctt gaacacccgc atgaattctc tttgggaaca ttgagtctac 1080

gtctgtcttc caaactcagt ttttaaagtg agggtgttca ttgtgtgtgt tcacagctcc 1140

ccgactgaga aatacctggt caaactagat catctagcag ctgcgtttgg tttgtggaag 1200

aatcgagccc gatgcaccaa gatgtgaccg tgcacacata agagaaagga aaaccctagg 1260

cccagactca cagctcctcc ccactgcgag ctactctgcc tccaggaatt tccactaggc 1320

tctttgcccc cctttcccta ttacactacc ccaccacggc ctttaaaggg ttaagagaaa 1380

cccccacggc cttccctcca cccagctcct gggcccggag gctagctcgc tcactcgcag 1440

ctcacagagt ctgctgccat ctccgcgcct cccccccctc cagtccttca gccgggaggg 1500

agcctgcatg cctgtctaga ccgatctatc accggcacac caacaacacc cgcgagagcg 1560

cggagaccct gcccaggtcc cccggcccgc acggttcacg gcggacagaa cccggggagc 1620

gtgacggtgc ctgcggggtg ggggtagggg gcccacaggc actggccgct ccgagcgcac 1680

cacgggtcct ctccagctcc ggcccccgc gctcccacca gcccggctgc cggggcacag 1740

tcacagctcc gcgcgctggg cagtcctatt tttatcttgt ctaatcctaa actgggcggg 1800

gagcacaggc gtcgtgctta gagaacaaga gataggcgcg ggaggcgggg aggagcagcc 1860

aggcagcgac gcgagcggga gcgagttaaa gacacagtgg aggcgaccgc gggcaggaag 1920

aaagagcggg ctgcgcgggc ctcgaggcgc cgcacgcaac gcgccgcccc gcgaagttac 1980

actcgcttcc ccgccgtccc tcgctaccgg cccctcccca cttcccagcg gcgctccccg 2040

cagcctggac agcgaggccc cggcctgtgt gggaggcgga ggccaggacg gccctgcaaa 2100

gggctggaga gaatcgctaa tgagctgtgc agcccattga tccggagaac acttcctaat 2160

taactctcag tcacctactg gtgacagcgc ctcaaaggac agcgctgggg ccagccgagc 2220

cccagccgcg acttgccggc cgggcgcctg ctgcccaaaa cccgcgcgct cccctaaaca 2280

ccccaaggca attggtgtgc gtggcggtgt atacccgcac actgaaggcc agcctcggat 2340

taaacctgga tggcatatta atcatcgcct tcccccccca ctccgctcta cgcgctgaaa 2400

ccctgcccaa aataaacacg aaaaccaaca ctgcaaaacc cacaccagat aacaggtcga 2460

gagaggagtg ggcagggtca aggaacgagc gtattcccta agtgtccacg tacaggaatc 2520

tttgaggctg acaattaaaa tgccttccac gcaagcaatt ctgttggcaa aaccgaaccc 2580

aacacttcgt aaggattgat ttcggcccgc ggtgtccgtt tccagtgcca caggaagtgt 2640

cagatggcca gaaataccca agtctgtctg cactcaccac ccaccacctt ctggggtggg 2700

ccatttcctc ctagtcttta ctcccaggcc attttaagga tctttctcca aaagactacc 2760

cctagaacca aataccacac tacccctcct ctagcgtctt ctcaaaacga aaaaaggggg 2820

ggaaaacaca gctaaaaccc cgagctgtct agtctagacg cagcgaggct gtgcgaatac 2880

ctatttgaag cgcgttggtt tctttttaag gtttgtttct aaatacacat aaccctcctc 2940

cttccttctt cgctcttcca ttcccccac ccccaccctc cagacgccgg cggcgcgcgc 3000

cctggcgcgc gggcactcac agcctctagc tggagatcgc ggactttctg gaaaagattc 3060

attaacagca aattacgcct cgcccggcca cggcctacac gctgccagac tcgtccgtct 3120

ccgggacccc gggaagggcc cactcgagcg tgcgagcggc gggaagcccg ccgggaccac 3180

gcgtgacttc ggccgcccta ctccacacac ggtgccccaa ggctctctcc gtaaacagca 3240

ggcgctcgtc accgccgccg cgagcccccg agttggcgga ggctctgcgg cgcagccgcc 3300

cgccggctcc gagcgcgggc cccgcggcgc ctcgccctcc ccgccttggc cgaggatctt 3360

gccccggtgc gcaagttcct cttcgcctac agcctccttg ccacacgccc tccgagtggg 3420

accaacttcc tccggccgca gactttcaca cgcccatcag ggctacccac cagactcccc 3480

attgcccagc ggagggccca ggtctcgccc gcccccgccc tccctccacg acgctgcccc 3540

cgcacaagca cattctacac ccacccctgg gggctcataa cttaaaacct acgctgaaca 3600

tcggggaggg agaggagggg gaaagcggga gggggatcgc agattataat cttaaaagaa 3660

catttacaaa acaaagcgtc tgacctggct ggcgggcggg ccaagtcgga acccccttgct 3720

ctgctcgatt tcttagtagt tttttttttt aatccacgtg attcgtgctt tggggcaagc 3780

caagaaaaaa cgggactccc ctcccgagtt gcgcggcagc ggcggcaggt cgagctcggc 3840

tcggcccgag gcactcgcca cacaccctcg cgcacgcaca cgccgacgtt ccacttggag 3900
gaacgaccgg cccccggaga ggcgagacga ggcggcggtg gcggcggctg ggggttttta 3960
cagttctttc ccgagtcgaa aaagaaagca gacggcaacc cgctgccctc cccttcctgc 4020
tcgcgagcta acgccgaacc cagcttccca gcttccagcc cacctccccc ccctccgccc 4080
gactcgcggc gctcagcggc gaccgcgctc ccgcgcacag acacacacag gctgcaaact 4140
tccactctgc gaactccctg ctctcctcct ggcccaccgg gaggcgccag gcccccagcc 4200
gcgcccccctc cctccctggt gcccacgcgg ggccgcttag gttggctgca ttttaaataa 4260
catagctatg aaataaaaac gaaagcgagc caagcagcca aagggaagcg cgaggcaaaa 4320
gcttcgcggg gtacgctgcg ggaggggggcc ccgggagagg gggccgactg cagccctcag 4380
cgcgcccgcc cgcccacccg tccgcggtct cccgggggaa aggtgttccg ggcactgacc 4440
tgaaatcccc ggtgggggag taggcaggga gcctgcgatc cggctctttg aagttttccc 4500
ccaagcggac tcgaggcggc gagaaggagc gggggcggt tgggtgggtg gagagagata 4560
tgaggggtgg ggggggttgg cgacgttaac gagcggagaa ggagccgggc cggagaagcg 4620
cggcgagcag caaagtttgc ggtccccggc tgcccgcagc ccgctcgcgg cgccgcgctc 4680
actcgactcc ctcgccgtct gctggcagcc ggctgccctc ggggtcggcc ccgccgaggg 4740
gtgggctctg ggccgggtgg gggcggggcg gggcgggctc ggggcctccg ggcaggtggc 4800
gacagccccg tgcgatcctc cgggctcccg cagctggact gggcttggcc gagctcagca 4860
gctcccgcgc tgccgccgcc gcccgcgctc gggcaggagc cccagcgcca tgttgacggt 4920
tcgccgcgag cgcccgctcg ggctgggtgt gtgtgagtgt gtgtgagtgt tggccaagtc 4980
gtccctgcgc ggcgacagcg cggcttgggc tctccgcccg gcggctcgcg ggctccccct 5040
ccgccgccgc cgcccgccta gctcccgctc tcggtcgcgg tctgggctcc tgcgcgtctc 5100
ccccgcagcc gccgagctcg gcccgcgttc agcgaggagc gcagctctgc ctcaccttgc 5160
cgctgggagc ccaggctccg tctgccgccg cacgccgcga tcccaacgcg tccccccctcc 5220
ctggttccct cctcttcctc ctccccctcc ctccgcccgt cctccctccc gcccgcccta 5280
cctccctccc tcccaacagc cgcctcccct ccccccgcag gcgcagcgct cgggcgacag 5340
ccgcccgccc gccgccgcct ccagctctcg caagtttgag ctccgccagt ctccgctcac 5400
cctccgctgc ggcgcctccg cggcggggtt tcgggagggg tgattcccca ggaaaggttt 5460

gcccgagttt ccccggccgt catcgatgca cggcccggca cgttttggcc tctctggatc 5520
cgaaccccgg accacttggg tggcgccccc cctcctcgtc ccccggcctc ccctccccag 5580
ctcgggccga gttggctgcg cacgcccgac ccgggcgcac gtcctgggcg agccggagac 5640
cgacttgggc ggcggcggcg gcgagggctg ccggcagagc cagagccgaa gggatggggg 5700
cggcgggggc ggctctccca gccgcgcgcc gagactcccc tcgctaccca gaaggccagt 5760
ggaggaccgc aattaccata aagcgcctcg cactccgctc agccaaagct gtcaaacccc 5820
agcggcagca gccgctgccg ccgcctcttg ccaccagcgc tgcgctctcc cggcctcgtc 5880
ccctccgaga cccagcggag cgtggcctgt gtgctcccgt gctcaccccc accccagttt 5940
gccccttcct cccagaggag ccctttccca tttcggtgtt aaaagctacc tgaaaacacc 6000
accccaggca acggtggccg ttccgcctga actccgcgcc cgagttcccc ggacgtttca 6060
attgtgtggg aattgtttgg aggtggggga agggggagc agcccgtgga ggcaccggga 6120
ggcgtgtggg gggcggtcgg cgagcggaga tccgcctgta tttctcccgg ttctcgagca 6180
ttttcacggt ctgtacctct gatggttgga ggggctgccc aggcccctcg ccccggcgtt 6240
gggagattat ctcccgcgtt ttattttcct tgacagggag caacttttgg cttacaccgt 6300
tcgccccata aatttccact cggtaacgtc gtgctgatcg gtgcttggcg gcggcgtaca 6360
gcctgcgccc gcagcagttc aattcaattg acacgtattg agcttctcca gcgcacccgg 6420
cgcggctctg ggcgcggggg gagggcgggg gcaggcggga gggaggggga agcggagaag 6480
cgaggttccg gcctccccgg agcgcaaagc ctcaagggcg agccgagaag ccctagttgg 6540
tgaagctggc gggaaggggt aggagtgggg gagggggtat tccggactaa ccgcgcccag 6600
actgcgggcc cggacttcgc ggccaatggt gaccccattc ggctgcccac tagggtcttt 6660
ttatactggg aagggggtgg ggtgtctctt tctgcctaag gacaagatgg tgcaggctcc 6720
atgaaacata aatctgaaat ggaggtggct tgtaaatcca cacggaaagt ttttaaaata 6780
tcctggagtg tccccatgcc aaaaataatc aaaagcatct caagttgatt actaagtcaa 6840
ggtcaaggcc aaaatcagct ctagctagca gatattttcc cttttaaaaa ttagactcac 6900
tactgactgt gggactggta ttaggtgcga ttttattcat tctgttttag ttctcagcac 6960
atagttataa aaacagccct tctcaaagca gaagtgaaac atctctaccg tttgaaaccc 7020
gaaacttcgg cagaattaaa tcttcatgct ccagagctgc ttataacaac tgatgcaaaa 7080

gacaggcgag caagtgcccc cgcgagcagt gcccagccag gctgggccgc gaacgcttcc 7140

aggaccagcg cctgcagaat ggaatgcacg ctttttcttc cctggtcttt ctgggggtgg 7200

atatctctgc aagccaaccc acacattttt ttaatctccg gtaacaagca gaaagacacc 7260

tggactatta tgctgtaaaa ctatttttta taaagacatt ttaggaccag acattaaaag 7320

ttattagatt gaacgcatgc gagttacaga cacagctgga gaaaaggcct ctggttgtgt 7380

gttgtggcaa catttctttt tgaagcgact ccttttattc aaaacaagtc aaaaataacc 7440

gggaaggctg aagctgggta tttgtgggga aactctgaac gtggttccag gcgaggaggc 7500

cgctctgctg ctgcaggctg gacgggagaa ctggcctggg ccagtgtgga gccgtgccag 7560

gctcttccgg ctcggttagg ccgaaccgca gaggccctgg cccggcgccc agccaccttc 7620

tatcccctcc cctcgtctcc ccaactctgc ccccaggcac aggcttttct cggagttcga 7680

agtcgcatgt aaacgtccag aacttttaga aaatccagga accgagctcg ggcctgcag 7740

cctctcagag tcttacggcc tcccggggct gctgacaaag aggcctgttt gtctaacttt 7800

aaaagtccta gaattcggga ggctgagcca ggagaatcgc ttgaacccgg gaggcggagg 7860

ttgcggtgag ccgagatcgc gccgttgcac tccagcctgg gcaacaagag cgaaactccg 7920

tctcaaaaaa aaaaaaagaa aagaaaaaga aaaaaaaaag tcctagagtt aaaatgaaca 7980

ggggtggtgg gggtgctgta aatgcgaagg ggccgagggg agggctgact gggggagaaa 8040

tttgatgctg gagacaaaca ggccagtaaa ttcgcctttg acgtaatttg caacatcctc 8100

ccccaccttt tctagggatc ttgcctccac ggtaggctgg taggattaaa gggtttctcc 8160

tccatctcct agggatacat ttttcaactc cgtgggattt caaaaatatc ttattaaggg 8220

agtcctaaga ggcatgagtg gtgggggga ttcgtgactc actgggaatt aaaatgtggg 8280

attttcccga ggcgctataa ggcagagggg gggaggggag attacaccta ttggcccggt 8340

tagttccaat tgttctgggg aaaacaagca gaaagctgtc gaagggcaat cccatcttgt 8400

gtcctcactt aagaaggggc tcagggagct ggggatgccg cggggcctgc aatgggtgcc 8460

ggcggtttgg cccttctcct gccccctcct ctcctgccct cccacaagcc cttcgggagc 8520

tgacccgggg gtcaggctgc tatacccagc actcagcaag gggaggattt caatgaaaac 8580

agaggaggag gagagagtga gcaaaggagt gcatgggagg ggaggccgtg cagcccacgg 8640

agaaataaag aaataggtgc cgaaatgaga ggtgctgcgg atggagcagc gggggctgcc 8700

cctccgccac caatctccct tcccatggaa gtgtctcctg attataacaa accactcgac 8760

ctcaggctaa ccaggccctg caactgggca atcgagctgg cccttctagg agcaggcctt 8820

gagcctggct gcccttctca aggcaggttg actctgcaag acccgctagg gcctctccca 8880

cacacctcca gctccaccct ggcctagggg aaaggccagg catcctgtgt ttggggggac 8940

cctggccttc taaccttgtg tgcaagattt atttataacg tatactagga tccacatcat 9000

aaagcttata acgtatgtgt aggcttccga tggtcctcct cggaggctgt tctcagggcg 9060

atgacacttg gtactggaac aggtgggact cggatgagta cagataatgt atttttacat 9120

aatgtgaggt agcctttctt taaaaacaca cccacactta tagtcttctg gtcactttgt 9180

acaagaactc tgggcttatc caatggcact ttaaaaaaca tccgctgccc catcctccaa 9240

gcacaaggca cagctggctt cccctctgaa actcaaacga ggtaacaaac aggaggcgca 9300

tcctcctccc aggatttcac tcatctggaa acatctaaag gtagctgcct cccagggcag 9360

aagactgctg gacttaaggg tcgccaggct gaacatgact gttctttctt cttcctagcc 9420

tttggagggt tattgggtgt ttggggaagg aaggccctaa gggcagcctc tccgtggttc 9480

ctagcaacca tcttggaatt tctggtctgg gctcgatttg ggtcaggccc tgagctcccc 9540

gaacacaggc acgtgtgggt gaactggcat ctacactacg gggcccgca gggtggttgg 9600

gtgtcgacca aatgggcata atacaggagg cttctgggag gctggccgcc tcttgcgctc 9660

ggcaagggct taagagccct cgacgccgcc gcgctgggcc gggtgctcct gcagataaac 9720

ccggacgcca cgtcccgcct cggctgctta tttagcaagg cgccttctgc gcatatttta 9780

cccggctggg ggcagtgagt gggaaccgcc gggagagggg gcggggaacc accaactact 9840

cgccttcgga cccagtgtgc cggcctccgg ccccagccat tttgtatcca gcttccctcc 9900

tccactccca ccgggtccct gcgaagacgc accaccccga accccttcgc ggtaccactc 9960

ccgaactgga ggggtagaga aggctctgcc agcccctcgc ctgcagcagg aaggtgggta 10020

cggaccagca gaagggaaag cggtgctgat ctgtctctaa atacctctgc cctcccgccc 10080

ccaacaaagt cggtcacaga ggtgagtggc gcgggatggt tgcctcgaga aggtcgcagc 10140

caggagcaaa gctttggggc tcacaacgga ctgggcattc caaacggtgt aatcttcggc 10200

acatttcacc cgctcccatt ccaccttccg aaccactctg ctctcggtgc tcatcctcct 10260

ctccatacct gcgttcccag cgcgttagca gccgcgcagc cccggccct gagtgcgggg 10320

ggtggggtgg gggggctcag ggtggtttgt gtggtgcggc ctgacagaac cttctgtgtg 10380

cggcggaggg aggaggctaa tgcataatgc acagcgcctg gaagcccggc cattagcggc 10440

ctgtcggtga cacagacaaa cgactgagag ggaggagatc cagctcgctc tggagtttaa 10500

atagaccagc gtggagggga aacgccatga tttaaaagtg tgtgtttgca gacactcgca 10560

tatattttaa tgatttccag caggctatgt gtcctagcct tgcatccccc cctccccaaa 10620

aggaggatgt gtctagagga gggaggatga aggcagagtg agaggcctca acccagaccc 10680

accagcagct tttgagagat gaaggagggg agaagttcag aaagcgaggc ctggggaagc 10740

cacacgaccc aaggccaagc ccagattttg ttcttaaaat aataatttaa aaaaagagcc 10800

acccagcctt ccaccctgga ctacaccctg tgttgtacca gaaaggcctg ggggaaagaa 10860

taaattctgc caccccttgct tcccccggga gcccacattt ttgaatcttc cttttccact 10920

gacactccag agacctcctt cctcccggtt taacacacta gtgtttttta gcaattaagg 10980

cgagaagggg gtggggataa agataagcca attttttttt ccgccatgca agtgtgagag 11040

ataagataac gctaagctgg ggcaaagcgg ctgcttacaa cctcccctta gcgattttga 11100

tcggtcactt cttatctcgc aaaaatgctc ttttcgaaaa ctggatcaaa aaagaaaaag 11160

agaaaaagaa aagaaagaaa gattttcatt tggaagtgac atttaaaacc cacctcccct 11220

ccactgacaa cccaccccccc attggcgggg ctccccagga aaagtcgcac ctcgactgca 11280

ggatttcagg ctttctcgcg gggggcagga ttactagtgc aattaggctg aataaaacca 11340

gggatgcgtg atgaggattt atcctgtatc caacccaagc tcgcggctgg gttttccttc 11400

tcttagttac tggtcgatgt ttgtgtttcg ccatcgcact cagtgccttc aaactaaaca 11460

ggtagttata cttgggaggg tcatataact atgtcaacat gaagtggttg ttaatgcgaa 11520

ataagaagaa actcttctga tatttatagg cccctataaa gggagagaaa ggaaatcaca 11580

ctccctagaa cccgaaagct cccagccgaa ccggaccctc ggggagtttt agaaatcctt 11640

ctttccgcac aggaatttcg cgaaggggtt tccggaagac tttgtcgagg aaggttttgg 11700

tatcagactc cacgacctca gagcgccctg caaattcccc ccggcccagt ggctagcccc 11760

agaatgcaaa cctcaaggcg ggcgccccag accaagctga cctagcctct gcccaccccc 11820

agctcacaac cccgaggctc ccacccccctg cccccgaccg ggtcggcacc gtggcgtccg 11880

gcacatgcca agtctaccgt ctccaagcca ggctgcaaga tccaaaggtt ggggcctgcc 11940

gcggacggat tgtccctggg gagctgaaac cgcccagcgg ggaaacccct cctctcgccc 12000
ttccccgaaa agcttggccg cggtctaagc tccgagcaag cgggtatccg ctgacagccg 12060
ggcctcacgt aaggcccaca cgcgtcccat tagtcagccc gagttcccct cgggcgagcc 12120
gcctggcgcg gccccaaggc cgggctcagg cggggagagg gtgcggggcc gcagcgagct 12180
gcgggcggag gtgcgggctc cgcggagccc ggccaggctc ggttccgcgt tcctgggccc 12240
gcgggtaagc tgagtcggcg gggcaggccg ctccctggct ggcgccgggg cgccaagctg 12300
cgcttcacgt gcccgccata tcagcagcag cggcggcggc ggcgaggagg gggtgctggg 12360
aggcgcgcgg ccgttcccgc ggggtctccg gaccccgcct ccccgcccct ccccgtgggg 12420
gctgggccga gaccaccctg cggcccccat cccagcgcaa gccgaaagcg gcgctgccag 12480
acgcagagag gctcctgggg cgccgttccg agagccgcgc ggcggcagca gccaggggag 12540
ggtgctcctc gcggccgccc cgccgccgcc gccgcagtca cggggaccgg aagccctcgc 12600
gccgccccga gcggcctccg ctggccccag ggctgttcca gacgagggct ggccgcgcgt 12660
cacccctgat ccctcctcgc cccaggcgac cacccgggcc ctgcggtatc ccggtgtaag 12720
ccgagaggac ccttcctgcg gtccgatgag atgtccctgt ccctctgcta ccaccataag 12780
cacgcccccc cgcaccgctc gcagctccgc agctgggacc ggagggggcgc ggagccgcca 12840
ctccggccgg gcaggaacac ctgctcccag gccctgcagc tgggcgcgtg ctgctgtgag 12900
aggctgccta ctgttgtgtg cagagatcgc ggaggagaaa atagggctcc gaagctcagg 12960
gccaggagca gccaagcctc ggccaccccc atctcccacc cactgcaggc gacagagcgc 13020
gaagccagag ggcctgctgg gggcccgggt ctcctttcga aggtcgaagg gccgccaccc 13080
ctgggatccg gcttctggcc tgaagaggtc gttcataaaa cagatggggc aaaacctgtt 13140
ttgggagcct tcccagtgtc cgcccccgct ctccaaacca gacacactca tctcccctc 13200
agccctactc tagcctccgg cgggggacgg gagtaggtca tggcggcacc ggtactccac 13260
ccgcccccct ccacgtaggg acgtagggtc ggctgcgggg ctcggacttt gccactccac 13320
ctagggaagg gccaatggac gctccccgtc tcctccgtgt ccggaggaga tccggcggcg 13380
gagctgacct gcaaggcttg ccctgcctca cccacccccc acctccgtcc caccctcccc 13440
gactctcctg gcccagggcc cggcccggga aggcgttgtt ttgccagcgg gagccgagcg 13500
agtgcacaga gaaaagcctc cctactctaa tgagagtgca cacagcgctg cagcgaggag 13560

```
gatgattacc cggcgggcgg ggggcgcggg ctcgggccgc gggcggcgtg tgcggcggcc    13620
ccgcgggccc ggagtccccg cctgtctggc tgctaatcgc acgctttctg ctgttacgga    13680
ggcaggcggg agccggcggc cgagcccagc gccgacgact cggcgggtga cacggaaaca    13740
tggcgcagcg cccctccccc gcccgcgctc ccgcctcccg ccctccccgc gctcccgcct    13800
cccgcctcgc acttcctccc gcccgcgcgg cttttgcagt tttaatgctc tgctttcggc    13860
ctggcaccat tacgctagca gccgcttctc cgcggccccg cgcgtctctt tcccctttgt    13920
tttcggggtc tatagaaaac gtttgctttt gtcccgagct ctcgttaaac caaacttgtg    13980
gtggcaacgg aaggcggtgc aggcggggag gggtccgggg attttggcag tagccggcca    14040
gagcgtggtg gaaacttctc tcgctgggtt ctccgctccc tcccactccc cctccccaac    14100
acagagccgc cggcctcccg acgcttctcc cttcgccccc gagaggcccc agtgctccag    14160
gaaggtctgg gccacagaga ctgtgaccag gggtgaagaa ctcagagtgc gtgtctgtga    14220
gccccgaggt tttggcactc gatgaccttg ctccctgcag ccccgactta cctctttccc    14280
tttggtcatt cggggctttg tatcagtcgg tgaagaaggg gagggaagag aaagtgtttc    14340
tttcgtgcgt gtggttttta ttttaaggat ttgttggaat ctgtcacccc caactgcaca    14400
gccccctccc gtataggatc tagggcgaaa gcggggaaac gtcggattta gggggaaagg    14460
gtcgggtcag aaacctagcc ctggaggctg gatcgtactc tcctggcggg ctcagccaca    14520
cttggtcggc acgcggggcg gggcgaccca gcagagtggg ggacgcaagg gccaggtccc    14580
tcggtccagg agacgccaaa cgttccaacc ttggagcgag agagcaggaa cccccgcccc    14640
cacccaagcg cgcgcgctcc ctagctcgac gcgcaagccg tggctctccg cgccagagct    14700
gctcaggcgg caatttttta agcctgcaat taagcgaggc gtcgccgtgt cctctgctag    14760
tgtcggtcct gtcagtcgca catggccttg gtgctccggt ccgaggcccc gcgattagtc    14820
acaggctcgc ttcgctcctt ccctaatccg cctctgcagg cagcctgggg acgcggccga    14880
cttggcggcg ctggcaggga gccactgccc gcggggcggg cctcctgacc agtgccagcc    14940
ccgctgcgcc cgagccggcg cgggccctgg ggtgcgggtt tggcgcccct cccctctcac    15000
agccccctgg ctggggtggc ggcgcccctc ctcctcccgc ccctctcccg ggcctgcgag    15060
atctatttgt gtggtgtcat ttgcataaaa ataggacaag tgtttctgtg cgttcatacg    15120
cgggaggcga aatccttatt gatgtgtctg tgtggagcct tcgattgtcg gagcggtttg    15180
```

atttagggtg tttgtgttgc cttctacaag aaaggagaga aagtccttct caactccttc 15240
ggcggcctgg gcccccagaa gcatgtccct ctgcgcggcc ggaatggtcc accacttggg 15300
ccccaagcgc gccggatcca cagcgagcag gttgagggcc aggcgggagg cccattatga 15360
ctcataaaat cgggcggcct aatcgccagc tgccaaccca tttacatgtg gagcttccca 15420
atgctccctt cgcccgggcc cggcttcctg agggtttgcc ccgacacagc tagagccgcc 15480
aagtgggagc taatctagtg gcctctcctt cccgtcggca gcgtggcttc ccttccccgg 15540
cgacccccgc tctcttggcc tactgcgctg ctcgccagca actcctcagc ttggacaaat 15600
agctgggagg gcgccggccg cgcggagcca gtaaatcaat cattaactcc caagcgaggc 15660
ctggaccagg ggagggagcg gcggccagag ccgagtccgc gaggcgagcg caaagcctcc 15720
accggggtta ggagccaccc acgcgccaac gaaccgggtg cgtgaaggcg ggtcccgcca 15780
tggcctcccg ggcccctcca gaagcctccg cccgccgggc ctttcaagcc cgcaccttgc 15840
ctggccaggc aggtcgcgtt acagcgctgc ccctgggaga gcttgaacat taacgagtcg 15900
cctcgggcct cagcggcctg ctctccacag gagagcgtcc cgcgcttggg caagctcctc 15960
ggatccggcc gggctcctcc tggctcaggc ttgctaaacc aaactgatcg caccctgtat 16020
cgaccaatct ccctgcggca ccctcttccc tcagcccacg gtggagccgg cacgcagggg 16080
gttaaaagcc ccggttcgct ctggctcctc ggctgcgagg agaaaacgct gatcaaagtg 16140
cttaagggga aggtaacaca tgcacattcg gtcccgggtc gaaggaggcc gactggagac 16200
ggctcccgag ccacccgcct cgacccgctt cccacactcc cgggaacgcg gcggcggcgg 16260
cggccctggc ccgcacgatt cgctcggcgg agcgctgcgc tgctgggctg ggagggcggc 16320
ggggaggagg agagacgagg gggcgggagg cgaacccggg ctgcgcgcca agaggcccgg 16380
agccgcagat tagtcaccgc tcggctctgc gctcccgggt gcgcccggga accgggaggg 16440
caggcgtcgg ccccgctcag ccgggctccc cggtcctcgg cgcgggtgcc cccaccccca 16500
cccccgcgcg ctcctggcgc ggctggctcg actctcgggg acccgcgcag ccgagatcca 16560
cttgtcaggt caagggcagg acagaaactc tgcaagcgga gtcttgcttg cgaacaggaa 16620
atttctcctt gatgaggtgc tgcccctccc ccacgccggg cttcaaaaca aaactccaga 16680
gcgggcggcc aggtacccag cctcgctcgc catggccggc gacctccgct cctcggcccc 16740
ggccccgggc ggcggagcca agccaacaga gccggcggac ggactcggcg ccggccccgc 16800

ggagggcgcc aggctgccac cctccggggc ccggacgggg tcggcggggg tgcggagccc 16860

cggagccccc gcgcctcccc aggttctaca gccaccgagc ggttttgcgg agctgcccga 16920

ggccgccggg tcagcggctc cccggagggc gtgggggcg gggaggccgc gcggcgccgc 16980

cgactcggcg aatcccgctg cgggagcccg ggagggctgc gagctcggcc tggcaaagtt 17040

cctgtggaaa ctccatgttt tgaaactcct gcggcgtcgc agaaggaggg ggagagggga 17100

gcgcgggaag cgcgcggggg cggtctgcac ccgcctctcc ccgccgggga gtgggttccg 17160

cggcccccg ggcctcatgg gaaagctgga gggcggggac ggccgggtcc ccgggcccg 17219

<210> 19
<211> 2264
<212> DNA
<213> Homo Sapiens

<400> 19

gaccagcctg gccaacatgg taaaaccccg tctctactaa aaatacaaaa attagctggg 60

catggtggtg tgcgcctgta atcctagcta ctcgggaggc tgaagcagga gaatcgcgtg 120

aacccaggag gcgcaggttg cagtgggcca gagatcgcgc cattgttctc cagcccgggc 180

gactctcagg ttaaatcgtg ttcgatataa aagtctatat cgatatagac ttttcgatat 240

aaaagtctat ctccctttga caggtctgat cgaccctcct tcctataaag acaaaagttg 300

tcttaacatc taagaggaac catattgcgg tgaaaggggt gctacgaatc ctgggtttta 360

gctcttgcac tacgtctcag tgtgacattg gatcccaatt tcctcaccac caaagccaac 420

gggctattat aactccctta gaagtagctt taatccctaa agacaagact aaaaatatta 480

acagtgcttg agcctctgga accgaaccag cactggagtt ttgtttggtc gtcccctctc 540

ttccccctca agattaacga tctggaggga attctggtga gttcgagttc tcgtaacttg 600

ttagggacta accagaagcc cggcgagcag ccagtaacac aatctctggg tattccttca 660

gcctcatcag tacatctctt cctacctcgc cttcctccgc tgcagcttct ttcaagtcca 720

catctgcgtc tccgtcagtt tcctctggga ttccatcggt gtcggtagcc cccgggtccc 780

cgggcacagc ggccgtctca tccgactccc ctgtctctcc acctttgtcg gagggcgact 840

cgtgggcatc gtccatcacg gataggattg gaggggagag gagatcgcgg agatgcctga 900

ggcagaagct cggaaacccg agctgcacag gccaggatct tacttcctgt cgtcgcgcag 960

cgatgacatc acccctaccg ctctcctgag gggtcatttt gaggcgcgcg tagaggacta 1020
gaggactcat tttaactttg gtttggcgac agtgggaagc gggaaaattg gacgtaaaga 1080
gctcagacta tggatcctcc tcgctaggaa accagcactt gtgaaggagc gcagttttgt 1140
ttactaaata actttttcct tttcgcctgt ctaaaaatgc aatgtcagtt aatcccttcg 1200
ttaacccctc agagtaaagt tgggggactg ggagaaaact cagttccgtt gtatggcggt 1260
gtcgtggaat gaggctcccc gggactggta aactttcagg caaggactca accttgtata 1320
tttcaactgg cccgaaaccg cgggcctgtg tgtacataga attgctagca tttcccctcc 1380
attccgcaag caccttaaca acaaaaagtc atatagctcg agcagaattt atccaattca 1440
tttattcatc cacaactatt gactgcctac ttcgatttag aaacagtgct agtaccaggg 1500
aacaaaacac acatagtttc tacccaatta aatgattttt ttcagttcgc cagtactgaa 1560
gattgaggtt tgtttttaca agtctgacac ttttctatca gtttggttaa tctcattaaa 1620
actaagagga aagcctaaag agaggaaggt gtctcctttg aacgtttcac tttaatgaac 1680
tagttcacgt actgggagga tgaagtggtg aggatatttc tgttttaaaa ttttcttctc 1740
ttccctcatt gagatacgag ggggagggcg ttttattcac ctgggtatga ctaggtcccc 1800
gcacctatta catatttaat ttatgcgtgc tgaaagaatg gacttggaaa tgtaggtgcg 1860
cgttacattt atctctttgt ttctacgctg cctggacgaa ttaccgtatt tcctgtccca 1920
ggagctccca aaacctttgg ggaaggagcg cacttcggga ggcgcgtaca ggtgggcctg 1980
tttgtccctt tccacctgca taatgggggc ccacaaggcg ctgtccccat gccccgagag 2040
gcgtgtctcg gcacagcaaa gtccacctgc gctggtccgc ggagccccgc ccagctaggt 2100
gagctagggg gcggggcgcg ggggcgcggt gacgcgtgac gccggatccc ggaagtgacg 2160
cgctcgtggg gaaaaggcag ggaggggtg gtgtccccag ccggtttggg gggtgcgttg 2220
cccggagacg gaaagtttgg gagcccgagc aggctcggct gcag 2264

<210> 20
<211> 3104
<212> DNA
<213> Homo Sapiens

<400> 20

gcgggcgctg tcgagcacgg ggaggtgctg aaatagtcct ggcgtgctga ttcaagcttt 60
gattggcaga gccacccggt gactgacagg gggtctccat ggcgcccgcg ccgccaatcc 120

gcccacccca atagcggagc cagctcgcct gccggcgtgc ctgagccgag ccgagcccga 180
accccaagcc gcggagccag cacctcctcc agtcggggtc gtccgctccc ggccgttgag 240
ccaccgccgc cacccggtag tgtgtcccgc tgccccaatc cgcctcatca acaagcgcct 300
ggcacactca gccaggcccg cgggcatctg ctgcgtgtcc cgctccgggc tcagtgccct 360
cgccgccgcc ggcactgcct cgatgttcca gctgcccatc ttgaatttca gcccccagca 420
agtggccggg gtatgtgaga ccctggaaga gagcggcgat gtggagcgcc tgggtcgctt 480
cctctggtcg ctgcccgtgg cccctgcggc ctgcgaggcc ctcaacaaga atgagtcggt 540
gctacgcgca cgagccatcg tggcctttca cggtggcaac taccgcgagc tctatcatat 600
cctggaaaac cacaagttca ccaaggagtc gcacgccaag ctgcaggcgc tgtggcttga 660
agcacactac caggaggctg agaagctgcg tggaagaccc ctgggacctg tggacaagta 720
ccgagtaagg aagaagttcc cgctgccgcg caccatttgg gacggcgaac agaagacaca 780
ctgcttcaag gagcgcacgc ggcacctgct acgcgagtgg tacctgcagg atccataccc 840
taaccccagc aaaaaacgtg agctcgccca ggcaaccgga ctgaccccta cgcaggtggg 900
caactggttc aaaaaccgcc gacaaaggga ccgagcggct gcagccaaga acaggtcggt 960
acctagaggc ctccgcgctt tgagcgcacc ggggaggagg cgggtggagg cacctctggc 1020
gcccttaccc agtccctggc gactccaatt cagcaggagt tgggagcgcg gtctgtcttg 1080
ggttaagagc cctgcgttct gggctcctgg ccgggagttc ccttgccggc tctgcttccc 1140
cacccgctgg ctccccacgc ctgcgggcag ctgcagcagc tggtcccggt caccaaacca 1200
aggcttcact gggacggaga ggggaagaga aataaaaaat taaaatccta caaacagtta 1260
gggaccccaa gacccaaagc taattcttgt cagcctgggc acaggctcct actattaatc 1320
gaagcctggc ttattagcaa tgtgtcggtt tcatgttaat tatcattttc aaagcccagg 1380
tatatccctc cctaatgctt tgaaaacagt tttcaatgga cttttgagaa atgggaagtc 1440
gagttttcct cttcccatgc gctgcctgcc actcttgtct caaaacagca aactagtccg 1500
tgggccgagg cttttcgttt cccggagtgt ggatctcgat tagccaaaca ttttgcggaa 1560
gagcccggcc tcatccccca ggcccaaatg ctccttacaa tcctttttgc ctttaggtcg 1620
ggccgacccg atccaacgcg atcgcgggag cacttgctca ggcgtaagcc ccaggcagac 1680
gcaccgttag aaatggtatc ccatgtccct gggaccgatc tgtccttgtc acccacactt 1740

cgtttatttc ctgacagtcc tgtaaatctc ccaaaagtgc acaacaaaca gggaggacac 1800

tgcaagccca gtatataaaa gacctgggag ctgcggcgct gagaaagggc gcgaatcatg 1860

gtggggcaca acagtaggga cccgcggagg ggcggccgcg gactcctgcc cgacctctgt 1920

cgccttgccg agtaatcctc gccttaactg ctggggtctt cggaagaacc tctagccgcc 1980

gggctggagg gacgcaggag gtggtggggg cgggcgacgg gcggctgtgt tacgagctgt 2040

gacccgtgtt ccctttcttc cccgtagact ccagcagcag gtcctgtcac agggttccgg 2100

gcgggcacta cgggcggagg gcgacggcac gccagaggtg ctgggcgtcg ccaccagccc 2160

ggccgccagt ctatccagca aggcggccac ttcagccatc tccatcacgt ccagcgacag 2220

cgagtgcgac atctgagttg cccatccagg atgctcagaa gcagattcca gtgtaaaaac 2280

gagaaaaaca aaatgaaaga ggggaagaag atgagagacc tgcaaatcca gcgccacaga 2340

agccaggtga ccagggaccc gcgggctcgg gttgccgttt cccgccccac cccgcggccg 2400

gcctggcttc actggcgccc tttggccgcg accacgggaa ccagcgtgag gcctgaccca 2460

gcaccacgtt cttcttgctt tgctttttcc taaggatttt gctgcaaagt ctcttcggaa 2520

cccgaactgc aagctgagcg cctgcccaga ttctcccatg ggtatttcac gtcgaaagga 2580

cgctgttaca tatgtataac tttcgcttta aagttttttt ttaacaaaac atatatatgc 2640

tgtttattta cttatttaag agaccgccat ggtaggtttc tctgtagctt ggggaacttg 2700

ctgtttctaa acatgcaggc tggtggtgat gggttctgtg tggagaagcc aaacaataaa 2760

acaacctagt gggcaacctt cttaattaag ggagctgctc tcagattcct tttcttctta 2820

ttattattaa tatcattccc ttcaccaggc atgcagggac ccttgagcaa atggtctccg 2880

gaaggtctta cctatatatc ttttgccttc tttggtgctt cctggtatat gttttaaaca 2940

acctatggta ggtctataac cacctcccac gtcaaattac acgtatgtgc atatatgtat 3000

gtacctatac aaacatatgt atgtacctat acaaacatat gtatgtatgc atacacgtga 3060

tatatttaag gctagaaatt ggcaacatgt gagcgtcctc tcta 3104

<210> 21
<211> 2493
<212> DNA
<213> Homo Sapiens

<400> 21

actggcatga ctagagtaga aggagggcat cctaattccc agccttatcc accgctttgc 60
aagaaccagg aaaatgaatt cctgttggcc ataaaactag ttgcaggtga tgtaacccat 120
cctattcggc ctcccatatt tacagctata caggaacaac tctctgaaac tcagtctcct 180
cacccgtaaa atgggatggt aatacgtgcc cctgggagcc agcgagaatt aaaagggata 240
agatgcactg gctcccaggg gcaagcacta gcagacaagg aatagggcag tcctcccttc 300
acactccaca gagaagaaat gtcctccaac tctcccagga ggcagagccc agctctcctt 360
ccaaacagcc ttggagttgc ttggcctctc cacttcttca acgaagttgt gggcagaaga 420
caaatgcaga gttctcttgt acctgacaag gagagagaag cagaccctgt gtgagtctct 480
tgctgttgac gttgatccac agggggtgca ggatgggact gaggcaggcc cacctgcctg 540
gaaagcctgc ctgtaccctg accccatggg gccctaacac acaccctccc agccacaggg 600
gctctgcaga gctaagccag ctgtcctatg gaggtgggga ccctcctcct gccctgccac 660
tctctctttc tcctggagag gacaggcagg tgggcatggc cacaggagaa cttctcgaag 720
gtgctaactg gcccagagaa agaaaggtgg cctggtaggg agttgtctac cagagattga 780
gagagcatgg gagaagttga gagggaggag gaaagtgcac gccatcctgc gcgcagcctc 840
gccttaggga aagcatgtgt gagaagtggt ggtaggcggt tgctagcctt ggaaacaaac 900
aaacagccaa ccaaaaggct tcggaaggaa ggagtgtcta attaatcagc cccgagggac 960
aggtccgcac tgcctgactg tgctcctcag cctggtttca ggccaccgag tccaagtttc 1020
tgccacagtt ccagggccga ggctgtttcc aaagagccct gtaattgttt tccacctgtg 1080
tctcacccaa acaccaaggc tggcgcaggt ggacaccttc ccacttttct ccctccaggc 1140
tgggccccag aaatcagtag aggagggagg aatcagtcag cgtggccatg cctgggagga 1200
gaggcccgtg tgggtctgtg gggctaagag gcaaaggcgg gtggcggatg tgggccagcg 1260
ggagcctgga ggggttgaca ccgcctgctc caccgcaagc ccctggagga agagccccgc 1320
tgtgcccgag agcgagcgcg ggcaggtgta actacccggg gctggggctc cgggggctcc 1380
gcgcagcctc cttccctccc agggacaccg cccagctgcg ccccgcgccc cgccgactgc 1440
gcgggccttg agacgctggt ggctgcctcg gggttggcct gctcctcgcg cacatgttca 1500
gggtcatccg cgctgcgcct ctgcttcagg tgcttggcta gagaaagggc ggcaagacgg 1560
ggcagtgcgt gtgcgcgcgc gggcaagtgc atgtgagtgc acacttatgt gagcgcatgt 1620

gtgtctgcgc ttgtgcgtgt ccaggggaac cacagggagc accctcattc taagcctcca 1680

gaggactgcc tgaagccgct agatagaaac tcccctagaa tgtaagctcc gggggggagg 1740

gagctttgtt tgatggctgc tgtattccca gtgcccattg aagtactggg gacacattag 1800

atgcttaata aacagctgtt gagttaatca acggactcta ggaatggagg cagaccggcc 1860

cttctggaac tggagaaagg acaaggccac atttcaccgt ctcctctgtt cttggtacca 1920

gccctggcgt ccccggcaac gccaatcctt ctcacatcca gcttgtgcca agttttcctc 1980

cctgctcgtg tttacacctt ctcccctttg tctctgtttc ctgctggccc ctgtgtgtaa 2040

gcaccccagc agtcagagtg agaggaagac gaaagaagca gaattctgga aatagagagc 2100

taccagggtg gcctcttcct tctggaagga cacggaaaga tggggtgaga ctggcgctct 2160

tcttcagctc ctccctttgg gaattgggac tttgagcttc agtttcctca ctttgaaata 2220

gcagctattc cacctgcctc gcaggattgt tgcaagaagt caggcaggag gctgaaaagt 2280

tctggagcac atgataggtg ctacagaaac gttcgtttcg tctcctgact agcatccaac 2340

agtctgggag tgccccaggt gtggctgagg gtggttatgg tttgataacc accaccagaa 2400

cacccctcct tttttcacaa aatgccttga ggagaattta atggctgtcg aaaattaagg 2460

caagcttcat ttctaaaaca ttcaggagtc aat 2493

<210> 22
<211> 2315
<212> DNA
<213> Homo Sapiens

<400> 22

ctgtacctgg cgtgaggtct cctggccacg gacgttgttg gcgtcacacc tgtggcacct 60

gaggtgaatt gttcagatgg aaacggcacc atcaggcaac tggcaacaga agcctaaagc 120

cctgggtgcc tcaggtcaga ggaaggaagg aatgcaaggg ccccgctccc cccaatgcca 180

ggagcgtgtg ttcacagctt tagacaggac aacccactgc aaatccaaag cagaaaacat 240

ggtccaactc catctctgtg gcaccttcat agatggtgcc cacctgcctt ggcagtgatg 300

accgacactg cgaacgctgt gctggcagtg tgtttaaggg ctttaactga atcatctcga 360

tggagctgaa atccagcccc attttacaga agagaaaact gaggcttaga gagatcaggt 420

gtcaagttct atgccaagca cagggaagca aaaacaggac acactcaaca taaggtggcc 480

ccaggaatca gtggccagga cacgggaggt gtgctgagcg cagatgggca gatgcaagct 540

gaggtggcct gttggagggt cctggacctt ccagaaaaga ggacatcagc tgctcaggga 600
gggaaggttt gagatggatt ctggctggtg tggatcagcc catggtgtgt ttgcaggcag 660
ggacaccttg aacagacatg ggctgagggg agtttgtgtg gagcgtggcc agtgaaggct 720
gggctggccg gcagggtccg ctggtcatgg gaggcctggc ctgcagcagc agggcccatc 780
aaagacatgg ggatggatgg ctgatggaca agatggctga tggacagcgt tttagaaggt 840
cgcaggcttg tttaggctga ggccagggag ggttagggtc ctcagggaag gagagtaagc 900
taggggagcc tcctgcatag aagacttcct acctgaggcc gtcccctagg agttcagcca 960
ccccgtggag agcctggcgc tgactgtgga agaggtgatg gacgtgcgcc gtgtgctggt 1020
gaaggccgag atggaaaagt tttgcagaa caaggagctc ttcagcagtc tgaagaaggg 1080
gaaggtgagg ctgcctagac gtggggctac gctcttgccc gctgggtcag gggcgggtgc 1140
cgagagggcc agttcccagg actgtttgct catgatctgg ttgggagccc tgagggggta 1200
gcagggacag ggcgggaccc cagggagctt tggacaaagc ggggctctaa ccagtctctc 1260
ctgacagatt tgctgctgct gccgggccaa gttcccgctg ttctcgtggc cgcccagctg 1320
tctcttctgc aagaggtgag ccttcccttt agctgtcagt tcacaaggga aggaggaggc 1380
gagaaacctc ggggcagtac cgcccacaga acttcctgtg attccaggaa tgttctagca 1440
tgttcgatgg ctgatgagtc cacaagatat ggttagtacg aatgaggagt taaattttaa 1500
attgtattta aattaaatta aatttaaata gctaagcaac tcccagtttc agccaagatg 1560
gggtaatagg catcttccac agccaagaac aaacaggatg tgaaacaatg gttttcaaga 1620
cagcaggcaa caaaaaggaa acacaacggg gcaagcctgc catcaccaca gtcattgcct 1680
ggagagtttc taggtcatga gccacagctg ccggactccc cagatggagg aggtcaggct 1740
gagaatccag ggagaccaca gcagctcgag tccgccgtgt agaactccag aggagagggc 1800
tgcgccgaga aagcctcaga gaaggtcccc ctctctgaag gaaatgactg aaggctgcag 1860
aacatttatc ctaaagcaga gattggcaga ctatagccac gggccaaatc tggtcctcgg 1920
cctgttttgc aagtaaaatc tcactggaca cagccacacc catttattac tcgctcagcc 1980
acgcctaaaa ggtttattct ctggcccttt acacaaaaaa tttaccttcc cttgttctaa 2040
agatctgaag ggacggtgct caggggcagg aatgagtaac atcggtttcc accggccaca 2100
ggcagaaatg tcataattca cagggcacta actggaatgc acacaaagga ctcgcctcac 2160

taacgtggag taagtagccc aaagtgattg ttgctctagg cctgactgat acatcataaa 2220

agcaagacct gaaaggatca aactgttcac aagtatctta actgattctg agaacaaagc 2280

tcaagaataa gaatttttta aacatcaaca aaaaa 2315

<210> 23
<211> 2304
<212> DNA
<213> Homo Sapiens

<400> 23

cgattctctt gcctcagcct cccgagtagc tagaattaca ggtgtgagat atgcacccag 60

ctaatttttt gtgtctttta gtagaaacgg ggtttcacca tgttggccac gctggcctcg 120

aactcctgac ctcaagtgat ccacccacct tcgcctccca aagtgttggg atttcaggag 180

tgatcccgca tgcccagcca tcaataaata cttactaagt gcctactttg ttcccaagag 240

tgctgggaac ttagcagtga acaaaataaa taccttccct cggggacctt gaactcaagt 300

ctagtcaggg atcactctat taagagatca agtcaacact caattctcct acagactcag 360

ataggatagt attttaattt ctcgttgatc tgctccctca gaccctagac catgggcact 420

gcagattttt gcaccttttg tagaggcagg gtttcaccac taccagagac tttaccatca 480

tttctatgtg aagggaaaca gtgccctttc agtagacaac gttaaatcag ccatcagacg 540

tgggtcatgg tgggtttgcc gtgactgcca caaacacgcc tcaccgaagt ctgccagggg 600

acgccatacc ctcccctaga aagtaagaaa cagagaaagg ctttctcctg ccccggactg 660

tctttctctc agctctaaga aatttcttta cagctgttac tttatcctgg cctgtctctt 720

gagtaagaaa actgctggaa agagtctgcc attcactcag atcataatca gggagcctgc 780

tgtcactcag gaagggcttt aaaaaattct tctgatttac tgtgagtgca aatctctgat 840

attcctgcat ctccccatcc atcttatttt gattttacac atagtgcgat tttgaaagga 900

agcggtgttt ttggagaggt agaaaaactc tggaaaataa ggcttatgac gttggccatc 960

agcacggggc tgctgtctgc tgtgtgacat gctcaggggt gacgcgcctc ccgaaagaga 1020

cacttggtag agaaggcacg ggcgcgaagc ccggctgcgg gagagctggg agcctgaatg 1080

tcgctggctg ccgttcacag caggctctct ctgagcaccg tggggaggca gtgacacccg 1140

ctggcctcgc agcgacactc cggcagctgt cctgttttcc agcctggacg tgataggtgt 1200

gtcttggagg acagtcagag gaagacaggc aagccatact ggcctccctc gaggttatac 1260

cctcgcttag gttaggttgc ttctaacaga catcctccgc gggcgttgag gcagccagct 1320

tcactgtcac ccagacaatt tttctctcgc tctgccaacc ctgttcccct ttcaagaatg 1380

tttaaactct tcattcaagc gatgagaggg aaacacttga gctgcacact caaggagatg 1440

tgggtttcca ttctggcctt gcgatatatt attggtggga ccgtgggtga gtcacttaac 1500

ttctctagtc ctcagttttc ttatctgtaa agtgggctcc agcatacggc ttaactgagg 1560

taacacacgt tgttcatgtc agctgttgct ttcatgccgg ctctgcctag gtctccccat 1620

ggcaacagga taggagttct ctgcctgtgc gtcagttttt cactagcgtg ggaatgctgt 1680

gaggaggggg cggaactcgg tgtagcaagg gtttctgtcc gtatcttcta tttttttttt 1740

tttttttttt ttgagactga gtcttgctct gtcgctcagg ctggagtgca gtggcgcgat 1800

ctccgctcac tgcaagctcc gcttccaggg ttcacgccat tctcctgcct cagcctccca 1860

agtagctggg actacaggcg cccgccacca agcccagcta atttttgta tttttagtag 1920

agacggggtt tcaccgtttt agccgggatg gtctcgatct cctgacctcg tgatccgccc 1980

gcctcggcct cccaaagtgc tgggattaca ggcgtgacta ccgcgcccgg cccgtatctt 2040

ctattaagaa ccagcaacca cgggctgggc gtggtggctc acgcctgtaa tcccagcact 2100

tagggaggcc aaagtgggcg gatcacgagg tcaggcgatc cagaccatcc tggataacac 2160

ggtgaaaccc cgtctctact aaaaatacaa aaaatttggc aggcgtggtg gcgggcgcct 2220

gtagtcccag ctactcggga ggctgaggga ggagaatggc gtgaacctgg gaggcggagc 2280

ttgcagtgag ccgagatggc acca 2304

<210> 24
<211> 2470
<212> DNA
<213> Homo Sapiens

<400> 24

gtggaacaca tcctccatat agccacacta ctcaaaattc actgtcttta agtctttact 60

caaatgcttc tccgtgaagc catccctgag ccctctttaa aattgcagcc ccccactcct 120

gtactcctta ttgtctctca tttttaattt ttgttattgt agttagaacc atctgacaca 180

tagcttttat tccattggtt ttttgttatg tctttcttta caagaatttg aagtccatca 240

ggccgggagt tttgtttgtt gtgtttgctg ctatctccca gtgcctaaaa ttgcctggca 300

```
tacagtaggc atttaataat ctttgaatca gtgaaaacca gatggtggct tggcatttcc     360
acataggaat gagccaggtg gaaatcatcc aggatataag tagatcttga agtgataagg     420
aagggtcatc ataatcatgt ggggcccatt ttgccctttc ttgtttcttt tctctaggct     480
cagcaacagc ctcaccaagg actccatgaa tatcaaagcc catatccaca tgttgctaga     540
ggtgagagca gctcacccca ctaccagact ctgtgtttag ggtggtgacc tgaagaagga     600
agagagcgaa agaagggaag gaccatcttt ccctctaaac tggagtcaag ggagggaggt     660
cagagcaagc ctgggggcgt aacccagacc cagtctttgt tcaatctctt ctgtcctctt     720
tttcaggggc ttagagaact acaaggcctg cagaatttcc cagagaagcc tcaccattga     780
cttcttcccc ccatcctcag acattaaaga gcctgaatgc ctttgagtca catggccctt     840
ctttttcccc ataaaccctg ctagttgcca cgggggcctg ttcctagggc acaaagttac     900
tgagagaccc agagatccag tctctctgtg gaacctccaa aatgccccag cagtcctgcc     960
tccagcctgt tgcctggcag tatttgccag ttgaccttat gcactgccct ctcttctgtc    1020
atcttggcaa cctgggcccc ccacctcacc ctttcttcca ttccttttcc tagacgccac    1080
cctttgaatt gccatagaga atgggccaat tcatggtgga ggtttgttca ttcccacaac    1140
aatcaaaggg ccctaaggtt tacgctttca cacacccaat cattccccag gtaccccaaa    1200
ttacacccaa accctaactc agccctacct tgtcttagcc cctgcttgta agtgttccag    1260
cttccaatgg gcacagacct ggatcccgct ctccctagtc gggcccctat tctaggttga    1320
gtccagccac cgccaatcaa tgcagagcca ggttcctccc ctttttaact ctggccgcag    1380
ttcaaccctg ccctctgaaa gcatttcgtt tctgccactc aatgctcttt cgtgtgcctg    1440
acagccatcc tgccctccta cctccgctgt gttctaaatt cgtctttacc cagctctatg    1500
gtcttgtcta accgtagagc tgccctgccc gcctacgcgg agcccagtcc gacccactcc    1560
cgccggctgc tacgcccatt tctatacaag ccctgcttcc gctgagcagc atggcgtgcg    1620
acaccgcccc cttggtgttt tggcaggggt ctagaagtcc ctcgtccgcc aatcagagaa    1680
aaacagggcc acccaccccc gtccttgggg gctgtcttcc atcaatccca tgtaagccaa    1740
tcagtgtgag gcagaccccc gcccccccga cacaggcccc gagccttttc agttgctcac    1800
agtccgtcag tccttgagcc aatcggcgtg gagcaccgtg aaggccgaac gcgcctcctc    1860
gggactccag gggccgtgag cgttccatca tttcccctta cgccaatcac ggcacagctc    1920
```

tgtagggaag ggcccgtccc ccaacccctc gaggccttgc ggccgattaa tagcgctttg 1980

gccaatcagc gagcggcggg acattgggct cctcctcctc gggcccacgt gagctgtagg 2040

gaaacgcagg ggcggcttct aggtgctgcc gccgccaccg ccaccaccac ctccaccgcc 2100

gcctcggaac ccaggcctgg ggggcggtgg ggccgcgtat ggagcccccg ccccccggag 2160

ctgccaacat tgccaacgcc accgccacgc tacacacagg tgagctctgg gcctggaggg 2220

tggagggccc agtccgtgac cccacgtatc cttcccgccc ccgcgcagag gatgtggctt 2280

ggccggtggc ctgctggtgt tcgactcccc gccgccacca ccacggctgg tggacctgcg 2340

tgtggcattg ctcaagccct tcgtccctta tagtggatct gaccgtggcc taacctcccc 2400

ctcccgttgt ataatggatc ggtctgcgtg cttatgtttt tccccacgcc aacttagggt 2460

ggactcgtcc 2470

<210> 25
<211> 2470
<212> DNA
<213> Homo Sapiens

<400> 25

tccaaaaagc tctgtaaacc aaaagtttgg gggtaaactc atttggtagc aaattttgac 60

ctgaggctat ttatagtcta tattctgtat tctttctact tagtatgaat aagcatgtaa 120

gttttactgc atgtttgatt tcagcatgtt cccccagact ctctgggggt gtttacgtat 180

gccggtgggg gaaagagacc aactctcaaa tattatctca aacagttggt ttcactgtgc 240

ttgcttgggt agcacatata ccaaaattgg aatgacccct gcacagggat gaaatgcaaa 300

ttcgtgaagc atactgtatt tttcttagca cataccacct ttggcaatat tctttttttt 360

tttttgagag ggagtcttgc tctgtcgccc aggctggagt gcagaggcgc gatctcggct 420

cactgcaagc tccgcctccc gggttcacac cattctccta cctcagcctc cccagtagct 480

gggactacag gcgtgtgcta ccacgccagg ctaatttttt gtatttttag tagaggcggg 540

gtttcactgt gttagccagg atggtctcga tctcctgacc tcgtgatccg cccacctccg 600

cccccccccc gaagtgccga gtgctgggac tacaggcgtg agccactgcg cccggccccc 660

gccttttttt tttagattga ttttattact tgcctagcaa aggagaacct tctggcagaa 720

cagtctccaa gaacaaggca aacaactaat tttacatagg tttttaccaa tgtacagctg 780

ttgattgtga ctggtttccg gcaatctgga tttcacaatc tggataaggg gacaaacaat 840
tgtctgtctt ccactatctt tcttgaattt gaatagaacc tttttattct catagcctct 900
tagctttctt tctttttttt ttgagacgga gtttcgctct tgtcgcccag gctggagtgc 960
agtggcgcga ccttggctca ctgcaaacgc tgcctcccag gttcaagtta ttctcctgcc 1020
tcagcctccc aagtagctgg gattacaggc gcatgccacc acgcccggct aatttttgga 1080
tttttagtag agacggggtt ttcaccatgt tgactaggct ggtcttcaac gcctgacctc 1140
aggtgatccg cccgcctcgg catcccaaag tgctgggatt acaggcgtga gccactgcgc 1200
ccggcctctc atagtctctt agctttctaa aatttgaaaa atcctgtaaa gacacacctg 1260
ggtcaaaggg ctcagataac ggactgtggc ccttaagtac ttacgtcaca ggttattgag 1320
aggatcgatt tagttaccag atgtaaaatg ctgggatcag tgcctggcaa aggaaaactt 1380
tgtacagctg caggctttca ccatacacaa cagcatcgct aacgaatgct attacaatat 1440
tcatttagcg tttaccaagt gcctactcta tacaaatctt gagaatacaa cgtgaaggtg 1500
aactgctgac taaagtttgg tccctttcgc tccgtctcct tgcgaaaatg ctctaacggc 1560
aggaggtcac gcgagcgctg gacgcgtttc tccccgcgag ccccttttccg aggcctttcg 1620
ggtcccccccg gttatccccg cccgggcggt gcgcgccccc gctgttcccg cttccgctcc 1680
agagaggcag ggctttccga gcctgctagc cccgcggccg caactaaccc cgggtcggag 1740
tgttccggcc cggccagccc cgcggcgtga gggaagggga gctcagcagt tccccgcgcg 1800
gggcccaggc gtcggcggca gggcgggccc ctcaccgcca gcgtgccagc cccgcccccta 1860
cccaccagtg tgccagcccc gcccttcccc acgtcgccgc gcgcccgggg gcggggcctg 1920
gcgcgcaccg cccgcgcacg gcgaggcgcc tgttgattgg ccactggggc ccgggttcct 1980
ccggcggagc gcgcctcccc ccagatttcc cgccagcagg agccgcgcgg tagatgcggt 2040
gcttttagga gctccgtccg acagaacggt tgggccttgc cggctgtcgg tatgtcgcga 2100
cagagcaccc tgtacagctt cttccccaag tctccggcgc tgagtgatgc caacaaggcc 2160
tcggccaggg cctcacgcga aggcggccgt gccgccgctg cccccggggc ctctccttcc 2220
ccaggcgggg atgcggcctg gagcgaggct gggcctgggc ccaggccctt ggcgcgctcc 2280
gcgtcaccgc ccaaggcgaa gaacctcaac ggagggctgc ggagatcggt agcgcctgct 2340
gcccccacca ggtagcgggg tgggggtggg gtcgaaggcg ggggcatagc ggcggggcgc 2400

ttggaacccg gcgaggggag gctcgcacag ggggttgggg gggtgcacgg cctggccctg 2460

ggctcggagg 2470

<210> 26
<211> 2470
<212> DNA
<213> Homo Sapiens

<400> 26

atatattata tcgtgtatgt aatgtataag tatttatttc gtttgcttgg ggttttgttt 60

gcttttgctg agtccgaccc ctctacctgc cgcctggccc ttgcctcacg ctccagtgcc 120

actgagatca aggagagaac gaatttgccg ctgactgggc agagcgagcg cgtggatcgc 180

ggccaccgcc cgttcatcac ccgcgcgcat ctgggctggc accgggcgaa gaatcgtgcg 240

ggtctgggac ctgggggccc agagggagcg agctcctgcg cgggcgctcg gtccgcaggt 300

ttcgcaggct caggggcgtg cctcgttctc acccccactc cggaccccgg tcctcttccc 360

tagacagcgg ccccctccac ccctggctcc cgcaggccgc tagtagtccg cgccaggccc 420

cgccggcgcc tctagggccc cccagatcgc gcagaccctg acatccccgc ctggccctgg 480

gttctgggag ctgagagccg gccagggtcc tgctcgtacc tccgggcgcc cagcctcggg 540

tctgctcccc gcggacgccc caacctcccc ggccgaatgg atggtggtgc gcgcgcgtcc 600

tactccggcg gtgccggcct tttctgttgc caaaactaga cccaaacctc tgcatgggat 660

tcgtctttgg gtccccaccc cgtgcgccca gcaaacagtg ggtgagccat gaagatgtgc 720

gagtcagccg gaccctcccc gtcaggcgcg gacccgctgc ggccagagaa cccagtctgc 780

gccagcccgg ctcgctcgcg aagccacggg cttcactgac gcgactttcc aagacgtggg 840

ggtcaccatg ggcagaggac atcggttcgg agccagatca cgggccccat aagcatcaga 900

ccataagcag cgccgccact gagagccgct cggaactcgc ccagcatgtc gggtccccta 960

gccagggcct ggtgtacgtg gtcgagggcc ctggaagccc cgatggccta ggaggagcag 1020

gcgggcgggg cggcgggtgt cgctggccgg tagagagctt cggcctgacc tagcgcaggt 1080

ctggtgcgcg cagagaacaa ctccaagcgc accgacgccc gcgagctcct tccaaacacc 1140

gaacgggatc cagagcccga gcccacaggc ggcggccggg ggagggagca gggtgctggc 1200

cgccgcccgg gagtgttcgc gtcctgggtg acccctggaa ggacgtgggg cccaaactcc 1260

ggctggggtt gggagagcag cccccagagg ctctccgcgg gatcctctgc cgggcgggac 1320

cgtggctcca caggagaagt gggtggcaag ccctgcttgg cggaaagcag ccgttcccct 1380

cctcctgggc ctggggcggc gcccctcacc cctgttcccc gcccctcacc cctgttcccc 1440

gccggccaca tcccctgccc cttggattcc aagcgccccg cgcgccgagg agcccagcgc 1500

tagtggcggc ggccaggaga gacccgggtg tcaggaaaga tgggccgtct gggggacagc 1560

agggagtccg ggggaaacgc aggcgtcggg cacagagtcg gcaccggcgt ccccagctct 1620

gccgaagatc gcggtcgggt ctggcccgcg ggaggggccc tggcgccgga cctgcttcgg 1680

ccctgcgtgg gcggcctcgc cgggctctgc aggagcgacg cgcgccaaaa ggcggcggga 1740

aggaggcggg gcagagcgcg cccgggaccc cgacttggac gcggccagct ggagaggcgg 1800

agcgccggga ggagaccttg gccccgccgc gactcggtgg cccgcgctgc cttcccgcgc 1860

gccgggctaa aaaggcgcta acgcccgcgg ccgcctactc cccgcggcgc ctcccctccc 1920

cgcgcccata taacccgcct aggggccggg cagcccgccc tgcctccccg cccgcgcacc 1980

cgcccggagg ctcgcgcgcc cgcgaagggg acgcagcgaa accggggccc gcgccaggcc 2040

agccgggacg gacgccgatg cccggggctg cgacggctgc aggtaggagg cccagggccg 2100

gggggcggtt cggctccgcg ggcgggggct ggagcgcagc gctgggcagg cacctgggct 2160

cgcagctccg aagctgggag gtgaggggag agcgatcggg gacgagctgg gacaaggcga 2220

cacaggggct ccctcggagt tggatcggcc cctgggactt ggcgctcgcg agaggctgga 2280

gcggccagag tctagcctgc gaggagacgc gggtcctgcc ctcagcgccg gccgcctttg 2340

gcgccaaaga cagccccgca ggggttccgg gagggccctc ctcctgctgt cccctctcca 2400

ccccgggctc cgagggccgt tgggagggta accccgggaa gaggccgggg tgcggggcgc 2460

gggtgcaggt 2470

<210> 27
<211> 2470
<212> DNA
<213> Homo Sapiens

<400> 27

aaaattgaac atctgagaac actggaccca cattcctgag ggcaacaatc tgctagagtt 60

gagcagctgt tccttttagt tatggcatgg atattccaat ttgccatttt ccctatcatt 120

ccccattgtc ttacacaagg ccagtctcac tcatttgctt tacttgtctg gcctctgtag 180

ccatttgaat ctgcaaacac tatctagacc tttcctgtct tccatcaaag cttactttgt 240
tctgtcccaa gtccttcatc agtgtttcaa agcaagagcc aggtaatctg atcaaatata 300
ggtcctttaa catgtgcttt ctggagacag catcttaaca gaaagagatg actctgcagg 360
tagaaattac aggcaccact ggattacaat caggatggca actgtctgtt ctactttctc 420
tgttatcttg acgtagccat acctcactgt tctaaagcag accaaaggaa ctggttctat 480
ggtagaacaa tggacgctgg tcttatgtat gaaatcttct caagctgcac ttttatagat 540
caccctagtt ccaaaagatc caaagctacc acgggctcct ctgacccccc catttcctcg 600
gaggcttcag gatatccggc ctgacggcat ttcccctcac ctgctttcgg ggcccgtttt 660
gcgctgggca gttgcgccca aggagcgcgc gcatccagca tgagctcatt tctcatgggc 720
gtttccagag gcccggccgg gccgcgcaca ccaagcgcag caagcctctg tatcaatggc 780
ccccgcggct gggcggggcg cggccgccca aaggcgctgc ctttctggaa gctttgttcc 840
cattttagcg tctgagagct tgcagccggc tgggaaggcc cccttggtcc gtctggccct 900
ttcggggaag aggccaacac tcggcacacg cgatccacgg cagaggggag ccttgggcgc 960
gcagaattgg ctgcgccccg ccgagagcct cctgtgggtg gggagagccc ctccacccct 1020
ctgctcgctt gagcgctcag agcccagggc cgccgaccgc agcactttcc gatttgctgc 1080
accgagggcc cgcggtccct gtgtgcggtt tccaccgttg ttggaggcgg ccgcaggcga 1140
accgtcgggt cgtcagccac gacccgagtc aggcatctcc ccgctcctgg gaccggggcc 1200
gaaggccaat cacactgcag ctaggtcttg cgattggacg gcagtgagag ccgattggcc 1260
gccgccgcga gtttcgggct ccctccctct ccccttttgcc ccaactccag cgcctaagct 1320
tcaggccaat gagacagcgc tttatagacg ccctcccttc gctttcttct ccccaccttg 1380
gagagggagg ggaagtcctg actggccaga ctgtcctcgg aagcccccctt ctcttcacca 1440
atcaccgaag gaggtacgct cccagcggag ttttccagcc aatcacaaag cggcggcggc 1500
gcccagccgt gcagtttcac cagcgtctct gggtttcacc gtcctcaact cttcaagcct 1560
cttcgtaagg gccggcgact ctgattggcc actgttgcca ttgtcgaatg tctcctccag 1620
ccagccaccg aacaaggcga attcaccctt tccgttcggc taccttcggc attttccgct 1680
ctttgggcgt ggcttcccag cgtcactttc taattggttt ctcaggctga tcggcttttt 1740
ccgggaggag ccgcaaacaa acgacgtccg tgattggctc cgttcgggct tcggctccca 1800

gccgaagcgg gcgagcgtgg ggctcggccg gcgattccca gacgcctgtt acgcgggcgg 1860
cggggcgctg ggcggtgtaa ggctgggtgg gggaggaagg aggtggagga cgagtaggag 1920
gggggaggag gagtggggaa gtgcaaggcg gctgcgcaga cagcgctcct cacacagagc 1980
agctcctgac ccgggcgaat gcgggcttgt gccgccgccg ccgccgccgc cgcccgggcc 2040
aagtgacaaa ggaaggaagg aagcgaggag gagccggccc cgcagccgct gacagggctc 2100
tgggctgggg caaagcgcgg acacttcctg agcgggcacc gagcagagcc gaggggcggg 2160
agggcggccg agctgttgcc gcggacgggg gagggggccc cgagggacgg aagcggttgc 2220
cgggttccca tgtccccggc gaatggggaa cagtcgagga gccgctgcct ggggtctgaa 2280
gggagctgcc tccgccaccg ccatggccgc tggatccagc cgccgcctgc agctgctcct 2340
ggcgcaatga ggagaggagc cgccgccacc gccaccgccc gcctctgact gactcgcgac 2400
tccgccgccc tctagttcgc cgggcccctg ccgtcagccc gccggatccc gcggcttgcc 2460
ggagctgcag 2470

<210> 28
<211> 2470
<212> DNA
<213> Homo Sapiens

<400> 28

ctttgttttt atgtttagtc tattgaagct tgctctcagg ccactggagc tgagctttct 60
gaacacttct ggttcagagt gctgcagatt tatgaattgt tgtttgctga aataaggtgt 120
aaagtttatt ttgtctaaag tttttaacag gagtgataat tcaggcaaaa tgttcacata 180
atagtgggaa atcaaaagct tgcgatacag aaaagtgata tatatttgta aactccttta 240
acaaaggcat cgtagcataa gcatgactgt agagccagac tgtcaatgtt caaatttttt 300
ctgggcctta atttctttat ctgaaaatcg ttaacagtgg aacctagttg atagggttgt 360
tgtggagcaa gcgttcttaa tctggagtcc atgaaatttc agcatctgtg aactttgtat 420
tgaaaaaatc gtaactttat gtctattaac ctcacaacag tttcgtttct ttgaattata 480
aaggcaggca acaaaccaca gtagtattat cagtacctac aactttgttt cctacagaaa 540
ttattttcat actcagtaag ttgttgcaga tatcttgaac aattatacgt atatattcct 600
taagtattat atatataata tttttatatt ctgtatatat ttttggagac agggttttgc 660
tctgtctccc aggctggagt gctgtagcat gatcggaact cactgtaacc tcaaactcct 720

gggctctagt gatccttcca gcctcagtct tggaatagct aggactacaa gtactcccca 780
ccatacccag ctgttaattt tttttttttt cgtaaagaca aggtctcact atcttgccca 840
ggctgctctc caactcttgg acacaagcct cctgcatagg tctcccaaag cattgggttt 900
acaggtgtga gccaccgtgc ctggctaata ctatgtattt aatttcatgc gtttagaaat 960
atgtgctatt ttaagaaggc gtctacaggt tttatcacat taccaaaggt gttcatggca 1020
caagtgaaga aatctctaga tactagagta ataaaaacca tgatgcatta gaacggatca 1080
ggcccatatc aaacgttcaa catacagtct gctgctatgg ttcttttttt tttttttttt 1140
ttttttttga gacagagtct cattctgtct cccaggctgg agtgcagtgg agtgatctcg 1200
gctcactgca acctctgcct cccgggttta tgccattctc ctgcctcagc ctcccaagta 1260
gaggaactgc aaggcgctcc accatgcccg gctacttttt gtatttttag tagaaatggg 1320
gtttccccat gttacggcag gctggtcttg aactcctgac ctcaggtgag ccacctgcct 1380
cagcctccca aagtgctggg attacaggca tgagccacca tgtccagtct gctatggttc 1440
ttaaaatatt catagaatag aaattgaagt aagtctattc gtttttatat taaaagtagc 1500
tagtacggtg ctctgtactt atgcactcca tggatgctga atgagtcatt gaatgaagtg 1560
gagtctaggt tgtgcctcag gccttccagt ggggctggaa ggaacagttg tgaagttgtg 1620
gggcggcttc tgtgcatcgg gggactctgg agttagatgg tttcccaggg ctctgaagga 1680
gaagagctgt gttcctaata atgatctcaa tgggaataga gattgctggg gaccgcaggg 1740
gccaggttgt cattccccaa agcttcccct tcatcatcca agaaggcatt caggtctttc 1800
tgtgctaggc cccaggtaaa gtgctggact acccagtaat tgggttcagt agcaggatgg 1860
cctcagattg aggtcccagg gccaaaggac cactcctctc ctcagcgctg gtccgggaaa 1920
ggcaagctcc gggcgggagc gcacgccgcg cccccgaagc ctggctccct cgccacgccc 1980
acttcctgcc cccatcccgc gcctttccag gtcttctccc ggtgaaccgg atgctctgtc 2040
agtctcctcc tctgcgtcct cggccgcggc ccgggtccct cgcaaagccg ctgccatccc 2100
ggagggccca gccagcgggc tcccggaggc tggccgggca ggcgtggtgc gcggtaggag 2160
ctgggcgcgc acggctaccg cgcgtggagg agacactgcc ctgccgcgat gggggcccgg 2220
ggcgctcctt cacgccgtag gcaagcgggg cggcggctgc ggtacctgcc caccgggagc 2280
tttcccttcc ttctcctgct gctgctgctc tgcatccagc tcgggggagg acagaagaaa 2340

aaggaggtag aatggatccc cttggccttc ccctgtgggc gggggcgggc cagggtgggc 2400

cgcgttgcca ggcagccctg ccgtgttgct aggcagcctg gtcgccggcg tgggcgatgc 2460

cggcgctggg 2470

<210> 29
<211> 2470
<212> DNA
<213> Homo Sapiens

<400> 29

agctgctctg tctagaagcc gattttctga tgcctccaac gtctggtcta attgatctgt 60

tttaatggag tcttcgtcgg tgaggagcga gatgccaccg actagaatgc tgggatctgc 120

tgcttaattg ccaggagtga gagacactga gattcagaaa tctttggagg tgggaggggga 180

gagggacagt ctcggacgga ggcggagatg taagataaag ggatggattt cacacaggaa 240

aaaaaaaaag atttcgttga ggcactgagg tgctgcacga tcacatctct caaaggagaa 300

gttaaaaagc aaggaagtgg gaggaggttg gaggttaaag tacttaaaag gattactcgg 360

gtacaatttg tttttctgct ggtgtctgca aaggatagat agtcccgttt tcaaagtata 420

tgaatgcctc ttttaagtga ttgggaatgg acactaattg cctgttaaat gttatcaaat 480

gctctcctaa attcagggga cacagaaaga ggggcacaaa aggagaattt aaatagaaaa 540

agggaggatc cggaggcttt tgaaagcggg gggagaagaa ggaggaggga taacagagag 600

gaatagagaa ggagagcgga gagaagataa acaaaaacaa aaacaggaat cactgaataa 660

tcacacacca aaaagaaagc tcttccctat ggggcatcca aaacactgag actgcaatag 720

tgaccccggt catggaagaa agatgttcct ctccaccctt gtccccgaaa gctcttggtc 780

ccgttactgg cgactaaaat tccattaggc taaagagtgt gtctaactgc ctgaagaatg 840

cagcagacgg aaggcgggtc ccgctatgcc gtttgccctt cccgctggag agaatgaaag 900

aaacgcgcag agccagagac tcctgccgag ttagaccttc tctcgtcgcc ccaggtcacc 960

ggccatccgg caaagacccg agtaaggaac gcagggtcac tgcctgggcc aacaaatgga 1020

gcccgctctc cccttcccgg acgccgctgc ccggccgatg ctcccggcaa cccacccgcg 1080

gcgtatgcag aggagccttt ctctttctct cagaccactt gtcccgacca atctgacctt 1140

ccaaacacat ctgaccgcac ctcccaggtg gacacactaa taggctacgg gctggagagg 1200

agcgggtgat gaggagaggg attcaaacct gcgaacgctt gggctgggtc ggagctgcgg 1260

ggggcctggg aggagagagg ggagaagaga gaaggaagga gagcgcctgc cgggatggct 1320

gagctgcctc ggcgagcagc cttggggttg cacgctcttg tgggagatgc tgctgttgct 1380

tccaggtcgg caagagcggt tctaacacca tcgcctctca ccctctttcc tgtaaatccc 1440

tagagaaacg tccctggcct ctccgccgcg acattcccag cctgcatccc cctacagcct 1500

aggcggcgcg ctcccgcacg ctggagcgcc ggtcgccagc aggacgccct ctcccgcgcc 1560

gactcgcccc tctctgccct gctgctgctg ctcctctgac acctccgccc ccaccatctc 1620

cagctcggag agacgccacc cagccgcggc ccgcactcgc ggcccggggt cacgcgcgga 1680

agaggggcgc tagtccggac cccgccttcg gtagggggcg tcctggagcg gagagtgagg 1740

cgaatggtat atgagtgtgc gggtagccca ccctgaagcc cgagcttctc atttgagcca 1800

tccccgccta gccccactcg ggccagcgcc tggcgagcga gcccatctgt ggcttccgcg 1860

gccgcctcct ccttgcatcc ttgcacctcc tcgtcgaccc ctccctcccg ggacctgcat 1920

cctgctccac caatcagagc ccgactgcct cttcccacgt gaccccgggc gggctgagga 1980

cctgctgctt cccaaacgcc agagggatgc gggcggcaga gctcgagagg cggctgccgg 2040

gctgcggggc gccttgactc tccctccacc ctgcctcctc gggctccact cgtctgcccc 2100

tggactcccg tctcctcctg tcctccggct tcccagagct ccctccttat ggcagcagct 2160

tcccgcgtct ccggcgcagc ttctcagcgg acgaccctct cgctccgggg ctgagcccag 2220

tccctggatg ttgctgaaac tctcgagatc atgcgcgggt ttggctgctg cttccccgcc 2280

gggtgccact gccaccgccg ccgcctctgc tgccgccgtc cgcgggatgc tcagtagccc 2340

gctgcccggc ccccgcgatc ctgtgttcct cggaagccgt ttgctgctgc agagttgcac 2400

gaactagtca tggtgctgtg ggagtccccg cggcagtgca gcagctggac actttgcgag 2460

ggcttttgct 2470

<210> 30
<211> 2470
<212> DNA
<213> Homo Sapiens

<400> 30

acatttaaat tgcatatttg gcttgtaata tattacatat ttaaaagaat tactattttt 60

ctgaatacca ttaatactaa taatagcatc acagtgacta agagtggact taaataaagg 120

ctggcttgaa actcaggtct gccatttatt agcaagcttc taaaaattct gagcccttag 180
ttttctcaca tgtgaaatgg agaaaataaa tctgcaaaat taattaaatc tgtccattca 240
tatttttctc tccagtgtat attaactggc attcctcgtt aggccagaat gtgctctcaa 300
ccatgctcca aatccgcttt gtgccaaccc cactgccaga accctttcta ccttgagaac 360
cagaaaagga aacattatgc ctggcaatgc ctacaccctc caaaataaat ctgcaggaaa 420
gaacacccag taagtgatga gagcagcaac gactgccttt atcattttaa atttacaaca 480
ccacctttc tagagcctct taagcattgt agataattcc ccactcatta aaaaataaat 540
tgtaaccata agtattcagg gttgatactg cttttgaatt agacagtgct catatcagtt 600
gcataagacc aacctaaagt agaggatgaa atcttttttc tgaacctttt tcagaacgta 660
acttagtgaa tatattaaaa ctaaactttc tttgaatggg agtaatttct acggattaat 720
ctgtaatctc ttagaccaca cctaaggtaa tgtagaggtt gttgtatcat aggctttgtg 780
attagagacc actggatttg tctttggaaa agtcactcat gattctttgg gctttggttt 840
cctcatcttt tataccggct taataatgac caccgtagag ttgtcatgga agttaaatga 900
actcatgtac cataagtgac caatacaatg attgacactc agtgatttat caataaatta 960
aaacatttat tatcaatatg acagagaagg tgccgctaaa atagacaata ggtttttgga 1020
agaggtgatt aaatggatgc aaaatttatg gattgtttat tccgtctacc tttgctgtgt 1080
cccctggttg tggcatacac acgtgtgggt ataaaatcgt aaatcctatg tagtcgcgta 1140
gtgcatgcgc agaaggctta gacacgaaat gtcatttcag caatgtgcct agagaagctc 1200
tgacgccgcc ttggaagtaa gtcgttgctg cctgaccttt gggcgtctgg gacggatgcc 1260
tatacctgca cccagcagca ctggaagggg cccaggccct tcgcagcaca gcctatcccc 1320
agaccgctta gtccttcata acatatatct ccacggaaaa gggtatttcc tcccgtcaga 1380
aaaagcgccc cagtctggtc tgggttggtt tttatttcac gttgttgcaa gtaggcgaag 1440
tcccttctgt ctcctccctt ggggtaagtg gaaaggagtc cggcaggggg cccgcagtgg 1500
cctgcacagg ggaactgggt agcgagagag ttccaggcaa ttccgggggc tgccccacag 1560
aagcaggtgg ggatcgacag tggctctccg gcccagggag gagagcgcgg tcgcgggtcc 1620
ctcccctcag cctggaggct gcagccgctc gagtcggccc gggtgggggc ggggtggggg 1680
cggcgcggag ggcacggaga ttacggcggc gccacccggg acatccaggg ccccgaggcc 1740

ctgggcggtc cccacgcgag atcgcaaacc atgacaatag gcagtcaccc gaggtcaaat 1800

aaaaacggag tgggtccccc gcgcgccgcc gcccccgcg tccctggcgg cctcccccga 1860

ggcccccggc ggcctcacga gcccgcagta gccggtggcg acgtcgcccc cgccccacct 1920

ccctgcgcaa gtgcgaggct gccggcagcg cggcgcacgc tccggccgtt cccggcttcc 1980

gcgcaaaact tccatcctgt ccacgtgaag ttgtcgctgc cttagagagg gggaaagagc 2040

tgcgggaaaa gccggggagt gacgactgcg gcggctgggc gcgctctctc attttctttt 2100

cttctccttt ccccctgtc gcagtccgga gttttggctc ctctcctttc ctcctccccc 2160

tcggagccgg cttctccctc cgccccgctt ctcccccgct tgtgtacgct atttgttgtg 2220

gggtggccga aggggatgtc ctgttttcac cagaggcaca gcgcgaaggg gaaacttcga 2280

cactggaagg aacgagaata aatacttaat tacggacgca ctgaaccgcg gctgggacag 2340

acacttcggg aacccgaggc ggaccgggcg acgaggtgag tgaccccttc ttccaacccc 2400

cgccccaggg ctcccggggg agcctgagtt gagagaaccc ccaaactttc cgggaaagtg 2460

cgcgaggctc 2470

<210> 31
<211> 947
<212> DNA
<213> Homo Sapiens

<400> 31

ctgctgctct caggggccct ggccctgacc gatacttggg cgggtgagtg cggggtccag 60

agagaaacgg cctctgtggg gaggagtgag gggcccgccc ggtgggggcg caggactcag 120

ggagccgcgc ccggaggagg gtctggcggg tctcagcccc tcctcgcccc caggctccca 180

ctccttgagg tatttcagca ccgctgtgtc gcggcccggc cgcggggagc cccgctacat 240

cgccgtggag tacgtagacg acacgcaatt cctgcggttc gacagcgacg ccgcgattcc 300

gaggatggag ccgcgggagc cgtgggtgga gcaagagggg ccgcagtatt gggagtggac 360

cacagggtac gccaaggcca acgcacagac tgaccgagtg gccctgagga acctgctccg 420

ccgctacaac cagagcgagg ctggtgagtg aacccggccg gggcgcaggt tcacgaccac 480

cccccatccg ccacggaccg cccgggtccc tcagagtctc cggatccgaa atctaccccg 540

aggcagcggg acccgcccag accctccacc cgggagagtc ccaggcgcct ttacccaggt 600

tcattttcag tttaggccaa aatccccgcg ggttgggcgg ggagggggcg gggctagctg 660

ggcggggctg actgcgggga ccggctaggg tctcacaccc tccagggaat gaatggctgc 720

gacatggggc ccgacggacg cctcctccgc gggtatcacc agcacgcgta cgacggcaag 780

gattacatct ccctgaacga ggacctgcgc tcctggaccg cggcggacac cgtggctcag 840

atcacccagc gcttctatga ggcagaggaa tatgcagagg agttcaggac ctacctggag 900

ggcgagtgcc tggagttgct ccgcagatac ttggagaatg ggaagga 947

<210> 32
<211> 3120
<212> DNA
<213> Homo Sapiens

<400> 32

aactttaagt ttaggccggg cacagtggct cacgcatgta atcccagtac ttggagggac 60

cgaggtgggc ggatcacaag gtcaggagat tgagaccatc ctggccagca tggtgaaacc 120

ccgtctctac taaaaataca aaacttagct gggcatgatg gcacgtgcct gtagtcccag 180

ctacttggga ggctgaggca ggagaatcgc ttgaacccgg gaggtggagg ttgcagtgag 240

cggagattgc accactgcac tcgcctggtg acagagcaag actctatctc aaaaaacaaa 300

caaagaaaac ttgaagtata gtatcctttt aaattttaaa tagataatag aaactggttt 360

ccccccattt aaaccagaat ttaagtttaa ctttatatat tcttgacagt ttggattttg 420

tccttcaacc tcataaaatt gggaatttaa gcatcacctg gttcgattta aatgcaatgt 480

agaatttgca ttaaaatact acattaaagc ctcagatttg tagtagctaa cagcacttct 540

atgtatgtgt cagggactgc tctaaatact tcatatatat taactcctct attctgtact 600

tctgttcccg ttttatacag caggaaattg aaacactgag aggttaagta actaaagtta 660

cagagctaga gtgacaggag taaagcttca actcaggcaa cccagacttc cagagttctg 720

atctccacta ctaagctgct agcatagctt ttctggtaac tatttttaat tcaaatataa 780

ttcgagtgat ctatctaaca agtcatcact ctgacaactc agtgacttgt aatgtaaaat 840

tattcattgt aattcattta atattattgt ttctctgtgc tgcaaaaatc atagcaatcg 900

agatgtaatt tattactctc cctcccacct ccggcatctt gtgctaatcc ttctgccctg 960

cggacctccc ccgactcttt actatgcgtg tcaactgcca tcaacttcct tgcttgctgg 1020

ggactggggc cgcgagggca tacccccgag gggtacgggg ctagggctag gcaggctgtg 1080

cggttgggcg gggccctgtg ccccactgcg gagtgcgggt cgggaagcgg agagagaagc 1140
agctgtgtaa tccgctggat gcggaccagg gcgctcccca ttcccgtcgg gagcccgccg 1200
attggctggg tgtgggcgca cgtgaccgac atgtggctgt attggtgcag cccgccaggg 1260
tgtcactgga gacagaatgg aggtgctgcc ggactcggaa atggggtagg tgctggagcc 1320
accatggcca ggcttgctgc gggggg aggg gggaaggtgg ttttccctcg cactgtctta 1380
aaccgatggc ctttccttgg cacagggtcc actgcagcat gccaaacgag gaggcagggg 1440
cgtcgtcccc ccgcccccca ctgcagcact ggagatggat ttcctgtact tcggatccag 1500
ggtttttgac agaagaggaa gaagggggag gggtagaagt gttaagggga gtctgctgag 1560
aaaagctgtt tttgaagcca gaaggggttt ttgtttttat aatgccattt gacagagtgg 1620
aataacagta tctaaggaaa cgggtagagg acaacaaaga atggagcata ttcatggcga 1680
ggagcaaaag ctctacccca ttgaaaggct tcttttcctc cctggcgaca aggacacatg 1740
cattggtggc caaaagagag aggagacaaa accgctgcag atggctgatg tgaatctagt 1800
ggaaagagct actggggatg agagaaagag gaggaggcag gtactgcaga gcgtgagtgg 1860
tggtgttggt tggtgaaata ctggtcacca gtagtgtgcc tgcttttgta aaacatctaa 1920
gtaaactccc tgtgaacagg gtggcaaaca gataccagtg tctttgttag ttacaaaatg 1980
cagtggtagt ggctttttgc ggacgactgc agcagtgctt tttctccctc tgttaggccg 2040
aaaagacaac tgcagaggaa taagaaacct tgcagcaaat gctggggtag aagcccattt 2100
acaagaagcc atagtttata aatgcagcct gaacagcaga aaaaaaatta ctgtttttta 2160
aagtaggaat aatgtcaggc tatgaatgtt ttgtcattgg aatgtattgg acactttgat 2220
tctacatcac gaaagtgatg ctcaaattct ttgatttaac ataaatccta tacgaaatct 2280
taataaatta tgtatgaaac agtggatctt ttcttttgtt agtgaagctt ttatgccatt 2340
aattaggtca ttcaagagca aaccacttta caacgtaaat tactttgtca aaaattatgg 2400
tgaacaaatt tttgtaggcc taatatttaa gacctatagt taagtaattt tatatttctc 2460
tttggttgct tttagataac actgaataaa tatttaagat attaattcag tgtgcaaata 2520
ttttaaatta aagcaacatg gcttttcttc tagatgtatt tctgtttagt gagtactcat 2580
gagatatact cttgatataa agtgtttttc attgagcttt tttttccttt acctaaatgt 2640
aaaagcctat ctttatgcat acttatagta gccagcctgc cacacctccc cactccctga 2700

acaaggatgg cagtggcttt gtaaagccct ccagggtcag tgacagtgtc tcttaaataa 2760

tgtttatgta atagaaagtc ttaagatgac ctattatatt gtttcagtaa tattttcaag 2820

ataatgcgga attgggctgg cttagataaa taagactaca taagtttttc actgataaat 2880

ttaaatagtt ctttaaaaaa ttatttctgc tttaagaatt acttgatcat ggatatcagg 2940

gttaatagta cttaggagcc gggcgtggtg gcccatgcct gtaatacaag ctaaacagga 3000

ggctgaggga agctgaggca ggaggatcac ttgagccttg gcgttcaaga ccagcctggg 3060

caaccaagca agaccctatc tcaaaaaaaa gtacttagag tctctttttt aaaatctgat 3120

<210> 33
<211> 2501
<212> DNA
<213> Homo Sapiens

<400> 33

gtgttctagc tcctgaatcc atgctgttcg tcactacact gtactgcctg tggatgactt 60

acttgtccct gtagtttcat ctgaagaatt cctcctcctt ttcctttgag gcctgcctca 120

aatatcactt cccctgtgaa gactgcctgg tgttctccag gagagagtgt gactcccttc 180

tctaggaatg gtagcacccc aaacacacac attttgcagc atatttcacc ttgcatggta 240

atggcctgct tgtgagtttt attccgtacc agagggtgag ggctctgaag atagcgccag 300

gtcttattta cctcgatacc ccacaacact cattacatgt ctgatgaatg aatgcatagg 360

gggatggctg ggtgcatttc tctcaacttt tcaatttctt gaaataaata acaaaatctt 420

accttccctc agaagtcctg gcatggttcc tttcatcttg ccacagccac tgataatcac 480

tttcattttc tgtgtaactc accaggcaag aagtccatgc agatttactt ttagtagttc 540

acatgacaaa taaatactgc gtttgatttc caaacattaa accatagtat attatagata 600

gatatagagt tatcattcaa agtatgatat ttcaatctca aaaggcttcc cctgaagaat 660

attacaaact cttcctctct ttaagatctg ctgggtagga aagatgggag aaaatgaatt 720

aatgtttaca cagaaaggag gataatgggg gcaaaaataa tagatgaacg tatgggtgga 780

tgagagaatg gataaaatga taggtggata tgttgatctt ggacagatgg gaaatgagtg 840

gatatatcaa taaacagata tgtgggtgga tgggtggaga agaggatggt ggatggttgt 900

ggttttatga agagatgtga aaaaggaagt gtggaatgat ggatgagaag ttgtatggga 960

agatgaatag aagaataggt ggttgaataa attaaaaggt gtgtggttgg atgaatgaat 1020

gagtgggatg atagatggac ctaagtggtt agtggatgga caggaggatg gatggatgtg 1080

agagccccag aaggacataa ggaaagatgg gtggatagat ggatgggcgg atggaaggat 1140

atttaggagg atgaatgagc atgtgtgtgg agagaggtgc ccattcacac tggcttgaac 1200

acatgggtta gctgagccaa atgccagccc tatgacaggc catcagtagc tttccctgag 1260

ctgttctgcc aagaagctaa aattcattca agccatgtgg acttgttatt gaggggaaaa 1320

agaatgagct ctccctcttt ccacttggaa gattcaccaa ctccccaccc ctcactcccc 1380

actgtgggca cggaggcact gcgccaccca gggcaagacc tcgccctctc tccagctcct 1440

ctcccaggat atccaacatc ctgtgaaacc cagagatctt gctccagccg gattcagaga 1500

aatttagcgg gaaaggagag gccaaaggct gaacccaatg gtgcaaggtt ttacggttcg 1560

gtcatcctct gtcctgacgc cgcggggcca gcgggagaag aaagccagtg cgtctctggg 1620

cgcaggggcc agtggggctc ggaggcacag gcaccccgcg acactccagg ttccccgacc 1680

cacgtccctg gcagccccga ttatttacag cctcagcaga gcacggggcg ggggcagagg 1740

ggcccgcccg ggagggctgc tacttcttaa aacctctgcg ggctgcttag tcacagcccc 1800

ccttgcttgg gtgtgtcctt cgctcgctcc ctccctccgt cttaggtcac tgttttcaac 1860

ctcgaataaa aactgcagcc aacttccgag gcagcctcat tgcccagcgg accccagcct 1920

ctgccaggtt cggtccgcca tcctcgtccc gtcctccgcc ggcccctgcc ccgcgcccag 1980

ggatcctcca gctcctttcg cccgcgccct ccgttcgctc cggacaccat ggacaagttt 2040

tggtggcacg cagcctgggg actctgcctc gtgccgctga gcctggcgca gatcggtgag 2100

tgcccgccgc agcctgggca gcaagatggg tgcggggtgc tcagcgcgga cccggcggca 2160

gcccctccgg ctgagtcggc cctgggggac tggagtcaag tgagctgtct gcgaagtgca 2220

ttgggctccg gaaagcaggg ctgggatttg cgctaaaccg ttggagaatg tgtctgtgga 2280

agcaccattt ggttgaaaga aaaagagaaa gagaagaaag tttgttgggc aggctgccgg 2340

cgcgcagttt tgggcgaggt cgctagagct gcagcacatg gcagaaagta accgttctcc 2400

cggatgcgca cagtcgttgt ctggactaac aggctcctgt gcccaagggc tcgccaagcc 2460

ccaccgggct gtgtctaggc agggcagagc tgggcggggc a 2501

<210> 34
<211> 2501
<212> DNA

<213> Homo Sapiens

<400> 34

```
agatttactc aaatttaaga atgagaatac aaatccacat cttgaagtgt ttcacagaaa      60
ggtctatctt aatgtctgga gtatatattt caatgaacat tcattttatt ttatttctct     120
ccattcctga atcaagcaat cttgaatcta aagttgctat gattagcact gaaaagacca     180
ctggactatt aattgtgtga ctttgggaca gtaactttct gcaccttagt ttgtttacat     240
gttatacatg aaggttgaag tctgattctg ctctgtgact atcattctaa acatctgatg     300
aaatcaaatt tcagtgtttg gaatggtagt acaataaatt tactaagaat aaataattca     360
ctgcaaaaac acattgattt ccaaatgatg taactgacag ttatattact gcagagggct     420
gataaataac aaaagaaatg aaagatgcac atggtgagaa ctgaaattat cctgacaagt     480
cttctacctg tttatcactt aaaatcaatg accatgctga atgcctacaa attacaaaat     540
ataaaagaaa tcttataaat gcgcatgtac aggagtctaa gttactaaaa gttttaaagc     600
ataagtttaa accaaactaa tcaaagaagt tgagaggaaa aattggcttt catctttaat     660
cactactgtt ttgaggtcct atgtttaata taattttcta agtagaggct tcagagagaa     720
gagttgtgag gatactttca tatttgtgta gaaggaaaag tttgccatcc attctagtat     780
ccctagtgtt atactgatgt gcaccttgga tttattttgt tcctattgta taaactcata     840
cttgacttca aagaaaagga aaatccaaag tccctctttt ctaaggggac agaaatcctt     900
tgtgtcaact gtttgaccct tttctctgta aggtcctatt ggaaatcttt tgtaacacaa     960
tgcaggggac tcttccatgt gttgatgctg tttacacagt ggggtgggcc tgactgaaga    1020
aaaaaaatcg catatacgca tgaaagatta tggtcttatt tccggaaagc atgaaaggtg    1080
attgatactt ccaagaagtc cctgttactc aggaaaatta tcaaatattc tactcagaga    1140
tacttggaaa gactgaagga aaggaagaac gaagaaagca gaatctagac ttatgtgggg    1200
agagatttgt ggcagaggaa aagtattctc tttgaatccg acaagggatt tgcctggggg    1260
aatttcctgt ccagcctttt attaccaggg tcttttgaag ccgggctccc cattgggcag    1320
ttccctggga gtgcagtggg gaattcttac actttccctc taggtccccg aaggatctcg    1380
ttttctcagt gtctctttca ggttggcagg agccttgagc ctgacacttc cctttgatgg    1440
gacaggcaag ctctgtgggc gcgtaaacac gctgtaacca agttctttgc tgattttaca    1500
```

gttttgtgtg ctcccgagaa gaagtgatcg tactcaattg tctattgctg gcctgccccc 1560
taagagcctg ggggctcctt tcccctaacc cagaactagc tgcacggggg gcggggaaat 1620
gggggtgggg aaggagtggg agggcagtgg tttccgcgag cagagcgatg ttactgagtg 1680
agtccctgaa tggggagcgc tgctgtcccc aagccgattg gtacttcttg tcaggaagaa 1740
acgccaagag gtgggagtgc ctggggaggg aggcaggcgg tccctaccgc aggcgcgggg 1800
agctgccttt ccgcccctcc gcctgctttc caagcctgga ctcttaggag tggctgaagc 1860
tgcggagcgc ttttggagcc tgtgaatgaa ccctcctcct ctccctcctc cttcttctcg 1920
ctgagtctcc tcctcggctc tgacggtaca gtgatataat gatgatgggt gtcacaaccc 1980
gcatttgaac ttgcaggcga gctgccccga gcctttctgg ggaagaactc caggcgtgcg 2040
gacgcaacag ccgagaacat taggtgttgt ggacaggagc tgggaccaag atcttcggcc 2100
agccccgcat cctcccgcat cttccagcac cgtcccgcac cctccgcatc cttccccggg 2160
ccaccacgct tcctatgtga cccgcctggg caacgccgaa cccagtcgcg cagcgctgca 2220
gtgaattttc cccccaaact gcaataagcc gccttccaag gtaatcacgt ttcttttgtt 2280
ccccccttaa aaaacaaaaa caaaaaactt atagaaaaaa acccgcgagc ttagaaaaaa 2340
gaagcaattg gtagaaggct ttaattaagg caaagagctg taaggcgaag ttaagaaaat 2400
gtaggcactt aaaaaatgca ggtaactttc ataagggctt ttggggagag gcatacagag 2460
ggaccttggt gttgaaaaag attcagacaa aagaaaccca g 2501

<210> 35
<211> 3000
<212> DNA
<213> Homo Sapiens

<400> 35

acatttaaat tgcatatttg gcttgtaata tattacatat ttaaaagaat tactattttt 60
ctgaatacca ttaatactaa taatagcatc acagtgacta agagtggact taaataaagg 120
ctggcttgaa actcaggtct gccatttatt agcaagcttc taaaaattct gagcccttag 180
ttttctcaca tgtgaaatgg agaaaataaa tctgcaaaat taattaaatc tgtccattca 240
tatttttctc tccagtgtat attaactggc attcctcgtt aggccagaat gtgctctcaa 300
ccatgctcca aatccgcttt gtgccaaccc cactgccaga accctttcta ccttgagaac 360

```
cagaaaagga aacattatgc ctggcaatgc ctacaccctc caaaataaat ctgcaggaaa       420
gaacacccag taagtgatga gagcagcaac gactgccttt atcattttaa atttacaaca       480
ccaccttttc tagagcctct taagcattgt agataattcc ccactcatta aaaaataaat       540
tgtaaccata agtattcagg gttgatactg cttttgaatt agacagtgct catatcagtt       600
gcataagacc aacctaaagt agaggatgaa atcttttttc tgaacctttt tcagaacgta       660
acttagtgaa tatattaaaa ctaaactttc tttgaatggg agtaatttct acggattaat       720
ctgtaatctc ttagaccaca cctaaggtaa tgtagaggtt gttgtatcat aggctttgtg       780
attagagacc actggatttg tctttggaaa agtcactcat gattctttgg gctttggttt       840
cctcatcttt tataccggct taataatgac caccgtagag ttgtcatgga agttaaatga       900
actcatgtac cataagtgac caatacaatg attgacactc agtgatttat caataaatta       960
aaacatttat tatcaatatg acagagaagg tgccgctaaa atagacaata ggtttttgga      1020
agaggtgatt aaatggatgc aaaatttatg gattgtttat tccgtctacc tttgctgtgt      1080
cccctggttg tggcatacac acgtgtgggt ataaaatcgt aaatcctatg tagtcgcgta      1140
gtgcatgcgc agaaggctta gacacgaaat gtcatttcag caatgtgcct agagaagctc      1200
tgacgccgcc ttggaagtaa gtcgttgctg cctgaccttt gggcgtctgg gacggatgcc      1260
tatacctgca cccagcagca ctggaagggg cccaggccct tcgcagcaca gcctatcccc      1320
agaccgctta gtccttcata acatatatct ccacggaaaa gggtatttcc tcccgtcaga      1380
aaaagcgccc cagtctggtc tgggttggtt tttatttcac gttgttgcaa gtaggcgaag      1440
tcccttctgt ctcctccctt ggggtaagtg gaaaggagtc cggcaggggg cccgcagtgg      1500
cctgcacagg ggaactgggt agcgagagag ttccaggcaa ttccgggggc tgccccacag      1560
aagcaggtgg ggatcgacag tggctctccg gcccagggag gagagcgcgg tcgcgggtcc      1620
ctcccctcag cctggaggct gcagccgctc gagtcggccc gggtgggggc ggggtggggg      1680
cggcgcggag ggcacggaga ttacggcggc gccacccggg acatccaggg ccccgaggcc      1740
ctgggcggtc cccacgcgag atcgcaaacc atgacaatag gcagtcaccc gaggtcaaat      1800
aaaaacggag tgggtccccc gcgcgccgcc gcccccgcgg tccctggcgg cctcccccga      1860
ggcccccggc ggcctcacga gcccgcagta gccggtggcg acgtcgcccc cgccccacct      1920
ccctgcgcaa gtgcgaggct gccggcagcg cggcgcacgc tccggccgtt cccggcttcc      1980
```

gcgcaaaact tccatcctgt ccacgtgaag ttgtcgctgc cttagagagg gggaaagagc 2040

tgcgggaaaa gccggggagt gacgactgcg gcggctgggc gcgctctctc attttctttt 2100

cttctccttt ccccccctgtc gcagtccgga gttttggctc ctctcctttc ctcctccccc 2160

tcggagccgg cttctccctc cgccccgctt ctccccccgct tgtgtacgct atttgttgtg 2220

gggtggccga aggggatgtc ctgttttcac cagaggcaca gcgcgaaggg gaaacttcga 2280

cactggaagg aacgagaata aatacttaat tacggacgca ctgaaccgcg gctgggacag 2340

acacttcggg aacccgaggc ggaccgggcg acgaggtgag tgacccccttc ttccaacccc 2400

cgccccaggg ctcccggggg agcctgagtt gagagaaccc ccaaactttc cgggaaagtg 2460

cgcgaggctc cgccggggac gccgagcgct gggtactgag gacgcgcagc tggacggtgc 2520

gtgggcgcct gcgtccccgg ggggcgcttg gaggccgggt gccccacgcc tgagggcccg 2580

ggccgctcgg accgcagcgg tgctctctgc cctagaagac gtccccaagc cccaagggtc 2640

ccttccgagc ctgcctgtcc cttccggggt cggcgcggag cctgcgcgta acggagttca 2700

tccagcagtc cagcgcgcgg cttctacctg caccccgcct ccacctggca gaggcgcgag 2760

catcggggtc tcccccacat ctttcttatg acgtgtatta ctttctgatg accccctaga 2820

tggtccaggc gcgaggatgc tgacccagag tccttcggag ggtcacaggc gcctgggctt 2880

tcccggtgcc gggtgcgtgt gtactttaaa ggctcgcgtt ctaatctcca ggcactgatc 2940

gggcttttca actgcggcga tcccacttta atagttttta tgtggcgtgg actgaatgtc 3000

<210> 36
<211> 2501
<212> DNA
<213> Homo Sapiens

<400> 36

gtcacttcca tgtgtcctgc agttctctga aggggcgtgg gacctaccga tgccaattat 60

ccagcattat ctccagattc caagaagttg ggtgtgagc cagcaatcag tacagaaaag 120

agataccaaa ataagtttga gttggggagt gttccttcaa cttcagtttt ctggaagaga 180

tctttttttt ttttttgaga cagagtttcg ctcttattgc ccaagctgga gtgcagtggc 240

acgatctcgg ctcaccgcaa cctcctcctc ccgggttcaa gcgattctcc tccctcagcc 300

ttctgagtag ctgggattac agacatgcac ctgtaatttc tactaaaaat acaaaaatta 360

gccgggcgtg gtggcgcacg cctgtaatct cagctactgg ggaggctgag gcaggagaat 420

cgcttgaaac caggaggcgg agattgtacc aagatagttt gttccagcta aacaacctgg 480
cgctagtgca ggaaaaggtg gaaggcacgg ggctagcaca ggagggttca atattttcaa 540
ccttatcaag ccatattttg gcaactcttg tttttcacga gaagcccccg ctgggcttgt 600
cccagcgctg tcctgaggct tccccccatga gttccgatag ggcagaggcc gccctgagcg 660
tttctctttc ccctggtcca agagtggctc aaaagaagga tttttgactg gaattggcca 720
ctttgtgtta ctttttgacc cttgacctcg ccccaaaggg ggatgcgggg gaggggctct 780
ggtaggggtg gccccgctcc ttccaggtcc gcaagcccag gttcccgccc accgggctca 840
gcccaccctg cggccgttca gggaggccgt tggcacccgt gacctacgac cccccttcccg 900
agccccaccg aggtcacagc cgtggcctcg tctccccatg cctgcttccc gccccctgcc 960
cgtgacgggc gtctccgagg accaatgagc gcgctgtatc cacccctcgg gcggggccaa 1020
gcgccgacca atcgccgctc gggcgcccgg ccgggtccaa acgctccaat cgtcagcggc 1080
ggcggggcgg gcagagggcc ggggatggca ggttcaacca acgggtgggc acgtcgtcct 1140
cgcgaggagg cgtgccctgc ggccgggcgt gcggtgtccg cggcggcgca gggaggggga 1200
gggaggtaaa caagatggcg gcggcgtgtc gggcgcggaa gggggaggcg gcccggggcg 1260
cccgcgagtg aggcgcgggg cggcgaaggg agcgcgggtg gcggcacttg ctgccgcggc 1320
cttggatggg ctgggccccc ctcgccgctc cgcctcctcc acacgcgcgg cggccgcggc 1380
gagggggacg cgccgcccgg ggcccggcac cttcgggaac cccccggccc ggagcctgcg 1440
gcctgcgccg cctcggccgc cgggagcccc gtggagcccc cgccgccgcg ccgccccgcg 1500
gaccggacgc tgagggcact cggggcgggg cgcgcgctcg ggcagacgtt tgcggggagg 1560
ggggcgcctg ccgggccccg gcgaccacct tgggggtcgc gggccggctc gggggcgcc 1620
cagtgcgggc cctcgcgggc gccgggcagc gaccagccct gagcggagct gttggccgcg 1680
gcgggaggcc tcccggacgc ccccagcccc ccgaacgctc gcccgggccg gcgggagtcg 1740
gcgcccccccg ggaggtccgc tcggtcgtcc gcggcggagc gtttgctcct gggacaggcg 1800
gtgggaccgg ggcgtcgccg gagacgcccc cagcgaagtt gggctctcca ggtgtggggg 1860
tcccgggggg tagcgacgtc gcggacccgg cctgtgggat gggcggcccg gagaagactg 1920
cgctcggccg tgttcatact tgtccgtggg cctgaggtcc ccggaggatg acctagcact 1980
gaaaagcccc ggccggcctc cccagggtcc ccgaggacga agttgaccct gaccgggccg 2040

tctcccagtt ctgaggcccg ggtcccactg gaactcgcgt ctgagccgcc gtcccggacc 2100
cccggtgccc gccggtccgc agaccctgca ccgggcttgg actcgcagcc gggactgacg 2160
tgtagaacaa tcgtttctgt tggaagaagg gtttttccct tcctttttggg gtttttgttg 2220
cctttttttt ttcttttttc tttgtaaaat tttggagaag ggaagtcgga acacaaggaa 2280
ggaccgctca cccgcggact cagggctggc ggcgggactc caggaccctg ggtccagcat 2340
ggaggtggtg gacccgcagc agctgggcat gttcacggag ggcgagctga tgtcggtggg 2400
tatggacacg ttcatccacc gcatcgactc caccgaggtc atctaccagc cgcgccgcaa 2460
gcgggccaag ctcatcggca agtacctgat gggggacctg c 2501

<210> 37
<211> 2501
<212> DNA
<213> Homo Sapiens

<400> 37

ggaggataga aatataaatt aaagaatgac acaaataatt ataaagttac agctgttaaa 60
agaaaagcat atggtgccaa gagaacgtgt aatacaagat ctactcatgg aggtgaggga 120
aagcttgccc atcaaagaag ttatgattca atccacgaag accaggagtt ggctgggtga 180
agaaaaaaag gtcagaggaa ggaagtccac actggggaag gctctaagca taaagggtag 240
gaggattaca gaggcatatt cacgaaattt ggagaaggct ttcagtaagc aaggagaagc 300
caaatgaaag tttacgggag agttggaggc ttgaagacac gttcaaggat ctggttttta 360
tcttctcttt atctcaagag cagtgggaag ccattaaatg attttaatca gagggttggt 420
ataactagtt ttgtattttg aaaagctgaa ttcagctctc gtttgagaaa ctgagtgaaa 480
gagcccagaa cggccgtggc tgagggtgac tcgtgggaga ctcctacaca agccatggca 540
gtggcatggg ctggtggcag aagagggaat agggagaaga tttggaactc aatcttcctc 600
cattgacaaa gtcactccag ctttggcaag gcaattaatt ggtgggaaag aagatgccta 660
gccctcctga tttcactgca ctttctgcat cttcaacatg agtactggga agtggcaaaa 720
catccagagg cagcttgggt gctaggtgga gcatgagtta aaattccagg atgaagcaaa 780
tgaacactta gaatgacagg aaagatttgg gagttgggtt tgggggaggg ctatttacct 840
ttattccctg gagaccctgg cacaaaccct tgcctctgca atcttcctct caggtaaagg 900

aattcattaa atgaattgct agaagatcta ctgaccagag ggctgtacag aatcatatct 960
ttgagagtgg gaagtaggtt gatcacatag tttattatcc aatcaggaca tatctgaaag 1020
agaaaggggg ttctattaat atttaaacta caaaacatgt acaccaggaa tgtcttgggc 1080
aaatctggtt gccctagcaa gaaaggaaat ttgaaagttt atactgttct gctcccatgt 1140
taccccgttt gcacatgaga gggtaagtat tctctttctt cacctgcatt aagggaataa 1200
aagcacaagc attcaggtga ctcccaaccc acttttaatt ttacagtttc tgctatactc 1260
tatacattct gaaaattaca tttcccacca ctatcacttc gtgataggtg atcatttaca 1320
attactcact gactcagtcc cgggaagagg cggtgcaaaa tgggacgctc tatccaggtg 1380
ctcattagaa atgcagaatc tctgcctgcc tcctagacct actgaattag aatctgcatt 1440
tttaaataag atttccaggt gatcaatatg tacattaaaa cttgagaaaa acctctagac 1500
ttcgacctaa agaaaaacat tttacaactt gacagtgtat gcacatacat acatgcatat 1560
agacacaact gaagcacaaa tttaatgaag tagaatttac cgttactatt ttatttggga 1620
aagaaatgtg ctcgcgactc aatagattgg agtattcact cctggatctc aacttgcaat 1680
ttgaaaacgc atctctaaag cacctaggag caatctgaag aaagctgagg ggaggcggca 1740
gatgttctga tctactaggg aaaacgtgga cgttttctgt tgttactttg tgaactgtgt 1800
gcacttagtc attcttgagt aaatacttgg agcgaggaac tcctgagtgg tgtgggaggg 1860
cggtgagggg cagctgaaag tcggccaaag ctctcggagg ggctggtcta ggaaacatga 1920
ttggcagcta cgagagagct aggggctgga cgtcgaggag agggagaagg ctctcgggcg 1980
gagagaggtc ctgcccagct gttggcgagg agtttcctgt ttcccccgca gcgctgagtt 2040
gaagttgagt gagtcactcg cgcgcacgga gcgacgacac ccccgcgcgt gcacccgctc 2100
gggacaggag ccggactcct gtgcagcttc cctcggccgc cgggggcctc cccgcgcctc 2160
gccggcctcc aggccccctc ctggctggcg agcgggcgcc acatctggcc cgcacatctg 2220
cgctgccggc ccggcgcggg gtccggagag ggcgcggcgc ggaggcgcag ccaggggtcc 2280
gggaaggcgc cgtccgctgc gctgggggct cggtctatga cgagcagcgg ggtctgccat 2340
gggtcggggg ctgctcaggg gcctgtggcc gctgcacatc gtcctgtgga cgcgtatcgc 2400
cagcacgatc ccaccgcacg ttcagaagtc gggtgagtgg tccccagccc gggctcggcg 2460
gggcgccggg ggtcttcctg gggtccccgc ctctccgctg c 2501

<210> 38

<211> 1508
<212> DNA
<213> Homo Sapiens

<400> 38

```
attctacaat tataaacatt tagtgtattt tgcaaatatg gcataacctg ttggcataaa      60
attccattgt tccagaaaat atcggtaata aaattataga aaagttaaag atcttcattt     120
cttatttcga agcgtttggg agacatttca gaaacggatg ggaaatgtta aattctgcat     180
gcctgcttat ttttctttcc acaccgacta gatgtaaacg agtgtcacca aaagtacacc     240
acaggcaccc acacagattc cttccataag ggatccacaa agtttagatg tgaaatgtac     300
cctaaagggt tcctagccgt ctctcatccc tccctctgtg aaacagggaa gacacatgtg     360
ttttcaaggg cagagatgga acttgggcca atgggccggg gggtgggggg aagttggaag     420
ggactgcctt aggacatggc aggatagtgg attgtttctg ccgccttgtt gcccataccg     480
gggctctctg caggcgcgtc ggctccctcc ccccctgctg agaagaagcc ctgccaaaac     540
atccgcctcc cccgggaacc cccccgtggg ttcaaagcgg gaaaatgttg cctcaggttt     600
aaaataatct gtccaagccc cccagcgcgg gagaaacgtt cttactcgct ctctgccccc     660
gggggtgctc cccgccctct gctgccagaa ccttggggag tgcctagacc cggggcagca     720
cacgtccggg ccaaccgcga gcagaacaaa cctttggtgg gcggccagga agctccctcc     780
cagccaccgc ccccctccag tgcctttttt tccccccata caatacaaga tcttccttcc     840
tcagttccct taaagcacag cccaaggaaa cctcctcaca gttttcatcc agccacgggc     900
cagcatgtct gggggcaaat acgtagactc ggaggtaggc atccgtgggg gggcgccggc     960
tcgggcgtgc ggggaagtgt ccgcttctgc tatctgcctc tccaaatatc ccgactgctg    1020
atctggcccc ccagccctct ttccacttcg gagcactcct ctggcgttgg caccgctgaa    1080
gaatgggcct gggcggggag gtgaagagaa gccaggaatg ttttatgttt tcctaatgga    1140
gagggggcct cgggagcccc tgagctagga ggacacggaa aaggggattg ggtcctgag     1200
attgggtctg ttgggcccaa gacgcgtttt ctggatgggt ctaggatgct cccctgtcgc    1260
gggaaccccg cggtccggcc ctgcctgctg ggggttcgaa aaagtggatt gcagggtgga    1320
aggtgttatt tacccgagtc ctggggacag tccccgggac tctccgccaa gcgcccaaaa    1380
cggcaggtcc ccaagcggcg cgcggtgttt ttgcactttc caaaattctt gaaacatctc    1440
```

aaaaatcctt ctgcatcttg gcgtctggca gggttttcca aaaaaagtaa acctccctcc 1500

caaatgca 1508

<210> 39
<211> 286
<212> DNA
<213> Homo Sapiens

<400> 39

tttgcactag gctggaagtg gccgccagtc ccccgtgcaa ttccattctc tggaaaagtg 60

gaatcagctg gcattgccca gcgtgatttg tgaggctgag ccccaacagt ccaaagaagc 120

aaatgggatg ccacctccgc ggggctcgct cctcgcgagg tgctcacccc gtatctgcca 180

tgcaaaacga gggagcgtta ggaaggaatc cgtcttgtaa agccattggt cctggtcatc 240

agcctctacc caatgctttc gtgatgctgc tgctgatcta tttggg 286

<210> 40
<211> 2501
<212> DNA
<213> Homo Sapiens

<400> 40

tctatacaca ttatgtcttt taaatgacac actagccttc tgagggtaac ttatattggc 60

aacagttttc agatgtggaa actgtgaaga caatgttggt gatgtggaag caacataaac 120

tttggagtct ttcagaccca ggtttgaatg tcagactgct ttttattcag agtaacttca 180

gagcattatt tctcacctta atttttttc aggcctcttt gtgtctatgt gtcctcttca 240

ctcctgtcca ttgttcattc agtgattttt gcaccttcct tcactgttag tgtgtagaca 300

catagttctc ctggctctga gacctatgtt aattccattc taccatcctg ccagcccact 360

caattcctat tgagcaatgc tagttgaaag ttgtggtggg attaaatgtt gcaatgagta 420

ttcaaatgag gttgaagtat ctacgcattc tacttacata tggtgaggta tattcaagga 480

aggctgtagc cattaaaatc tcaggaaata atttttcacc tcctcaggtg aaagggtctt 540

caggcctttg tgttctggaa ggttcattta tagccatttc ccaaatgaca atgcgattga 600

tgagtctaga gtctagctca aatagcaatg gactggaaga ctagtttagg ttttactaat 660

gtggaacata gaacaaatta tgtccttgtt tcagcctgtt catctgtgaa atagagccta 720

tcatatccag tcttccttgc ctttaggttt gagttacctt ctttggtcaa ggtaagtaaa 780

tgcctatgat gtttggctgt gcacaagata aagctacaac aaagctacaa cccatctttt 840

ctctgtagaa gactgcaaaa agcaaaagag acccaggcaa aaatctcgga atgacttttg 900

gaacagagag cctccccaga atcagaagtc aaaggaattt aaaacatagg gaggcccagg 960

gtctctactg acataaagga aagatgtttt ccttataggt ttacgtttac attttctctc 1020

tctttccatt cccacttgca tctccacctt tacacagggc ttatgggacc tcctccacaa 1080

aagagcagtt gcagtaaccc acatcatcct ctacgcctgg ctgtccatca agaggcgaaa 1140

agcagcccta tataggttct atccttggat agttccagtt gtaaagttta aaatatgcga 1200

aggcaacttg gaaaagcaag cggctgcata caaagcaaac gtttacagag ctctggacaa 1260

aattgagcgc ctatgtgtac atggcaagtg tttttagtgt ttgtgtgttt acctgcttgt 1320

ctgggtgatt ttgcctttga gagtctggat gagaaatgca tggttaaagg caattccaga 1380

caggaagaaa ggcagagaag agggtagaaa tgacctctga ttcttggggc tgagggttcc 1440

tagagcaaat ggcacaatgc cacgaggccc gatctatccc tatgacggaa tctaaggttt 1500

cagcaagtat ctgctggctt ggtcatggct tgctcctcag tttgtaggag actctcccac 1560

tctcccatct gcgcgctctt atcagtcctg aaaagaaccc ctggcagcca ggagcaggta 1620

ttcctatcgt ccttttcctc cctccctcgc ctccaccctg ttggtttttt agattgggct 1680

ttggaaccaa atttggtgag tgctggcctc caggaaatct ggagccctgg cgcctaaacc 1740

ttggtttagg aaagcaggag ctattcagga agcaggggtc ctccagggct agagctagcc 1800

tctcctgccc tcgcccacgc tgcgccagca cttgtttctc caaagccact aggcaggcgt 1860

tagcgcgcgg tgaggggagg ggagaaaagg aaaggggagg ggagggaaaa ggaggtggga 1920

aggcaaggag gccggcccgg tgggggcggg acccgactcg caaactgttg catttgctct 1980

ccacctccca gcgccccctc cgagatcccg gggagccagc ttgctgggag agcgggacgg 2040

tccggagcaa gcccagaggc agaggaggcg acagagggaa aaagggccga gctagccgct 2100

ccagtgctgt acaggagccg aagggacgca ccacgccagc cccagcccgg ctccagcgac 2160

agccaacgcc tcttgcagcg cggcggcttc gaagccgccg cccggagctg ccctttcctc 2220

ttcggtgaag tttttaaaag ctgctaaaga ctcggaggaa gcaaggaaag tgcctggtag 2280

gactgacggc tgcctttgtc ctcctcctct ccaccccgcc tccccccacc ctgccttccc 2340

cccctccccc gtcttctctc ccgcagctgc ctcagtcggc tactctcagc caaccccccct 2400

caccaccctt ctccccaccc gcccccccgc ccccgtcggc ccagcgctgc cagcccgagt 2460

ttgcagagag gtaactccct ttggctgcga gcgggcgagc t 2501

<210> 41
<211> 2448
<212> DNA
<213> Homo Sapiens

<400> 41

ctgccctgcc tctacgacaa aagccaacgg gtcttcagta cttttattaa aaaatagtca 60

cgcagacagt gccctggtgg ctctgccccg catcccaact ctggggtggg ggaaaggggt 120

caacgttttc gcagccccaa accgggccat cacttgccca ccgagtcgaa tatgatgcgg 180

ttctgctcgg cgcgctcccg ctggctctgc gtccgcgcca ctccagcagg gtccgcagca 240

ggtgaaaggt gaggtcaatg gacagagaag ggttgtcccg ccgcggccgc tcgcctgccg 300

tcccgagtcc caatcggccg ggccccgcgc gggcgagaag cgtccgccag cagcaagagg 360

agcgcgcggg ccccgcaccc tggttccgtg ccccggggct ccagcgcaga ctcgggtcct 420

ggacccggcc gcctcggggc tcctctggct gctcccaggg cacagctgta ccaggagcag 480

cagcgcggcc agcagcgctg cgtcccgcct gcctcatggt gccgccggcc ctggacacac 540

aggggcagcg cagggtgagg tgcgcctggg acagctgcag gcgccgctcc cctccccgcg 600

ctcgcccggc gacccctccg gccgccgccc aatgcccgca gcctgccctc cagccccagc 660

cactgcgcca gtgctgttcc atcccttcca cccagcctcc ctccccggag gagcgtggtg 720

gctgagctga gcggagtcct ggtgcagggg gaggctgggc ggacgctctg agccactcac 780

aggttgtcgg cgagcgtctg tacggtccaa gattgagctc cagtctctgt ccctcggggc 840

aggctgaaga cgccttgggg aacacagggt ctgtcccggc gtgtcgccag ccttatatag 900

tgccaggaca gctccgactg acgtcagcga agaacatgca ctgattgtag aagcaatgac 960

agccacagct tcgagtctga cggctggcgg gccccggagg gcgcacctcc ctacaccagc 1020

cttcgtgccc ctgggcgtag cgctggaagc agcactggac gcaggaaagc gagaagccgg 1080

ctggggcgta aagactgctc tgggacccac gggagtccca tcgccccgct tagaacagcc 1140

agcaatgact agggccgtcc ccagaccaga ctagtccggg tcgcgtgttc tgacacacga 1200

aagggaggcg ggaccgtgag gctgtccatg gtgctgaccc cgtcctctgc ctctagaagt 1260

ggccagaaat gtgagaacgc tgtgccgctg gaacccactg cctccctgca gctatcagtt 1320
ctgcaaatgc aggcatcagg agtccaccgc tggcactcag ccaacacagt gggacccact 1380
gagaggcagt ctgccacaac caaagtctcc cagcattctt atctttttcc agccccagga 1440
tccaaatttg tagggctgtc tgacaggcac catgtaacag agggtctcta gcatccgagt 1500
gctttcagac cacaaatggc tggcccatct gcttttgagt ttgccaaagt agtttagcaa 1560
agttcagttt ctctcctggg aatcatagca gcagtgtaac tctgcccatg agcagcaaag 1620
aaatatgggt tcaagtttct gctaactcct ggtgtggaca ggtcacctcc ctcctaccaa 1680
cctcagggtc ctcatttgta agactgggct tggccaggcg cggtggctca cgcctgtaat 1740
cccaacactt tgggaggccg aggcaggcgg atcacgaggt cagtagtttg aaaccagcct 1800
ggccaatatg gcgaaacccc atctctacta aaaatacaaa aattagccgg gcgtggcggt 1860
atgcgcctgt ggtcccagct actcaggagg ctgaggcagg agaactgctt gaacccagga 1920
ggcggaagct gcagtgaggc aagatcgcgc cactgcactc cagcctggga gacagagcaa 1980
gaccctgtct caaaaaaaaa aaaaaaaaaa aaaaaaaaaa ttgggcttgg tggtctcttg 2040
gcttctttcc agctctagaa atgactcccc aatagttttc tgcttaaaaa cttactcatt 2100
accactgcag attccggatt acatatttaa taacatattt actgaggcac catataaagg 2160
gttccgggag tctctaaaga gctggagcta caagaagcct aggcagggtt agagtaacaa 2220
atgtgtctat gaagagtggg gatgagtggc atttgctggg atatgggtgt aaagttgata 2280
aggtcatgaa ggttcaacag atatttatgg agtgcctagt atgtggtggg aataagacta 2340
ttatcaaggg ctctaaagca gtcagtgtac attttagagt gaagaggggc attgcagggt 2400
gctagtcctc ttaagctctg accggcaacc caaccccgtg gaaactgg 2448

<210> 42
<211> 2344
<212> DNA
<213> Homo Sapiens

<400> 42

gcctggcaca ctcaagactc ccacggaggt tcagttccac actcccctcc accctcccag 60
gctggtttct ccctgctgcc gacgcgtggg agcccagaga gcggcttccc gttcccgcgg 120
gatccctgga gaggtccgga gagccggccc ccgaaacgcg cccccctccc ccctcccccc 180
tctcccccttt cctcttcgtc tctccggccc caccaccacc accgccacca cgccctcccc 240

caccaccccc cccaccccac accaccacca ccaccaccac caccaccacc ccgccggccg 300

gccccaggcc tcgacgccct gggtcccttc cggggtgggg cgggctgtcc caggggggct 360

caccgccatt catgaagggg tggagcctgc ctgcccgtgg gcctttacaa gggcggctgg 420

ctggctggct ggctgtccgg gcaggcctcc tggctgcacc tgccgcagtg cacagtccgg 480

ctgaggtgca cgggagcccg ccggcctctc tctgcccgcg tccgtccgtg aaattgcggc 540

cggggctcac cgcgatggcc ctcccgacac cttcggacag caccctcccc gcggaagccc 600

ggggacgagg acggcgacgg agactcgttt ggaccccgag ccaaagcgag gccctgcgag 660

cctgctttga gcggaacccg tacccgggca tcgccaccag agaacggctg gcccaggcca 720

tcggcattcc ggagcccagg gtccagattt ggtttcagaa tgagaggtca cgccagctga 780

ggcagcaccg gcgggaatct cggccctggc ccgggagacg cggcccgcca gaaggccggc 840

gaaagcggac cgccgtcacc ggatcccaga ccgccctgct cctccgagcc tttgagaagg 900

atcgctttcc aggcatcgcc gcccgggagg agctggccag agagacgggc ctcccggagt 960

ccaggattca gatctggttt cagaatcgaa gggccaggcc ccgggacagg gtggcagggc 1020

gcccgcgcag gcaggcggcc tgtgcagcgc ggcccccggc gggggtcacc ctgctccctc 1080

gtgggtcgcc ttcgcccaca ccggcgcgtg gggaacgggg cttcccgcac cccacgtgcc 1140

ctgcgcgcct ggggctctcc cacagggggc tttcgtgagc caggcagcga gggccgcccc 1200

cgcgctgcag cccagccagg ccgcgccggc agaggggatc tcccaacctg ccccggcgcg 1260

cggggatttc gcctacgccg ccccggctcc tccggacggg gcgctctccc accctcaggc 1320

tcctcggtgg cctccgcacc cggggcaaaa gccgggagga ccgggacccg cagcgcgacg 1380

gcctgccggg cccctgcgcg gtggcacagc ctgggcccgc tcaagcgggg ccgcagggcc 1440

aaggggtgct tgcgccaccc acgtcccagg ggagtccgtg gtggggctgg ggccggggtc 1500

cccaggtcgc cggggcggcg tgggaacccc aagccggggc agctccacct ccccagcccg 1560

cgcccccgga cgcctccgcg cggcaggggc agatgcaagg catcccggcg ccctcccagg 1620

cgctccagga gccggcgccc tggtctgcac tcccctgcgg cctgctgctg gatgagctcc 1680

tggcgagccc ggagtttctg cagcaggcgc aacctctcct agaaacggag gccccggggg 1740

agctggaggc ctcggaagag gccgcctcgc tggaagcacc cctcagcgag gaagaatacc 1800

gggctctgct ggaggagctt taggacgcgg ggttgggacg gggtcgggtg gttcggggca 1860

gggcggtggc ctctctttcg cggggaacgc ctggctggct acggaggggc gtgtctccgc 1920

cccgcccccct ccaccgggct gaccggcctg ggattcctgc cttctaggcc taggcccggt 1980

gagagactcc acacagcgga gaactgccat tctttcctgg gcatcccggg gatcccagag 2040

ccggcccagg taccagcagg tgggccgcct actgcgcacg cgcgggtttg cgggcagccg 2100

cctgggctgt gggagcagcc cgggcagagc tctcctgcct ctccaccagc ccaccccgcc 2160

gcctgaccgc cccctcccca cccccacccc ccgcccccgg aaaacgcgtc gtcccctggg 2220

ctgggtggag acccccgtcc cgcgaaacac cgggccccgc gcagcgtccg ggcctgacac 2280

cgctccggcg gctcgcctcc tctgcgcccc cgcgccaccg tcgcccgccc gcccgggccc 2340

ctgc 2344

<210> 43
<211> 2350
<212> DNA
<213> Homo Sapiens

<400> 43

cagatgcact ggtgagacca ggccctggct gtgaccttgg gcctgcatca tgacccccga 60

cccttgaccc agatcacaac tatgacctgg ccgtaacctc ctgacctgga cctcagtccc 120

tcagtcccca gagcctgtga ctcctgaccc tgacctggac cacaactctt gaccctgaat 180

gtgactcctg agacccaact tgacagtaac atttgaccct aaccatcacc cttgatgata 240

gtccctgccc ataccctgat agcaattgtg accctcatga ctctggccct gaatgtgacc 300

tataactcct gaatttgact gtgacatttg accctaatca taaccactaa ccatagcact 360

gaccataacc ttgactgtgt gactcttggt cctatctctt tcacagcccc tgcctcttct 420

cagaatccct tctctggaac ctggttgggt tcataatccc tgaccctggc ccctgacttg 480

aggcagaatt ctggaccctg actataactt ctgatcctaa ctggattcag attccagtca 540

ctaactctga cccatggctc agccgaatcc catccaatga tcctagttta taatacttga 600

ctggtccctt gccctagttg atttctgatc cctgtctgat ccctctcctg actccaaaat 660

cctggcacag gtagttccta gaaacctaga cccacaggac ttgcaaccat ggtccccgga 720

acccgagaac cttggtcagc cctacccacc tctgccatag agccctcccc cagcctccaa 780

ctcatgagac ttcccaccac ctcccccaga caccctactc caaggggatt ggtcgggtgt 840

gtcccgagac tggacccttt ctgaacaccc ccaccccca cagatgtgga cgaatgcagt 900
tcgggtgccc ctccctgtgg tccccacggc cactgcacta acaccgaagg ctccttccgc 960
tgcagctgcg cgccaggcta ccgggcgccg tcgggtcggc ccgggccctg cgcaggtgag 1020
cagcataggg acccgccaga gagtctggga gtagggcctg ggttccaggg caaagccggc 1080
tggaaaggtg gaggcgggac caaggcgctg tgggaggagc ttagaaacct ggcattggtg 1140
ggggcggggt tactgcgatg tgggcggagc ttgtctggga ggccgggtcc cgtgactccg 1200
cccaatctcc cgcgtaccct agacgtgaac gagtgcctgg agggcgattt ctgcttccct 1260
cacggcgagt gcctcaacac tgacggctcc tttgcctgta cttgtgcccc tggctaccga 1320
cccggacccc gcggagcctc ttgcctcggt tcgtacccgg gctgatcctg gccccggaaa 1380
gggtgggctt agggcaggaa aaggcgggac ggggagaaga gggcgaaaag gggaaaacga 1440
gtttttagcc ggggtattcc agcaggatca gggggcagct ggtgggagtc tcgaggcagt 1500
gaggggggc ggggcgtgga gatgaaaggg ccgagtctgg gtatttggac cgtgattgta 1560
aagaagctgt tctaaacccg tcgggggcg gtgtttgcag ggagggaagt agcgtgaggc 1620
aggttgggga aggcgtgaga ggcctaggag agccgagggg cggtggaggg gtgtggccta 1680
gaatgttagg cggagcggga ggtgggccgg gccttcggac gccctgtccc gcagacgttg 1740
acgagtgcag cgaggaggac ctttgccaga gcggcatctg taccaacacc gacggctcct 1800
tcgagtgcat ctgtcctccg ggacaccgcg ctggcccgga cctcgcctcc tgcctcggtg 1860
agaggccccg ccccggcctg atccctcctc ccttcgactc cccgactcgc cgattggcct 1920
cccacctctg tctttcctcc tccgcttctc ccctcccctt acctctttcc cccgcctcct 1980
ctcctagcct ccccaactct cctctacccc aatcttctcc tgccctctcc tctgcttccc 2040
cggcctcccc cttctgactc tcctctcccc tctttgtatc ccccatcttg cctccctgct 2100
tcccacatcc gaccacccga cctctctcct cagacgtgga cgaatgtcgc gagcgaggcc 2160
cagccctgtg cgggtcgcag cgctgtgaga actctcccgg ctcctaccgc tgtgtccggg 2220
actgcgatcc tgggtaccac gcgggccccg agggcacctg tgacggtgag cctgccccca 2280
cccgccttcg ctagcgcttg caacgcggtg ctggaggctg ctcccttggg gactgaggag 2340
gggcgccctg 2350

<210> 44
<211> 2206
<212> DNA

<213> Homo Sapiens

<400> 44

```
gggcattccc tgggatggca agtggcacca gcgctgtgag atggtttcct tcctgccgtc     60
aggcgtcagg aggcctgctc gggtctgcat gtgcctgggc ttggcaggtg gggaggcggc    120
acccagggca gcccccgcat tggaagctct aggaacttgg atctaggctg aaggcctttg    180
ctctagagtt ccggattgct gagcttggaa cagctcctcc tagacacagt gtggcgcggg    240
agcagctgca tgagggccca cgtactctgc acacatcagc agcccatccg accttgtccc    300
tggtcatgcg aaacgcatgc ccatccgacc tccgaccttg ttcctggtca tgcaggttcc    360
gcctgggctg tgacaggagg ctcggcgccc tcaggcacct ggcaggcttt cccgaccttc    420
tccctgagcg acaggaatgg gaagtgagca aatccaagta gaattaggag gctctcctca    480
gaggagcctg gcggaacccg gtcctggctg tgtgccgagt ggcggggcac ccacagggag    540
atgagccagt gcctatctct tgcctcagtc tccccgtggt cgcctccttc ctcagaaggg    600
gctggcccag gctggtgcct ccgtggcccc tggagcatac ctgctgccgc cttccctggc    660
ctctggcgac agcaggcctt ccgtactcca cctgtccccc agccagcatg gctccacaca    720
gaacacggcc gcagacaacc ctcctgtgtc ctgcatagcg aactgccact ctgcgtgggc    780
cccactgcca ggttagggag gcaggagctg cccccacccc ctgcccggga gctgtcgtct    840
gtctttcgga tgagtcagga acaaatgcgt caacactggg tggccgccct cgccactcct    900
gcgcacgggc cctgtgggga gagccaggca gtgggtggcg tgggacaggt gatgcccatg    960
cccagtactc aggagacctg catggcagct cctaagatgg gtacaagcga tcataaggag   1020
ggatgtacgt gtctcatctc cccacccacc acgggatcag gttgaggccg tccagggagt   1080
gcatgggagg gctgcgctcg gcgcatctgc agagatcccc tgaagagggg ccctggctgc   1140
ctcccactgg ggtaggtgcg ggcccaggtc cccgacaccg tgacggtgct gtgggacttt   1200
gatggacctc acccggggag cccaggcctg ctccacggca aggtggccac gcactgcctc   1260
tgcagtcctg agcacaggct ccatacggga cctgaggcct gggctccatg acacttgggc   1320
tatgtgcttc cctggtgctt ccctcctggg gatgaaatca cacccatagg taaaggtttg   1380
ggagtggagg gagacagcag gagacaaagc aaggggacga ggactctggc agccacaaag   1440
gcagcgggag ggccctggag gctcctgttg ggcagcgcag tcaggaaagg gcccactagg   1500
```

tgggagaggc aggcgcctgg tgagtgctga gatctgcacc acgagaaggg ctgggctccc 1560

aaggcatgca tggggcctgg gagtggaggg agtgtgagcg ggtgttagga cggggtctgg 1620

ggcgagcggg tgttaggacg ggcctgggag cggagggagt gcgagcgggt gttaggacgg 1680

ggtctgggag tggagggagc gcgagcgggt gttaggacgg ggcctgggag tggagggagc 1740

gagagcgggt gttaggacgg ggtctgggag tggagggagc gcgagcgggt gttaggacgg 1800

ggtctgggag tggagggagc gcgagcgggt gttaggacgg ggtctgggag tggagggagc 1860

gagagcgggt gtcaggacgg ggtctgggag tggagggagc gcgagcgggt gtcaggacgg 1920

ggtctgggag tggagggagc gcgagcgggt gttaggacgg ggtctgggag tggagggagc 1980

gcgagcgggt gttaggacgg ggtctgggag tggagggagc gcgagcgggt gttaggacgg 2040

gtctgggagt ggagggagcg cgagcgggtg ttaggacggg tctgggagtg gagggagcgc 2100

gagcgggtgt taggacgggt ctgggagtgg agggagcgcg agcgggtgtt aggacggggt 2160

ctgggagtgg agggagcgcg agcgggtgtt aggacggggt ctggga 2206

<210> 45
<211> 2233
<212> DNA
<213> Homo Sapiens

<400> 45

ctgctggacg tggacgcaga cagcgggctc ctctacacca agcagcgcat cgaccgcgag 60

tccctgtgcc gccacaatgc caagtgccag ctgtccctcg aggtgttcgc caacgacaag 120

gagatctgca tgatcaaggt agagatccag gacatcaacg acaacgcgcc ctccttctcc 180

tcggaccaga tcgaaatgga catctcggag aacgctgctc cgggcacccg cttccccctc 240

accagcgcac atgaccccga cgccggcgag aatgggctcc gcacctacct gctcacgcgc 300

gacgatcacg gcctctttgg actggacgtt aagtcccgcg gcgacggcac caagttccca 360

gaactggtca tccagaaggc tctggaccgc gagcaacaga atcaccatac gctcgtgctg 420

actgccctgg acggtggcga gcctccacgt tccgccaccg tacagatcaa cgtgaaggtg 480

attgactcca acgacaacag cccggtcttc gaggcgccat cctacttggt ggaactgccc 540

gagaacgctc cgctgggtac agtggtcatc gatctgaacg ccaccgacgc cgatgaaggt 600

cccaatggtg aagtgctcta ctctttcagc agctacgtgc ctgaccgcgt gcgggagctc 660

ttctccatcg accccaagac cggcctaatc cgtgtgaagg gcaatctgga ctatgaggaa 720
aacgggatgc tggagattga cgtgcaggcc cgagacctgg ggcctaaccc tatcccagcc 780
cactgcaaag tcacggtcaa gctcatcgac cgcaacgaca atgcgccgtc catcggtttc 840
gtctccgtgc gccagggggc gctgagcgag gccgcccctc ccggcaccgt catcgccctg 900
gtgcgggtca ctgaccggga ctctggcaag aacggacagc tgcagtgtcg ggtcctaggc 960
ggaggaggga cgggcggcgg cgggggcctg ggcgggcccg ggggttccgt ccccttcaag 1020
cttgaggaga actacgacaa cttctacacg gtggtgactg accgcccgct ggaccgcgag 1080
acacaagacg agtacaacgt gaccatcgtg gcgcgggacg ggggctctcc tcccctcaac 1140
tccaccaagt cgttcgcgat caagattcta gacgagaacg acaacccgcc tcggttcacc 1200
aaagggctct acgtgcttca ggtgcacgag aacaacatcc cgggagagta cctgggctct 1260
gtgctcgccc aggatcccga cctgggccag aacggcaccg tatcctactc tatcctgccc 1320
tcgcacatcg gcgacgtgtc tatctacacc tatgtgtctg tgaatcccac gaacggggcc 1380
atctacgccc tgcgctcctt taacttcgag cagaccaagg cttttgagtt caaggtgctt 1440
gctaaggact cgggggcgcc cgcgcacttg gagagcaacg ccacggtgag ggtgacagtg 1500
ctagacgtga atgacaacgc gccagtgatc gtgctcccca cgctgcagaa cgacaccgcg 1560
gagctgcagg tgccgcgcaa cgctggcctg ggctatctgg tgagcactgt gcgcgcccta 1620
gacagcgact tcggcgagag cgggcgtctc acctacgaga tcgtggacgg caacgacgac 1680
cacctgtttg agatcgaccc gtccagcggc gagatccgca cgctgcaccc tttctgggag 1740
gacgtgacgc ccgtggtgga gctggtggtg aaggtgaccg accacggcaa gcctaccctg 1800
tccgcagtgg ccaagctcat catccgctcg gtgagcggat cccttcccga gggggtacca 1860
cgggtgaatg gcgagcagca ccactgggac atgtcgctgc cgctcatcgt gactctgagc 1920
actatctcca tcatcctcct agcggccatg atcaccatcg ccgtcaagtg caagcgcgag 1980
aacaaggaga tccgcactta caactgccgc atcgccgagt acagccaccc gcagctgggt 2040
gggggcaagg gcaagaagaa gaagatcaac aaaaatgata tcatgctggt gcagagcgaa 2100
gtggaggaga ggaacgccat gaacgtcatg aacgtggtga gcagcccctc cctggccacc 2160
tcccccatgt acttcgacta ccagacccgc ctgcccctca gctcgccccg gtcggaggtg 2220
atgtatctca aac 2233

<210> 46
<211> 2398
<212> DNA
<213> Homo Sapiens

<400> 46

actgtatttc tggccccaca gtcttctact gccgacttta ggtctctctg gatctcaggc 60
ccccttctct aagatgcatc ctagaggacc aaaaatacac tttatttggg cttcgcctgc 120
ttttgtggaa gggtagttta ctagaggata taatctcgtg ttttaatttg ctctctctcc 180
taaaggaaat gtggagaaaa aaaaaaagca gaaattggaa ataaccaata tttagtttat 240
ttcattcgat tcttagggga actggtgagg agcctaagat gattttccct tcctagagaa 300
agaatccaaa gtccagggaa atagcgacag gggagttcaa gactgcccct gctagtcctt 360
ccttggctac tctccgctgc gatcgcagga tagctctcat tagcaggaga atcgggcaag 420
tgtgtggata agtagagagt gtgttgaaca acttgtaacg ttttatgaaa tacgcattgt 480
catggttccc taaaaggctt tgcggaagcc gtttgtcttt actaatcaag tctttactta 540
cacaaaagta gaagtagaag tagttttaga aaacatacta acaatcttct atccccttga 600
agaccagagt agcagaaaac aggtgatttg cattataaaa ttgcactcac tttttcctcc 660
tttcagattt cacattacat tagcccattt gtgttacggt gtataaaaaa tggaacaggc 720
gcctccactg cattgttctc ctttaaaaat agatcactta caccctaact ttgttttcct 780
taaattcgat tcttaacagg agagctttct attatttcag atggagtgag gttgcacgac 840
tgggatggaa gaaaggaatc ccttaaattt gggggaattt ctgttctctg ttccaagacc 900
attttacttg gggtgtgggg gtgggcgcgg cggtcagggc agtggaacgc agtcgcggct 960
gcgccatccc tgcacttcca ggcgcgcggg agggaccggc ggggacgcga gctgcggact 1020
ctggcgaact cgggggaggc agacaggggg aggcggacac ccagccggca ggcgtctcag 1080
cctccccgca gccggcgggc ttttctcctg acagctccag gaaaggcaga ccccttcccc 1140
agccagccag gtaaggtaaa gactgctgtt gagcttgctg ttactgaggg cgcacagacc 1200
ctggggagac cgaagcttgc cactgcggga ttctgtgggg taacctgggt ctacggaagt 1260
ttcctgaaag aggggagaag ggtttgcatt tttcctatgg aggattcttc tctctctagc 1320
atttcgtttg atgtattcaa ctggtagaag tgagatttca acaggtagca gagagcgctc 1380
acgtggagga ggtttggggc gccgcggcgc cacccccacc cctcctcggg accgcgccta 1440

tttctaaagt tacacgtcga cgaactaacc tatgctttaa attcctcttt ccagccccgt 1500

gagtccgcgg cgacattggg ccgtggggtg gctgggaacg gtcccctcct ccggaaaaac 1560

cagagaacgg cttggagagc tgaaacgagc gtccgcgagc aggtccgtgc agaaccgggc 1620

ttcaggaccg ctgagctccg tagggcgtcc ttgggggacg ccaggtcgcc ggctcctctg 1680

ccctcgttga gatggacaac gcctcgttct cggagccctg gcccgccaac gcatcgggcc 1740

cggacccggc gctgagctgc tccaacgcgt cgactctggc gccgctgccg gcgccgctgg 1800

cggtggctgt accagttgtc tacgcggtga tctgcgccgt gggtctggcg ggcaactccg 1860

ccgtgctgta cgtgttgctg cgggcgcccc gcatgaagac cgtcaccaac ctgttcatcc 1920

tcaacctggc catcgccgac gagctcttca cgctggtgct gcccatcaac atcgccgact 1980

tcctgctgcg gcagtggccc ttcggggagc tcatgtgcaa gctcatcgtg gctatcgacc 2040

agtacaacac cttctccagc ctctacttcc tcaccgtcat gagcgccgac cgctacctgg 2100

tggtgttggc cactgcggag tcgcgccggg tggccggccg cacctacagc gccgcgcgcg 2160

cggtgagcct ggccgtgtgg gggatcgtca cactcgtcgt gctgcccttc gcagtcttcg 2220

cccggctaga cgacgagcag ggccggcgcc agtgcgtgct agtctttccg cagcccgagg 2280

ccttctggtg gcgcgcgagc cgcctctaca cgctcgtgct gggcttcgcc atccccgtgt 2340

ccaccatctg tgtcctctat accaccctgc tgtgccggct gcatgccatg cggctgga 2398

<210> 47
<211> 2114
<212> DNA
<213> Homo Sapiens

<400> 47

aaagttacag cagaagggaa gctgaagttt gaaaacacat tacattttgt cccttggttc 60

tcaaaccccc tcagttaaaa atctaggcaa aattattata tccaatacag taaccatctg 120

taaaacttca gaacagtaaa caaaggacat aaagaaatgt atcttcatct cccttaaatc 180

gaaaacaagc aaaacactac agaatcagat tttgctccat tatataggaa ttgtctggca 240

aagatccctc tttttttcct aaaggtgaaa tttcaggctg ctgaacattg aatgggtaaa 300

gatttttaca atgctgtatc taagtgagca ctaatgactt cacctactaa taacgactac 360

ttcttgggaa taagaattta ctaggaagca atttttaaaa ctttacaaag gatccaattg 420

gtccctacaa catggccagt agtcacatgt aaattctatc tggaagattg gggtgaaatt 480

aaggggttgg tgaaggaatc caggaaaatt aaaacattaa atccattttt aatagctgac 540

aatgctatgc ctcaagagaa gaaaaagagt atattctgct acagtgtgtg agaaacccaa 600

tttttgtttt tggatgatgg tggtatgtca cttcccttaa agttaggcac cagtgtgagt 660

agtgtcccag agaggtgaac gtgtagctgt gtcagacatt tatagcccca ttcttaaaag 720

acacatggga aaacagtcaa aagtcctccc ataactgcac caatttgacc aataggaaaa 780

caagtggaaa atgcagtttc accaaagcag agtagaggtt ccttttcatc aaaccctcag 840

gagggggagg ggagaacaca gaatggccag ggggtacttt ctggaagccg aagagagatt 900

aaatgatgac tcttacatcc cccccgctgt ttaagtgctg aaactaattc agatcccact 960

ttctccttca tgttaactct ggattccccg agtaatttgg agcctatgct ccaggtttcc 1020

tgaagattag aagctcaaga agatgggaat ttttagaaca ataactaggg gaaactgttt 1080

acttgaaggg ccaagggtag ggggctgcgt ttggtatttt actgagaccc cgtccattga 1140

aaacattgtt tctaacgaaa tcacccagca ccttgctcta tttgcgtggc tgttttcctt 1200

acagtggtgt aacttgtgtt agctgattgt ctttaggatt gttgtaaatc ttctgatgag 1260

cagcacttag aaggggcttt agtaaaaacc atgcgcccca aatttctaaa gcttcgtttg 1320

ggagagaggt tgacatttgc acatgaagtc tgtcttttgg aaacagcagg aggacgcagg 1380

aggggcgaag taaccccggg aagacgttct tccccctccc ttacggattc agtgtagttc 1440

ctggcataag caattctgtc accgggcggt cttttctaat tcgtgtgggg gctgatatta 1500

acataagcag ccgcctcttc gggtgcgagt gggatcccag gtccgggatg tggtgggtgc 1560

ggtgcgggag aggcacccgc cgccccttc actcggatgt tagaggcaag ttgttcgtgt 1620

gaactcgagc gaacgactgg aggttggctg ttgtaggttc actccttcag gatgtgggtt 1680

tcagggggcg ttttgatgtg atttaattcc gaccgtgaaa acaattgcta ggggctggct 1740

ggattgctgt tggtttttcc cctcctggtg gctttgcttg gtttgggttt agtttgggaa 1800

caaacttgtt ttatatcaac acttccttct acatcgggcg ccgtcgcctc aactgcgcgt 1860

ggggggcgag gcgcccaccc cgcacaagaa cagaggagcg ggggagaaga gacggcctcg 1920

ctgctcactg ggtaagcgga gcccagcccc gctcgggagc gggcaggaga gacagggtag 1980

tgaccccac tcacaccaga aaacccctcc ccccggcgat tttgctgcca actcgagtcg 2040

gctacagaca gtccagtgag tccttaatta caaggacgag agaaaagcac ggtgcccctc 2100

gagtcctctc cgcc 2114

<210> 48
<211> 2382
<212> DNA
<213> Homo Sapiens

<400> 48

aggccctgga ggtggccact gtcggtccag gcacggcttc gctcgggact actggctgcc 60

ctcgtggggt gccccgcctg gggttccctc ttaccctggg acgttccagg cgcgttcagc 120

ctgagctggt ggagagggcg ggggcggggg cggctggggt cccgaagtcc aggtccctct 180

tcccacttcc ccgccggccc tgccgctgcg gccctcgctc ccgcgctcgc tcgctctcga 240

gtctctccct ctctctcttc tcttcctctc tctctctctg cagtagtaac aacctgatcc 300

cgcttccccc acccgcctct tgagatgctc ctcacatccg cctgcacaca gcgcgtgcgg 360

cccctccgaa ggcgatcccc gcaaaccgcg acgcaggtcc tccgccccag gcaatccccg 420

ctgcgggaga ggccgcctcc tggcgcccac gccccccttc tgcagtcctt actgccggcc 480

gagggggaaa tgggcgacag gggaagggga ggtgtgtgcg ggacggacta ggctggggca 540

gaggggttta aactggcgcg gtcctacaga agtttgagga gggcggggcc ggctccgagc 600

ccccggagcc ctacgggact cccccgattc tactgaggag tccccgccag ctccgtgcac 660

tcctgcaaca ctccccaccc cacccgccag ctccgagttt acagcctctc gggtccgggg 720

attggctggg ggagggggacg ggggggaggg gaccccctgg ctgtagggaa cggcgtgcgg 780

gcgggggtgg gagggaaacg atttgcttca ggagataggg atgaaggttt tcctgagtca 840

agggagggag aagagaggtg gaacaaaagg cagatgctgg aggggaaggg gagctgggga 900

gctcgccccg agggctccgg cagcccgcgg tcccctgcct cagtctgtcc cagagggtga 960

ggtcaaggct ggtgccaggg ctcttcaccg gccacctgga tcccacgcgc ccagcaccca 1020

cttctctccg cacgcccact tcatgcacct ccccgcgccc ttcccacggc ttctctgcgg 1080

cgagtcgcct ttgcttcccc gcaggtcccc ggtcccagcg ctaaggcacc gcggcttctc 1140

tcgccttctc tccgcgttga acccgggctc tccgcgggga gaaataggtt gggggcgagg 1200

ggttcccgag ataattcagg acacctcccc ggggtctagc caggtaattc cgacgcccat 1260

ggatttccgg attacagttc ccaccgcggg ctcagtccta ctcttagttt atcccgcggt 1320

gagcgccaag ccccaaaagt cggagtgtca ccgtttggtg accccgcgtc cgcccgcgcc 1380

tcttaaccta tggtcatctg tctgtcacag cgaacctttc cctaggaaag aatctgccct 1440

ctgcaaagag gatgtgtaat gctggaaagc ccgcctttct ccccatcagc tggattctga 1500

gagttgggag agatgaccaa tgaacaggct gagggtgtca cagccctgct actttgggat 1560

ggggggtgca gtaggggagc tacagtcttc ccagaggcca ggtcattcta cccagctgca 1620

ccccatcccc aacctttcag tgggcgacac ctgcagggtg ttgggactgg gagaaacctc 1680

agatacctta aattccaaac cccattttcc agatgaagat actgaggccc agagtgggtc 1740

agtgagttgc ccaaggtcat aggacaggtc agaatggaag ctttccgact ctccccgggg 1800

cgctgggttc acagctccac cttgccaccc cctgccccgg aagtgagcac tgaagtcact 1860

taagagaggc cagtgaccaa aggcagcgat tcccaggtgc agaggctggg gcagacgtgc 1920

ccatgagctt gttcccccgc gcaccctccc ccacctcgtc cccattcctg tcttggttac 1980

catgtgcctc catcatcctg agattcagag aggtgcagcg gcttgtcgaa ggtcacacag 2040

cacactgagt agggccctcg tttccactgc ccagctgccc cctcccattg cccacccggg 2100

cctctgcaag cttgccagga tccccgggag cctgcttcct ccaatgcaca gagccgtggc 2160

gcgtgtcaaa gtgtgggaaa gttcctggga gagggaaggg gtagaaaata cagccgctca 2220

tctcaacctt ggacggctgc ggcaaggcag gggcgctcag aaaggcagcc agtcctgcgc 2280

accgccggtg cctccggcac accccactct gcgccagggg ctccccaggg tcgcgcgagc 2340

atgttcttcc agctcctcct cagcgaagca ggcgcggcgg gg 2382

<210> 49
<211> 2192
<212> DNA
<213> Homo Sapiens

<400> 49

ccatataatt tttaaaaaga aagaaacccc taaattacag atgtgcctgg aaccagaacc 60

cagaaagcat ctgggtaggg aaaatagttt ctagtgttca aggccaaagc cagtagggcc 120

tccttggatg ggaaagagat atgagactgc aagtaattcc tggacattac ctatttgtta 180

ttgtgttttc atcccttccc tcagtcacct acacgttttc tttttccagc taaaatgaat 240

ctaatctttg ttacagtact tcaccttgtt aataaaaccc aggactgaag aatgagaata 300

aaagggtttt atactcacca tttcttaatt tctgattcta gtctggacag aacaatcact 360
cattctagaa aattggcata gggagcattt attagcagtt gtaacatatc actgccaaag 420
ggagggattc tgtaaggcgg cactgggtgt ctcatatgta tgggaaaccc gaggccacgt 480
tcagtgccaa ccacaagcat tttcatctct ggccaggcaa gccttgatcc ctgaattttg 540
agctctcaac ctcttttaaa atgagaaaga gatggtttct tccagagtct ttcagcttct 600
aacttacagg caaacactac cgtatgctgt cccccagcag agattggttt taaccaaggc 660
aaataagtta ggatggactt tgttgaccgt gatggctcag tgctacatct catatatcct 720
accacctttc agaacattta atagtgctgc tcctggcagc cctggggttg tagtccttgt 780
agagaggagg aactggcatt gaaagaaata gttgagtttc actacgccac gtggggcgct 840
cccattgcta agagcaatct cccctccctg ggaagaaaag cgtgggcggg gccggggcag 900
tccctcccct ccgcgttgcc ttggagacca tgggcgtcca gctgcagttg tcgctggacc 960
ggccctggct gccttgagag gtgctttctg caggcgggga cgcggccagg ccggacgcaa 1020
ggcctccctc caatctaccg ggctcagggg gacggccagg ggctgctcag tctacttggc 1080
gggctaggga gggggatggc agaggctggt tgggtcttct cagcacacag taggcgccta 1140
attagcgtga cctgaatcag gttactctct ccctccaatt ctcctccctg tgtcccatgg 1200
agggtggatt agggtcccgg atgcgaattc tgcgccgcat tagagttcca gtcccaaccg 1260
acaccttgag cgccgttaac ttttccccga agagcatggc agagtgaagc acaagcaata 1320
atcctgtatt attcgcgttc ccagagtccc ttcggatttg cgccatgcgc ggcggggaga 1380
accggcctcc tgctcgagtt cagagctcat ctgaggttag tttcatcgtt tcgttgaaag 1440
ttaaaaccct aagtcgacgt tccccagccg cctacccccc agcagagagt ccttccccgg 1500
ggagtcctgc cctggggtgc cgcctcgagg ccagacgtcg tcccgcctgg acctgaacct 1560
gagtttggga cgggcgattt cccaacctca ggaattggaa ttgagacacc aaagcctaga 1620
cgcgtttcct ggacgacggc ttcccggcag gggcatccag ccagcggcca agatgtcgtc 1680
agtggggaag gtgacccagg ttccgaatgg gaaagcctac cagcagatct tccaggctga 1740
ggtaggagcc gccctctgtc ccgcttttct ccatccccct tcccttgctt tcttccaaac 1800
ttccctccct cccagggctt ccctactgga aattgaataa aacaagggct ttgaaattac 1860
tttgatttta aaaataattc ttgcttgttc ttaaatcgta ccaatacaaa attagaatgt 1920

cagttcttga aaattagaaa acttaaatta aaattttaaa tgtaaattac tcagaaatag 1980
cctggatgga accactgggt gacctctgtt tttcccatgc ataaaaacat gactaatttt 2040
tgtatttaaa aaataggttt cattctgtac tagtgttttg aaatctgctt tttcttcata 2100
acagtatctc acagaccctt ttctatgccc gtgggcgccc atgtatttca ccatactgag 2160
cagctcccca cttgttgacc taggactccc at 2192

<210> 50
<211> 2192
<212> DNA
<213> Homo Sapiens

<400> 50

ccatataatt tttaaaaaga aagaaacccc taaattacag atgtgcctgg aaccagaacc 60
cagaaagcat ctgggtaggg aaaatagttt ctagtgttca aggccaaagc cagtagggcc 120
tccttggatg ggaaagagat atgagactgc aagtaattcc tggacattac ctatttgtta 180
ttgtgttttc atcccttccc tcagtcacct acacgttttc tttttccagc taaaatgaat 240
ctaatctttg ttacagtact tcaccttgtt aataaaaccc aggactgaag aatgagaata 300
aaagggtttt atactcacca tttcttaatt tctgattcta gtctggacag aacaatcact 360
cattctagaa aattggcata gggagcattt attagcagtt gtaacatatc actgccaaag 420
ggagggattc tgtaaggcgg cactgggtgt ctcatatgta tgggaaaccc gaggccacgt 480
tcagtgccaa ccacaagcat tttcatctct ggccaggcaa gccttgatcc ctgaattttg 540
agctctcaac ctcttttaaa atgagaaaga gatggtttct tccagagtct ttcagcttct 600
aacttacagg caaacactac cgtatgctgt cccccagcag agattggttt taaccaaggc 660
aaataagtta ggatggactt tgttgaccgt gatggctcag tgctacatct catatatcct 720
accacctttc agaacattta atagtgctgc tcctggcagc cctggggttg tagtccttgt 780
agagaggagg aactggcatt gaaagaaata gttgagtttc actacgccac gtggggcgct 840
cccattgcta agagcaatct cccctccctg ggaagaaaag cgtgggcggg gccggggcag 900
tccctcccct ccgcgttgcc ttggagacca tgggcgtcca gctgcagttg tcgctggacc 960
ggccctggct gccttgagag gtgctttctg caggcgggga cgcggccagg ccggacgcaa 1020
ggcctccctc caatctaccg ggctcagggg gacggccagg ggctgctcag tctacttggc 1080
gggctaggga gggggatggc agaggctggt tgggtcttct cagcacacag taggcgccta 1140

attagcgtga cctgaatcag gttactctct ccctccaatt ctcctccctg tgtcccatgg 1200

agggtggatt agggtcccgg atgcgaattc tgcgccgcat tagagttcca gtcccaaccg 1260

acaccttgag cgccgttaac ttttccccga agagcatggc agagtgaagc acaagcaata 1320

atcctgtatt attcgcgttc ccagagtccc ttcggatttg cgccatgcgc ggcggggaga 1380

accggcctcc tgctcgagtt cagagctcat ctgaggttag tttcatcgtt tcgttgaaag 1440

ttaaaaccct aagtcgacgt tccccagccg cctacccccc agcagagagt ccttccccgg 1500

ggagtcctgc cctggggtgc cgcctcgagg ccagacgtcg tcccgcctgg acctgaacct 1560

gagtttggga cgggcgattt cccaacctca ggaattggaa ttgagacacc aaagcctaga 1620

cgcgtttcct ggacgacggc ttcccggcag gggcatccag ccagcggcca agatgtcgtc 1680

agtggggaag gtgacccagg ttccgaatgg gaaagcctac cagcagatct tccaggctga 1740

ggtaggagcc gccctctgtc ccgcttttct ccatccccct tcccttgctt tcttccaaac 1800

ttccctccct cccagggctt ccctactgga aattgaataa aacaagggct ttgaaattac 1860

tttgatttta aaaataattc ttgcttgttc ttaaatcgta ccaatacaaa attagaatgt 1920

cagttcttga aaattagaaa acttaaatta aaattttaaa tgtaaattac tcagaaatag 1980

cctggatgga accactgggt gacctctgtt tttcccatgc ataaaaacat gactaatttt 2040

tgtatttaaa aaataggttt cattctgtac tagtgttttg aaatctgctt tttcttcata 2100

acagtatctc acagaccctt ttctatgccc gtgggcgccc atgtatttca ccatactgag 2160

cagctcccca cttgttgacc taggactccc at 2192

<210> 51
<211> 2244
<212> DNA
<213> Homo Sapiens

<220>
<221> unsure
<222> (2126, 2128, 2131, 2132)
<223> unknown base

<400> 51

ttccattttc tctttctctt ccgctcttcc cctactttcc cttctccctt ttctttttct 60

ttcttactct ctccttgtcc ctgagctttc attgaccgac cccccccat ttcattcgcc 120

ctcccctcaa tgtgccaacc tttgccctat ttccgatctt cccaggtact gggaggcggg 180
atgggggtgt gcgttttcct ctaggagccc tgtctttcca agacccacag aaaccaggac 240
ctgcccttat tcaaaacccc atgcacttca agtctctttt agacaacaca tttcaatttt 300
ccgggctgac tagtctccct gtgcagaggc agttgagagg ctttgctctg cagagggaaa 360
agagctctct actctcccac ccaccatata ggcaaactta tttggtcatt ggctgaaggc 420
acagccttgc ccccgcgggg aaccggcggc caggatacaa cagcgctcct ggagcccatc 480
tctggccttg gcgttggcgc agggactttc tgaccgggct tgaggggctc gggccagctc 540
caatgtcact acctacagcg agggcagggt gtaaggttga gaaggtcaca ttcaccgctt 600
tgggaggacg tgggagaaga gactgaggtg gaaagcgctt tgccttgctc accggccgtc 660
cttgccccgg tcccagcgtt tgctgggatt tgccaggatt tgccggggct ccgggagacc 720
ctgagcactc gcaggaagag gtgctgagaa attaaaaatt caggttagtt aatgcatccc 780
tgccgccggc tgcaggctcc gcctttgcat taagcgggcg ctgattgtgc gcgcctggcg 840
accgcgggga ggactggcgg cccgcgggag gggacgggta gaggcgcggg ttacattgtt 900
ctggagccgg ctcggctctt tgtgcctcct ctagcggcca agctgcgagg tacagccctc 960
tattgttcta ggagcacaga aacctcctgt gtgggcggcg ggtgcgcgag ctagagggaa 1020
agatgcagta gttactgcga ctggcacgca gttgcgcgct tttgtgcgca cggaccccgc 1080
gcggtgtgcg tggcgactgc gctgccccta ggagcaagcc acgggcccag aggggcaaaa 1140
tgtccaggtc cccgctgggg aaggacacac tataccctat ggcaagccag ggtgggcgac 1200
ttcccatgga tcgggtggag gggggtatct ttcaggatcg gcgggcggtc taggggaaca 1260
attcgtggtg gcgatgattt gcatagcgcg ggtcttggga tgcgcgcggt tccgagccag 1320
cctcgcacag ctcgcttccg gagctgcgag ctcaggtttc cacccccgat cccccgggct 1380
ttcctcgcac cgctgagccc agcttgtggg gtgcactcga ccaacgcccg acagggctgg 1440
ggaatgtgac aggcagcagg ttcacccggg cttggggagg gggagtttcc gctttgacag 1500
cattttcctt tgccgtctgc tggtggattc ctattcccag tcggtaatcg ccccgcagtg 1560
ttgatctaag aaggtaaaga aaactaggtt tccctgcaaa gagcctcccc caaatcggcg 1620
gactccggat actttgagtg gatttagaaa tttatgtaat ctttctcctt tagtttattt 1680
ttcatcctct cctacagttt tctctgattt gctgttggtt cggggcaaga taaagcagcc 1740

agtagagagc gataataata gcggcgggaa atgaactgga gactggctga cagttcttaa 1800

cattttgtca tagatccccc cgaatgtccc aggctgtctc tggtgggttt tagtacccgc 1860

cggcttcttg ggcaccgggg accagaagga acttggcagc tggtcttagg ggtacagtta 1920

aaggcaggat gacagctatt ctcctgctca tctcagagcg ctgccgcccc ctcatgccgg 1980

tcgcgcaaag aacacagctt ttaaaaaaca cgtgccttct gcccatatag gtctgaaagt 2040

gatgaggaaa gtaatgcttc gcctattagc gagtttcagc ttttaaaatg atcccaagcg 2100

ttgctgagat gagaaagcgt ggcatncncc nngggggtcc tcagccccac ccgcgcccat 2160

ggtgcaagtc tgcagggaca ggcccgggac agcactgccc acgctgctag attttccgca 2220

gaggatcgct gaagctgcct tcgt 2244

<210> 52
<211> 2420
<212> DNA
<213> Homo Sapiens

<400> 52

tctctctcct cgggatgagc agggagagcg cgcggaggtt cccgactccc tcgactacaa 60

ccaagaaaga ataattttca aagtgttcaa catccccgcc cccaagctcc ccaaaacaca 120

ggggcaggga acaccaaaac actcggctct cattaggaag atcacggctc tgaaaggaaa 180

tagtagacac gatacttcat ctcatctgga tttatgacca aaaaaacaaa aacaaaaacc 240

caaagagttc gcttgcattt tttccttcca aatctcggtt cggctcgaag gcagggaatc 300

taaaagaccg aggccgatgg aagagagcca gcggggcgag cgagcgggca gcctcccttt 360

ttgcctcccg gagttaccca gaaggacagg ggaagggaag gaagaagagg cgaggaaaaa 420

gaggagggag ggaagcggag gccaggagcg acggagcaag gaaagcagtt tgcaagcgag 480

aaaagaggga aaaaacacag ccgcacgaat ccagagagat cacaagccgt acgcaagcag 540

cagcagaaag agcgagagcg cgagcgcgcg tcctctccgc ggtctggggc cagacagccc 600

ccagactagc ccgaatcacc ccccaagcac tgtctcgtcc tctctgctcc ggccgccccc 660

taattcccct ccttcctctc ctccacctcc tttccaaaaa ccaaaacaac acaagggagg 720

gtggcaaaag cctccccaaa ccggccgatt cactcaaaga caacaataat aataataaat 780

acataacaat ctatatccta tggtgggaga gacgtgggac taatcttcgg catttatttt 840

aacacctgac agctagaata aataaatata tacatttata tcaatagata cacatagaaa 900

```
acttggagcc aaagcatttg gcaagagcgg aaaaaaaaag aattaaaagg taaaataatg      960
atcatgagca gcggcggcgg cagcggcacc agcggcaaca gcggcggcgg cggcagtagc     1020
agcagcagcg gcggcagcaa cagcaataat cacctggtgt ccggcctttc ctagaaactt     1080
cttgcatcac cacttctaag aaccccagtt ctaagaatca acagagctca attctcggaa     1140
tttgagcttc ggactttacc actgctacgt ggcaggggag gacttggtgt cagctctccg     1200
agatttttac tgcccctggc caaccaaaag ccctcaaagc cacaagattt tttcactggc     1260
cggcatattt cgaggtcctc ataagcagag cgtctcggat ttggaggttc cggttcgagg     1320
ctcgaggggc ctgaaggtgg ctctccctcc ccgggcccaa gacgatggta tggcctgctc     1380
cgccaccatc acgtgggctc ctcctctgtg acgtcggcgc cttcgctgta gcaaagctcg     1440
gcctctggaa ttctgagaac taatttgcta ttcggtgaca taagaggggg agtgcgcttt     1500
gctttcccgg ggtctggggc taattccttc tttcttaccc ataaactcag cagatcgagc     1560
taaatgcaca aaagggagcg agaggtttga accactggga aaagtatgtt atatatatag     1620
tagggttaga gaggcgagta agagaaaaat aaaataaaat aaacatcaca gctctttcca     1680
actagaatat taggcaccac gagaaaaata tttgccaagc agttttcggt gggttcattt     1740
gctttatttt tatttaggac aggggttttt gctgttgttc tgggtttttt tctttctggt     1800
gtggtggctt gggattttg gtttctgtat tttgatggtt tatggatttt tgcttctgat      1860
tttttgcctt ttgcaagttt gtggtgttac gtaaatcaca ggatcggcat cggttggatt     1920
tttttgtacg tgccttttct ttccctatct aatccctcaa gcgttttaaa gatgtattat     1980
ttcaatacta atactattga aagaagctta aatttttggc catatgtaac aatcccagcc     2040
cccacttttt tttttttttt ttcctttggt gcaattttct ttttcccct tggacttttg      2100
ctgaagtgtg tctctcctgc acttcagaga aatgttcaaa ggatttgttt tggtttggtt     2160
tgtttctttc caggacagca agtggtgggt ttaatctgtt attgttgact cttgggaaat     2220
ttcttgttgc aagaaacgtg tgtgtggggg ggagggtggg ggtggcgggg tggtatgtgt     2280
gtgttttcta caaaattctg tgagccaaat acctgtttgt gttttgtttt ctcttaaggt     2340
cttgagattt ttgttttcga ggctcgtttc aaggtcgttg taaaaaaatc tcttcagtct     2400
gtgtttaaga gatcagccgg                                                 2420
```

<210> 53
<211> 2344
<212> DNA

<213> Homo Sapiens

<400> 53

gcctggcaca ctcaagactc ccacggaggt tcagttccac actcccctcc accctcccag 60

gctggtttct ccctgctgcc gacgcgtggg agcccagaga gcggcttccc gttcccgcgg 120

gatccctgga gaggtccgga gagccggccc ccgaaacgcg ccccccctccc ccctcccccc 180

tctcccccctt cctcttcgtc tctccggccc caccaccacc accgccacca cgccctcccc 240

caccaccccc cccaccccac accaccacca ccaccaccac caccaccacc ccgccggccg 300

gccccaggcc tcgacgccct gggtcccttc cggggtgggg cgggctgtcc cagggggggct 360

caccgccatt catgaagggg tggagcctgc ctgcccgtgg gcctttacaa gggcggctgg 420

ctggctggct ggctgtccgg gcaggcctcc tggctgcacc tgccgcagtg cacagtccgg 480

ctgaggtgca cgggagcccg ccggcctctc tctgcccgcg tccgtccgtg aaattgcggc 540

cggggctcac cgcgatggcc ctcccgacac cttcggacag caccctcccc gcggaagccc 600

ggggacgagg acggcgacgg agactcgttt ggaccccgag ccaaagcgag gccctgcgag 660

cctgctttga gcggaacccg tacccgggca tcgccaccag agaacggctg gcccaggcca 720

tcggcattcc ggagcccagg gtccagattt ggtttcagaa tgagaggtca cgccagctga 780

ggcagcaccg gcgggaatct cggccctggc ccgggagacg cggcccgcca gaaggccggc 840

gaaagcggac cgccgtcacc ggatcccaga ccgccctgct cctccgagcc tttgagaagg 900

atcgctttcc aggcatcgcc gcccgggagg agctggccag agagacgggc ctcccggagt 960

ccaggattca gatctggttt cagaatcgaa gggccaggcc ccgggacagg gtggcagggc 1020

gcccgcgcag gcaggcggcc tgtgcagcgc ggcccccggc gggggtcacc ctgctccctc 1080

gtgggtcgcc ttcgcccaca ccggcgcgtg gggaacgggg cttcccgcac cccacgtgcc 1140

ctgcgcgcct ggggctctcc cacagggggc tttcgtgagc caggcagcga gggccgcccc 1200

cgcgctgcag cccagccagg ccgcgccggc agaggggatc tcccaacctg ccccggcgcg 1260

cggggatttc gcctacgccg ccccggctcc tccggacggg gcgctctccc accctcaggc 1320

tcctcggtgg cctccgcacc cggggcaaaa gccgggagga ccgggacccg cagcgcgacg 1380

gcctgccggg cccctgcgcg gtggcacagc ctgggcccgc tcaagcgggg ccgcagggcc 1440

aaggggtgct tgcgccaccc acgtcccagg ggagtccgtg gtggggctgg ggccggggtc 1500

cccaggtcgc cggggcggcg tgggaacccc aagccggggc agctccacct ccccagcccg 1560

cgcccccgga cgcctccgcg cggcaggggc agatgcaagg catcccggcg ccctcccagg 1620

cgctccagga gccggcgccc tggtctgcac tcccctgcgg cctgctgctg gatgagctcc 1680

tggcgagccc ggagtttctg cagcaggcgc aacctctcct agaaacggag gccccggggg 1740

agctggaggc ctcggaagag gccgcctcgc tggaagcacc cctcagcgag gaagaatacc 1800

gggctctgct ggaggagctt taggacgcgg ggttgggacg gggtcgggtg gttcggggca 1860

gggcggtggc ctctctttcg cggggaacgc ctggctggct acggaggggc gtgtctccgc 1920

cccgcccccct ccaccgggct gaccggcctg ggattcctgc cttctaggcc taggcccggt 1980

gagagactcc acacagcgga gaactgccat tctttcctgg gcatcccggg gatcccagag 2040

ccggcccagg taccagcagg tgggccgcct actgcgcacg cgcgggtttg cgggcagccg 2100

cctgggctgt gggagcagcc cgggcagagc tctcctgcct ctccaccagc ccaccccgcc 2160

gcctgaccgc cccctcccca cccccacccc ccgcccccgg aaaacgcgtc gtcccctggg 2220

ctgggtggag acccccgtcc cgcgaaacac cgggccccgc gcagcgtccg ggcctgacac 2280

cgctccggcg gctcgcctcc tctgcgcccc cgcgccaccg tcgcccgccc gcccgggccc 2340

ctgc 2344

<210> 54
<211> 2366
<212> DNA
<213> Homo Sapiens

<400> 54

tctccttctt ctcctggcag aactgctcga tggccttgat catgcccagg cccatctcga 60

tgcagcagta ggcatggtcg gcccggggct cgggacagcc cgccacgcag tagtaacagt 120

ctcccagggt gctgattttc tcacacttgg tctcctcaca caggcggtcg aagcgaccga 180

acagatcgtt caggagaccc accagggcgt gggcagactt gttggcactc atcttggtga 240

agcccacgat atctgcaaat aaaatactga cttcttcgat ctgctgcatc ttaaaagggc 300

ggaaggctat aggagctttt tggatggaag actttttctt cctgttcttg gggctcgagg 360

tggcatgcct cttgacagaa ttctcactct cctcatctcc ctgcttcatt aagtcatcgg 420

ctatgattct tggcatcacg gaatgaatca tcctctcttt gagggctttt tccacttcca 480

ggtccttccc gtgcataatg gattgcccca ccttgaggaa ggtgctcctg gacctcacct 540

gggacatgac gaacaggtgg accccgatgg cgtggatgca gccgtggagc agccccctgc 600

tcagcagctc ccagtgcagg gccccggctc cgggcgaggg gaagcaggct tcatcccgga 660

aatggtagcc aaaggtctcg aaaaggacag agtaggccac ccccagacac aaactcaggt 720

acaaaggtaa gtgcatgacg gtatagagca aaaagagcac ttcgatgcac atggagaagc 780

tccccacttg agataagcaa gtatctgtgg gccgggctgt ggccgtaagg ttggagctgt 840

cgccgcgtcc tgagacaggc gtcaagacct ggaactgcgc agccagggtc agggcgaaca 900

ccagcagggt gagagccagc gaggtccacg cgtaatgccg ggcgtacagc ttggtgaagg 960

taaacagaaa gaagcccaca cacaccagga ggaagcacag cgcgggggcg accatgacga 1020

tcagtctgga tctcatgtgg accgcaaaat agatgctcca cagaaggcag gcgaagccga 1080

tgtagaagag cgcataccgg aaccggcgct gggtctgcgg gaagcagcgc tccaggcagg 1140

cctcctccag gttcaccgag tcgaacttgg ggtcccacca gcggctggag gccctctcga 1200

acagctgggg cagcttcttc tgcctgcgca gccggcctcc gccgcccact cgccggggga 1260

cgcccccgga gtccccagag ctgctgcagc tagaggagat gctgtatttg cagtgcttgg 1320

ggtggctgtt ggaggacagc tgcttggggt tgatcttgac gcgcacgctg ttgctgtccc 1380

cgctggagtc gcagctcacc tcggtgctgt ggtgatgcag cagctgctgg tggggtgggg 1440

aagccatgtt gtcgagtccc ggggcctgcc ccggccgggg tcaccagtac ctgccagcaa 1500

aacggggaga gttagcggcg ctcccaccta ggcatgcacg cctagaggcc cgggacctgc 1560

tcctgtccta aggggcggct ccagcacgcg acctggacag gcaccatctg ttcctgtggt 1620

tcccggctca gcggtgctcc caccgccccc accgccccca ccttcgaggc gcacgagaac 1680

cgctcgggac ggacctagaa cgcccggggg tccccgccgc gtggccgccg tggctccggg 1740

accgctttgc tcgctcgcct tccgcgcctc tcgccccgag ggtggcctcc gcgccgcgcg 1800

gcttctcctc ctcgcgcgct cgcctcctcc agctgcggct ccggagggaa gttcagacct 1860

tgagcgctcc cagccccgcg agccgcctgc gcacaaacaa ctccgctggc ggcgcggagc 1920

cctccatcct gcaggagccg cgctccgatg cgtcaaaggc ggcgcgcggc cggccccggg 1980

cccggacccc gacccggagc agcgagcttc ggcgggcgcc cccggctcgc gctccccggc 2040

cgcccccgc gctccgggcc ggccctgccc gcggcggcgg gcgctggggg tgggggcgcc 2100

ccgggctgcg agtgcgcgga gcccgtcggc gcggcccgtc tagtggggcc tgcttccccc 2160

cgagtgcgcc gccgctcccg ccgcggccgc agctgtcccg ggaccgagcg cgtggaacca 2220

cggacgcggg cccctcgccg ggcggcggtg cagacgctgc cgcagagccg ggctcccgcg 2280

acgccggccg ggacgcccgc ccgcccgcgc ccacgccggg ggccgcctcc ccgagctaga 2340

gatgcggccg ccgccgcgcc cgccgc 2366

<210> 55
<211> 2264
<212> DNA
<213> Homo Sapiens

<400> 55

gaccagcctg gccaacatgg taaaaccccg tctctactaa aaatacaaaa attagctggg 60

catggtggtg tgcgcctgta atcctagcta ctcgggaggc tgaagcagga gaatcgcgtg 120

aacccaggag gcgcaggttg cagtgggcca gagatcgcgc cattgttctc cagcccgggc 180

gactctcagg ttaaatcgtg ttcgatataa aagtctatat cgatatagac ttttcgatat 240

aaaagtctat ctccctttga caggtctgat cgaccctcct tcctataaag acaaaagttg 300

tcttaacatc taagaggaac catattgcgg tgaaaggggt gctacgaatc ctgggtttta 360

gctcttgcac tacgtctcag tgtgacattg gatcccaatt tcctcaccac caaagccaac 420

gggctattat aactcccttta gaagtagctt taatccctaa agacaagact aaaaatatta 480

acagtgcttg agcctctgga accgaaccag cactggagtt ttgtttggtc gtcccctctc 540

ttccccctca agattaacga tctggaggga attctggtga gttcgagttc tcgtaacttg 600

ttagggacta accagaagcc cggcgagcag ccagtaacac aatctctggg tattccttca 660

gcctcatcag tacatctctt cctacctcgc cttcctccgc tgcagcttct ttcaagtcca 720

catctgcgtc tccgtcagtt tcctctggga ttccatcggt gtcggtagcc cccgggtccc 780

cgggcacagc ggccgtctca tccgactccc ctgtctctcc acctttgtcg gagggcgact 840

cgtgggcatc gtccatcacg gataggattg gaggggagag gagatcgcgg agatgcctga 900

ggcagaagct cggaaacccg agctgcacag gccaggatct tacttcctgt cgtcgcgcag 960

cgatgacatc acccctaccg ctctcctgag ggtcattttt gaggcgcgcg tagaggacta 1020

gaggactcat tttaactttg gtttggcgac agtgggaagc gggaaaattg gacgtaaaga 1080

gctcagacta tggatcctcc tcgctaggaa accagcactt gtgaaggagc gcagttttgt 1140

ttactaaata actttttcct tttcgcctgt ctaaaaatgc aatgtcagtt aatcccttcg 1200

ttaacccctc agagtaaagt tgggggactg ggagaaaact cagttccgtt gtatggcggt 1260

gtcgtggaat gaggctcccc gggactggta aactttcagg caaggactca accttgtata 1320

tttcaactgg cccgaaaccg cgggcctgtg tgtacataga attgctagca tttcccctcc 1380

attccgcaag caccttaaca acaaaaagtc atatagctcg agcagaattt atccaattca 1440

tttattcatc cacaactatt gactgcctac ttcgatttag aaacagtgct agtaccaggg 1500

aacaaaacac acatagtttc tacccaatta aatgattttt ttcagttcgc cagtactgaa 1560

gattgaggtt tgtttttaca agtctgacac ttttctatca gtttggttaa tctcattaaa 1620

actaagagga aagcctaaag agaggaaggt gtctcctttg aacgtttcac tttaatgaac 1680

tagttcacgt actgggagga tgaagtggtg aggatatttc tgttttaaaa ttttcttctc 1740

ttccctcatt gagatacgag ggggagggcg ttttattcac ctgggtatga ctaggtcccc 1800

gcacctatta catatttaat ttatgcgtgc tgaaagaatg gacttggaaa tgtaggtgcg 1860

cgttacattt atctctttgt ttctacgctg cctggacgaa ttaccgtatt tcctgtccca 1920

ggagctccca aaacctttgg ggaaggagcg cacttcggga ggcgcgtaca ggtgggcctg 1980

tttgtccctt tccacctgca taatgggggc ccacaaggcg ctgtccccat gccccgagag 2040

gcgtgtctcg gcacagcaaa gtccacctgc gctggtccgc ggagccccgc ccagctaggt 2100

gagctagggg gcggggcgcg ggggcgcggt gacgcgtgac gccggatccc ggaagtgacg 2160

cgctcgtggg gaaaaggcag ggaggggggtg gtgtccccag ccggtttggg gggtgcgttg 2220

cccggagacg gaaagtttgg gagcccgagc aggctcggct gcag 2264

<210> 56
<211> 1334
<212> DNA
<213> Homo Sapiens

<400> 56

gggcgccttg actctccctc caccctgcct cctcgggctc cactcgtctg cccctggact 60

cccgtctcct cctgtcctcc ggcttcccag agctccctcc ttatggcagc agcttcccgc 120

gtctccggcg cagcttctca gcggacgacc ctctcgctcc ggggctgagc ccagtccctg 180

gatgttgctg aaactctcga gatcatgcgc gggtttggct gctgcttccc cgccgggtgc 240

cactgccacc gccgccgcct ctgctgccgc cgtccgcggg atgctcagta gcccgctgcc 300
cggcccccgc gatcctgtgt tcctcggaag ccgtttgctg ctgcagagtt gcacgaacta 360
gtcatggtgc tgtgggagtc cccgcggcag tgcagcagct ggacactttg cgagggcttt 420
tgctggctgc tgctgctgcc cgtcatgcta ctcatcgtag cccgcccggt gaagctcgct 480
gctttcccta cctccttaag tgactgccaa acgcccaccg gctggaattg ctctggtaag 540
tccagaaccc ccgtccccga ccctttaact ccgcagaaga acacgcgtat ccagcacaga 600
ccagcctacc ctagcgcgcc tcctcagccc ctcacctcct actgccctag acccctaata 660
ccacccacct ctatccagag aaacaagggg aactgttgca ggcccggggg tgaggggtgg 720
ttctgggatg ggcagaaagt gcaggtgtag caggaaacct ttgcatgctt gcgcttacat 780
tggagctgcg aggattttga gaaatattaa acgggatggt tttctgggtt cactgttttg 840
aaagagcacc aatcctaggg gaaacactga aacagaagct ttgtcatcat taaagaaaaa 900
agtcttacta ggatgaggaa gaaataactt tatgagaaag aatgagcgag aaagcaataa 960
atcaaatggt gactgcaggg gaatcgctga ttcctggcaa aggtgccatg aggtcgcact 1020
ggtctcccgt tgaagaccag gtcacacaga ttctagagga gctgggtttc aatagaattt 1080
ctctctctct ctctctctct ctctctctct atctctctct ctctctcatt cccttctctc 1140
ctaggcggca aaagacattg gttttgcagt ccagatatgc ccctctcttt gcttccctaa 1200
gcttcaaggt agtacagggg agttgagaaa aagaacactt tgcgggtctc ccaggccgga 1260
gtgggcatga ctgaggctgg tcaggctcca tgtaggcgag ccgagggcgg aacccgactt 1320
cagtgggcgc tgac 1334

<210> 57
<211> 2501
<212> DNA
<213> Homo Sapiens

<400> 57

tctttgaaaa ttgctaataa aaactcgctg gttttacggc tcagggggca tcacggaacc 60
tgcggacatg tgatgtctcc cctggacacc cggctttaaa atgtctctct tttgtactct 120
ttccctttat ttctcaggcc agccgacact tagggaaaat aggaaaggac ccacgtgaaa 180
tattgggggc tgaatttccc ccgataataa gtctcttaaa aaagactttt aaatgaaatt 240

tctttgtttt ttaactttta tttctgtttt gggggtacat gtgaaggttt gttacataag 300
taaactcgtg tcttcggtgt ttgttgtaca gaatatttca tcacccaggt attatgccga 360
gtacccaata gttctctttt ctgctcctct ccttcctccc atcctgcacc ctggagtcaa 420
ccacagtgtc tgttgtttcc ttgtttgtgt tataagttct catcatttag ctcccactta 480
caagtgagaa catccagtat ttggatttct gttcctgcat tagtttgcta aggataatag 540
cctctagctc catccatgtc ccacaaaaga catgatctag ttctttttaa tggctgcatt 600
aaatgaagtt ttaaagatac aacataaaca ccaacctctt ccccaccaca aaaatccctt 660
gctgaatttg attacactta aattaacgag ttttgtttca tgaaagactc cttggacaaa 720
cttgacagtt gatggaatag gagaagctgt ctgtcatgtc taaagccaac aagagatcaa 780
tatctagaat aaatggagat ctgcaaatca acagaaagta ggcagcaaag ccaaagaaaa 840
tagcctaagg cacagccact aaaaggaacg tgatcatgtc ctttgcaggg acatgggtgg 900
agctggaagc cgttagcctc agcaaactca cacaggaaca gaaaaccagc gagaccgcat 960
ggtctcactt ataagtggga gctgaacaat gagaacacat ggtcacatgg cggcgatcaa 1020
cacacactgg tgcctgttga gcggggtgct ggggagggag agtaccagga agaatagcta 1080
agggatactg ggcttaatac ctgggtgatg ggatgatctg tacagcaaac catcatggcg 1140
cacacaccta tgtaacaaac ctgcacatcc tctacatgta ccccagaact tcaaataaaa 1200
gttggacggc caggcgtggt ggctcacgcc tgtaatccca gcactcttgg gaagccgagg 1260
cgtgcagatc acctaaggtc aggagttcga gaccagcccg gccaacatgg tgaaaccccg 1320
tctctactaa aaatacaaaa atcagccaga tgtggcacgc cacctataat tccacctact 1380
cgggaggctg aagcagaatt gcttgaaccc gagagcggag gttgcagtga gccgccgaga 1440
tcgcgccact gcactccagc ctgggccaca gcgtgagact acgtcataaa ataaaataaa 1500
ataacacaaa ataaaataaa ataaaataaa ataaaataaa ataaaataaa ataaaataaa 1560
aaaataaaat aaaataaaat aaaataaaat aaagcaattt cctttcctct aagcggcctc 1620
cacccctctc ccctgccctg tgaagcgggt gtgcaagctc cgggatcgca gcggtcttag 1680
ggaatttccc cccgcgatgt cccggcgcgc cagttcgctg cgcacacttc gctgcggtcc 1740
tcttcctgct gtctgtttac tccctaggcc ccgctgggac ctgggaaaga gggaaaggct 1800
tccccggcca gctgcgcggc gactccgggg actccagggc gcccctctgc ggccgacgcc 1860

cggggtgcag cggccgccgg ggctggggcc ggcgggagtc cgcgggaccc tccagaagag 1920
cggccggcgc cgtgactcag cactggggcg gagcggggcg ggaccaccct tataaggctc 1980
ggaggccgcg aggcttcgct ggagtttcgc cgccgcagtc ttcgccacca gtgagtacgc 2040
gcggcccgcg tccccgggga tggggctcag agctcccagc atggggccaa cccgcagcat 2100
caggcccggg ctcccggcag gctcctcgcc cacctcgaga cccgggacgg gggcctaggg 2160
gacccaggac gtccccagtg ccgttagcgg ctttcagggg gcccggagcg cctcggggag 2220
ggatgggacc ccgggggcgg ggaggggggg cagactgcgc tcaccgcgcc ttggcatcct 2280
cccccgggct ccagcaaact tttctttgtt cgctgcagtg ccgccctaca ccgtggtcta 2340
tttcccagtt cgaggtagga gcatgtgtct ggcagggaag ggaggcaggg gctggggctg 2400
cagcccacag cccctcgccc acccggagag atccgaaccc ccttatccct ccgtcgtgtg 2460
gcttttaccc cgggcctcct tcctgttccc cgcctctccc g 2501

<210> 58
<211> 2327
<212> DNA
<213> Homo Sapiens

<400> 58

ccccccaccc cccccaccgc caccccttgcc tccgggccac tgcccctctc tgcaagcttt 60
gtccgctgga ctccgttgcc cttgcgcggc cgccccatca ctcacgcttg actggaggaa 120
ataaaattgg cgttggcttt ttaattccaa gcttcgtgct tgattggggg aggggttatt 180
taggtatttt ttttttcaaa cagaagaaat ttaggtacta attgtaaaca ccccgttgct 240
cttcttgaaa gtatgtatca tattagccga ggccttggag acacacggcc tattactcat 300
ttccagcctc taggcaaaga attctatgga gatccaccag taaaaccaga tttcttaagg 360
gaaattacca ggacgtcttc agactctctt caagggatat tactggttgt cgattacact 420
tgagcacact cttctctagc ccacccctac cattttattt tgggatcacc ctaaggaaat 480
tagccatttg cagaaagctt gaaatcccga aaggagaggc acaacctagg gtaaaagaga 540
ccgagtgaag cttttcagat tgattgtctc cactggggcc acttttcata aggtcgggag 600
tggagaggag tgtctgcaga agagagacta cctttaaagg ggctctcccg aactcaggag 660
gtgaggggga cttcctagag ttgtcaccac ctgtaggtgg atttgtcatc actatgtagt 720
gtctcctggg gagccagcct gttctcccca gagtccccct caggtactgt cccagagttc 780

aggggagcct ttagcgcttt ctccgtttgc cctgagggta ttgcatgtac aaaagctcct 840
tcacgcgcgc gcgcacacat agacatccag cgggtttgga gtgtacccct gtttatttgt 900
caccacgtgg taaaccccag atttggaagt catcactatg cgggcgcgca tcaggatcca 960
ggggagactg tgtggggttg ggactgcgca caaacacagg ggccgcacgg aagccttgca 1020
tattttaaag tgtaacctga gccttcgcgg tttcagcttc acttaaaaca tgcaaattct 1080
tgaaattgaa aaatctgaaa aacttccgaa gagttctatc tgaataaatc caaatccatt 1140
gggagtcgct ttgaggagac aaaacgcaca gcgatttggg gtgagggata tttgtgggga 1200
ggcaggacgt gctggattgg gtttccaggg tcaaggtgtc tctgggcctt cgacgatagc 1260
cttagcgcag agcagggaag tggcaccgct aggcagcaag ctcagttgct ctacttttgt 1320
gacccatgag ccagtcctga ggctctggct tcagggccta gtttccattt atgccgcgtt 1380
tttgagagtc taatactgtg tctggcacat ggtaggtgct cactgaatag tcgtggtatg 1440
aatgaatgaa cgaatgaatg aatgaatgaa tgaatgaata taagtttaat gggggaaacc 1500
cgggcctcct aataaaggta ggggctgggg gatacctagg ggcttcccca ggaggatttc 1560
tttttttcatc atcccacccc tgggagaaag gtccacgcag gatggtcgct tccccccttgc 1620
tgagagtttt gccttcagcc tatctgggcc gctggaaaag aggagaagaa taaacaagag 1680
acaagcaact actcccctac cggcgttccg tccttgtcct cactgccaaa tccactccaa 1740
agccgaggat ggtgagactg tgaagttgca aagaaacaca gagcccaccc ccttaaagaa 1800
ttacgatata tttaaagttt gcctctttca ggtttctctc cttggctcct gcccctttcc 1860
cctcccggct ccttgtcctt gactgaacct catgggacag agaacctcct gtcccccacg 1920
aggcaaggcg cgaacccgca gagatctggg gtgccctttg gttccctgcg ctgccctgga 1980
ggcgtccata gaggcctttg ccgccaagga cagcaattgt tttattttcg atggttgctc 2040
gccaggcctg cgggtcgcgg gcccacccag ccgtcgaact ttccagtcgt tatcagcgct 2100
gctcctaact taatggaata atgcaaatta tagcctgccc agctgacacg tccctgcgaa 2160
tgcgccgggg ctgagctctg gccagccgct ctctcgacgt cctggacggc cggagggaat 2220
gaagctctga attgtgacaa aagtgggggg ggggcacccc aaattctcaa agcaatgttc 2280
tttttttttt ctttttctt aagcaattga gccttaccaa atgtcgg 2327

<210> 59
<211> 2494
<212> DNA

<213> Homo Sapiens

<400> 59

```
tttctatgaa gttacaaagc tgcaatcccg ttctcatgtg cagcaccaca ggttttgctt      60
tcaaatatct gcaggtgcat cctcctgcac tcgttaaact aagggttttc cttttctgtg     120
ctagacccgc ttttacgacc acgctctcca cctgattcac agaaagggtt ctacgaccgt     180
caggtcaccg ccgcctttgt actttattgg ggagtcgaag gcctcagcgc tgctcgcgat     240
ttccctaagg tggtcgggaa ggtcccaacc acgttcttct gtaaaccaga taaggaaggc     300
ctggggattc cggccgcgga aaggcacaga ggaggtgaac gctccggatg cccaagtgac     360
gcacttgtag gcggattcgg aaactcgtgc tcttgagccg gaggcggagc ccccagactc     420
agaccgaccc cccagaaccg ggcggccttg cccgaggcat gaaggctttc taggcccgct     480
tctaaaagct gtgtacaatg gagactcctg gaataggcac atatctctac tgtaatcaga     540
acgagatgta acatctaaga cgcagggcag ggctctggca aagacacgct gggggaacgt     600
gaaagaaagg gccaaaacta cgcagctggt cctcagagac caaagactac gacccccagc     660
attcaatgca gtgaacccct aggacgttgc atgccgggag ctgtagtttc tcaccaccgc     720
caccccccgg cccccccgcc atctgaaagg gttctagggg atttgcaacc tctctcgtgt     780
gtttcttctt tccgagaagc gccgccacac gagaaagctg gccgcgaaag tcgtgctgga     840
atcacttcca acgaaacccc aggcatagat gggaaagggt gaagaacacg ttgccatggc     900
taccgtttcc ccggtcacgg aataaacgct ctctaggatc cggaagtagt tccgccgcga     960
cctctctaaa aggatggatg tgttctctgc ttacattcat tggacgtttt cccttagagg    1020
ccaaggccgc ccaggcaaag gggcggtccc acgtgtgagg ggcccgcgga gccatttgat    1080
tggagaaaag ctgcaaaccc tgaccaatcg gaaggagcca cgcttcgggc atcggtcacc    1140
gcacctggac agctccgatt ggtggacttc cgccccccctc acgaatcctc attgggtgcc    1200
gtgggtgcgt ggtgcggcgc gattggtggg ttcatgtttc ccgtcccccg cccgcgagaa    1260
gtgggggtga aaagcggccc gacctgcttg gggtgtagtg ggcggaccgc gcggctggag    1320
gtgtgaggat ccgaacccag gggtggaggc ggctcctgcg atcgaagggg acttgagact    1380
caccggccgc acgccatgag ggccctgtgg gtgctgggcc tctgctgcgt cctgctgacc    1440
```

ttcggtgagt gattctggag gagcagacgt ccccctcca cacacgcggc cgcttctcga 1500

aggtcctggg ggcgttgaac gtgggagggg ggatcccggg gcctgcggtg ggccaaggga 1560

cgtcaccatt cctcgaaaga gggcaaggga attacgttta ttctcttctt cctctggaaa 1620

taaaaaaaga gagcaacatc atctaactgc cctacagatc taatagtatc tcgcccggag 1680

gtctcagcga cctcggggtg gggcctctct gaggctctgg ctcccccggg agctgtgcga 1740

gcccctcccc cctccccgcc cggaggactt tttcggccac cgcaaccttc ggccatccca 1800

gctttcccgt cccctaattc cccagactcc gactcctcgc aagcccgtgt cccttcttag 1860

atgctggcct cgggagcgtg aggcctgggg ccagcggaat ggcagtgtca gcgtgtttga 1920

ttcagttttg ctggagttcg gttcttaaca tttctccccc ttctcatgcc tctgggctca 1980

atttcagttc ccctcgccag aggttgcata aatcctattt gcattctgac ctgtgctaac 2040

gaaggccgaa gttttggctt gatcagttgg gaggccttgg ctcggcctgc gcgcaacacg 2100

tgtgggtgta gaagaggcct ggtatcagtc tgcttaacgt tatttataaa tgatcggaga 2160

cacactgttt agaagtggtg actgcctccg tatttcagga tctactggag acctcagcat 2220

ctgaacttaa atagatgttc tgtcttggct ttacctttgg tatttaaacc agggaatgca 2280

ctctttcatc cccacctccc ttagttacca actgaatttt gcaaccccta cccttcccta 2340

tttgatcatc ctccttggag aagtgtgatg ttgaagagtt tgcccgtgtt aaatcttcag 2400

ggtcggtcag agctgacgat gaagttgatg tggatggtac agtagaagag gatctgggta 2460

aaagtagaga aggatcaagg acggatgatg aagt 2494

<210> 60
<211> 2497
<212> DNA
<213> Homo Sapiens

<400> 60

tgaagactgt gtggagtggg aggtctgtgc ctatttttct cattcattgg ggaatgggct 60

gagtggctgg gggcaaagtg ggactcaggg gctctgccca gtccttactg caggctaagc 120

cctcccttgc ggccaagagc cactgggccc tccacctccc tctggcttct acccgcagat 180

ctagatggtt tcatggtgac tcagaggagg atgcactcgg gcgtcccttt tgaggatggc 240

tgcagggacc atccagggac tgagccgagg tgcagataca gccacggatt tgggtgttgt 300

tagacacaga agggtgtgta gcaaagtgag ccccacgcat gcgtagactc ttccctcggc 360

ttctgctctc catggaaaca caactgatca catcatgatt gatcacggta gccctctagg 420
gctttctttc ttttcttttt tttttttcc agaagtcata aagggatata gaatatactc 480
aaagaccacc tcatgctttt agtggagagg caaaaaaggt tcgctccgga atggggaagc 540
ggctgcgccc tggacggaga ggggcgggga cttcgcgact gcaggcggag ggagggcggg 600
tgtcgctggc gcaggcggtg acagggagac accgccgcca ctgagtattc ctatgcaagt 660
ttcttcatct tcctgtgcat cagtgtttac actggggtaa tgataaatgc tgtgttgaaa 720
aattatttga tggggccatg gaaggaacgg aaggaacggc gtcctggccc gctcggggcc 780
cgcgcacgcc gccaccaagc cgcgggggcg ggtcggaggg gagagttgcg tcagccaggc 840
cgctgtcaga tgacgagccc ggggcgtgac ggggtggagc atccccaaaa aagtgcatgc 900
ctaggatccc gcccagtgta tccctgcgcg cggcgggccg ggctgggcag ctttataaac 960
agccgtggtg tgagcctcga agggaaccat cagcgcctcc tgtccacgga gctccaggtc 1020
tacaatggca gcggccgcca gccccgcgtt ccttctgtgc ctcccgcttc tgcacctgct 1080
gtctggctgg tcccgggcag gatgggtcgg tgagttcggg gatgtagcct aagcagggcg 1140
ggggccaaac ctgggaggtt gtggactgca gcgggtttca gaggagggga ggcttctgga 1200
aggaccggcg cgatctccct gaacgaacat cgcggtctcc ccgaacgtcg cggtccctcc 1260
gaacgtcgcg gtctccccga acatcgcggt gcccccgaac atcgctgtct ccccgaacat 1320
cgcgatctcc ccgaacatcg tgatctcccc agacatgccc agctgaaggc actcagttcc 1380
cctcggtggc tcctttccgc cgggtccgct tcctgcggct gctgcttgcc cctcaggcca 1440
ggaggtttct ggaaggaccg gtgctgtctc cccgaacatc gtggtctccc cgaacatcgc 1500
ggcctctccg aacatcgccc tctctccgag caacgcgatc tccccgaaca tcgcggtctc 1560
cccgaaaatc gcgatctccc cgaacattgc catctcaccg aacatcgcga tctcgccgaa 1620
catgcccggc tgaaggcact cagttcccct ccgcggctcc tttccgccgg gtctgattcc 1680
tgcggctgct gcttgccccg caggccagga ggcttctggt agcaccggcg cgatgccccc 1740
gaacatcgcg ttctacccca acatcgcgat ccctccgaac atcgtgatcc cccccgaaca 1800
tcgccgtccc cccgagtaac gcggtctccc cgaacatcgc ggtccccccg aacatcgcgg 1860
tacccccgaa catcgccgtc tccccgtaca ttgcgatccc ccgaaacatt gcgatctccc 1920
cgaacatcgc gatctcgccg aacatgcccg gctgaaggca ctcagttccc ctccgcggct 1980

cctttcctcc gggtccgctt cctgcggctg ctgcttgccc cataggccag gaggcttctg 2040

ggtggaccag cgcgatctcc ccgaatatcg cggtctaccc gaacatcgcg gcctccccga 2100

acatcgcggt ctccccgaac atcgcgatcc cccagaacat cgcggcctcc ccgaacatcg 2160

cggtctcccc gaacatcgcg atcccccaga acatcgcggt ctacccgaac atcgcggcct 2220

ccccgaacat cgcggtctcc ccgaacatcg cgatccccca gaacatcgcg gtctccccga 2280

acatcgctgt ctccccgaac gtgcctggct gaaggcactc agttcccctc cggggctcct 2340

ttccgccgag tccgcttcct gcagctgctg ctagcaccgc agtccagggg gagtgtcaaa 2400

gaaggctgaa aaggaattgc aggagggtgg agggaccaaa aggctacaga gggcaaggta 2460

gggcggggat ccctggtgca gacccgcagc cccactg 2497

<210> 61
<211> 2455
<212> DNA
<213> Homo Sapiens

<400> 61

ggaccctcca ttccggctgc agcttcccgc gcagtgagaa ggcagcggtc cctgagagcc 60

cagcagtgcc ctgggagcct gcagaacgca ggggcggatt ccgcgtcgca ctggccgtca 120

aggggcggcc gcggagggaa ggggtgggtc ggtgggtctg acagcgggtc tgcgtaggcg 180

gcagcgtctg tccctcccag cctctcgctc cgcgccatgg gcgggccccg ggctctgctg 240

gccgcactct gggcgctgga agccgccggg accgccgcgc ttcgcatcgg agccttcaac 300

attcagagct tcggtgacag caaagtgtcg gaccccgctt gcggcagcat catcgcgaag 360

gtggggcccg ggccggggcg gggcggcgtt taggggtgct gaccgcgctg acccccgcac 420

ccgccgctcc tccccagatc ctggctggct atgacctcgc gctggtgcag gaggtgcgag 480

acccagacct cagcgccgtg tccgcgctca tggagcagat caacaggtgt ggtgggcagg 540

gcccctcgtc gcgacccccc gccgggatct cgccccggcg cggcgccccg accctgagcg 600

ggcccctgtc tccacagcgt gtccgagcac gagtacagct ttgtgagcag ccagcccctg 660

ggccgggacc agtacaagga gatgtacctg ttcgtgtaca ggtgaggggc gggccgcagg 720

gaggggcgcg cggggccgca ggtcgggggc tcagcgggtc ctccccatct cctaggaaag 780

acgcggtgtc ggtcgtggac acctacctgt acccagaccc cgaggacgtc ttcagccgcg 840

agcccttcgt ggtcaagttc tcggcccccg gcaccggtga gcgggccccg cccctcccct 900
cccgccgagc tctgacgccc ccaccccttc ccgcagcagc acagaacctg gtgctgatcc 960
cgctgcacgc ggcgccgcat caagccgtgg cggagatcga cgcgctctac gacgtgtacc 1020
tggacgtgat cgacaagtgg ggcaccgacg taagcccacc cctcggtccc ggggtccctg 1080
caggcgcgcc ccgggggtct ggttcatgag ggcgggacct cgacggctcc tgcggcggct 1140
cagacacggc tccgcggcgc ccgcaggaca tgctgttcct gggcgacttc aacgccgact 1200
gcagctatgt gcgggcgcag gactgggccg ccatccgtct gaggagcagt gaggtcttca 1260
agtggctcat ccctgacagc gccgacacca cggtgggcaa ctcagactgc gcctacgacc 1320
gcattgtggc ctgtggcgcc cgcctgcgcc ggagcctgaa gccccagtcg gccaccgtgc 1380
acgacttcca ggaggaattc ggcctggacc agactcaggc gagtgggccg tggggcggtg 1440
ctggtcttgg gtccccaccg cccgggcgca cgcaggcagc agggagggtc cagcgcccaa 1500
ggcagggcca cctcagcttc ctccctgcac cagggctcgt ttccaaggac cctggagagc 1560
ccggcccacc ccatgtggag ctctgggcag gcagggtacc ttgcctttcc ctcaaagggc 1620
caatggtgac accctctgcc ccggcccctg cccctgcccc accgcaggca gcagcagggg 1680
tgggcaccca ggcctcaccg gcccctccca tccctgtggg tggggtgcac ttttcccctg 1740
aggttcactc tgtccccaca ggctcttgcc atcagcgacc actttccagt ggaggtgacc 1800
ctcaagttcc accgatgact cgaggcctgg ctggggcatg ccacctgcag accctggctc 1860
tgaggaatgg cccaacagtg gccccttcag ggtggcagcc acccttcagt gaggccccaa 1920
ggcagagtcg gctgggcgtg gaccaggggc catggacacg tgatgtgctg ctctgtacct 1980
ccgttcccca tctgtgggac gggctgcatc cagcgacctc atggggtgtt gtgagcccca 2040
tgaggggtgc agggggcact gcccggcaga tgtctgctca ataaatgagc tgctcccggt 2100
cgggccccac agcttggctc ccaggcctct gtccttcctc aggacccagc ctcagctcct 2160
taggggcagc catggcctgg gctgcccacc ccatgtcctg gtgggacgtg gctgcccccg 2220
gagcctgctg gccccagaag gcagctcagg gatgctcctt gctcatcctc tgtggaaact 2280
acaaacgctc ccaggagcct gtactgtgag cacagctggg acagggatgg cgccaacaca 2340
ccagcctcct gggccctcgg ggctctcaga acagctctcc ccaggcccac cccgcagccc 2400
ccattccagg gccctgcagg ggcccccagg cacttgtctg tctcaagagc ccggt 2455

<210> 62

<211> 2738
<212> DNA
<213> Homo Sapiens

<400> 62

```
tgtattgttg agggtcaccg accaccgaga atcaggttca gttctcagag gaagcgccag      60
aggagggga gggtgggcga gggtctcggg gtggtggggg tgccggcccc agcatgtgga     120
ggcgacagga tccgtaagac cccacgcgcc gccggggcag gaccgcacag ggccgccatt     180
cactcacttg gggcgtccct aatgggcact cgtgcattcc agctcgtgcg gtgagcgcac     240
gcacgccgcc gctgccgcgt tcacacggcc ccccaaagtc gggggagggg tcgtggagcg     300
aaggagctgt aggcagattt tctttctctt tccgagctgg gaaaagggcc agccccgtgc     360
cctgcacgcc tggctctgct ttgcagcttg ccggtgagcc aagagccggt agagcagagg     420
aggcgcacgg ttcgggtcgt gtcaccattt cttggcttgc tgacctggcc aggagctggg     480
gatggcggca gcaccgcgga ggtacgcttt gcgctgccct cacgctccct tccgaaagca     540
gtgcccccag ggggaaagtt cgcaatgcca tttgtctcgc cggcaaagtt ctgggatgtc     600
gggcatcttt tgaggagggg gaagacgcgg cacccttact gaggccggca ggccaggaag     660
agaagttctc cgggcgcagg gcgcagccca gctccgggga ccctctgggc ctcccgcgtg     720
ccggccggga tgaagcgcgc tttgcaccgg acccgggatt cgggggcggg ggctggcgct     780
ggcagcgtca gacgctgctt catttcccct cgaccctggg gaggacagca acaagtcctg     840
ccccagccat ttcatcaccc tgctagcacg tgcagattcc gggccctcgg agcccggcgc     900
ggacctcgcc ttgcctcgcg cttccctttg cacgtcccgt gccatcccaa gagtttgcgt     960
ggccctgggt gggcgcctgg ctcccggcga aacgcctggg gcgcgccgcg tgggtgcacg    1020
gtgtcctctc ccagtagctc ccggctgcgg gggcacatgc acttacctgt attctgcagg    1080
gacaggcggc ctttctgctg ggagctcctg tcttggggac cctcgggtgg gttggtggcg    1140
tcggtccccc gcgccgggtc aaaggtggcc aacaccaaag ccagggtcat gaactggccc    1200
agccgctcgg cacacatggt tcttggtatt aacctcccgt cgggagacct ggatcctttg    1260
tgctcccctc ttcctcctcc tcctcttcct ccttcgccgc ttcccctcct cctcccactc    1320
ttcctttttg ctcgcctttt ccctcctcct cgcggccgcg gctcggatag aggttaccca    1380
gcgccctccc gtagctctcc ggagagcatg tgaccaggcc gttagcagcg ccgcgagtgc    1440
```

ccggcaatgg cagggatggt gcctcaaata tccctggaag ctctggtgtc acttgaatga 1500

aggagtcgag caggtgttgt cccggcggct ggaccaatcc gagaccccgg ccccttttgac 1560

aacacggccg ggaggcgggg cctgcgctca actactacag gaggaagccg agcgccgctg 1620

ggagcgccgg acaaagttgc cgacctggcg gcggccacgc gtttgcgtgc gtgtgtatga 1680

gtgtgtgtga gcgcggccgg agatgcgtgc gcgtgtgcgg gtgctccagg gcaatggtcg 1740

aaaggactgt acaatataag agcgaatcca agctccagtt gctgttaaag ggggacggtg 1800

tgctctggga cagtgtccct ggggcccctg gctcgcgtca cacccacatc gggttgggaa 1860

agtttcctct ttccgccgtt tggaattcag tgagattccc agcccagagg acttgataaa 1920

ttgaagcagt ttcctaggca caatgatgct gtgggccaac tgagcacaca tgggaattcc 1980

tggccttgtg acactttgca ttaaaaagaa ccccacgatt gaagatagga gcagaagcac 2040

tagatacagt atttggtctg agcaactgcc ctatcttccc gctttggtgg gccgtgcaac 2100

acctggagtg tcgtttctcg tctttcccct aatccagacg cattccaggc tgggctgcag 2160

ggatccaggt gacagtcagg acttgcatca tttccaggag ggtggcaggg aggcccaagg 2220

cagccctgag tcagcccgcc gcggagcatc gcgtgcccgg aggccagagc ttgaaaggca 2280

actttacgcg tctccaaaca cactcctgca ttttctcaaa gcgctgctcg ccaccccgct 2340

ccgggttccc ttttactctc cagtcatccg atggaagtgg tgagaacaaa aacaaagaca 2400

aaaaagctaa atccggggaa gttatctcca tctcctccct tttcttgttc ggccccctcc 2460

ccaagaaccg ccgggtcggg atagcccccg gctactttct cacccggccc gtggagggaa 2520

ggagaaagtt gggcgccgct tggagcgcct cttcccctgc tgtgtgtgta cgtgtgcacg 2580

ctcacgtctc ccccgcgccg gggccgggga caggaactgc tctcggtccc gtcaccccac 2640

ggcgcagctc gggggctcg cagggctggg ggagggcggg gacacgagcg ccgcgcccaa 2700

gcgccccagg gcacgcaccc ggcgttggcg cgcactcg 2738

<210> 63
<211> 2401
<212> DNA
<213> Homo Sapiens

<400> 63

gcggcaggcg aggcggggtg caggtgtgag tggggtggga ggaaggggaa aggatcgggg 60

tcgccgcagc ctttctggtc agtttcgctg ggttttgtca gttccgtttg agaccgaggc 120
gcacggacta gcggccgcca gcacctcaca ggtggaggac acctcttgcg ccccaaggtc 180
aatgtcatcc cgatgggtcc cgtgccgtcc tgactctccc tgacctaatt ctttggttat 240
gactttctca ccttctgggc ttcgcctctg ccagctttcg ccgacgtcgc ccttcccgcc 300
gcccgcccgg gccttcctac ccgctctcct ccctccctgg cccgacggtc ccactgaggc 360
aaaaacaaac agccctgagg gcacttcaag gagcgtgcag tggccccgac ggtactctcg 420
gggggataaa agaggtggcc agccacgccg tcggcccccg gccgaataat gggcgcgcag 480
cagattgcca gggggcactt gaaactcgag gggagggaaa tacttgagcg cggtccctcc 540
ccgcgaggac gcacgacgca gcctcgcgga tcgtcccttg ttcgctctct ttcatgcact 600
ctccttttgt gtggaattat ctaaacggga gatccctggc agctttatgc taatgcgccg 660
aacaaaggca acggcccgcg ggcgggaggc ccggaaggtg cctggggccg ggccgggcgg 720
ggtggcggcc aggcgggctg cgcgtgtctc tcgcgtgccc ctgggactga cacaacaacc 780
cctcattcct gcaaatacaa agcctctcac cggctcgggt cgggcgcacc cggcgcaggg 840
aggcgctggt ccctccgggc ggcgggcact gcagcggtaa cgcacggttc ctgccggttg 900
tggctcgggg gactgagggc cgggagtggg ggcttctgtg aagccagaag agaagtctgc 960
agcagagtgg tgatgagggg cgctgacacc tcacccccaa cgccgacgtc tccaaacgcg 1020
ataccctctg cggggtgaga atgcgggccc gcccggctcc tcccgtgagg ccagggcctc 1080
ctgttctcct agacacccca aggagccaac tcctccgcag aagttccccg cttctgctct 1140
tatttccaag cttcgcgctt tctacaaact ccctgttgcc ttgactttga tttccagccg 1200
tggtgagggt cagagtgaac cccggcgcgc tccccgacgg catccccgca caccaggata 1260
ggagaaattg gagggcctgg ggcctcgggc tccgcagtcg tcggaggaag aacccaccgc 1320
ggggtccgca agggaaagtg aagaggcccg ggatttttcc aaagcgctgg ccaggacccc 1380
gaaggaaggg gaggagtcac ctgaagccgg ggaaggcccc ttgggtgctc tgccttggat 1440
ccttatgttc actgactttc gcgaagcccc cctggagggg ggcaaatccg cgctgtttcc 1500
cccaacttaa cttcacgcgg cccattctcc gaatcgcgtg cgccaaggcc ggcgtcgggg 1560
gcgcactgtg atggcgcttt gctcttccca ggcgaagaca aatatctcga cccgatgtcc 1620
ccatgcatgc gggtgcacag gttctgagaa aatcacgcag aaggcggctg cgatccacgg 1680

cccgggctgg gctacacctg cttggaggtc gctggtgtgt gcgtgcgcgc gtgcgcagcc 1740
taactccttc cagccccggg tggtgaaatt agtttccaat actagtacct ttcaagtttc 1800
catttatttt gactgtaccc gtctgaaagc agatttcatt tacgctagca agactggaag 1860
ccacaaataa agaaaaagaa acggattcat tgaatctcag tatctcagta gcgtttaata 1920
gattcccttg gctaaattca gagccctgtt tgaattcact cacgcaggag ttgttttgtt 1980
tttttgttg gtagaggtgc cggtttccca tccccacggc ttatttacaa ttatgggtgt 2040
tgttattttt aaaggattca caaggtgact aactctctgt tatctgttga atggaaggag 2100
tgttacatat gcaagaccga atggggcagg ccgagccccc gccctatgct attctacaat 2160
ccagatggga ttcgtgatta gaaaataaaa ttggccaaag ctgtctcgga atccttttcc 2220
cacttctaat ttcatcagca ctattaatca gagctcaaaa gagcaaggaa ataacatcag 2280
ttacttttag atagaaatat gtttataaat tttagataaa ctcattttaa gatttctaac 2340
ttttggggag actagcactc accacagcat ttttaaaact agggtgagac ttttcaaaat 2400
a 2401

<210> 64
<211> 2476
<212> DNA
<213> Homo Sapiens

<400> 64

aattactagg aggctggggg aaaagagggg aaaaacgggt aaggaaggtt gcaaatgtta 60
agatgccaaa caggaaccca accgaagtcc gaagtgaaat acagcctcct ctggcccaac 120
tgtcatgcaa acaacttcta agcatcttca gatgtaaaga aaaacagtca ttgtgcacag 180
gcgttagctg aatgttcgag agttggatga atggggaaga cttgttatac acctagtggt 240
gggtggattt cactaggctg cagcctccac acagcttttc tttgctgcac tgcagaggcg 300
ccatcttctc cgtcttaatc ttcactcaat tcgatccttt tatctgcacc ctaaaaaaaa 360
gacccttcgg accctcccca gcatcctgtg cccccagggg aactaaacca gagtccccag 420
agctgccagg gaagaggccg cgccttaatc agacaaacgc ctccttggat gcacaggaca 480
gagcccgtgc ctccccacca tccgccactg ccctttccag acgcacatcc cactctccgc 540
cttatttcct ccccgccaag gactttacag cccagcacag aattaagcct cggaggtgat 600
ccgagggtgg gaagcagagg ggccccgagg ctccgccaga agccccggca gcgtctctgc 660

cccctcccag gcggacgcgc agccccggga agggagccgg gcgggagcct cggtggcgca 720
ctgagactaa cgcggctcgg cccacggcga gggcggggtg tcgcggcttt tgttcccgcg 780
gaccttctgc ctctcagaca gagcttaggg tgcgagcgaa ctcccgggac gtccagactt 840
ggggaaggcg cggggtggct atgggtctcc gacccctccc atttctcgca agttacacaa 900
aagggagacg cggacatgca caaagtttgc ctgtggaagg cggtgcgcga ccccgacgcc 960
ggctcgggct agggtcgcac ccaggctgcc gccgccagca gccgcgaaaa gaggttggag 1020
caaggaaggg ggatgggggt gagagaggaa ggtgaaacga ggcggagaac gcagggaaaa 1080
gcgaggggct cctagcttcc tgggctgtcc cgactcctcc tcctcctccc cagggcccaa 1140
gagaaaggaa agggcaacga ttcaagagcg aaggactggc tttagggacg ctgcgttttc 1200
ggttttcccc acgtgctcct tccccttcc cccctgtctg aatgtctctc gcctcccccg 1260
acccccgctt ccaaggcagg gactaagtcg gggtctgggc tcagggtcgc cccctaccct 1320
tctcgccccg ggctggcgcg gaaccaggga gaccagcgcc tcgcccgctc cctctcagcg 1380
ggccggagcg cgactccctg gggcagggct gggccgaaag cggggatgcg ctggacgtcc 1440
gcaagcgggg gcggagagga gaggggtccc attgacggac ccccggcttc tctgcattaa 1500
agacttgggt taaggtcttg gggggtatcg cgtccccacc gtgcagcctc cccccgcctt 1560
ccgaggcgcg gcacccacct ccagcgcccg agccgtccag gcggccagca ggagcagtgc 1620
caaaccgggc agcatcgcga ccctgcgcgg ggcaccgagt gcgctgctgt gcgagtggga 1680
tccgccgcgt ccttgctctg cccgcgccgc caccgccgcc gtctcccggg gcccccgcgc 1740
acgctcctcc gcgtgctctc gcctaccgct gccgaggaaa ctgacggagc ccgagcgcgg 1800
cggcggggct cagagccagg cgagtcagct gatccggccc accccgctcg gcacccgaga 1860
gagaccccta gcggcgccgc cggggaactg cgcccgctcg cgccgggagg ggccctcgcg 1920
ccccgcgccc acaggtgcac gcgcccttgg cgccgcctgc accccacgcg ccccctccgc 1980
tccccggccg acggcccacc tgggcttcgt gaacagtggg agggagagtc tggggccagg 2040
agagggacgg tgcaggatca gggaaaggtg agtcctagga cgctgaggct ctagaaaagt 2100
cgagagcgct cctgctcgtc cccgtgagct tgaatcatcc gaccccgcag gcctcccggg 2160
ggtgtcgtat aaaggactgc tgctagggtg cgtttttatc cgcatttcgt tttttctctc 2220
ggtttggaga ggtggggcag gcgtttctgg aagagaatga gaacgagtga agcttaaagg 2280

aaataggatt ttctttgctt gttgagacag caaaacctca ttttttaacc cccaacgtca 2340

aaagcaggca cgagcaacct ggaatttcta tctttagaac gaaccaaagg agcaaggcgc 2400

aggacctggc aaagaagcgg ccaacccatt tacttcttct tgaaagcagg gttctgggct 2460

tgggttttgt ttcttt 2476

<210> 65
<211> 2229
<212> DNA
<213> Artificial Sequence

<220>
<223> chemically treated genomic DNA (Homo sapiens)

<400> 65

tttttttcg gcgttggttg gtgcgggttg gggttaggtg gagaagtcgt tttttgttaa 60

ggtgatagaa cgtgttgggg gtgggggtcg gggttagggt cggtgtaatt agggggtcgt 120

tgttttttt tggatatagt ggaagttttt ttcgtattat taaattttg ttattttttt 180

tgagggattt gtttttaggt agtacgtaag ttgttgtttc gggtttattt cgtattttt 240

tattgggtga ggaaggagta ttttgaatgg agatggggt gttttcggtt tatatatttg 300

tagagaagag gtgtgtcggg ttgtattttt ggaggtcgcg gtaattgata ttagagaaga 360

tttcggttgt agttgggaag gtttattggt tggaaagagg tgttttttt ttttagtaaa 420

gggttttgtt tggaagggtt gtttttttatt tgtttagtgg tattatagga cggtcggttt 480

ttattcgaat tttttcggac ggtattatta tatagtcggg ttttcgtagt gttggttttt 540

taattcgatg attgttattt cggtgaggat ttgtgttgat ggtcggagaa ttttgcgttg 600

cgggcgtata tggttaggtg gcgtttggta ggcgacgttc gggtgtagga cggcgttttt 660

atcgttttat tttaaatcgt tgtttgggtt taggtttttc ggttttttga ataggggttt 720

ggggggttaa ggacgttgag gtttcggggg taggaagttt tttttggtta agcgtttttt 780

tttttttcg gtatatattt ttttatttat ttatttcgtt tattttcggg gcgagaggtt 840

tattaaggta gggcgcgttt tttttatgaa ttattttaag gtttttgagt cgcgggggtt 900

tcgggtaatt atttttttt ttttttggtt ttaggtattt tagtttaggg gtttgtagag 960

aagttcgaag ttcggataaa cgcgtcggac gttaataatt ttttattttt ggtagtagta 1020

aaggttaata tatttttatt ttttatttta gtttgttatt aaaataaagt tgcgcgcggt 1080

tgagggtagg aaggcgttga gatcgagaag aagggacgtt tcggagaaag tgcgtttagt 1140

tgattttaga aattagagtt tttcgggatt tcgtcgagat tttttgtagg gcgttttaat 1200

ttgttttttt attgcgtgtc ggcgtcgtag cgcgtgcggt ttagggtttg gtgatttcgg 1260

tttagttcgg cggtcgcggc gaggtttttg gcgtagtcgt ttggaatttc gtattagaat 1320

cgggatcgcg taaatgtttt ggttgaagtg ttattttatt taagaaatat tgttgttagg 1380

aataaaatgg ggttttcggt gtttcgaagt atttttttgaa atttttttaa aataatttat 1440

aaaaaatgtt tttgttttaa cgttttataa cgtttaagga aatatgtaaa tggtttgttt 1500

ttttatcgag atggtcgttt taattaatag tgtatatata tataataatt tttttaattt 1560

tttttttag agttaagtat tttattatat gtaaattata ataaagaaaa gattgtgtaa 1620

gattatgtaa gtcgattgat ttaaaatatt gagttttaat ttaggttttt tgttttttta 1680

tttaataatt tttgtgtttg gattagattg gtgaagtagg ttatggaaat taataaagta 1740

aaaaattaaa agtatttttt ttcgttattt tttttttaa aattaaataa tagtcgtttt 1800

tttttgagta ggttttagtt ttaggttcga gttttttgc gattatttta tagttattta 1860

tagtagttgt tgttgttttt gtcgggtttt cgtttttgtt ttttttgggt cgttttttgt 1920

atataaaata tattttagtt ttttaattaa atttaaatac gatttcggta gaatttatat 1980

atttcgtggt gtatggattg tgtcggtgta ggggaaataa atattttttg gtatttaatt 2040

attgagttta attcgaaaaa tcgggattgg gtttttaggc ggtattttag gggttttaat 2100

ttggttcgcg ttttttttaga ttttggcgtt gagagcgttg tttttgcggg tgggtggacg 2160

gagaggtaat aatttgtttt taataaaaat ttgtcgttat cgaatcgaaa gcgaaaggga 2220

agggagaag 2229

<210> 66
<211> 2229
<212> DNA
<213> Artificial Sequence

<220>
<223> chemically treated genomic DNA (Homo sapiens)

<400> 66

tttttttttt tttttttcgtt ttcgattcgg tggcgatagg tttttgttga aagtagattg 60

ttatttttttc gtttatttat tcgtaaaagt agcgttttta gcgttaaggt ttggggaggc 120

gcgggttagg ttggagtttt tggggtgtcg tttaggggtt tagtttcgat ttttcgaatt 180
agatttagtg gttaaatatt agagggtatt tatttttttt gtatcgatat aatttatgta 240
ttacgaaatg tgtaaatttt gtcggggtcg tatttgaatt tagttagaga attggggtgt 300
gttttgtata taagagacga tttaaagaag gtagaaacga aaattcgata gaagtagtaa 360
tagttgttgt gggtgattgt ggggtgatcg taggaaaatt cgagtttggg attgaagttt 420
gtttaggaag gggcgattgt tgtttaattt tggagggagg gatggcgaag gaagatgttt 480
ttaatttttt attttgttaa tttttatagt ttgttttatt agtttggttt aaatataaaa 540
gttgttaaat agaaaaatag agggtttgga ttaaaattta atattttaag ttaatcgatt 600
tgtatgattt tgtataattt tttttttatt ataatttata tatagtgaag tgtttagttt 660
tgaggaggaa agttggaaga attgttatgt atgtgtatat tgttagttag gacgattatt 720
tcgataaaga aatagattat ttatatgttt ttttaaacgt tgtaaaacgt taaagtaaaa 780
atattttttg taagttgttt taagaaaatt ttagaagata tttcggagta tcggggattt 840
tattttgttt ttgatagtag tgttttttga atagggtgat attttagtta gggtatttgc 900
gcggtttcga ttttaatgcg aagttttaag cggttgcgtt aggaatttcg tcgcgatcgt 960
cgggttaagt cggagttatt aagttttgag tcgtacgcgt tgcgacgtcg gtacgtagta 1020
ggaaaataga ttaaaacgtt ttatagaaaa tttcggcgaa gtttcggagg attttggttt 1080
ttaagattag ttgggcgtat ttttttcggg acgttttttt ttttcggttt tagcgttttt 1140
ttgtttttag tcgcgcgtag ttttgttttg gtggtaaatt gaaataagaa atggaaatat 1200
attggttttt gttgttgtta gggatgagag gttgttgacg ttcggcgcgt ttgttcgggt 1260
ttcgggtttt tttgtagatt tttggattgg ggtgtttgag gttaggagag gagggggata 1320
gttgttcgga gttttcgcgg tttagaggtt ttgggatgat ttatgggggg ggcgcgtttt 1380
gttttggtga gtttttcgtt tcgagggtag gcgaggtggg tgggtagggg agtgtatgtc 1440
ggagagaaga gagaacgttt aattagagag aatttttttgt tttcggagtt ttagcgtttt 1500
tagtttttta aatttttgtt taggaagtcg aaggatttag gtttaggtaa cggtttgggg 1560
tggggcggta agagcgtcgt tttgtattcg gacgtcgttt gttaggcgtt atttggttat 1620
gtgcgttcgt agcgtagggt ttttcggtta ttagtatagg tttttatcga ggtgatagtt 1680
atcggattgg gaaattaata ttgcgaggat tcggttatgt gatgatatcg ttcgggggaa 1740

ttcgagtgga agtcgatcgt tttgtggtgt tattagatag gtgagaagta gtttttttaa 1800

atagggtttt ttgttggaag gaggaggtat tttttttag ttagtgagtt tttttagttg 1860

taatcggggt ttttttaat attagttatc gcggttttta gaggtgtagt tcggtatatt 1920

tttttttgt agatgtataa atcggggata tttttatttt tatttaagat gttttttttt 1980

tatttagtag aggggtgcgg agtaaattcg ggataataat ttgcgtgttg tttggaagta 2040

ggtttttag aaaggatgat aaaaatttgg tgatgcggaa gaagttttta ttgtgtttag 2100

gaaagggtag cggtttttta gttgtatcgg ttttggtttc ggtttttatt tttagtacgt 2160

tttgttattt taataaagag cggttttttt atttgatttt aattcgtatt agttagcgtc 2220

gaggaaaga 2229

<210> 67
<211> 2229
<212> DNA
<213> Artificial Sequence

<220>
<223> chemically treated genomic DNA (Homo sapiens)

<400> 67

ttttttttg gtgttggttg gtgtgggttg gggttaggtg gagaagttgt tttttgttaa 60

ggtgatagaa tgtgttgggg gtgggggttg gggttagggt tggtgtaatt agggggttgt 120

tgttttttt tggatatagt ggaagttttt tttgtattat taaatttttg ttattttttt 180

tgagggattt gtttttaggt agtatgtaag ttgttgtttt gggtttattt tgtatttttt 240

tattgggtga ggaaggagta ttttgaatgg agatgggggt gtttttggtt tatatatttg 300

tagagaagag gtgtgttggg ttgtattttt ggaggttgtg gtaattgata ttagagaaga 360

ttttggttgt agttgggaag gtttattggt tggaaagagg tgtttttttt ttttagtaaa 420

gggttttgtt tggaagggtt gtttttattt tgtttagtgg tattatagga tggttggttt 480

ttatttgaat ttttttggat ggtattatta tatagttggg tttttgtagt gttggttttt 540

taatttgatg attgttattt tggtgaggat ttgtgttgat ggttggagaa ttttgtgttg 600

tgggtgtata tggttaggtg gtgtttggta ggtgatgttt gggtgtagga tggtgttttt 660

attgttttat tttaaattgt tgtttgggtt taggtttttt ggtttttga ataggggttt 720

ggggggttaa ggatgttgag gttttggggg taggaagttt tttttggtta agtgtttttt 780

tttttttttg gtatatattt ttttatttat ttattttgtt tatttttggg gtgagaggtt 840

tattaaggta gggtgtgttt tttttatgaa ttattttaag gtttttgagt tgtgggggtt 900

ttgggtaatt attttttttt ttttttggtt ttaggtattt tagtttaggg gtttgtagag 960

aagtttgaag tttggataaa tgtgttggat gttaataatt ttttattttt ggtagtagta 1020

aaggttaata tatttttatt ttttatttta gtttgttatt aaaataaagt tgtgtgtggt 1080

tgagggtagg aaggtgttga gattgagaag aagggatgtt ttggagaaag tgtgtttagt 1140

tgattttaga aattagagtt ttttgggatt ttgttgagat tttttgtagg gtgttttaat 1200

ttgttttttt attgtgtgtt ggtgttgtag tgtgtgtggt ttagggtttg gtgattttgg 1260

tttagtttgg tggttgtggt gaggtttttg gtgtagttgt ttggaatttt gtattagaat 1320

tgggattgtg taaatgtttt ggttgaagtg ttattttatt taagaaatat tgttgttagg 1380

aataaaatgg ggtttttggt gttttgaagt atttttttgaa attttttttaa aataatttat 1440

aaaaaatgtt tttgttttaa tgttttataa tgtttaagga aatatgtaaa tggtttgttt 1500

ttttattgag atggttgttt taattaatag tgtatatata tataataatt tttttaattt 1560

ttttttttag agttaagtat tttattatat gtaaattata ataaagaaaa gattgtgtaa 1620

gattatgtaa gttgattgat ttaaaatatt gagttttaat ttaggttttt tgttttttta 1680

tttaataatt tttgtgtttg gattagattg gtgaagtagg ttatggaaat taataaagta 1740

aaaaattaaa agtatttttt tttgttattt ttttttttaa aattaaataa tagttgtttt 1800

tttttgagta ggttttagtt ttaggtttga gtttttttgt gattatttta tagttattta 1860

tagtagttgt tgttgttttt gttgggtttt tgtttttgtt ttttttgggt tgttttttgt 1920

atataaaata tattttagtt ttttaattaa atttaaatat gattttggta gaatttatat 1980

attttgtggt gtatggattg tgttggtgta ggggaaataa atattttttg gtatttaatt 2040

attgagttta atttgaaaaa ttgggattgg gtttttaggt ggtattttag gggttttaat 2100

ttggtttgtg tttttttaga ttttggtgtt gagagtgttg tttttgtggg tgggtggatg 2160

gagaggtaat aatttgtttt taataaaaat ttgttgttat tgaattgaaa gtgaaaggga 2220

agggagaag 2229

<210> 68
<211> 2229
<212> DNA
<213> Artificial Sequence

<220>
<223> chemically treated genomic DNA (Homo sapiens)

<400> 68

```
tttttttttt ttttttgtt tttgatttgg tggtgatagg tttttgttga aagtagattg 60
ttattttttt gtttatttat ttgtaaaagt agtgttttta gtgttaaggt ttggggaggt 120
gtgggttagg ttggagtttt tggggtgttg tttaggggtt tagttttgat tttttgaatt 180
agatttagtg gttaaatatt agagggtatt tatttttttt gtattgatat aatttatgta 240
ttatgaaatg tgtaaatttt gttggggttg tatttgaatt tagttagaga attggggtgt 300
gttttgtata taagagatga tttaaagaag gtagaaatga aaatttgata gaagtagtaa 360
tagttgttgt gggtgattgt ggggtgattg taggaaaatt tgagtttggg attgaagttt 420
gtttaggaag gggtgattgt tgtttaattt tggagggagg gatggtgaag gaagatgttt 480
ttaatttttt attttgttaa tttttatagt ttgttttatt agtttggttt aaatataaaa 540
gttgttaaat agaaaaatag agggtttgga ttaaaattta atattttaag ttaattgatt 600
tgtatgattt tgtataattt ttttttatt ataatttata tatagtgaag tgtttagttt 660
tgaggaggaa agttggaaga attgttatgt atgtgtatat tgttagttag gatgattatt 720
ttgataaaga aatagattat ttatatgttt ttttaaatgt tgtaaaatgt taaagtaaaa 780
atattttttg taagttgttt taagaaaatt ttagaagata ttttggagta ttggggattt 840
tattttgttt ttgatagtag tgttttttga atagggtgat attttagtta gggtatttgt 900
gtggttttga ttttaatgtg aagttttaag tggttgtgtt aggaattttg ttgtgattgt 960
tgggttaagt tggagttatt aagttttgag ttgtatgtgt tgtgatgttg gtatgtagta 1020
ggaaaataga ttaaaatgtt ttatagaaaa ttttggtgaa gttttggagg attttggttt 1080
ttaagattag ttgggtgtat tttttttggg atgttttttt tttttggttt tagtgttttt 1140
ttgtttttag ttgtgtgtag ttttgttttg gtggtaaatt gaaataagaa atggaaatat 1200
attggttttt gttgttgtta gggatgagag gttgttgatg tttggtgtgt ttgtttgggt 1260
tttgggtttt tttgtagatt tttggattgg ggtgtttgag gttaggagag gagggggata 1320
gttgtttgga gtttttgtgg tttagaggtt ttgggatgat ttatgggggg ggtgtgtttt 1380
gttttggtga gtttttgttt ttgagggtag gtgaggtggg tgggtagggg agtgtatgtt 1440
```

ggagagaaga gagaatgttt aattagagag aatttttgt ttttggagtt ttagtgtttt 1500
tagtttttta aatttttgtt taggaagttg aaggatttag gtttaggtaa tggtttgggg 1560
tggggtggta agagtgttgt tttgtatttg gatgttgttt gttaggtgtt atttggttat 1620
gtgtgtttgt agtgtagggt tttttggtta ttagtatagg tttttattga ggtgatagtt 1680
attggattgg gaaattaata ttgtgaggat ttggttatgt gatgatattg tttgggggaa 1740
tttgagtgga agttgattgt tttgtggtgt tattagatag gtgagaagta gtttttttaa 1800
atagggtttt ttgttggaag gaggaggtat tttttttag ttagtgagtt tttttagttg 1860
taattggggt tttttttaat attagttatt gtggttttta gaggtgtagt ttggtatatt 1920
ttttttttgt agatgtataa attggggata tttttatttt tatttaagat gttttttttt 1980
tatttagtag aggggtgtgg agtaaatttg ggataataat ttgtgtgttg tttggaagta 2040
ggttttttag aaaggatgat aaaaatttgg tgatgtggaa gaagtttta ttgtgtttag 2100
gaaagggtag tggtttttta gttgtattgg ttttggtttt ggtttttatt tttagtatgt 2160
tttgttattt taataaagag tggttttttt atttgatttt aatttgtatt agttagtgtt 2220
gaggaaaga 2229

<210> 69
<211> 19
<212> DNA
<213> Artificial Sequence

<220>
<223> Detection primer for

<400> 69
ctcctccttc caacaaaaa 19

<210> 70
<211> 22
<212> DNA
<213> Artificial Sequence

<220>
<223> Detection primer for

<400> 70
gtttagaggt tttgggatga tt 22

<210> 71
<211> 25
<212> DNA
<213> Artificial Sequence

<220>

<223> Detection primer for

<400> 71
tgaatagggt gatattttag ttagg 25

<210> 72
<211> 22
<212> DNA
<213> Artificial Sequence

<220>
<223> Detection primer for

<400> 72
ataaatcatc ccaaaacctc ta 22

<210> 73
<211> 16
<212> DNA
<213> Artificial Sequence

<220>
<223> chemically treated genomic DNA (Homo sapiens)

<400> 73
gacgtcggta cgtagt 16

<210> 74
<211> 18
<212> DNA
<213> Artificial Sequence

<220>
<223> chemically treated genomic DNA (Homo sapiens)

<400> 74
gatgttggta tgtagtag 18

<210> 75
<211> 17
<212> DNA
<213> Artificial Sequence

<220>
<223> chemically treated genomic DNA (Homo sapiens)

<400> 75
ttcgggggaa ttcgagt 17

<210> 76
<211> 17
<212> DNA
<213> Artificial Sequence

<220>
<223> chemically treated genomic DNA (Homo sapiens)

<400> 76

tttgggggaa tttgagt 17

<210> 77
<211> 16
<212> DNA
<213> Artificial Sequence

<220>
<223> chemically treated genomic DNA (Homo sapiens)

<400> 77
tattgcgagg attcgg 16

<210> 78
<211> 16
<212> DNA
<213> Artificial Sequence

<220>
<223> chemically treated genomic DNA (Homo sapiens)

<400> 78
attgtgagga tttggt 16

<210> 79
<211> 16
<212> DNA
<213> Artificial Sequence

<220>
<223> chemically treated genomic DNA (Homo sapiens)

<400> 79
gtgcgttcgt agcgta 16

<210> 80
<211> 17
<212> DNA
<213> Artificial Sequence

<220>
<223> chemically treated genomic DNA (Homo sapiens)

<400> 80
tgtgtttgta gtgtagg 17

<210> 81
<211> 16
<212> DNA
<213> Artificial Sequence

<220>
<223> chemically treated genomic DNA (Homo sapiens)

<400> 81
ggacgtcgtt tgttag 16

<210> 82

<211> 17
<212> DNA
<213> Artificial Sequence

<220>
<223> chemically treated genomic DNA (Homo sapiens)

<400> 82
ggatgttgtt tgttagg 17

<210> 83
<211> 16
<212> DNA
<213> Artificial Sequence

<220>
<223> chemically treated genomic DNA (Homo sapiens)

<400> 83
agagcgtcgt tttgta 16

<210> 84
<211> 17
<212> DNA
<213> Artificial Sequence

<220>
<223> chemically treated genomic DNA (Homo sapiens)

<400> 84
agagtgttgt tttgtat 17

<210> 85
<211> 17
<212> DNA
<213> Artificial Sequence

<220>
<223> chemically treated genomic DNA (Homo sapiens)

<400> 85
tttcgagggt aggcgag 17

<210> 86
<211> 17
<212> DNA
<213> Artificial Sequence

<220>
<223> chemically treated genomic DNA (Homo sapiens)

<400> 86
ttttgagggt aggtgag 17

<210> 87
<211> 18
<212> DNA
<213> Artificial Sequence

<220>
<223> chemically treated genomic DNA (Homo sapiens)

<400> 87
tttcgatttt aatgcgaa 18

<210> 88
<211> 19
<212> DNA
<213> Artificial Sequence

<220>
<223> chemically treated genomic DNA (Homo sapiens)

<400> 88
tttgatttta atgtgaagt 19

<210> 89
<211> 16
<212> DNA
<213> Artificial Sequence

<220>
<223> chemically treated genomic DNA (Homo sapiens)

<400> 89
aggaatttcg tcgcga 16

<210> 90
<211> 17
<212> DNA
<213> Artificial Sequence

<220>
<223> chemically treated genomic DNA (Homo sapiens)

<400> 90
aggaattttg ttgtgat 17

<210> 91
<211> 16
<212> DNA
<213> Artificial Sequence

<220>
<223> chemically treated genomic DNA (Homo sapiens)

<400> 91
tttgagtcgt acgcgt 16

<210> 92
<211> 17
<212> DNA
<213> Artificial Sequence

<220>
<223> chemically treated genomic DNA (Homo sapiens)

<400> 92
ttttgagttg tatgtgt 17

<210> 93
<211> 22
<212> DNA
<213> Artificial Sequence

<220>
<223> bisulfite treated nucleic acid for analysis of methylation status of SEQ ID NO: 5

<400> 93
ggtattatag gacggtcggt tt 22

<210> 94
<211> 22
<212> DNA
<213> Artificial Sequence

<220>
<223> bisulfite treated nucleic acid for analysis of methylation status of SEQ ID NO: 5

<400> 94
accgaaataa caatcatcga at 22

<210> 95
<211> 37
<212> DNA
<213> Artificial Sequence

<220>
<223> bisulfite treated nucleic acid for analysis of methylation status of SEQ ID NO: 5

<400> 95
ggacggtatt attatatagt cgggttttcg tagtgtt 37

<210> 96
<211> 23
<212> DNA
<213> Artificial Sequence

<220>
<223> bisulfite treated nucleic acid for analysis of methylation status of SEQ ID NO: 5

<400> 96
ccgaaataac aatcatcgaa tta 23

<210> 97
<211> 22
<212> DNA
<213> Artificial Sequence

<220>
<223> bisulfite treated nucleic acid for analysis of methylation status of SEQ ID NO: 5

<400> 97
ttataggacg gtcggttttt at 22

<210> 98
<211> 22
<212> DNA
<213> Artificial Sequence

<220>
<223> bisulfite treated nucleic acid for analysis of methylation status of SEQ ID NO: 5

<400> 98
aattcgatga ttgttatttc gg 22

<210> 99
<211> 21
<212> DNA
<213> Artificial Sequence

<220>
<223> bisulfite treated nucleic acid for analysis of methylation status of SEQ ID NO: 5

<400> 99
gataaaaacg ccgtcctaca c 21

<210> 100
<211> 24
<212> DNA
<213> Artificial Sequence

<220>
<223> bisulfite treated nucleic acid for analysis of methylation status of SEQ ID NO: 5

<400> 100
aggtggcgtt tggtaggcga cgtt 24

<210> 101
<211> 22
<212> DNA
<213> Artificial Sequence

<220>
<223> bisulfite treated nucleic acid for analysis of methylation status of SEQ ID NO: 5

<400> 101
agtggtatta taggacggtc gg 22

<210> 102
<211> 21
<212> DNA
<213> Artificial Sequence

<220>
<223> bisulfite treated nucleic acid for analysis of methylation status of SEQ ID NO: 5

<400> 102
gtggtattat aggacggtcg g 21

<210> 103
<211> 22
<212> DNA

<213> Artificial Sequence

<220>
<223> bisulfite treated nucleic acid for analysis of methylation status of SEQ ID NO: 5

<400> 103
tggtattata ggacggtcgg tt 22

<210> 104
<211> 22
<212> DNA
<213> Artificial Sequence

<220>
<223> bisulfite treated nucleic acid for analysis of methylation status of SEQ ID NO: 5

<400> 104
attcgatgat tgttatttcg gt 22

<210> 105
<211> 22
<212> DNA
<213> Artificial Sequence

<220>
<223> bisulfite treated nucleic acid for analysis of methylation status of SEQ ID NO: 5

<400> 105
caccgaaata acaatcatcg aa 22

<210> 106
<211> 23
<212> DNA
<213> Artificial Sequence

<220>
<223> bisulfite treated nucleic acid for analysis of methylation status of SEQ ID NO: 5

<400> 106
attataggac ggtcggtttt tat 23

<210> 107
<211> 23
<212> DNA
<213> Artificial Sequence

<220>
<223> bisulfite treated nucleic acid for analysis of methylation status of SEQ ID NO: 5

<400> 107
taattcgatg attgttattt cgg 23

<210> 108
<211> 23
<212> DNA
<213> Artificial Sequence

<220>

<223> bisulfite treated nucleic acid for analysis of methylation status of SEQ ID NO: 5

<400> 108
tagtggtatt ataggacggt cgg 23

<210> 109
<211> 20
<212> DNA
<213> Artificial Sequence

<220>
<223> bisulfite treated nucleic acid for analysis of methylation status of SEQ ID NO: 5

<400> 109
gcgttgagat cgagaagaag 20

<210> 110
<211> 22
<212> DNA
<213> Artificial Sequence

<220>
<223> bisulfite treated nucleic acid for analysis of methylation status of SEQ ID NO: 5

<400> 110
cgaaatcccg aaaaactcta at 22

<210> 111
<211> 33
<212> DNA
<213> Artificial Sequence

<220>
<223> bisulfite treated nucleic acid for analysis of methylation status of SEQ ID NO: 5

<400> 111
gacgtttcgg agaaagtgcg tttagttgat ttt 33

<210> 112
<211> 22
<212> DNA
<213> Artificial Sequence

<220>
<223> bisulfite treated nucleic acid for analysis of methylation status of SEQ ID NO: 5

<400> 112
attataggac ggtcggtttt ta 22

<210> 113
<211> 21
<212> DNA
<213> Artificial Sequence

<220>
<223> bisulfite treated nucleic acid for analysis of methylation status of SEQ ID NO: 5

<400> 113

cgatgattgt tatttcggtg a 21

<210> 114
<211> 24
<212> DNA
<213> Artificial Sequence

<220>
<223> bisulfite treated nucleic acid for analysis of methylation status of SEQ ID NO: 5

<400> 114
tattatagga cggtcggttt ttat 24

<210> 115
<211> 20
<212> DNA
<213> Artificial Sequence

<220>
<223> bisulfite treated nucleic acid for analysis of methylation status of SEQ ID NO: 5

<400> 115
ataaaaacgc cgtcctacac 20

<210> 116
<211> 21
<212> DNA
<213> Artificial Sequence

<220>
<223> bisulfite treated nucleic acid for analysis of methylation status of SEQ ID NO: 5

<400> 116
acgaaatccc gaaaaactct a 21

<210> 117
<211> 22
<212> DNA
<213> Artificial Sequence

<220>
<223> bisulfite treated nucleic acid for analysis of methylation status of SEQ ID NO: 5

<400> 117
gtattatagg acggtcggtt tt 22

<210> 118
<211> 21
<212> DNA
<213> Artificial Sequence

<220>
<223> bisulfite treated nucleic acid for analysis of methylation status of SEQ ID NO: 5

<400> 118
ccgaaataac aatcatcgaa t 21

<210> 119

<211> 21
<212> DNA
<213> Artificial Sequence

<220>
<223> bisulfite treated nucleic acid for analysis of methylation status of SEQ ID NO: 5

<400> 119
accgaaataa caatcatcga a 21

<210> 120
<211> 21
<212> DNA
<213> Artificial Sequence

<220>
<223> bisulfite treated nucleic acid for analysis of methylation status of SEQ ID NO: 5

<400> 120
ttcgatgatt gttatttcgg t 21

<210> 121
<211> 20
<212> DNA
<213> Artificial Sequence

<220>
<223> bisulfite treated nucleic acid for analysis of methylation status of SEQ ID NO: 5

<400> 121
cgataaaaac gccgtcctac 20

<210> 122
<211> 20
<212> DNA
<213> Artificial Sequence

<220>
<223> bisulfite treated nucleic acid for analysis of methylation status of SEQ ID NO: 5

<400> 122
acgataaaaa cgccgtccta 20

<210> 123
<211> 40
<212> DNA
<213> Artificial Sequence

<220>
<223> bisulfite treated nucleic acid for analysis of methylation status of SEQ ID NO: 5

<400> 123
ggcgtatatg gttaggtggc gtttggtagg cgacgttcgg 40

<210> 124
<211> 21
<212> DNA
<213> Artificial Sequence

<220>
<223> bisulfite treated nucleic acid for analysis of methylation status of SEQ ID NO: 5

<400> 124
tcgatgattg ttatttcggt g 21

<210> 125
<211> 20
<212> DNA
<213> Artificial Sequence

<220>
<223> bisulfite treated nucleic acid for analysis of methylation status of SEQ ID NO: 5

<400> 125
gataaaaacg ccgtcctaca 20

<210> 126
<211> 19
<212> DNA
<213> Artificial Sequence

<220>
<223> bisulfite treated nucleic acid for analysis of methylation status of SEQ ID NO: 5

<400> 126
aggcgttgag atcgagaag 19

<210> 127
<211> 29
<212> DNA
<213> Artificial Sequence

<220>
<223> bisulfite treated nucleic acid for analysis of methylation status of SEQ ID NO: 5

<400> 127
gggacgtttc ggagaaagtg cgtttagtt 29

<210> 128
<211> 22
<212> DNA
<213> Artificial Sequence

<220>
<223> bisulfite treated nucleic acid for analysis of methylation status of SEQ ID NO: 5

<400> 128
gacgaaatcc cgaaaaactc ta 22

<210> 129
<211> 23
<212> DNA
<213> Artificial Sequence

<220>
<223> bisulfite treated nucleic acid for analysis of methylation status of SEQ ID NO: 5

<400> 129
tattatagga cggtcggttt tta 23

<210> 130
<211> 21
<212> DNA
<213> Artificial Sequence

<220>
<223> bisulfite treated nucleic acid for analysis of methylation status of SEQ ID NO: 5

<400> 130
tataggacgg tcggttttta t 21

<210> 131
<211> 20
<212> DNA
<213> Artificial Sequence

<220>
<223> bisulfite treated nucleic acid for analysis of methylation status of SEQ ID NO: 5

<400> 131
taaaaacgcc gtcctacacc 20

<210> 132
<211> 25
<212> DNA
<213> Artificial Sequence

<220>
<223> bisulfite treated nucleic acid for analysis of methylation status of SEQ ID NO: 5

<400> 132
ttaggtggcg tttggtaggc gacgt 25

<210> 133
<211> 24
<212> DNA
<213> Artificial Sequence

<220>
<223> bisulfite treated nucleic acid for analysis of methylation status of SEQ ID NO: 5

<400> 133
tttaattcga tgattgttat ttcg 24

<210> 134
<211> 19
<212> DNA
<213> Artificial Sequence

<220>
<223> bisulfite treated nucleic acid for analysis of methylation status of SEQ ID NO: 5

<400> 134
aaggcgttga gatcgagaa 19

<210> 135
<211> 29
<212> DNA
<213> Artificial Sequence

<220>
<223> bisulfite treated nucleic acid for analysis of methylation status of SEQ ID NO: 5

<400> 135
aagggacgtt tcggagaaag tgcgtttag 29

<210> 136
<211> 21
<212> DNA
<213> Artificial Sequence

<220>
<223> bisulfite treated nucleic acid for analysis of methylation status of SEQ ID NO: 5

<400> 136
ttataggacg gtcggttttt a 21

<210> 137
<211> 18
<212> DNA
<213> Artificial Sequence

<220>
<223> bisulfite treated nucleic acid for analysis of methylation status of SEQ ID NO: 5

<400> 137
tttttattgc gtgtcggc 18

<210> 138
<211> 22
<212> DNA
<213> Artificial Sequence

<220>
<223> bisulfite treated nucleic acid for analysis of methylation status of SEQ ID NO: 5

<400> 138
ccgattctaa tacgaaattc ca 22

<210> 139
<211> 22
<212> DNA
<213> Artificial Sequence

<220>
<223> bisulfite treated nucleic acid for analysis of methylation status of SEQ ID NO: 5

<400> 139
ggcggtcgcg gcgaggtttt tg 22

<210> 140
<211> 23
<212> DNA

<213> Artificial Sequence

<220>
<223> bisulfite treated nucleic acid for analysis of methylation status of SEQ ID NO: 5

<400> 140
ttaattcgat gattgttatt tcg 23

<210> 141
<211> 20
<212> DNA
<213> Artificial Sequence

<220>
<223> bisulfite treated nucleic acid for analysis of methylation status of SEQ ID NO: 5

<400> 141
gtaggaaggc gttgagatcg

<210> 142
<211> 27
<212> DNA
<213> Artificial Sequence

<220>
<223> bisulfite treated nucleic acid for analysis of methylation status of SEQ ID NO: 5

<400> 142
agaagggacg tttcggagaa agtgcgt 27

<210> 143
<211> 18
<212> DNA
<213> Artificial Sequence

<220>
<223> bisulfite treated nucleic acid for analysis of methylation status of SEQ ID NO: 5

<400> 143
aaaacgccgt cctacacc 18

<210> 144
<211> 25
<212> DNA
<213> Artificial Sequence

<220>
<223> bisulfite treated nucleic acid for analysis of methylation status of SEQ ID NO: 5

<400> 144
tggttaggtg gcgtttggta ggcga 25

<210> 145
<211> 22
<212> DNA
<213> Artificial Sequence

<220>

<223> bisulfite treated nucleic acid for analysis of methylation status of SEQ ID NO: 5

<400> 145
atagtcgggt tttcgtagtg tt 22

<210> 146
<211> 19
<212> DNA
<213> Artificial Sequence

<220>
<223> bisulfite treated nucleic acid for analysis of methylation status of SEQ ID NO: 5

<400> 146
acctaaccat atacgcccg 19

<210> 147
<211> 36
<212> DNA
<213> Artificial Sequence

<220>
<223> bisulfite treated nucleic acid for analysis of methylation status of SEQ ID NO: 5

<400> 147
tatttcggtg aggatttgtg ttgatggtcg gagaat 36

<210> 148
<211> 19
<212> DNA
<213> Artificial Sequence

<220>
<223> bisulfite treated nucleic acid for analysis of methylation status of SEQ ID NO: 5

<400> 148
taggaaggcg ttgagatcg 19

<210> 149
<211> 22
<212> DNA
<213> Artificial Sequence

<220>
<223> bisulfite treated nucleic acid for analysis of methylation status of SEQ ID NO: 5

<400> 149
tagtcgggtt ttcgtagtgt tg 22

<210> 150
<211> 19
<212> DNA
<213> Artificial Sequence

<220>
<223> bisulfite treated nucleic acid for analysis of methylation status of SEQ ID NO: 5

<400> 150

ggcgttgaga tcgagaaga 19

<210> 151
<211> 19
<212> DNA
<213> Artificial Sequence

<220>
<223> bisulfite treated nucleic acid for analysis of methylation status of SEQ ID NO: 5

<400> 151
gaaggcgttg agatcgaga 19

<210> 152
<211> 28
<212> DNA
<213> Artificial Sequence

<220>
<223> bisulfite treated nucleic acid for analysis of methylation status of SEQ ID NO: 5

<400> 152
gaagggacgt ttcggagaaa gtgcgttt 28

<210> 153
<211> 25
<212> DNA
<213> Artificial Sequence

<220>
<223> bisulfite treated nucleic acid for analysis of methylation status of SEQ ID NO: 5

<400> 153
cgaaataaca atcatcgaat taaaa 25

<210> 154
<211> 19
<212> DNA
<213> Artificial Sequence

<220>
<223> bisulfite treated nucleic acid for analysis of methylation status of SEQ ID NO: 5

<400> 154
aacgataaaa acgccgtcc 19

<210> 155
<211> 19
<212> DNA
<213> Artificial Sequence

<220>
<223> bisulfite treated nucleic acid for analysis of methylation status of SEQ ID NO: 5

<400> 155
aaaaacgccg tcctacacc 19

<210> 156

<211> 22
<212> DNA
<213> Artificial Sequence

<220>
<223> bisulfite treated nucleic acid for analysis of methylation status of SEQ ID NO: 5

<400> 156
cgatgattgt tatttcggtg ag 22

<210> 157
<211> 19
<212> DNA
<213> Artificial Sequence

<220>
<223> bisulfite treated nucleic acid for analysis of methylation status of SEQ ID NO: 5

<400> 157
taaaaacgcc gtcctacac 19

<210> 158
<211> 20
<212> DNA
<213> Artificial Sequence

<220>
<223> bisulfite treated nucleic acid for analysis of methylation status of SEQ ID NO: 5

<400> 158
ataggacggt cggtttttat 20

<210> 159
<211> 17
<212> DNA
<213> Artificial Sequence

<220>
<223> bisulfite treated nucleic acid for analysis of methylation status of SEQ ID NO: 5

<400> 159
acgtcgccta ccaaacg 17

<210> 160
<211> 31
<212> DNA
<213> Artificial Sequence

<220>
<223> bisulfite treated nucleic acid for analysis of methylation status of SEQ ID NO: 5

<400> 160
gatttgtgtt gatggtcgga gaattttgcg t 31

<210> 161
<211> 25
<212> DNA
<213> Artificial Sequence

<220>
<223> bisulfite treated nucleic acid for analysis of methylation status of SEQ ID NO: 5

<400> 161
ttttaattcg atgattgtta tttcg 25

<210> 162
<211> 21
<212> DNA
<213> Artificial Sequence

<220>
<223> bisulfite treated nucleic acid for analysis of methylation status of SEQ ID NO: 5

<400> 162
acgaacgcac ataaccaaat a 21

<210> 163
<211> 22
<212> DNA
<213> Artificial Sequence

<220>
<223> bisulfite treated nucleic acid for analysis of methylation status of SEQ ID NO: 5

<400> 163
tttcggagtt ttagcgtttt ta 22

<210> 164
<211> 29
<212> DNA
<213> Artificial Sequence

<220>
<223> bisulfite treated nucleic acid for analysis of methylation status of SEQ ID NO: 5

<400> 164
aacggtttgg ggtggggcgg taagagcgt 29

<210> 165
<211> 22
<212> DNA
<213> Artificial Sequence

<220>
<223> bisulfite treated nucleic acid for analysis of methylation status of SEQ ID NO: 5

<400> 165
aatacaaaac gacgctctta cc 22

<210> 166
<211> 21
<212> DNA
<213> Artificial Sequence

<220>
<223> bisulfite treated nucleic acid for analysis of methylation status of SEQ ID NO: 5

<400> 166
gttttcggag ttttagcgtt t 21

<210> 167
<211> 35
<212> DNA
<213> Artificial Sequence

<220>
<223> bisulfite treated nucleic acid for analysis of methylation status of SEQ ID NO: 5

<400> 167
ttaggaagtc gaaggattta ggtttaggta acggt 35

<210> 168
<211> 21
<212> DNA
<213> Artificial Sequence

<220>
<223> bisulfite treated nucleic acid for analysis of methylation status of SEQ ID NO: 5

<400> 168
tgttttcgga gttttagcgt t 21

<210> 169
<211> 22
<212> DNA
<213> Artificial Sequence

<220>
<223> bisulfite treated nucleic acid for analysis of methylation status of SEQ ID NO: 5

<400> 169
atcgaaacta aacccctaaa cg 22

<210> 170
<211> 23
<212> DNA
<213> Artificial Sequence

<220>
<223> bisulfite treated nucleic acid for Analysis of methylation status of SEQ ID NO: 5

<400> 170
ttcgtaaaag tagcgttttt agc 23

<210> 171
<211> 22
<212> DNA
<213> Artificial Sequence

<220>
<223> bisulfite treated nucleic acid for analysis of methylation status of SEQ ID NO: 5

<400> 171
ggggaggcgc gggttaggtt gg 22

<210> 172
<211> 20
<212> DNA
<213> Artificial Sequence

<220>
<223> bisulfite treated nucleic acid for analysis of methylation status of SEQ ID NO: 5

<400> 172
ttgttttcgg agttttagcg 20

<210> 173
<211> 22
<212> DNA
<213> Artificial Sequence

<220>
<223> bisulfite treated nucleic acid for analysis of methylation status of SEQ ID NO: 5

<400> 173
tcgtaaaagt agcgttttta gc 22

<210> 174
<211> 22
<212> DNA
<213> Artificial Sequence

<220>
<223> bisulfite treated nucleic acid for analysis of methylation status of SEQ ID NO: 5

<400> 174
caaataacgc ctaacaaacg ac 22

<210> 175
<211> 22
<212> DNA
<213> Artificial Sequence

<220>
<223> bisulfite treated nucleic acid for analysis of methylation status of SEQ ID NO: 5

<400> 175
actcgaattt tcctacgatc ac 22

<210> 176
<211> 20
<212> DNA
<213> Artificial Sequence

<220>
<223> bisulfite treated nucleic acid for analysis of methylation status of SEQ ID NO: 5

<400> 176
attttgtcgg ggtcgtattt 20

<210> 177
<211> 41
<212> DNA

<213> Artificial Sequence

<220>
<223> bisulfite treated nucleic acid for analysis of methylation status of SEQ ID NO: 5

<400> 177
tagaaacgaa aattcgatag aagtagtaat agttgttgtg g 41

<210> 178
<211> 20
<212> DNA
<213> Artificial Sequence

<220>
<223> bisulfite treated nucleic acid for analysis of methylation status of SEQ ID NO: 5

<400> 178
cgaacgcaca taaccaaata 20

<210> 179
<211> 24
<212> DNA
<213> Artificial Sequence

<220>
<223> bisulfite treated nucleic acid for analysis of methylation status of SEQ ID NO: 5

<400> 179
attcgtaaaa gtagcgtttt tagc 24

<210> 180
<211> 21
<212> DNA
<213> Artificial Sequence

<220>
<223> bisulfite treated nucleic acid for analysis of methylation status of SEQ ID NO: 5

<400> 180
tcgaaactaa acccctaaac g 21

<210> 181
<211> 21
<212> DNA
<213> Artificial Sequence

<220>
<223> bisulfite treated nucleic acid for analysis of methylation status of SEQ ID NO: 5

<400> 181
ttcggagttt tagcgttttt a 21

<210> 182
<211> 22
<212> DNA
<213> Artificial Sequence

<220>

<223> bisulfite treated nucleic acid for analysis of methylation status of SEQ ID NO: 5

<400> 182
gaatacaaaa cgacgctctt ac 22

<210> 183
<211> 20
<212> DNA
<213> Artificial Sequence

<220>
<223> bisulfite treated nucleic acid for analysis of methylation status of SEQ ID NO: 5

<400> 183
atcacataac cgaatcctcg 20

<210> 184
<211> 21
<212> DNA
<213> Artificial Sequence

<220>
<223> bisulfite treated nucleic acid for analysis of methylation status of SEQ ID NO: 5

<400> 184
gttcgtagcg tagggttttt c 21

<210> 185
<211> 34
<212> DNA
<213> Artificial Sequence

<220>
<223> bisulfite treated nucleic acid for analysis of methylation status of SEQ ID NO: 5

<400> 185
ggtttttatc gaggtgatag ttatcggatt ggga 34

<210> 186
<211> 20
<212> DNA
<213> Artificial Sequence

<220>
<223> bisulfite treated nucleic acid for analysis of methylation status of SEQ ID NO: 5

<400> 186
ttttcggagt tttagcgttt 20

<210> 187
<211> 19
<212> DNA
<213> Artificial Sequence

<220>
<223> bisulfite treated nucleic acid for analysis of methylation status of SEQ ID NO: 5

<400> 187

ttttgtcggg gtcgtattt 19

<210> 188
<211> 22
<212> DNA
<213> Artificial Sequence

<220>
<223> bisulfite treated nucleic acid for analysis of methylation status of SEQ ID NO: 5

<400> 188
ctaataaccg aaaaacccta cg 22

<210> 189
<211> 21
<212> DNA
<213> Artificial Sequence

<220>
<223> bisulfite treated nucleic acid for analysis of methylation status of SEQ ID NO: 5

<400> 189
ggtaagagcg tcgttttgta t 21

<210> 190
<211> 31
<212> DNA
<213> Artificial Sequence

<220>
<223> bisulfite treated nucleic acid for analysis of methylation status of SEQ ID NO: 5

<400> 190
tgttaggcgt tatttggtta tgtgcgttcg t 31

<210> 191
<211> 20
<212> DNA
<213> Artificial Sequence

<220>
<223> bisulfite treated nucleic acid for analysis of methylation status of SEQ ID NO: 5

<400> 191
ataaccgaat cctcgcaata 20

<210> 192
<211> 21
<212> DNA
<213> Artificial Sequence

<220>
<223> nucleic acid for analysis of methylation status of SEQ ID NO: 5

<400> 192
ctaacgcaac cgcttaaaac t 21

<210> 193

<211> 21
<212> DNA
<213> Artificial Sequence

<220>
<223> bisulfite treated nucleic acid for analysis of methylation status of SEQ ID NO: 5

<400> 193
cggagtatcg gggattttat t 21

<210> 194
<211> 33
<212> DNA
<213> Artificial Sequence

<220>
<223> bisulfite treated nucleic acid for analysis of methylation status of SEQ ID NO: 5

<400> 194
agttagggta tttgcgcggt ttcgatttta atg 33

<210> 195
<211> 19
<212> DNA
<213> Artificial Sequence

<220>
<223> bisulfite treated nucleic acid for analysis of methylation status of SEQ ID NO: 5

<400> 195
cgaaaaaccc tacgctacg 19

<210> 196
<211> 22
<212> DNA
<213> Artificial Sequence

<220>
<223> bisulfite treated nucleic acid for analysis of methylation status of SEQ ID NO: 5

<400> 196
ggtaagagcg tcgttttgta tt 22

<210> 197
<211> 30
<212> DNA
<213> Artificial Sequence

<220>
<223> bisulfite treated nucleic acid for analysis of methylation status of SEQ ID NO: 5

<400> 197
ttcggacgtc gtttgttagg cgttatttgg 30

<210> 198
<211> 25
<212> DNA
<213> Artificial Sequence

<220>
<223> bisulfite treated nucleic acid for analysis of methylation status of SEQ ID NO: 5

<400> 198
tattcgtaaa agtagcgttt ttagc 25

<210> 199
<211> 21
<212> DNA
<213> Artificial Sequence

<220>
<223> bisulfite treated nucleic acid for analysis of methylation status of SEQ ID NO: 5

<400> 199
cataaccgaa tcctcgcaat a 21

<210> 200
<211> 23
<212> DNA
<213> Artificial Sequence

<220>
<223> bisulfite treated nucleic acid for analysis of methylation status of SEQ ID NO: 5

<400> 200
actaataacc gaaaaaccct acg 23

<210> 201
<211> 22
<212> DNA
<213> Artificial Sequence

<220>
<223> bisulfite treated nucleic acid for analysis of methylation status of SEQ ID NO: 5

<400> 201
tcgaaactaa acccctaaac ga 22

<210> 202
<211> 23
<212> DNA
<213> Artificial Sequence

<220>
<223> bisulfite treated nucleic acid for analysis of methylation status of SEQ ID NO: 5

<400> 202
cgtaaaagta gcgtttttag cgt 23

<210> 203
<211> 23
<212> DNA
<213> Artificial Sequence

<220>
<223> bisulfite treated nucleic acid for analysis of methylation status of SEQ ID NO: 5

<400> 203
gaatacaaaa cgacgctctt acc 23

<210> 204
<211> 21
<212> DNA
<213> Artificial Sequence

<220>
<223> bisulfite treated nucleic acid for analysis of methylation status of SEQ ID NO: 5

<400> 204
atacaaaacg acgctcttac c 21

<210> 205
<211> 21
<212> DNA
<213> Artificial Sequence

<220>
<223> bisulfite treated nucleic acid for analysis of methylation status of SEQ ID NO: 5

<400> 205
acgtcgtttg ttaggcgtta t 21

<210> 206
<211> 27
<212> DNA
<213> Artificial Sequence

<220>
<223> bisulfite treated nucleic acid for analysis of methylation status of SEQ ID NO: 5

<400> 206
tggttatgtg cgttcgtagc gtagggt 27

<210> 207
<211> 24
<212> DNA
<213> Artificial Sequence

<220>
<223> bisulfite treated nucleic acid for analysis of methylation status of SEQ ID NO: 5

<400> 207
cgaaggattt aggtttaggt aacg 24

<210> 208
<211> 22
<212> DNA
<213> Artificial Sequence

<220>
<223> bisulfite treated nucleic acid for analysis of methylation status of SEQ ID NO: 5

<400> 208
tggggtgggg cggtaagagc gt 22

<210> 209
<211> 22
<212> DNA
<213> Artificial Sequence

<220>
<223> bisulfite treated nucleic acid for analysis of methylation status of SEQ ID NO: 5

<400> 209
ctcgaatttt cctacgatca cc 22

<210> 210
<211> 21
<212> DNA
<213> Artificial Sequence

<220>
<223> bisulfite treated nucleic acid for analysis of methylation status of SEQ ID NO: 5

<400> 210
tatttcggag tatcggggat t 21

<210> 211
<211> 21
<212> DNA
<213> Artificial Sequence

<220>
<223> bisulfite treated nucleic acid for analysis of methylation status of SEQ ID NO: 5

<400> 211
tcgaattttc ctacgatcac c 21

<210> 212
<211> 20
<212> DNA
<213> Artificial Sequence

<220>
<223> bisulfite treated nucleic acid for analysis of methylation status of SEQ ID NO: 5

<400> 212
taacgcaacc gcttaaaact 20

<210> 213
<211> 21
<212> DNA
<213> Artificial Sequence

<220>
<223> bisulfite treated nucleic acid for analysis of methylation status of SEQ ID NO: 5

<400> 213
aattttgtcg gggtcgtatt t 21

<210> 214
<211> 23
<212> DNA

<213> Artificial Sequence

<220>
<223> bisulfite treated nucleic acid for analysis of methylation status of SEQ ID NO: 5

<400> 214
aatcgaaact aaacccctaa acg 23

<210> 215
<211> 19
<212> DNA
<213> Artificial Sequence

<220>
<223> bisulfite treated nucleic acid for analysis of methylation status of SEQ ID NO: 5

<400> 215
ataaccgaat cctcgcaat 19

<210> 216
<211> 22
<212> DNA
<213> Artificial Sequence

<220>
<223> bisulfite treated nucleic acid for analysis of methylation status of SEQ ID NO: 5

<400> 216
gtcgtttgtt aggcgttatt tg 22

<210> 217
<211> 22
<212> DNA
<213> Artificial Sequence

<220>
<223> bisulfite treated nucleic acid for analysis of methylation status of SEQ ID NO: 5

<400> 217
gttatgtgcg ttcgtagcgt ag 22

<210> 218
<211> 20
<212> DNA
<213> Artificial Sequence

<220>
<223> bisulfite treated nucleic acid for analysis of methylation status of SEQ ID NO: 5

<400> 218
cgtcgtttgt taggcgttat 20

<210> 219
<211> 29
<212> DNA
<213> Artificial Sequence

<220>

<223> bisulfite treated nucleic acid for analysis of methylation status of SEQ ID NO: 5

<400> 219
agcgttaagg tttggggagg cgcgggtta 29

<210> 220
<211> 20
<212> DNA
<213> Artificial Sequence

<220>
<223> bisulfite treated nucleic acid for analysis of methylation status of SEQ ID NO: 5

<400> 220
cataaccgaa tcctcgcaat 20

<210> 221
<211> 29
<212> DNA
<213> Artificial Sequence

<220>
<223> bisulfite treated nucleic acid for analysis of methylation status of SEQ ID NO: 5

<400> 221
ggttatgtgc gttcgtagcg tagggtttt 29

<210> 222
<211> 18
<212> DNA
<213> Artificial Sequence

<220>
<223> bisulfite treated nucleic acid for analysis of methylation status of SEQ ID NO: 5

<400> 222
attcggtggc gataggtt 18

<210> 223
<211> 45
<212> DNA
<213> Artificial Sequence

<220>
<223> bisulfite treated nucleic acid for analysis of methylation status of SEQ ID NO: 5

<400> 223
ttcgtaaaag tagcgttttt agcgttaagg tttggggagg cgcgg 45

<210> 224
<211> 21
<212> DNA
<213> Artificial Sequence

<220>
<223> bisulfite treated nucleic acid for analysis of methylation status of SEQ ID NO: 5

<400> 224

gtattatagg acggtcggtt t 21

<210> 225
<211> 22
<212> DNA
<213> Artificial Sequence

<220>
<223> bisulfite treated nucleic acid for analysis of methylation status of SEQ ID NO: 5

<400> 225
cgaaataaca atcatcgaat ta 22

<210> 226
<211> 19
<212> DNA
<213> Artificial Sequence

<220>
<223> bisulfite treated nucleic acid for analysis of methylation status of SEQ ID NO: 5

<400> 226
ttcgaatttt ttcggacgg 19

<210> 227
<211> 17
<212> DNA
<213> Artificial Sequence

<220>
<223> bisulfite treated nucleic acid for analysis of methylation status of SEQ ID NO: 5

<400> 227
agtcgggttt tcgtagt 17

<210> 228
<211> 21
<212> DNA
<213> Artificial Sequence

<220>
<223> bisulfite treated nucleic acid for analysis of methylation status of SEQ ID NO: 5

<400> 228
ggtattatag gacggtcggt t 21

<210> 229
<211> 20
<212> DNA
<213> Artificial Sequence

<220>
<223> bisulfite treated nucleic acid for analysis of methylation status of SEQ ID NO: 5

<400> 229
tattatagga cggtcggttt 20

<210> 230

<211> 22
<212> DNA
<213> Artificial Sequence

<220>
<223> bisulfite treated nucleic acid for analysis of methylation status of SEQ ID NO: 5

<400> 230
taattcgatg attgttattt cg 22

<210> 231
<211> 19
<212> DNA
<213> Artificial Sequence

<220>
<223> bisulfite treated nucleic acid for analysis of methylation status of SEQ ID NO: 5

<400> 231
gataaaaacg ccgtcctac 19

<210> 232
<211> 19
<212> DNA
<213> Artificial Sequence

<220>
<223> bisulfite treated nucleic acid for analysis of methylation status of SEQ ID NO: 5

<400> 232
ggcgtttggt aggcgacgt 19

<210> 233
<211> 16
<212> DNA
<213> Artificial Sequence

<220>
<223> bisulfite treated nucleic acid for analysis of methylation status of SEQ ID NO: 5

<400> 233
tgcgggcgta tatggt 16

<210> 234
<211> 22
<212> DNA
<213> Artificial Sequence

<220>
<223> bisulfite treated nucleic acid for analysis of methylation status of SEQ ID NO: 5

<400> 234
accgaaataa caatcatcga at 22

<210> 235
<211> 21
<212> DNA
<213> Artificial Sequence

<220>
<223> bisulfite treated nucleic acid for analysis of methylation status of SEQ ID NO: 5

<400> 235
ccgaaataac aatcatcgaa t 21

<210> 236
<211> 20
<212> DNA
<213> Artificial Sequence

<220>
<223> bisulfite treated nucleic acid for analysis of methylation status of SEQ ID NO: 5

<400> 236
ataaaaacgc cgtcctacac 20

<210> 237
<211> 21
<212> DNA
<213> Artificial Sequence

<220>
<223> bisulfite treated nucleic acid for analysis of methylation status of SEQ ID NO: 5

<400> 237
tattatagga cggtcggttt t 21

<210> 238
<211> 21
<212> DNA
<213> Artificial Sequence

<220>
<223> bisulfite treated nucleic acid for analysis of methylation status of SEQ ID NO: 5

<400> 238
accgaaataa caatcatcga a 21

<210> 239
<211> 22
<212> DNA
<213> Artificial Sequence

<220>
<223> bisulfite treated nucleic acid for analysis of methylation status of SEQ ID NO: 5

<400> 239
agtggtatta taggacggtc gg 22

<210> 240
<211> 21
<212> DNA
<213> Artificial Sequence

<220>
<223> bisulfite treated nucleic acid for analysis of methylation status of SEQ ID NO: 5

<400> 240
aattcgatga ttgttatttc g 21

<210> 241
<211> 19
<212> DNA
<213> Artificial Sequence

<220>
<223> bisulfite treated nucleic acid for analysis of methylation status of SEQ ID NO: 5

<400> 241
attataggac ggtcggttt 19

<210> 242
<211> 18
<212> DNA
<213> Artificial Sequence

<220>
<223> bisulfite treated nucleic acid for analysis of methylation status of SEQ ID NO: 5

<400> 242
taaaaacgcc gtcctaca 18

<210> 243
<211> 20
<212> DNA
<213> Artificial Sequence

<220>
<223> bisulfite treated nucleic acid for analysis of methylation status of SEQ ID NO: 5

<400> 243
ccgaaataac aatcatcgaa 20

<210> 244
<211> 21
<212> DNA
<213> Artificial Sequence

<220>
<223> bisulfite treated nucleic acid for analysis of methylation status of SEQ ID NO: 5

<400> 244
gtggtattat aggacggtcg g 21

<210> 245
<211> 20
<212> DNA
<213> Artificial Sequence

<220>
<223> bisulfite treated nucleic acid for analysis of methylation status of SEQ ID NO: 5

<400> 245
tggtattata ggacggtcgg 20

<210> 246
<211> 20
<212> DNA
<213> Artificial Sequence

<220>
<223> bisulfite treated nucleic acid for analysis of methylation status of SEQ ID NO: 5

<400> 246
ataggacggt cggtttttat 20

<210> 247
<211> 27
<212> DNA
<213> Artificial Sequence

<220>
<223> bisulfite treated nucleic acid for analysis of methylation status of SEQ ID NO: 5

<400> 247
ttcggacggt attattatat agtcggg 27

<210> 248
<211> 21
<212> DNA
<213> Artificial Sequence

<220>
<223> bisulfite treated nucleic acid for analysis of methylation status of SEQ ID NO: 5

<400> 248
ttcgtagtgt tggtttttta a 21

<210> 249
<211> 20
<212> DNA
<213> Artificial Sequence

<220>
<223> bisulfite treated nucleic acid for analysis of methylation status of SEQ ID NO: 5

<400> 249
tataggacgg tcggttttta 20

<210> 250
<211> 23
<212> DNA
<213> Artificial Sequence

<220>
<223> bisulfite treated nucleic acid for analysis of methylation status of SEQ ID NO: 5

<400> 250
ttaattcgat gattgttatt tcg 23

<210> 251
<211> 20
<212> DNA

<213> Artificial Sequence

<220>
<223> bisulfite treated nucleic acid for analysis of methylation status of SEQ ID NO: 5

<400> 251
attataggac ggtcggtttt 20

<210> 252
<211> 17
<212> DNA
<213> Artificial Sequence

<220>
<223> bisulfite treated nucleic acid for analysis of methylation status of SEQ ID NO: 5

<400> 252
aaaaacgccg tcctaca 17

<210> 253
<211> 16
<212> DNA
<213> Artificial Sequence

<220>
<223> nucleic acid for analysis of methylation status of SEQ ID NO: 5

<400> 253
tgcgttgcgg gcgtat 16

<210> 254
<211> 16
<212> DNA
<213> Artificial Sequence

<220>
<223> bisulfite treated nucleic acid for analysis of methylation status of SEQ ID NO: 5

<400> 254
ggcgtttggt aggcga 16

<210> 255
<211> 17
<212> DNA
<213> Artificial Sequence

<220>
<223> bisulfite treated nucleic acid for analysis of methylation status of SEQ ID NO: 5

<400> 255
tcctacaccc gaacgtc 17

<210> 256
<211> 17
<212> DNA
<213> Artificial Sequence

<220>

<223> bisulfite treated nucleic acid for analysis of methylation status of SEQ ID NO: 5

<400> 256
tgttgatggt cggagaa 17

<210> 257
<211> 22
<212> DNA
<213> Artificial Sequence

<220>
<223> bisulfite treated nucleic acid for analysis of methylation status of SEQ ID NO: 5

<400> 257
attcgatgat tgttatttcg gt 22

<210> 258
<211> 23
<212> DNA
<213> Artificial Sequence

<220>
<223> bisulfite treated nucleic acid for analysis of methylation status of SEQ ID NO: 5

<400> 258
tagtggtatt ataggacggt cgg 23

<210> 259
<211> 18
<212> DNA
<213> Artificial Sequence

<220>
<223> bisulfite treated nucleic acid for analysis of methylation status of SEQ ID NO: 5

<400> 259
gtcctacacc cgaacgtc 18

<210> 260
<211> 20
<212> DNA
<213> Artificial Sequence

<220>
<223> bisulfite treated nucleic acid for analysis of methylation status of SEQ ID NO: 5

<400> 260
tcgatgattg ttatttcggt 20

<210> 261
<211> 19
<212> DNA
<213> Artificial Sequence

<220>
<223> bisulfite treated nucleic acid for analysis of methylation status of SEQ ID NO: 5

<400> 261

taggacggtc ggtttttat 19

<210> 262
<211> 22
<212> DNA
<213> Artificial Sequence

<220>
<223> bisulfite treated nucleic acid for analysis of methylation status of SEQ ID NO: 5

<400> 262
ctaataaccg aaaaacccta cg 22

<210> 263
<211> 21
<212> DNA
<213> Artificial Sequence

<220>
<223> bisulfite treated nucleic acid for analysis of methylation status of SEQ ID NO: 5

<400> 263
ggtaagagcg tcgttttgta t 21

<210> 264
<211> 19
<212> DNA
<213> Artificial Sequence

<220>
<223> bisulfite treated nucleic acid for analysis of methylation status of SEQ ID NO: 5

<400> 264
gacgtcgttt gttaggcgt 19

<210> 265
<211> 18
<212> DNA
<213> Artificial Sequence

<220>
<223> bisulfite treated nucleic acid for analysis of methylation status of SEQ ID NO: 5

<400> 265
tggttatgtg cgttcgta 18

<210> 266
<211> 21
<212> DNA
<213> Artificial Sequence

<220>
<223> bisulfite treated nucleic acid for analysis of methylation status of SEQ ID NO: 5

<400> 266
taataaccga aaaaccctac g 21

<210> 267

<211> 16
<212> DNA
<213> Homo Sapiens

<400> 267
catccctggc agcagc 16

<210> 268
<211> 17
<212> DNA
<213> Homo Sapiens

<400> 268
taacaaagta aaaaatt 17

<210> 269
<211> 16
<212> DNA
<213> Homo Sapiens

<400> 269
tggaccagac tggtga 16

<210> 270
<211> 16
<212> DNA
<213> Homo Sapiens

<400> 270
gatcatgcaa gtcgat 16

<210> 271
<211> 16
<212> DNA
<213> Homo Sapiens

<400> 271
tgtaaattat aataaa 16

<210> 272
<211> 16
<212> DNA
<213> Homo Sapiens

<400> 272
agctaagcac ttcact 16

<210> 273
<211> 17
<212> DNA
<213> Homo Sapiens

<400> 273
gtgctccgaa gtatctt 17

<210> 274
<211> 18
<212> DNA

<213> Homo Sapiens

<400> 274
gcaactatcc ccctcctc 18

<210> 275
<211> 16
<212> DNA
<213> Homo Sapiens

<400> 275
cagtccaggg gtctgc 16

<210> 276
<211> 16
<212> DNA
<213> Homo Sapiens

<400> 276
ccggacgtca acaacc 16

<210> 277
<211> 20
<212> DNA
<213> Artificial Sequence

<220>
<223> chemically treated genomic DNA (Homo sapiens)

<400> 277
gctaagccgg agtcaccaag 20

<210> 278
<211> 19
<212> DNA
<213> Artificial Sequence

<220>
<223> chemically treated genomic DNA (Homo sapiens)

<400> 278
cgctgagacc gagaagaag 19

<210> 279
<211> 21
<212> DNA
<213> Artificial Sequence

<220>
<223> chemically treated genomic DNA (Homo sapiens)

<400> 279
ctctaaaccg cgaaaactcc g 21

<210> 280
<211> 21
<212> DNA
<213> Artificial Sequence

<220>
<223> chemically treated genomic DNA (Homo sapiens)

<400> 280
cgcgtttgtt cgggtttcgg g 21

<210> 281
<211> 23
<212> DNA
<213> Artificial Sequence

<220>
<223> chemically treated genomic DNA (Homo sapiens)

<400> 281
gggatgagag gttgttgacg ttc 23

<210> 282
<211> 21
<212> DNA
<213> Artificial Sequence

<220>
<223> chemically treated genomic DNA (Homo sapiens)

<400> 282
gagaggttta ttaaggtagg g 21

<210> 283
<211> 28
<212> DNA
<213> Artificial Sequence

<220>
<223> chemically treated genomic DNA (Homo sapiens)

<400> 283
ctactaccaa aaataaaaaa ttattaac 28

<210> 284
<211> 39
<212> DNA
<213> Artificial Sequence

<220>
<223> chemically treated genomic DNA (Homo sapiens)

<400> 284
aaaattatta acatccaaca catttatcca aacttcaaa 39

<210> 285
<211> 26
<212> DNA
<213> Artificial Sequence

<220>
<223> chemically treated genomic DNA (Homo sapiens)

<400> 285
gtttaggggt ttgtagagaa gttcga 26

<210> 286
<211> 17
<212> DNA
<213> Artificial Sequence

<220>
<223> chemically treated genomic DNA (Homo sapiens)

<400> 286
tcggataaac gcgtcgg 17

## Description

FIELD OF THE INVENTION

The present invention relates to genomic DNA sequences that exhibit altered CpG methylation patterns in disease states relative to normal. Particular embodiments provide methods, nucleic acids, nucleic acid arrays and kits useful for detecting, or for detecting and differentiating between or among colorectal cell proliferative disorders.

BACKGROUND

The etiology of pathogenic states is known to involve modified methylation patterns of individual genes or of the genome. 5-methylcytosine, in the context of CpG dinucleotide sequences, is the most frequent covalently modified base in the DNA of eukaryotic cells, and plays a role in the regulation of transcription, genetic imprinting, and tumorigenesis. The identification and quantification of 5-methylcytosine sites in a specific specimen, or between or among a plurality of specimens, is thus of considerable interest, not only in research, but particularly for the molecular diagnoses of various diseases.

*Correlation of aberrant DNA methylation with cancer.* Aberrant DNA methylation within CpG 'islands' is characterized by *hyper*- or *hypomethylation* of CpG dinucleotide sequences leading to abrogation or overexpression of a broad spectrum of genes, and is among the earliest and most common alterations found in, and correlated with human malignancies. Additionally, abnormal methylation has been shown to occur in CpG-rich regulatory elements in intronic and coding parts of genes for certain tumors. In colon cancer, for example, aberrant DNA methylation constitutes one of the most prominent alterations and inactivates many tumor suppressor genes such as p14ARF, p16INK4a, THBS1, MINT2, and MINT31 and DNA mismatch repair genes such as hMLH1.

In contrast to the specific hypermethylation of tumor suppressor genes, an overall hypomethylation of DNA can be observed in tumor cells. This decrease in global methylation can be detected early, far before the development of frank tumor formation. A correlation between hypomethylation and increased gene expression has been determined for many oncogenes.

In the United States the annual incidence of colorectal cancer is approximately 150,000, with 56,600 individuals dying form colorectal cancer each year. The lifetime risk of colorectal cancer in the general population is about 5 to 6 percent. Despite intensive efforts in recent years in screening and early detection of colon cancer, until today most cases are diagnosed in an advanced stage with regional or distant metastasis. While the therapeutic options include surgery and adjuvant or palliative chemotherapy, most patients die from progression of their cancer within a few months. Identifying the molecular changes that underlie the development of colon cancer may help to develop new monitoring, screening, diagnostic and therapeutic options that could improve the overall poor prognosis of these patients.

The current guidelines for colorectal screening according to the American Cancer Society utilizes one of five different options for screening in average risk individuals 50 years of age or older. These options include 1) fecal occult blood test (FOBT) annually, 2) flexible sigmoidoscopy every five years, 3) annual FPBT plus flexible sigmoidoscopy every five years, 4) double contrast barium enema (DCBE) every five years or 5) colonoscopy every ten years. Even though these testing procedures are well accepted by the medical community, the implementation of widespread screening for colorectal cancer has not been realized. Patient compliance is a major factor for limited use due to the discomfort or inconvenience associated with the procedures. FOBT testing, although a non-invasive procedure, requires dietary and other restrictions 3-5 days prior to testing. Sensitivity levels for this test are also very low for colorectal adenocarcinoma with wide variability depending on the trial. Sensitivity measurements for detection of adenomas is even less since most adenomas do not bleed. In contrast, sensitivity for more invasive procedures such as sigmoidoscopy and colonoscopy are quite high because of direct visualization of the lumen of the colon. No randomized trials have evaluated the efficacy of these techniques, however, using data from case-control studies and data from the National Polyp Study (U.S.) it has been shown that removal of adenomatous polyps results in a 76-90% reduction in CRC incidence. Sigmoidoscopy has the limitation of only visualizing the left side of the colon leaving lesions in the right colon undetected. Both scoping procedures are expensive, require cathartic preparation and have increased risk of morbidity and mortality. Improved tests with increased sensitivity, specificity, ease of use and decreased costs are clearly needed before general widespread screening for colorectal cancer becomes routine.

Early colorectal cancer detection is generally based on the fecal occult blood test (FOBT) performed annually on asymptomatic individuals. Current recommendations adapted by several healthcare organizations, including the American Cancer Society, call for fecal occult blood testing beginning at age 50, repeated annually until such time as the patient would no longer benefit from screening. A positive FOBT leads to colonoscopic examination of the bowel; an expensive and invasive procedure, with a serious complication rate of one per 5,000 examinations. Only 12% of patients with heme positive stool are diagnosed with cancer or large polyps at the time of colonoscopy. A number of studies show that FOBT screening does not improve cancer-related mortality or overall survival. Compliance with occult blood testing has been poor; less than 20 percent of the population is offered or completes FOBT as recommended. If FOBT is properly done, the patient collects a fecal sample from three consecutive bowel movements. Samples are obtained while the patient adheres to dietary guidelines and avoids medications known to induce occult gastrointestinal bleeding. In reality, physicians frequently fail to instruct patients properly, patients frequently fail to adhere to protocol, and some patients find the task of collecting fecal samples difficult or unpleasant, hence compliance with annual occult blood testing is poor. If testing sensitivity and specificity can be improved over current methods, the frequency of testing could be reduced, collection of consecutive samples would be eliminated, dietary and medication schedule modifications would be eliminated, and patient compliance would be enhanced. Compounding the problem of compliance, the sensitivity and specificity of FOBT to detect colon cancer is poor. Poor test specificity leads to unnecessary colonoscopy, adding considerable expense to colon cancer screening.

Specificity of the FOBT has been calculated at best to be 96%, with a sensitivity of 43% (adenomas) and 50% (colorectal carcinoma). Sensitivity can be improved using an immunoassay FOBT such as that produced under the tradename 'InSure™', with an improved sensitivity of 77 % (adenomas) and 88.9% (colorectal carcinoma.

Molecular disease markers offer several advantages over other types of markers, one advantage being that even samples of very small sizes and/or samples whose tissue architecture has not been maintained can be analyzed quite efficiently. Within the last decade a number of genes have been shown to be differentially expressed between normal and colon carcinomas. However, no single or combination of marker has been shown to be sufficient for the diagnosis of colon carcinomas. High-dimensional mRNA based approaches have recently been shown to be able to provide a better means to distinguish between different tumor types and benign and malignant lesions. However its application as a routine diagnostic tool in a clinical environment is impeded by the extreme instability of mRNA, the rapidly occurring expression changes following certain triggers (e.g., sample collection), and, most importantly, the large amount of mRNA needed for analysis (Lipshutz, R. J. et al., Nature Genetics 21:20-24, 1999; Bowtell, D. D. L. Nature genetics suppl. 21:25-32, 1999), which often cannot be obtained from a routine biopsy.

The use of biological markers to further improve sensitivity and specificity of FOBT has been suggested, examples of such tests include the PreGen-Plus™ stool analysis assay available from EXACT Sciences which has a sensitivity of 20% (adenoma) and 52% (colorectal carcinoma) and a specificity of 95% in both cases. This test assays for the presence of 23 DNA mutations associated with the development of colon neoplasms. The use of DNA methylation as colon cancer markers is known. For example Sabbioni et al. (Molecular Diagnosis 7:201-207, 2003) detected hypermethylation of a panel of genes consisiting TPEF, HIC1, DAPK and MGMT in peripheral blood in 98% of colon carcinoma patients. However, this does provide a suitable basis for a commercially marketable test, as the specificity of such a test must also be sufficiently high.

The current model of colorectal pathogenesis favours a stepwise progression of adenomas, which includes the development of dysplasia and finally signs of invasive cancer. The molecular changes underlying this adenoma-carcinoma sequence include genetic and epigenetic alterations of tumor suppressor genes (APC, p53, DCC), the activation of oncogenes (K-ras) and the inactivation of DNA mismatch repair genes. Recently, further molecular changes and genetic defects have been revealed. Thus, activation of the Wnt signalling pathway not only includes mutations of the APC gene, but may also result from β-catenin mutations. Furthermore, alterations in the TGF-β signalling pathway together with its signal transducers SMAD4 and SMAD2 have been linked to the development of colon cancer.

Despite recent progress in the understanding of the pathogenesis of adenomas and carcinomas of the colon and their genetic and molecular changes, the genetic and epigenetic changes underlying the development of metastasis are less well understood. It is, however, generally well accepted that the process of invasion and proteolysis of the extracellular matrix, as well as infiltration of the vascular basement membrane involve adhesive proteins, such as members of the family of integrin receptors, the cadherins, the immunoglobulin superfamily, the laminin binding protein and the CD44 receptor. Apart from adhesion, the process of metastasis formation also includes the induction and regulation of angiogenesis (VEGF, bFGF), the induction of cell proliferation (EGF, HGF, IGF) and the activation of proteolytic enzymes (MMPs, TIMPs, uPAR), as well as the inhibition of apoptosis (Bcl-2, Bcl-X). More recently other groups have compared the genetic and molecular changes in metastatic lesions to the changes found in primary colorectal cancers. Thus, Kleeff et al. reported the loss of DOC-2, a candidate tumor suppressor gene, both in primary and metastatic colorectal cancer. Furthermore, Zauber et al. reported that in their series of 42 colorectal cancers Ki-ras mutations in the primary cancers were identical in all of the 42 paired primary and synchronous metastatic lesions. Similarly loss of heterozygosity at the APC locus was identical for 39 paired carcinomas and synchronous metastasis. The authors concluded that for Ki-ras and APC genes the genetic changes in metastasis are identical to the primary colorectal cancer. However, other groups have found genetic and molecular changes in metastatic colon cancers, that are not present in the primary cancers. Thus, the development of LOH of chromosome 3p in colorectal metastasis has been reported. In addition, using comparative genomic hybridization several alterations were found in liver metastasis that were unique to metastastic lesions (-9q, -11q, and -17q).

Apart from mutations aberrant methylation of CpG islands has been shown to lead to the transcriptional silencing of certain genes that have been previously linked to the pathogenesis of various cancers. CpG islands are short sequences which are rich in CpG dinucleotides and can usually be found in the 5' region of approximately 50% of all human genes. Methylation of the cytosines in these islands leads to the loss of gene expression and has been reported in the inactivation of the X chromosome and genomic imprinting.

Recently several groups have also analysed the methylation of various genes in colorectal cancer and reported the transcriptional silencing by promoter methylation for p16INK4, p14ARF, p15INK4b, MGMT, hMLH1, GSTP1, DAPK, CDH1, TIMP-3 and APC among others. Thus apart from mutational inactivation of certain genes, the hypermethylation of these genes also contributes significantly to the pathogenesis of this disease.

In recent years several genes that are methylated in colon cancer have been identified by MS-APPCR. This group of genes, among others, includes TPEF/HPP1 which is frequently methylated in colon cancers and which was independently identified by two different groups using the MS-APPCR method (see, e.g., Young J, Biden KG, Simms LA, Huggard P, Karamatic R, Eyre HJ, Sutherland GR, Herath N, Barker M, Anderson GJ, Fitzpatrick DR, Ramm GA, Jass JR, Leggett BA. HPP1: a transmembrane protein-encoding gene commonly methylated in colorectal polyps and cancers. Proc Natl Acad Sci USA 98:265-270, 2001).

*Multifactorial approach.* Cancer diagnostics has traditionally relied upon the detection of single molecular markers (*e.g.*, gene mutations, elevated PSA levels). Unfortunately, cancer is a disease state in which single markers have typically failed to detect or differentiate many forms of the disease. Thus, assays that recognize only a single marker have been shown to be of limited predictive value. A fundamental aspect of this invention is that methylation-based cancer diagnostics and the screening, diagnosis, and therapeutic monitoring of such diseases will provide significant improvements over the state-of-the-art that uses single marker analyses by the use of a selection of multiple markers. The multiplexed analytical approach is particularly well suited for cancer diagnostics since cancer is not a simple disease, this multi-factorial "panel" approach is consistent with the heterogeneous nature of cancer, both cytologically and clinically.

Key to the successful implementation of a panel approach to methylation based diagnostic tests is the design and development of optimized panels of markers that can characterize and distinguish disease states.

*Development of medical tests.* Two key evaluative measures of any medical screening or diagnostic test are its sensitivity and specificity, which measure how well the test performs to accurately detect all affected individuals without exception, and without falsely including individuals who do not have the target disease (predicitive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present. In this context: Sensitivity = TP/(TP+FN); Specificity = TN/(FP+TN); and Predictive value = TP/(TP+FP).

Sensitivity is a measure of a test's ability to correctly detect the target disease in an individual being tested. A test having poor sensitivity produces a high rate of false negatives, *i.e.,* individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, *i.e*., individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cancer, often involve a plurality of genes and proteins. Additionally, certain proteins may be elevated for reasons unrelated to a disease state. n example of a test that has high specificity is a gene-based test that can detect a p53 mutation. Specificity is important when the cost or risk associated with further diagnostic procedures or further medical intervention are very high.

*Pronounced need in the art.* If colon cancer screening specificity can be increased, the problem of false positive test results leading to unnecessary colonoscopic examination would be reduced leading to cost savings and improved safety.

In view of the incidence of colon cancer and disadvantages associated with current colorectal cell proliferative disorder screening methods there is a substantial need in the art for improved methods for the early detection of colon cell proliferative disorders, in particular colon cancer, to be used in addition to or as a substitute for currently available tests.

## SUMMARY OF THE INVENTION

Various aspects of the present disclosure provide efficient and unique panels of genes, whereby methylation analysis of one or a combination of the members of the panel enables the detection of cell proliferative disorders of the prostate with a particularly high sensitivity, specificity and/or predictive value. The inventive colorectal cancer testing methods have particular utility for the screening of at-risk populations. The inventive methods have advantages over prior art methods (including the industry standard FOBT), because of improved sensitivity, specificity and likely patient compliance.

The present invention provides novel methods for detecting or distinguishing between colorectal cell proliferative disorders. Said methods are most preferably utilised for detecting or detecting and distinguishing between one or more of the following: colorectal carcinoma, colon adenoma, inflammatory colon tissue, grade 2 dysplasia colon adenomas less than 1 cm, grade 3 dysplasia colon adenomas larger than 1 cm, normal colon tissue, non-colon normal tissue, body fluids and non-colon cancer tissue.

The method disclosed may be used for the detection of aerodigestive cell proliferative disorders.

Disclosed herein is a method for detecting and/or for detecting and distinguishing between or among colorectal cell proliferative disorders in a subject. Said method comprises the following steps: i) contacting genomic DNA isolated from blood plasma, blood serum, whole blood, isolated blood cells, cells isolated from the blood obtained from the subject with at least one reagent, or series of reagents that distinguishes between methylated and non-methylated CpG dinucleotides within at least one target region of the genomic DNA, wherein the nucleotide sequence of said target region comprises at least one CpG dinucleotide sequence; and ii) detecting, or detecting and distinguishing between or among colorectal cell proliferative disorders afforded with a sensitivity of greater than or equal to 80% and a specificity of greater than or equal to 80%.

Preferably, the sensitivity is from about 75% to about 96%, or from about 80% to about 90%, or from about 80% to about 85%. Preferably, the specificity is from about 75% to about 96%, or from about 80% to about 90%, or from about 80% to about 85%.

The method is novel as no methods currently exist that enable the detection of colon cancer by analysis of bodily fluids, with a sensitivity and specificity high enough for use in a commercially available and regulatory body approved assay. Current methods used to detect and diagnose colorectal cell proliferative disorders include colonoscopy, sigmoidoscopy, and fecal occult blood colon cancer. In comparison to these methods, the disclosed invention is much less invasive than colonoscopy, and as, if not more sensitive than sigmoidoscopy and FOBT. The development of a body fluid assay represents a clear technical advantage over current methods known in the art in that it is anticipated that patient compliance for a single body fluid based test will be higher than the triplicate analysis of stool currently recommended for FOBT.

A particular embodiment the method comprises the use of the ALX 4 gene as methylation biomarker for the differentiation, detection and distinguishing of colorectal cell proliferative disorders in a sample selected from the group consisting of blood plasma, blood serum, whole blood, isolated blood cells, cells isolated from the blood.

Said use of the gene may be enabled by means of any analysis of the expression of the gene, by means of mRNA expression analysis or protein expression analysis. However, in the most preferred embodiment of the invention, the detection, differentiation and distinguishing of colorectal cell proliferative disorders is enabled by means of analysis of the *methylation status* of the ALX 4 gene and its promoter or regulatory elements.

The invention provides a method for the analysis of biological samples for features associated with the development of colorectal cell proliferative disorders, the method characterised in that SEQ ID NO: 5 is contacted with a reagent or series of reagents capable of distinguishing between methylated and non methylated CpG dinucleotides within the genomic sequence, or sequences of interest.

The present disclosure provides a method for ascertaining genetic and/or epigenetic parameters of genomic DNA. The method has utility for the improved diagnosis, treatment and monitoring of colorectal cell proliferative disorders, more specifically by enabling the improved identification of and differentiation between subclasses of said disorder. The invention presents improvements over the state of the art in that it enables a highly specific classification of colorectal cell proliferative disorders, thereby allowing for improved and informed treatment of patients.

Preferably, the source of the test sample is selected from the group consisting of cells or cell lines, histological slides, biopsies, paraffin-embedded tissue, bodily fluids, ejaculate, stool, urine, blood, and combinations thereof. Preferably, the source is selected from the group consisting of stool, blood plasma, blood serum, whole blood, isolated blood cells, cells isolated from the blood obtained from the subject.

Specifically, the present invention provides a method for detecting colorectal cell proliferative disorders, comprising: obtaining a biological sample comprising genomic nucleic acid(s); contacting the nucleic acid(s), or a fragment thereof, with one reagent or a plurality of reagents sufficient for distinguishing between methylated and non methylated CpG dinucleotide sequences within a target sequence of the subject nucleic acid, wherein the target sequence comprises, or hybridizes under stringent conditions to, a sequence comprising at least 16 contiguous nucleotides of SEQ ID NO:

5, said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences. Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the group consisting of SEQ ID NOS: 65 to 68 and contiguous regions thereof corresponding to the target sequence.

Additional embodiments provide a method for the detection of colorectal cell proliferative disorders, comprising: extracting the genomic DNA from a sample obtained from a subject; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NOS: 68 to 68 and complements thereof, wherein the treated DNA or the fragment thereof is either amplified to produce an amplificate, or is not amplified; and determining, based on a presence or absence of, or on a property of said amplificate, the methylation state of at least one CpG dinucleotide sequence SEQ ID NO: 5 or an average, or a value reflecting an average methylation state of a plurality of CpG dinucleotide sequences thereof. Preferably, at least one such hybridizing nucleic acid molecule or peptide nucleic acid molecule is bound to a solid phase. Preferably, determining comprises use of at least one method selected from the group consisting of: hybridizing at least one nucleic acid molecule comprising a contiguous sequence at least 9 nucleotides in length that is complementary to, or hybridizes under moderately stringent or stringent conditions to a sequence selected from the group consisting of SEQ ID NOS:65 to 68 and complements thereof; hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NOS: 65 to 68 and complements thereof; hybridizing at least one nucleic acid molecule comprising a contiguous sequence at least 9 nucleotides in length that is complementary to, or hybridizes under moderately stringent or stringent conditions to a sequence selected from the group consisting of SEQ ID NOS:65 to 68 and complements thereof, and extending at least one such hybridized nucleic acid molecule by at least one nucleotide base; and sequencing of the amplificate.

Further embodiments provide a method for the analysis of colorectal cell proliferative disorders, comprising: extracting the genomic DNA from a sample obtained from a subject; contacting the genomic DNA, or a fragment thereof, comprising the sequence of SEQ ID NO: 5 or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of the sequence of SEQ ID NO: 5 or an average, or a value reflecting an average methylation state of a plurality of CpG dinucleotide sequences thereof. Preferably, the digested or undigested genomic DNA is amplified prior to said determining.

Additional embodiments disclose novel genomic and chemically modified nucleic acid sequences, as well as oligonucleotides and/or PNA-oligomers for analysis of cytosine methylation patterns within sequences from the group consisting of SEQ ID NOS:1 to SEQ ID NO:64.

## BRIEF DESCRIPTION OF THE **DRAWINGS**

Figures 1, 5, 9, 13, 16, 20, 24, 28 and 32 show ranked matrices of data obtained according to EXAMPLES 1 and 2, and according to CpG methylation differences between the two classes of tissues, using a suitable algorithm. The figures are shown in greyscale, wherein the most significant CpG positions are at the bottom of the matrix with significance decreasing towards the top. Black indicates total methylation at a given CpG position, white represents no methylation at the particular position, with degrees of methylation represented in grey, from light (low proportion of methylation) to dark (high proportion of methylation). Each row represents one specific CpG position within a gene and each column shows the methylation profile for the different CpGs for one sample. The p-values for the individual CpG positions are shown on the right side. The p-values are the probabilities that the observed distribution occurred by chance in the data set.

Figures 2, 6, 10, 17, 21, 25, 29 and 33 show the uncorrected multivariate LogReg test results for each comparison. Each individual genomic region of interest is represented as a point, the dotted line represents the cut off point for the 25% false discovery rate.

Figures 3, 7, 11, 14, 18, 22, 26, 30 and 34 show ranked matrices of data, obtained according to EXAMPLES 1 and 2, of the accuracy of the genewise linear support vector machine cross validations between the two classes of tissues, for the best performing markers. The figures are shown in greyscale, wherein the most significant CpG positions are at the bottom of the matrix with significance decreasing towards the top. Black indicates total methylation at a given CpG position, white represents no methylation at the particular position, with degrees of methylation represented in grey, from light (low proportion of methylation) to dark (high proportion of methylation). Each row represents one specific CpG position within a gene and each column shows the methylation profile for the different CpGs for one sample. Accuracy values for each individual genomic region of interest are shown on the right side.

Figures 4, 8, 12, 15, 18, 23, 27, 31 and 35 show the accuracy of the genewise linear support vector machine cross validation for all genomic regions of each classification. The accuracy of each genomic region is represented as black squares, the specificity as unfilled diamonds, and the sensitivity as unfilled squares. The accuracy as measured on the X-axis shows the fraction of correctly classified samples.

Figure 36 shows the PCR amplification of samples according to example 11.Each individual sample is annotated on the x-axis as ’n’ denoting normal, or ’t’ denoting tumour. A bronchial tissue sample was accidentally analysed in some assays, these are annotated with an arrow, and are irrelevant in the context of the present invention. The Y-axis shows the level of methylation (’methylation rate ’) in the sample. The methylation rate {methylated/(methylated + unmethylated) are shown for the fragments corresponding to the primer pairs in Table 4.

Figure 37 shows the PCR amplification of samples according to assay 11

Figure 38 shows a ranked matrices of data obtained according to Examples 1 and 2 according to CpG methylation differences between the two classes of tissues, (aerodigestive cancer on the left and healthy aerodigestive tissues on the right) using an algorithim. The most significant CpG positions are at the bottom of the matrix with significance decreasing towards the top. Red indicates total methylation at a given CpG position, green represents no methylation at the particular position.. Each row represents one specific CpG position within a gene and each column shows the methylation profile for the different CpGs for one sample.

## DETAILED DESCRIPTION OF THE INVENTION

<u>Definitions:</u>

The term "Observed/Expected Ratio" ("O/E Ratio") refers to the frequency of CpG dinucleotides within a particular DNA sequence, and corresponds to the [number of CpG sites / (number of C bases x number of G bases)] x band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 kb, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG CpG dinucleotide sequences) within a DNA sequence include "unmethylated," "fully-methylated" and "hemi-methylated."

The term "hemi-methylation" or "hemimethylation" refers to the methylation state of a palindromic CpG methylation site, where only a single cytosine in one of the two CpG dinucleotide sequences of the palindromic CpG methylation site is methylated (*e.g.*, 5’-CC$^{M}$GG-3’ (top strand): 3’-GGCC-5’ (bottom strand)).

The term ’AUC’ as used herein is an abbreviation for the area under a curve. In particular it refers to the area under a Receiver Operating Characteristic (ROC) curve. The ROC curve is a plot of the true positive rate against the false positive rate for the different possible cutpoints of a diagnostic test. It shows the tradeoff between sensitivity and specificity depending on the selected cutpoint (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "hypermethylation" refers to the average methylation state corresponding to an *increased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a *decreased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "microarray" refers broadly to both "DNA microarrays," and ’DNA chip(s),’ as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylations. Further epigenetic parameters include, for example, the acetylation of histones which, however, cannot be directly analyzed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers*) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531,1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401A1.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "array SEQ ID NO," "composite array SEQ ID NO," or "composite array sequence" refer to a sequence, hypothetical or otherwise, consisting of a head-to-tail (5' to 3') linear composite of all individual contiguous sequences of a subject array (*e.g.*, a head-to-tail composite of SEQ ID NOS:1-71, in that order).

The terms "array SEQ ID NO node," "composite array SEQ ID NO node," or "composite array sequence node" refer to a *junction* between any two individual contiguous sequences of the "array SEQ ID NO," the "composite array SEQ ID NO," or the "composite array sequence."

In reference to composite array sequences, the phrase "contiguous nucleotides" refers to a contiguous sequence region of any individual contiguous sequence of the composite array, but does not include a region of the composite array sequence that includes a "node," as defined herein above.

<u>Overview:</u>

The present invention provides for molecular genetic markers that have novel utility for the analysis of methylation patterns associated with the development of colorectal cell proliferative disorders. Said markers may be used for detecting or distinguishing between colorectal cell proliferative disorders, thereby providing improved means for the classification and treatment of said disorders. In an alternative embodiment said molecular genetic markers may be used for the detection of aerodigestive cell proliferative disorders.

*Bisulfite modification of DNA is an art-recognized tool used to assess CpG methylation status.* 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, e.g., PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analyzed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyze individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (*e.g*., Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyze individual cytosine positions, or treated by enzymatic digestion (Xiong & Laird, Nucleic Acids Res., 25:2532-4, 1997). Detection by hybridization has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 9746705 and WO 9515373).

The present invention provides for the use of the bisulfite technique , in combination with one or more methylation assays, for determination of the methylation status of CpG dinuclotide sequences within a sequence defined by SEQ ID NO: 5. According to the present invention, determination of the methylation status of CpG dinuclotide sequences within the sequence of SEQ ID NOS:5 has diagnostic and prognostic utility.

*Methylation Assay Procedures.* Various methylation assay procedures are known in the art, and can be used in conjunction with the present invention. These assays allow for determination of the methylation state of one or a plurality of CpG dinucleotides (e.g., CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, *e.g.,* the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

*COBRA*. COBRA™ analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (*e.g*., as might be found in a typical COBRA™-based kit) for COBRA™ analysis may include, but are not limited to: PCR primers for specific gene (or bisulfite treated DNA sequence or CpG island); restriction enzyme and appropriate buffer; gene-hybridization oligo; control hybridization oligo; kinase labeling kit for oligo probe; and labeled nucleotides. Additionally, bisulfite conversion reagents may include: DNA denaturation buffer; sulfonation buffer; DNA recovery reagents or kits (*e.g*., precipitation, ultrafiltration, affinity column); desulfonation buffer; and DNA recovery components.

Preferably, assays such as "MethyLight ™" (a fluorescence-based real-time PCR technique) (Eads et al.,

Cancer Res. 59:2302-2306, 1999), Ms-SNuPE™ (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

*MethyLight* ™. The MethyLight ™ assay is a high-throughput quantitative methylation assay that utilizes fluorescence-based real-time PCR (TaqMan ™) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight ™ assay may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the "MSP" technique), or with oligonucleotides covering potential methylation sites.

The MethyLight ™ process can by used with a "TaqMan®" probe in the amplification process. For example, double-stranded genomic DNA is treated with sodium bisulfite and subjected to one of two sets of PCR reactions using TaqMan® probes; e.g., with either biased primers and TaqMan® probe, or unbiased primers and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (e.g., as might be found in a typical MethyLight ™-based kit) for MethyLight ™ analysis may include, but are not limited to: PCR primers for specific gene (or bisulfite treated DNA sequence or CpG island); TaqMan® probes; optimized PCR buffers and deoxynucleotides; and Taq polymerase.

*Ms-SNuPE.* The Ms-SNuPE™ technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (e.g., microdissected pathology sections), and it avoids utilization of restriction enzymes for determining the methylation status at CpG sites.

Typical reagents (*e.g.*, as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for specific gene; reaction buffer (for the Ms-SNuPE reaction); and labelled nucleotides. Additionally, bisulfite conversion reagents may include: DNA denaturation buffer; sulfonation buffer; DNA recovery regents or kit (*e.g.*, precipitation, ultrafiltration, affinity column); desulfonation buffer; and DNA recovery components.

*MSP.* MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (*e.g.*, as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

*MCA.* The MCA technique is a method that can be used to screen for altered methylation patterns in genomic DNA, and to isolate specific sequences associated with these changes (Toyota et al., Cancer Res. 59:2307-12, 1999). Briefly, restriction enzymes with different sensitivities to cytosine methylation in their recognition sites are used to digest genomic DNAs from primary tumors, cell lines, and normal tissues prior to arbitrarily primed PCR amplification. Fragments that show differential methylation are cloned and sequenced after resolving the PCR products on high-resolution polyacrylamide gels. The cloned fragments are then used as probes for Southern analysis to confirm differential methylation of these regions. Typical reagents (*e.g.*, as might be found in a typical MCA-based kit) for MCA analysis may include, but are not limited to: PCR primers for arbitrary priming Genomic DNA; PCR buffers and nucleotides, restriction enzymes and appropriate buffers; gene-hybridization oligos or probes; control hybridization oligos or probes.

Genomic Sequences According to SEQ ID NO:1 to SEQ ID NO:64, and Non-naturally Occurring Treated Variants Thereof According to SEQ ID NOS: 65 TO 68 were Determined to have Novel Utility for the Detection, Classification and/or Treatment of Colorectral Cell Proliferative Disorders

Herein disclosed is a method for detecting and/or for detecting and distinguishing between or among colon cell proliferative disorders in a subject. Said method comprises the following steps: i) contacting genomic DNA isolated from bodily fluids obtained from the subject with at least one reagent, or series of reagents that distinguishes between methylated and non-methylated CpG dinucleotides within at least one target region of the genomic DNA, wherein said contiguous nucleotides comprise at least one CpG dinucleotide sequence; and ii) detecting, or detecting and distinguishing between or among colon cell proliferative disorders afforded with a sensitivity of greater than or equal to 80% and a specificity of greater than or equal to 80%.

Preferably, the sensitivity is from about 75% to about 96%, or from about 80% to about 90%, or from about 80% to about 85%. Perferably, the specificity is from about 75% to about 96%, or from about 80% to about 90%, or from about 80% to about 85%.

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, *e.g.*, by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. Body fluids are the preferred source of the DNA; particularly preferred are blood plasma, blood serum, whole blood, isolated blood cells and cells isolated from the blood.

The genomic DNA sample is then treated in such a manner that cytosine bases which are unmethylated at the 5'-position are converted to uracil, thymine, or another base which is dissimilar to cytosine in terms of hybridization behavior. This will be understood as 'treatment' herein.

The above described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

The treated DNA is then analysed in order to determine the methylation state of one or more target gene sequences (prior to the treatment) associated with the development of colorectal carcinoma. It is particularly preferred that the target region comprises, or hybridizes under stringent conditions to at least 16 contiguous nucleotides of at least one gene or sequence selected from the group consisting the genes and sequences as listed in Table 1. It is further preferred that the sequences of said genes in Table 3 as described in the accompanying sequence listing are analysed. The method of analysis may be selected from those known in the art, including those listed herein. Particularly preferred are MethyLight, MSP and the use of blocking oligonucleotides as will be described herein. It is further preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more of SEQ ID NO: (IDBISEQFIRST) to SEQ ID NO: and (IDBISEQLAST) and sequences complementary thereto.

Aberrant methylation, more preferably hypermethylation of the ALX 4 gene is associated with the presence of colorectal carcinoma.

Analysis of this sequence enables for the first time detecting, or detecting and distinguishing between or among colon cell proliferative disorders afforded with a sensitivity of greater than or equal to 80% and a specificity of greater than or equal to 80%. Sensitivity is calculated as: {detected colon neoplasia/all colon neoplasia); and specificity is calculated as (non-detected negatives/total negatives).

Preferably, the sensitivity is from about 75% to about 96%, or from about 80% to about 90%, or from about 80% to about 85%. Preferably, the specificity is from about 75% to about 96%, or from about 80% to about 90%, or from about 80% to about 85%.

Colon neoplasia can be defined as all colon malignancies or colon malignancies and large (> 1 cm adenomas), or subsets thereof. Negatives can be defined as any disease other than colon malignancy and adenomas, or healthy individuals.

In one embodiment the method discloses the use of the ALX 4 gene as marker for the differentiation, detection and distinguishing of colon cell proliferative disorders.

Said use of the gene may be enabled by means of any analysis of the expression of the gene, by means of mRNA expression analysis or protein expression analysis. However, in the most preferred embodiment of the invention, the detection, differentiation and distinguishing of colon cell proliferative disorders is enabled by means of analysis of the methylation status of the ALX 4 gene and its promoter or regulatory elements. Methods for the methylation analysis of genes are described herein.

Aberrant levels of mRNA expression of the ALX 4 gene according to Table 3 is associated with colorectal carcinoma.

Accordingly, increased or decreased levels of expression of said gene or sequence is associable with the development of colorectal carcinoma and other colorectal cell proliferative disorders.

To detect the presence of mRNA encoding a gene or genomic sequence in a detection system for colon cancer, a sample is obtained from a patient. The sample can be a tissue biopsy sample or a sample of blood, plasma, serum or the like. The sample may be treated to extract the nucleic acids contained therein. The resulting nucleic acid from the sample is subjected to gel electrophoresis or other separation techniques. Detection involves contacting the nucleic acids and in particular the mRNA of the sample with a DNA sequence serving as a probe to form hybrid duplexes. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2d ed., 1989). Detection of the resulting duplex is usually accomplished by the use of labelled probes. Alternatively, the probe may be unlabeled, but may be detectable by specific binding with a ligand which is labelled, either directly or indirectly. Suitable labels and methods for labelling probes and ligands are known in the art, and include, for example, radioactive labels which may be incorporated by known methods (*e.g.*, nick translation or kinasing),biotin, fluorescent groups, chemiluminescent groups (e.g., dioxetanes, particularly triggered dioxetanes), enzymes, antibodies, and the like.

In order to increase the sensitivity of the detection in a sample of mRNA transcribed from the gene or genomic sequence, the technique of reverse transcription/polymerisation chain reaction can be used to amplify cDNA transcribed from the mRNA. The method of reverse transcription /PCR is well known in the art (for example, see Watson and Fleming, *supra*).

The reverse transcription /PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end primer. Typically, the primer contains an oligo(dT) sequence. The cDNA thus produced is then amplified using the PCR method and EYA4 specific primers. (Belyavsky et al, Nucl Acid Res 17:2919-2932, 1989; Krug and Berger, Methods in Enzymology, Academic Press,N.Y., Vol.152, pp. 316-325, 1987 which are incorporated by reference).

Also disclosed is a kit for use in detecting a colon cancer disease state through testing of a biological sample. A representative kit may comprise one or more nucleic acid segments that selectively hybridise to the mRNA and a container for each of the one or more nucleic acid segments. In certain embodiments the nucleic acid segments may be combined in a single tube. In further embodiments, the nucleic acid segments may also include a pair of primers for amplifying the target mRNA. Such kits may also include any buffers, solutions, solvents, enzymes, nucleotides, or other components for hybridisation, amplification or detection reactions. Preferred kit components include reagents for reverse transcription-PCR, in situ hybridisation, Northern analysis and/or RPA

The present disclosure further provides for methods to detect the presence of the polypeptide encoded by said genes or gene sequences in a sample obtained from a patient.

Aberrant levels of polypeptide expression of the polypeptides encoded by the ALX 4 gene according to Table 3 is associated with colorectal carcinoma.

Accordingly over or under expression of said polypeptide is associable with the development of colorectal carcinoma and other colorectal cell proliferative disorders.

Any method known in the art for detecting proteins can be used. Such methods include, but are not limited to immunodiffusion, immunoelectrophoresis, immunochemical methods, binder-ligand assays, immunohistochemical techniques, agglutination and complement assays. (*e.g.*, see Basic and Clinical Immunology, Sites and Terr, eds., Appleton & Lange, Norwalk, Conn. pp 217-262, 1991 which is incorporated by reference). Preferred are binder-ligand immunoassay methods including reacting antibodies with an epitope or epitopes and competitively displacing a labelled protein or derivative thereof.

.Certain embodiments of the present invention comprise the use of antibodies specific to the polypeptide encoded by the ALX 4 gene.

Such antibodies may be useful for diagnostic and prognostic applications in detecting the disease state, by comparing a patient's levels of colon disease marker expression to expression of the same marker in normal individuals. In certain embodiments production of monoclonal or polyclonal antibodies can be induced by the use of the coded polypeptide as antigene. Such antibodies may in turn be used to detect expressed proteins as markers for human disease states. The levels of such proteins present in the peripheral blood or tissue sample of a patient may be quantified by conventional methods. Antibody-protein binding may be detected and quantified by a variety of means known in the art, such as labelling with fluorescent or radioactive ligands. Further disclosed are kits for performing the above-mentioned procedures, wherein such kits contain antibodies specific for the investigated polypeptides.

Numerous competitive and non-competitive protein binding immunoassays are well known in the art. Antibodies employed in such assays may be unlabeled, for example as used in agglutination tests, or labelled for use a wide variety of assay methods. Labels that can be used include radionuclides, enzymes, fluorescers, chemiluminescers, enzyme substrates or co-factors, enzyme inhibitors, particles, dyes and the like for use in radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

One approach for preparing antibodies to a protein is the selection and preparation of an amino acid sequence of all or part of the protein, chemically synthesising the sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference). Methods for preparation of the polypeptides or epitopes thereof include, but are not limited to chemical synthesis, recombinant DNA techniques or isolation from biological samples.

In a first further embodiment the present invention is based upon the analysis of methylation levels within the ALX 4 gene, according to Table 3 and/or its regulatory sequences.

In a Further disclosed is the analysis of methylation levels within two or more genes or genomic sequences taken from the group consisting all genes and genomic sequences according to Table 3 and/or their regulatory sequences. It is further preferred that the sequences of said genes or genomic sequences are as according to SEQ ID NOS:1 to SEQ ID NO:64.

Particular embodiments of the present disclosure provide a novel application of the analysis of methylation levels and/or patterns within said sequences that enables a precise detection, characterisation and/or treatment of colorectal cell proliferative disorders. Early detection of colorectal cell proliferative disorders is directly linked with disease prognosis, and the disclosed method thereby enables the physician and patient to make better and more informed treatment decisions. The sequences of the group consisting SEQ ID NOS: 20, 41, 35, 3, 39, 33 & 27 have further utility for the detection of aerodigestive cell proliferative disorders.

FURTHER IMPROVEMENTS

The present invention provides novel uses for the genomic sequence defined by SEQ ID NO: 5. Additional embodiments provide modified variants of SEQ ID NO: 5, as well as oligonucleotides and/or PNA-oligomers for analysis of cytosine methylation patterns within SEQ ID NO: 5.

An objective of the invention comprises analysis of the methylation state of one or more CpG dinucleotides within the genomic sequences defined by SEQ ID NO: 5 and sequences complementary thereto.

The disclosed invention utilizes treated nucleic acids, derived from genomic SEQ ID NO 5, wherein the treatment is suitable to convert at least one unmethylated cytosine base of the genomic DNA sequence to uracil or another base that is detectably dissimilar to cytosine in terms of hybridization. The genomic sequences in question may comprise one, or more, consecutive or random methylated CpG positions. Said treatment preferably comprises use of a reagent selected from the group consisting of bisulfite, hydrogen sulfite, disulfite, and combinations thereof. In a preferred embodiment of the invention, the objective comprises analysis of a non-naturally occurring modified nucleic acid compri sing a sequence of at least 16 contiguous nucleotide bases in length of a sequence selected from the group consisting of SEQ ID NOS: 65 TO 68, wherein said sequence comprises at least one CpG, TpA or CpA dinucleotide and sequences complementary thereto. The sequences of SEQ ID NOS: 65 TO 68 provide non-naturally occurring modified versions of the nucleic acid according to SEQ ID NO: 5, wherein the modification of each genomic sequence results in the synthesis of a nucleic acid having a sequence that is unique and distinct from said genomic sequence as follows. For each sense strand genomic DNA, *e.g.*, SEQ ID NO:1, four converted versions are disclosed. A first version wherein "C" →"T," but "CpG" remains "CpG" (*i.e.*, corresponds to case where, for the genomic sequence, all "C" residues of CpG dinucleotide sequences are methylated and are thus not converted); a second version discloses the complement of the disclosed genomic DNA sequence (i.e. *antisense* strand), wherein "C" →"T," but "CpG" remains "CpG" (*i.e.*, corresponds to case where, for all "C" residues of CpG dinucleotide sequences are methylated and are thus not converted). The 'upmethylated' converted sequences of SEQ ID NO: 5 correspond to SEQ ID NOS:65 and 66. A third chemically converted version of each genomic sequences is provided, wherein "C" →"T" for all "C" residues, including those of "CpG" dinucleotide sequences (*i.e.*, corresponds to case where, for the genomic sequences, all "C" residues of CpG dinucleotide sequences are *un*methylated); a final chemically converted version of each sequence, discloses the complement of the disclosed genomic DNA sequence (i.e. *antisense* strand), wherein "C" →"T" for all "C" residues, including those of "CpG" dinucleotide sequences (*i.e.*, corresponds to case where, for the complement (*antisense* strand) of each genomic sequence, all "C" residues of CpG dinucleotide sequences are *un*methylated). The 'downmethylated' converted sequences of SEQ ID NO: 5 correspond to SEQ ID NOS: 67 to 68.

Significantly, heretofore, the nucleic acid sequence and molecule according SEQ ID NO: 5 was not implicated in or connected with the detection, classification or treatment of colorectal cell proliferative disorders , or aerodigestive cell proliferative disorders..

In an alternative preferred embodiment, such analysis comprises the use of an oligonucleotide or oligomer for detecting the cytosine methylation state within genomic or treated (chemically modified) DNA, according to SEQ ID NOS: 5, 65, 66, 67 and 68. Said oligonucleotide or oligomer comprising a nucleic acid sequence having a length of at least nine (9) nucleotides which hybridizes, under moderately stringent or stringent conditions (as defined herein above), to a treated nucleic acid sequence according to SEQ ID NOS: 65 to SEQ ID NO: 68 and/or sequences complementary thereto, or to a genomic sequence according to SEQ ID NO: 5 and/or sequences complementary thereto.

Thus, the present disclosure includes nucleic acid molecules (e.g., oligonucleotides and peptide nucleic acid (PNA) molecules (PNA-oligomers)) that hybridize under moderately stringent and/or stringent hybridization conditions to all or a portion of the sequences SEQ ID NOS: 5, 65, 66, 67 or 68, or to the complements thereof. The hybridizing portion of the hybridizing nucleic acids is typically at least 9, 15, 20, 25, 30 or 35 nucleotides in length. However, longer molecules have utility, and are thus within the scope of the present disclosure.

Preferably, the hybridizing portion of the inventive hybridizing nucleic acids is at least 95%, or at least 98%, or 100% identical to the sequence, or to a portion thereof of SEQ ID NOS: 5 or 65 to 68, or to the complements thereof.

Hybridizing nucleic acids of the type described herein can be used, for example, as a primer (*e.g.*, a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO: 5 (such as allelic variants and SNPs), rather than identical, it is useful to first establish the lowest temperature at which only homologous hybridization occurs with a particular concentration of salt (*e.g.*, SSC or SSPE). Then, assuming that 1% mismatching results in a 1°C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of oligonucleotides of length X (in nucleotides), as indicated by polynucleotide positions with reference to, *e.g.,* SEQ ID NO:1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:

$$n \text{ to } (n + (X-1));$$

where n=1, 2, 3,...(Y-(X-1));
where Y equals the length (nucleotides or base pairs) of SEQ ID NO:1 (2,280);
where X equals the common length (in nucleotides) of each oligonucleotide in the set *(e.g.,* X=20 for a set of consecutively overlapping 20-mers); and
where the number (Z) of consecutively overlapping oligomers of length X for a given SEQ ID NO of length Y is equal to Y-(X-1). For example Z= 2,280-19= 2,261 for either sense or antisense sets of SEQ ID NO:1, where X=20.

Preferably, the set is limited to those oligomers that comprise at least one CpG, TpG or CpA dinucleotide.

Examples of 20-mer oligonucleotides include the following set of 2,261 oligomers (and the antisense set complementary thereto), indicated by polynucleotide positions with reference to SEQ ID NO:1:

1-20, 2-21, 3-22, 4-23, 5-24, ......2259-2278, 2260-2279 and 2261-2280.

Preferably, the set is limited to those oligomers that comprise at least one CpG, TpG or CpA dinucleotide.

Likewise, examples of 25-mer oligonucleotides include the following set of 2,256 oligomers (and the antisense set complementary thereto), indicated by polynucleotide positions with reference to SEQ ID NO:1:

1-25, 2-26, 3-27, 4-28, 5-29, ......2254-2278, 2255-2279 and 2256-2280.

Preferably, the set is limited to those oligomers that comprise at least one CpG, TpG or CpA dinucleotide.

Dis closed are for *each* of SEQ ID NOS: 5, 65, 66, 67 and 68 (sense and antisense), multiple consecutively overlapping sets of oligonucleotides or modified oligonucleotides of length X, where, *e.g.,* X= 9, 10, 17, 20, 22, 23, 25, 27, 30 or 35 nucleotides.

The oligonucleotides or oligomers constitute effective tools useful to ascertain genetic and epigenetic parameters of the genomic sequence corresponding to SEQ ID NO: 5. Preferred sets of such oligonucleotides or modified oligonucleotides of length X are those consecutively overlapping sets of oligomers corresponding to SEQ ID NO: 5, 65, 66, 67 and 68 (and to the complements thereof). Preferably, said oligomers comprise at least one CpG, TpG or CpA dinucleotide.

Particularly preferred oligonucleotides or oligomers are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, e.g., a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers are typically used in 'sets,' which contain at least one oligomer for analysis of each of the CpG dinucleotides of the genomic sequence defined by SEQ ID NO: 5 and sequences complementary thereto, or to the corresponding CpG, TpG or CpA dinucleotide within a sequence of the treated nucleic acids according to SEQ ID NOS: 65 to SEQ ID NO: 68 and sequences complementary thereto. However, it is anticipated that for economic or other factors it may be preferable to analyze a limited selection of the CpG dinucleotides within said sequences, and the content of the set of oligonucleotides is altered accordingly.

Therefore, the present disclosure provides a set of at least two (2) (oligonucleotides and/or PNA-oligomers) useful for detecting the cytosine methylation state in treated genomic DNA (SEQ ID NOS: 65 to SEQ ID NO: 68), or in genomic DNA (SEQ ID NO: 5 and sequences complementary thereto). These probes enable diagnosis, classification and/or therapy of genetic and epigenetic parameters of colorectal cell proliferative disorders. The set of oligomers may also be used for detecting single nucleotide polymorphisms (SNPs) in treated genomic DNA (SEQ ID NOS: 65 to SEQ ID NO: 68), or in genomic DNA (SEQ ID NOS: 5 to SEQ ID NO:64 and sequences complementary thereto).

At least one, and more preferably all members of a set of oligonucleotides may be bound to a solid phase.

Further, the present disclosure provides a set of at least two (2) oligonucleotides that are used as 'primer' oligonucleotides for amplifying DNA sequences of one of SEQ ID NOS: 5, 65, 66, 67 and 68 and sequences complementary thereto, or segments thereof.

It is anticipated that the oligonucleotides may constitute all or part of an "array" or "DNA chip" (*i.e.*, an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminum, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (*i.e.*, each analyte) can be uniquely bound by a unique label and thus detected (each label is directly counted, resulting in a digital read-out of each molecular species in the mixture).

It is particularly preferred that the oligomers are utilised for at least one of: detection of; detection and differentiation between or among subclasses of; diagnosis of; prognosis of; treatment of; monitoring of; and treatment and monitoring of colorectal cell proliferative disorders. This is enabled by use of said sets for the detection or detection and differentiation of one or more of the following classes of tissues: colorectal carcinoma, colon adenoma, inflammatory colon tissue, grade 2 dysplasia colon adenomas less than 1 cm, grade 3 dysplasia colon adenomas larger than 1 cm, normal colon tissue, non-colon healthy tissue and non-colon cancer tissue.

Particularly preferred are those sets of oligomer that comprise at least two oligonucleotides selected from one of the following sets of oligonucleotides:

SEQ ID NOS:267 - 276; and

SEQ ID NOS: 73 - 92.

In one embodiment of the method, at least one of colorectal carcinoma tissue or colon adenomas is distinguished from at least one tissue selected from the group consisting of inflammatory colon tissue and normal colon tissue. This is achieved by analysis of the methylation status of at least one target sequence comprising, or hybridizing under stringent conditions to at least 16 contiguous nucleotides of a sequence selected from the group consisting SEQ ID NO: 5 and complements thereof. This is preferably achieved by use of a set consisting of at least one oligonucleotide, and more preferably at least two selected from one of the groups consisting of, or by use of a set comprising of at least two oligonucleotides selected from one of the groups consisting of:

SEQ ID NOS:267 - 276; and
SEQ ID NOS: 73 - 92.

In one embodiment of the method, colorectal carcinoma is distinguished from at least one tissue selected from the group consisting of non-colon healthy tissue, peripheral blood lymphocytes and non-colon cancer. In one embodiment this is achieved by analysis of the methylation status of at least one target sequence comprising, or hybridizing under stringent conditions to at least 16 contiguous nucleotides of a sequence defined by SEQ ID NOS: 5. This is preferably achieved by use of a set consisting of at least one oligonucleotide, and more preferably at least two oligonucleotides selected from one of the groups consisting of:

SEQ ID NOS:267 - 276; and
SEQ ID NOS: 73 - 92.

In one embodiment of the method, colorectal carcinoma is distinguished from at least one tissue selected from the group consisting of inflammatory colon tissue, normal colon tissue, non-colon healthy tissue, peripheral blood lymphocytes, colon adenomas and non-colon cancer tissue. This is achieved by analysis of the methylation status of at least one target sequence comprising, or hybridizing under stringent conditions to at least 16 contiguous nucleotides of a sequence selected from the group consisting SEQ ID NO: 5 and complements thereof. This is preferably achieved by use of a set consisting of at least one oligonucleotide, and more preferably at least two by use of a set of oligomers comprising at least two oligonucleotides selected from one of the groups consisting of:

SEQ ID NOS:267 - 276; and
SEQ ID NOS: 73 - 92.

In one embodiment of the method, at least one of colorectal carcinoma tissue, or colon adenomas is distinguished from at least one tissue selected from the group consisting of inflammatory colon tissue and normal colon tissue. This is achieved by analysis of the methylation status of at least one target sequence comprising, or hybridizing under stringent conditions to at least 16 contiguous nucleotides of a sequence selected from the group consisting SEQ ID NO: 5 and complements thereof. This is preferably achieved by use of a set consisting of at least one oligonucleotide, and more preferably at least two oligomers comprising at least two oligonucleotides selected from one of the groups consisting of:

SEQ ID NOS: 267 - 276; and
SEQ ID NOS: 73 - 92.

In one embodiment of the method, colorectal carcinoma tissue is distinguished from at least one of inflammatory colon tissue and normal colon tissue. This is achieved by analysis of the methylation status of at least one target sequence comprising, or hybridizing under stringent conditions to at least 16 contiguous nucleotides of a sequence selected from the group consisting SEQ ID NOS: 5 and complements thereof. This is preferably achieved by use of a set consisting of at least one oligonucleotide, and more preferably at least two oligomers comprising at least two oligonucleotides selected from one of the groups consisting of:

SEQ ID NOS: 267 - 276; and
SEQ ID NOS: 73 - 92.

In one embodiment of the method, at least one of colorectal carcinoma tissue, or colon adenomas is distinguished from at least one tissue selected from the group consisting of inflammatory colon tissue, normal colon tissue, non-colon healthy tissue, peripheral blood lymphocytes, and non-colon cancer tissue. This is achieved by analysis of the methylation status of at least one target sequence comprising, or hybridizing under stringent conditions to at least 16 contiguous nucleotides of a sequence selected from the group consisting SEQ ID NO: 5 and complements thereof. This is preferably achieved by use of a set consisting of at least one oligonucleotide, and more preferably at least two oligomers comprising at least two oligonucleotides selected from one of the groups consisting of:

SEQ IDNOS: 267 - 276; and
SEQ ID NOS: 73 - 92.

In one embodiment of the method, tissues originating from the colon are distinguished from tissues of non-colon origin by use of a set of oligomers comprising at least two oligonucleotides selected from one of the groups consisting of:

SEQ ID NOS: 267 - 276; and
SEQ ID NOS: 73 - 92.

In one embodiment of the method, cell proliferative disorders are distinguished from healthy tissues by use of a set of oligomers comprising at least two oligonucleotides selected from one of the groups consisting of:

SEQ ID NOS: 267 - 276; and
SEQ ID NOS: 73 - 92.

Further disclosed is a method for ascertaining genetic and/or epigenetic parameters of the genomic sequence according to SEQ ID NO: 5 within a subject by analyzing cytosine methylation and single nucleotide polymorphisms. Said method comprising contacting a nucleic acid comprising SEQ ID NOS: 5 in a biological sample obtained from said subject with at least one reagent or a series of reagents, wherein said reagent or series of reagents, distinguishes between methylated and non-methylated CpG dinucleotides within the target nucleic acid.

Preferably, said method comprises the following steps: In the *first step*, a sample of the tissue to be analysed is obtained. The source may be any suitable source, such as cell lines, histological slides, biopsies, paraffin-embedded tissue, bodily fluids, stool, colonic effluent, urine, blood plasma, blood serum, whole blood, isolated blood cells, cells isolated from the blood and all possible combinations thereof. It is preferred that said sources of DNA are stool or bodily fluids selected from the group consisting colonic effluent , urine, blood plasma, blood serum, whole blood, isolated blood cells, cells isolated from the blood.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Once the nucleic acids have been extracted, the genomic double stranded DNA is used in the analysis.

In the *second step* of the method, the genomic DNA sampl e is treated in such a manner that cytosine bases which are unmethylated at the 5'-position are converted to uracil, thymine, or another base which is dissimilar to cytosine in terms of hybridization behavior. This will be understood as 'pretreatment' or 'treatment' herein.

The above-described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

In the *third step* of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). The set of primer oligonucleotides includes at least two oligonucleotides whose sequences are each reverse complementary, identical, or hybridize under stringent or highly stringent conditions to an at least 16-base-pair long segment of the base sequences of one of SEQ ID NOS: 65 to SEQ ID NO: 68 and sequences complementary thereto.

In an alternate embodiment of the method, the methylation status of preselected CpG positions within the nucleic acid sequences comprising SEQ ID NO: 5 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridizes to a treated nucleic acid sequence according to one of SEQ ID NOS: 65 to SEQ ID NO: 68 and sequences complementary thereto, wherein the base sequence of said oligomers comprises at least one CpG dinucleotide.

In a further preferred embodiment of the method, the MSP primers are selected from the group consisting SEQ ID NOS: 93, 97, 98, 101, 102, 103, 104, 106, 107, 108, 109, 112, 113, 114, 117, , 120, 124, 126, 129, 130, 133, 134, 136, 137, 140, 141, 145, 148, 149, 150, 151, 156, 158, 161, 162, 165, 169, 174, 175, 178, 180, 182, 183, 188, 191, 192, 195, 199, 200, 201, 203, 204, 209, 211, 212, 214, 215, 220, 94, 96, 99, 105, 110, 115, 116, 118, 119, 121, 122, 125, 128, 131, 138, 143, 146, 153, 154, 155, 157, 159, 163, 166, 168, 170, 172, 173, 176, 179, 181, 184, 186, 187, 189, 193, 196, 198, 202, 205, 207, 210, 213, 216, 217, 218, 222, Detection: SEQ ID NOS: 224, 228, 229, 230, 237, 239, 240, 241, 244, 245, 246, 249, 250, 251, 257, 258, 260, 261, 262, 266, 225, 231, 234, 235, 236, 238, 242, 243, 252, 255, 259, 263,

A further preferred embodiment of the method comprises the use of *blocker* oligonucleotides. The use of such blocker oligonucleotides has been described by Yu et al., BioTechniques 23:714-720, 1997. Blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-termini thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridizes to a treated nucleic acid sequence according to one of SEQ ID NOS: 65 to SEQ ID NO: 68 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labeled amplificates have a single positive or negative net charge, allowing for better detectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analysed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of hybridization-based methods such as, but not limited to, array technology and probe based technologies as well as by means of techniques such as sequencing and template directed extension.

In one embodiment of the method, the amplificates synthesised in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within the sequence according to SEQ ID NOS:1 to SEQ ID NO:64, and the equivalent positions within SEQ ID NOS:304 to SEQ ID NO:535. Said oligonucleotides may also be present in the form of peptide nucleic acids. The non-hybridized amplificates are then removed. The hybridized amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes that are hybridised to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also see* United States Patent No. 6,331,393) employing a dual-labeled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a nonextendible interrogating oligonucleotide, called a TaqMan™ probe, which, in preferred embodiments, is designed to hybridize to a GpC-rich sequence located between the forward and reverse amplification primers. The TaqMan™ probe further comprises a fluorescent "reporter moiety" and a "quencher moiety" covalently bound to linker moieties (*e.g.*, phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, (hereby incorporated by reference in its entirety) also known as the MethylLight™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

A further suitable method for the use of probe oligonucleotides for the assessment of methylation by analysis of bisulfite treated nucleic acids In a further preferred embodiment of the method, the *fifth step* of the method comprises the use of template-directed oligonucleotide extension, such as MS-SNuPE as described by Gonzalgo & Jones, Nucleic Acids Res. 25:2529-2531, 1997.

In yet a further embodiment of the method, the *fifth s tep* of the method comprises sequencing and subsequent sequence analysis of the amplificate generated in the *third step* of the method (Sanger F., et al., Proc Natl Acad Sci USA 74:5463-5467, 1977).

Best mode

In the most preferred embodiment of the method the genomic nucleic acids are isolated and treated according to the first three steps of the method outlined above, namely:

a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or *blocking oligonucleotides*, and further wherein
e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID NOS: 654 to SEQ ID NO: 68 and sequences complementary thereto, wherein the base sequence of said oligomers comprises at least one CpG dinucleotide.

In a further preferred embodiment of the method, the MSP primers are selected from the group consisting of SEQ ID NOS: 93, 97, 98, 101, 102, 103, 104, 106, 107, 108, 109, 112, 113, 114, 117, 120, 124, 126, 129, 130, 133, 134, 136, 137, 140, 141, 145, 148, 149, 150, 151, 156, 158, 161, 162, 165, 169, 174, 175, 178, 180, 182, 183, 188, 191, 192, 195, 199, 200201, 203, 204, 209, 211, 212, 214, 215, 220, 94, 96, 99, 105, 110, 115, 116, 118, 119, 121, 122, 125, 128, 131, 138, 143, 146, 153, 154, 155, 157, 159, 163, 166, 168, 170, 172, 173, 176, 179, 181, 184, 186, 187, 189, 193, 196, 198, 202, 205, 207, 210, 213, 216, 217, 218, 222, 224, 228, 229, 230, 237,239,240,241,244,245,246,.

Step e) of the method, namely the detection of the specific amplificates indicative of the methylation status of one or more CpG positions according to SEQ ID NO: 5 is carried out by means of *real-time* detection methods as described above, and wherein the sequence of said hybridization probes is selected from the group consisting of SEQ ID NOS: 95, 100, 111, 123, 127, 132, 135, 139, 142, 144, 147, 152, 160, 164, 167, 171, 177, 185, 190, 194, 197, 206, 208, 209,211,.

For each genomic sequence listed, the following oligonucleotides as detailed in the sequence listing may be used for a combined MSP-RealTime analysis:

Taqman Real-time single probe detection assays:

SEQ ID NO: 5

left Primer: 93, 97, 98, 101, 102, 103, 104, 106, 107, 108, 109, 112, 113, 114, 117, 120, 124, 126, 129, 130, 133, 134, 136, 137, 140, 141, 145, 148, 149, 150, 151, 156, 158, 161, 162, 165, 169, 174, 175, 178, 180, 182, 183, 188, 191, 192, 195, 199, 200, 201, 203, 204, 209, 211, 212,214,215,220,
right Primer: 94, 96, 99, 105, 110, 115, 116, 118, 119, 121, 122, 125, 128, 131, 138, 143, 146, 153, 154, 155, 157, 159, 163, 166, 168, 170, 172, 173, 176, 179, 181, 184, 186, 187, 189, 193, 196, 198, 202, 205, 207, 210, 213, 216, 217, 218, 222,
Detection: 95, 100, 111, 123, 127, 132, 135, 139, 142, 144, 147, 152, 160, 164, 167, 171, 177,185,190,194,197,206,208,219,221,223,

SEQ ID NO: 5

left Primer: 224, 228, 229, 230, 237, 239, 240, 241, 244, 245, 246, 249, 250, 251, 257, 258, 260, 261, 262, 264,
right Primer: 225, 231, 234, 235, 236, 238, 242, 243, 252, 255, 259, 263,

Detection: 226, 232, 247, 253, 264,
Anchor: 227, 233, 248, 254, 256, 265,

In an alternative most preferred embodiment of the method, the subsequent amplification of d) is carried out in the presence of *blocking oligonucleotides,* as described above. Said *blocking oligonucleotides* comprising a sequence having a length of at least 9 nucleotides which hybridizes to a treated nucleic acid sequence according to one of SEQ ID NOS: 65 to SEQ ID NO: 68 and sequences complementary thereto, wherein the base sequence of said oligomers comprises at least one CpG, TpG or CpA dinucleotide. Step e) of the method, namely the detection of the specific amplificates indicative of the methylation status of one or more CpG positions according to SEQ ID NO: 5 is carried out by means of real-time detection methods as described above.
Step e) of the method, namely the detection of the specific amplificates indicative of the methylation status of one or more CpG positions according to SEQ ID NO: 5 is carried out by means of real-time detection methods as described above.
Additional embodiments of the disclosure provide a method for the analysis of the methylation status of genomic DNA (SEQ ID NO: 5, and complements thereof) without the need for pretreatment.
In the *first step* of such additional embodiments, the genomic DNA sample is isolated from tissue or cellular sources. Preferably, such sources include cell lines, histological slides, body fluids, or tissue embedded in paraffin. In the *second step*, the genomic DNA is extracted. Extraction may be by means that are standard to one skilled in the art, including but not limited to the use of detergent lysates, sonification and vortexing with glass beads.
Once the nucleic acids have been extracted, the genomic double-stranded DNA is used in the analysis.
In a preferred embodiment, the DNA may be cleaved prior to the treatment, and this may be by any means standard in the state of the art, in particular with methylation-sensitive restriction endonucleases.
In the *third step,* the DNA is then digested with one or more methylation sensitive restriction enzymes. The digestion is carried out such that hydrolysis of the DNA at the restriction site is informative of the methylation status of a specific CpG dinucleotide.
In the *fourth step*, which is optional but a preferred embodiment, the restriction fragments are amplified. This is preferably carried out using a polymerase chain reaction, and said amplificates may carry suitable detectable labels as discussed above, namely fluorophore labels, radionuclides and mass labels.
In the *fifth step* the amplificates are detected. The detection may be by any means standard in the art, for example, but not limited to, gel electrophoresis analysis, hybridization analysis, incorporation of detectable tags within the PCR products, DNA array analysis, MALDI or ESI analysis.
Subsequent to the determination of the methylation state of the genomic nucleic acids the presence, absence or subclass of colorectal cell proliferative disorder is deduced based upon the methylation state of at least one CpG dinucleotide sequence of SEQ ID NO: 5, or an average, or a value reflecting an average methylation state of a plurality of CpG dinucleotide sequences of SEQ ID NO: 5.

<u>Diagnostic Assays for colorectal cell proliferative disorders</u>

The present invention enables diagnosis of events which are disadvantageous to patients or individuals in which important genetic and/or epigenetic parameters within SEQ ID NO: 5 may be used as markers. Said parameters obtained by means of the present invention may be compared to another set of genetic and/or epigenetic parameters, the differences serving as the basis for a diagnosis and/or prognosis of events which are disadvantageous to patients or individuals.
More specifically the present invention enables the screening of at-risk populations for the early detection of colorectal carcinomas. Furthermore, embodiments enable the detection of aerodigestive cancers and thus are additionally suitable for this purpose. Further embodiments of the method may also be used as alternatives to cytological screening for the differentation of colorectal carcinomas from other colon cell proliferative disorders, e.g. colon polyps.
Specifically, the present invention provides for diagnostic cancer assays based on measurement of differential methylation of one or more CpG dinucleotide sequences of SEQ ID NO: 5, or of subregions thereof that comprise such a CpG dinucleotide sequence. Typically, such assays involve obtaining a tissue sample from a test tissue, performing an assay to measure the methylation status of at least one of one or more CpG dinucleotide sequences of SEQ ID NO: 5 derived from the tissue sample, relative to a control sample, or a known standard and making a diagnosis or prognosis based thereon.
In particular preferred embodiments, inventive oligomers are used to assess the CpG dinucleotide methylation status, such as those based on SEQ ID NO: 5, 65, 66, 67 or 68, or arrays thereof, as well as in kits based thereon and useful for the diagnosis and/or prognosis of colorectal cell proliferative disorders.

Kits

Moreover, an additional aspect of the present disclosure is a kit comprising, for example: a bisulfite-containing reagent; a set of primer oligonucleotides containing at least two oligonucleotides whose sequences in each case correspond, are complementary, or hybridize under stringent or highly stringent conditions to a 16-base long segment of the sequences SEQ ID NOs: 5, 65, 66, 67 or 68; oligonucleotides and/or PNA-oligomers; as well as instructions for carrying out and evaluating the described methodSaid kit may further comprise standard reagents for performing a CpG position-specific methylation analysis, wherein said analysis comprises one or more of the following techniques: MS-SNuPE, MSP, MethyLight ™, HeavyMethyl™ , COBRA, and nucleic acid sequencing. However, a kit can also contain only part of the aforementioned components.

While the present invention has been described with specificity in accordance with certain of its preferred embodiments, the following examples serve only to illustrate the invention and are not intended to limit the invention within the scope of the claims.

## EXAMPLES

Samples were received either as frozen tissue or extracted genomic DNA. DNA samples were extracted using lysis buffer from Qiagen and the Roche magnetic separation kit for genomic DNA isolation. DNA samples were also extracted using Qiagen Genomic Tip-100 columns, as well as the MagnaPure device and Roche reagents. All samples were quantitated using spectrophotometric or fluorometric techniques and on agarose gels for a subset of samples.

Bisulfite treatment and mPCR

Total genomic DNA of all samples was bisulfite treated converting unmethylated cytosines to uracil. Methylated cytosines remained conserved. Bisulfite treatment was performed with minor modifications according to the protocol described in Olek et al. (1996). In order to avoid processing all samples with the same biological background together resulting in a potential process-bias in the data later on, the samples were randomly grouped into processing batches. For bisulfite treatment we created batches of 50 samples randomized for sex, diagnosis, and tissue. Per DNA sample two independent bisulfite reactions were performed. After bisulfitation 10 ng of each DNA sample was used in subsequent mPCR reactions containing 6-8 primer pairs.

Each reaction contained the following:

0.4 mM each dNTPS
1 Unit Taq Polymerase
2.5 μl PCR buffer
3.5 mM MgCl2
80 nM Primerset (12-16 primers)
11.25 ng DNA (bisulfite treated)

Further details of the primers are shown in TABLE 1.

Forty cycles were carried out as follows: Denaturation at 95°C for 15 min, followed by annealing at 55°C for 45 sec., primer elongation at 65°C for 2 min. A final elongation at 65°C was carried out for 10 min.

1.1.2 Hybridization

All PCR products from each individual sample were then hybridised to glass slides carrying a pair of immobilised oligonucleotides for each CpG position under analysis. Each of these detection oligonucleotides was designed to hybridise to the bisulphite converted sequence around one CpG site which was either originally unmethylated (TG) or methylated (CG). See Table 2 for further details of all hybridisation oligonucleotides used for the ALX4 gene (both informative and non-informative.) Hybridisation conditions were selected to allow the detection of the single nucleotide differences between the TG and CG variants.

5 μl volume of each multiplex PCR product was diluted in 10 x Ssarc buffer (10 x Ssarc:230 ml 20 x SSC, 180 ml sodium lauroyl sarcosinate solution 20%, dilute to 1000 ml with dH2O). The reaction mixture was then hybridised to the detection oligonucleotides as follows. Denaturation at 95°C, cooling down to 10 °C, hybridisation at 42°C overnight followed by washing with 10 x Ssarc and dH2O at 42°C.

Further details of the hybridisation oligonucleotides for the ALX4 gene are shown in TABLE 2.

Fluorescent signals from each hybridised oligonucleotide were detected using genepix scanner and software. Ratios for the two signals (from the CG oligonucleotide and the TG oligonucleotide used to analyse each CpG position) were calculated based on comparison of intensity of the fluorescent signals.

The samples were processed in batches of 80 samples randomized for sex, diagnosis, tissue, and bisulphite batch For each bisulfite treated DNA sample 2 hybridizations were performed. This means that for each sample a total number of 4 chips were processed.

Data analysis methods

Analysis of the chip data:

From raw hybridization intensities to methylation ratios;

The log methylation ratio (log(CG/TG)) at each CpG position is determined according to a standardized preprocessing pipeline that includes the following steps:

For each spot the median background pixel intensity is subtracted from the median foreground pixel intensity (this gives a good estimate of background corrected hybridization intensities):

For both CG and TG detection oligonucleotides of each CpG position the background corrected median of the 4 redundant spot intensities is taken;
For each chip and each CpG position the log(CG/TG) ratio is calculated;
For each sample the median of log(CG/TG) intensities over the redundant chip repetitions is taken.
This ratio has the property that the hybridization noise has approximately constant variance over the full range of possible methylation rates (Huber et al., 2002).

Principle Component Analysis

The principle component analysis (PCA) projects measurement vectors (e.g. chip data, methylation profiles on several CpGs etc.) onto a new coordinate system. The new coordinate axes are referred to as principal components. The first principal component spans the direction of the largest variance of the data. Subsequent components are ordered by decreasing variance and are orthogonal to each other. Different CpG positions contribute with different weights to the extension of the data cloud along different components. PCA is an unsupervised technique, *i.e.*, it does not take into account the labels of the data points (for further details see e.g. Ripley (1996)).

PCA is typically used to project high dimensional data (in our case methylation-array data) onto lower dimensional subspaces in order to visualize or extract features with high variance from the data. In the present report we use 2 dimensional projections for statistical quality control of the data. We investigate the effect of different process parameters on the chip data and exclude that changing process parameters cause large alterations in the measurement values.

A robust version of PCA is used to detect single outlier chips and exclude them from further analysis (Model et al., 2002).

Hynothesis testing

The main task is to identify markers that show significant differences in the average degree of methylation between two classes. A significant difference is detected when the nullhypothesis that the average methylation of the two classes is identical can be rejected with $p<0.05$. Because we apply this test to a whole set of potential markers we have to correct the p-values for multiple testing. This was done by applying the False Discovery Rate (FDR) method (Dudoit et al., 2002).

For testing the null hypothesis that the methylation levels in the two classes are identical we used the likelihood ratio test for logistic regression models (Venables and Ripley, 2002). The logistic regression model for a single marker is a linear combination of methylation measurements from all CpG positions in the respective genomic region of interest (ROI). A significant p-value for a marker means that this ROI has some systematic correlation to the question of interest as given by the two classes. However, at least formally it makes no statement about the actual predictive power of the marker.

Class prediction by supervised learning

In order to give a reliable estimate of how well the CpG ensemble of a selected marker can differentiate between different tissue classes we can determine its prediction accuracy by classification. For that purpose we calculate a methylation profile based prediction function using a certain set of tissue samples with their class label. This step is called training and it exploits the prior knowledge represented by the data labels. The prediction accuracy of that function is then tested by cross-validation or on a set of independent samples. As a method of choice, we use the support vector machine (SVM) algorithm (Duda (2001), Christiannini (2000)) to learn the prediction function. If not stated otherwise, for this report the risk associated with false positive or false negative classifications are set to be equal relative to the respective class sizes. It follows that the learning algorithm obtains a class prediction function with the objective to optimize accuracy on an independent test sample set. Therefore sensitivity and specificity of the resulting classifier can be expected to be approximately equal.

Estimating the performance of the tissue class prediction: Cross Validation

With limited sample size the cross-validation method provides an effective and reliable estimate for the prediction accuracy of a discriminator function and therefore in addition to the significance of the markers we provide cross-validation accuracy, sensitivity and specificity estimates. For each classification task, the samples were partitioned into 5 groups of approximately equal size. Then the learning algorithm was trained on 4 of these 5 sample groups. The predictor obtained by this method was then tested on the remaining group of independent test samples. The number of correct positive and negative classifications was counted over 5 runs for the learning algorithm for all possible choices of the independent test group without using any knowledge obtained from the previous runs. This procedure was repeated on up to 10 random permutations of the sample set. Note that the above-described cross-validation procedure evaluates accuracy, sensitivity and specificity using practically all possible combinations of training and independent test sets. It therefore gives a better estimate of the prediction performance than simply splitting the samples into one training sample set and one independent test set.

Results

Figures 1, 5, 9, 13, 16, 20, 24, 28 and 32 show ranked matrices of data obtained according to Examples 1 and 2 according to CpG methylation differences between the two classes of tissues, using an algorithm. The figures are shown in greyscale, wherein the most significant CpG positions are at the bottom of the matrix with significance decreasing towards the top. Black indicates total methylation at a given CpG position, white represents no methylation at the particular position, with degrees of methylation represented in grey, from light (low proportion of methylation) to dark (high proportion of methylation). Each row represents one specific CpG position within a gene and each column shows the methylation profile for the different CpGs for one sample. On the right side p values for the individual CpG positions are shown. The p values are the probabilities that the observed distribution occurred by chance in the data set.
Figure 38 shows a ranked matrices of data obtained according to Examples 1 and 2 according to CpG methylation differences between the two classes of tissues, (aerodigestive cancer on the left and healthy aerodigestive tissues on the right) using an algorithm. The most significant CpG positions are at the bottom of the matrix with significance decreasing towards the top. Red indicates total methylation at a given CpG position, green represents no methylation at the particular position.. Each row represents one specific CpG position within a gene and each column shows the methylation profile for the different CpGs for one sample.
Figures 2, 6, 10, 17, 21, 25, 29 and 33 show the uncorrected multivariate LogReg test results for each comparison. Each individual genomic region of interest is represented as a point, the dotted line represents the cut off point for the 25% false discovery rate.
Figures 3, 7, 11, 14, 18, 22, 26, 30 and 34 show ranked matrices of data obtained according to Examples 1 and 2 of the accuracy of the genewise linear support vector machine cross validations between the two classes of tissues, for the best performing markers. The figures are shown in greyscale, wherein the most significant CpG positions are at the bottom of the matrix with significance decreasing towards the top. Black indicates total methylation at a given CpG position, white represents no methylation at the particular position, with degrees of methylation represented in grey, from light (low proportion of methylation) to dark (high proportion of methylation). Each row represents one specific CpG position within a gene and each column shows the methylation profile for the different CpGs for one sample. On the right side accuracy values for each individual genomic region of interest are shown.
Figures 4, 8, 12, 15, 18, 23, 27, 31 and 35 shows the accuracy of the genewise linear support vector machine cross validation for all genomic regions of each classification. The accuracy of each genomic region is represented as black squares, the specificity as unfilled diamonds, the sensitivity as unfilled squares. The accuracy as measured on the X-axis shows the fraction of correctly classified samples.

Colon Normal vs. Colorectal Cancer

In the first comparison 102 colorectal carcinoma samples were compared to 73 samples from normal colon, including colon polyps and colon inflammatory disorders.
Figure 1 shows the multivariate test results of the top 12 performing markers using the conservative Bonferroni corrected LogReg test.

Figure 2 shows the uncorrected multivariate LogReg test results for each comparison. Each individual genomic region of interest is represented as a point, the dotted line represents the cut off point for the 25% false discovery rate.

Figure 3 shows the accuracy of the top 12 performing markers.

Figure 4 shows the accuracy of the genewise linear support vector machine cross validation for all genomic regions of each classification.

The following genomic markers were evaluated as being significant:

SEQ ID NOS:1-12, 15-20, 22, 25-36, 38-49, 51-58, and complements thereof.

<u>Other Tissues vs. Colorectal Cancer</u>

In this classification 73 colorectal carcinoma samples were compared to an 'other tissue' class consisting of 140 samples from non-colorectal carcinomas, peripheral blood lymphocytes and other normal tissues of non-colorectal origin. These markers therefore enable the detection of colorectal carcinoma cells in', for example, body fluids such as serum.

Figure 5 shows the multivariate test results of the top 12 performing markers using the conservative Bonferroni corrected LogReg test.

Figure 6 shows the uncorrected multivariate LogReg test results for each comparison. Each individual genomic region of interest is represented as a point, the dotted line represents the cut off point for the 25% false discovery rate.

Figure 7 shows the accuracy of the top 12 performing markers.

Figure 8 shows the accuracy of the genewise linear support vector machine cross validation for all genomic regions of each classification.

The following genomic markers were evaluated as being significant:

SEQ ID NOS:1-23, 26-36, 38-43, 45-49, 51-58 and complements thereof.

<u>Colon Normal and Other Tissue vs. Colon Cancer</u>

In this classification 73 colorectal carcinoma samples were compared to 242 colon normal and 'other tissue' samples. The colon normal class consisted of healthy colon, colon polyps and inflammatory disorder colon tissue samples, the 'other tissues' consisted of samples from non-colorectal carcinomas, peripheral blood lymphocytes and other normal tissues of non-colorectal origin.

Figure 9 shows the multivariate test results of the top 12 performing markers using the conservative Bonferroni corrected LogReg test.

Figure 10 shows the uncorrected multivariate LogReg test results for each comparison. Each individual genomic region of interest is represented as a point, the dotted line represents the cut off point for the 25% false discovery rate.

Figure 11 shows the accuracy of the top 12 performing markers.

Figure 12 shows the accuracy of the genewise linear support vector machine cross validation for all genomic regions of each classification.

The following genomic markers were evaluated as being significant:

SEQ ID NOS:1-3, 5-13, 15-23, 26-36, 38-43, 45-49, 51-58, and complements thereof.

<u>Polyps vs. Colorectal Cancer</u>

In this classification 73 colorectal carcinoma samples were compared to 51 colon polyp samples.

Figure 13 shows the multivariate test results of the top performing markers using the conservative Bonferroni corrected LogReg test.

Figure 14 shows the accuracy of the top 12 performing markers.

Figure 15 shows the accuracy of the genewise linear support vector machine cross validation for all genomic regions of each classification.

The following genomic markers were evaluated as being significant:

SEQ ID NOS:11, 25, 27, 38, 40, 45, 53, and complements thereof.

Colon normal vs. Colorectal cell proliferative disorder

In this classification 124 colorectal cell proliferative disorder samples (consisting of colon polyps and colorectal carcinoma) were compared to 51 'normal colon' samples consisting of both healthy colon samples and colon tissue of inflammatory disorders.
Figure 16 shows the multivariate test results of the top 12 performing markers using the conservative Bonferroni corrected LogReg test.
Figure 17 shows the uncorrected multivariate LogReg test results for each comparison. Each individual genomic region of interest is represented as a point, the dotted line represents the cut off point for the 25% false discovery rate.
Figure 18 shows the accuracy of the top 12 performing markers.
Figure 19 shows the accuracy of the genewise linear support vector machine cross validation for all genomic regions of each classification.
The following genomic markers were evaluated as being significant:

SEQ ID NOS:1-58, and complements thereof.

Colon Normal vs. Colorectal Cancer

In this classification 73 colorectal carcinoma samples were compared to 51 'normal colon' samples consisting of both healthy colon samples and colon tissue of inflammatory disorders.
Figure 20 shows the multivariate test results of the top 12 performing markers using the conservative Bonferroni corrected LogReg test.
Figure 21 shows the uncorrected multivariate LogReg test results for each comparison. Each individual genomic region of interest is represented as a point, the dotted line represents the cut off point for the 25% false discovery rate.
Figure 22 shows the accuracy of the top 12 performing markers.
Figure 23 shows the accuracy of the genewise linear support vector machine cross validation for all genomic regions of each classification.
The following genomic markers were evaluated as being significant:

SEQ ID NOS:1-3, 5-23, 25-36, 38-49, 51-58, and complements thereof.

Colon Normal and Other Tissues vs. Colorectal cell proliferative Disorder

In this classification 124 colorectal cell proliferative disorder samples (consisting of colon polyps and colorectal carcinoma) were compared to a class consisting of 'colon normal' and 'other tissue' samples. The colon normal samples consisted of both healthy colon samples and colon tissue of inflammatory disorders, the 'other tissue' samples consisted of samples from non-colorectal carcinomas, peripheral blood lymphocytes and other normal tissues of non-colorectal origin.
Figure 24 shows the multivariate test results of the top 12 performing markers using the conservative Bonferroni corrected LogReg test.
Figure 25 shows the uncorrected multivariate LogReg test results for each comparison. Each individual genomic region of interest is represented as a point, the dotted line represents the cut off point for the 25% false discovery rate.
Figure 26 shows the accuracy of the top 12 performing markers.
Figure 27 shows the accuracy of the genewise linear support vector machine cross validation for all genomic regions of each classification.
The following genomic markers were evaluated as being significant:

SEQ ID NOS:1-36, 38-43, 45-58, and complements thereof.

Other Tissue vs. Colon Tissue

The following comparison was carried out in order to identify markers capable of discerning elevated levels of free floating colon DNA, especially in bodily fluids as a marker of tumor progression. In this classification the 'colon tissue' class consisted of samples from colorectal carcinoma, colon polyps, colon tissue of inflammatory disorders and healthy colon tissue. The 'other tissue' class consisted of samples from non-colorectal carcinomas, peripheral blood lymphocytes and other normal tissues of non-colorectal origin.
Figure 28 shows the multivariate test results of the top 12 performing markers using the conservative Bonferroni corrected LogReg test.

Figure 29 shows the uncorrected multivariate LogReg test results for each comparison. Each individual genomic region of interest is represented as a point, the dotted line represents the cut off point for the 25% false discovery rate.
Figure 30 shows the accuracy of the top 12 performing markers.
Figure 31 shows the accuracy of the genewise linear support vector machine cross validation for all genomic regions of each classification.
The following genomic markers were evaluated as being significant:

SEQ ID NOS:1-58, and complements thereof.

<u>Normal Tissue vs. Cell Proliferative Disorder Tissue</u>

In this classification the gene panel was assessed for its ability to accurately discriminate cell proliferative disorder samples from both colorectal carcinoma, colon polyps and non-colon origin cancers from 'normal tissues', namely healthy colon samples, colon tissue of inflammatory disorders, peripheral blood lymphocytes, other normal tissues of non-colorectal origin.
Figure 32 shows the multivariate test results of the top 12 performing markers using the conservative Bonferroni corrected LogReg test.
Figure 33 shows the uncorrected multivariate LogReg test results for each comparison. Each individual genomic region of interest is represented as a point, the dotted line represents the cut off point for the 25% false discovery rate.
Figure 34 shows the accuracy of the top 12 performing markers.
Figure 35 shows the accuracy of the genewise linear support vector machine cross validation for all genomic regions of each classification.
The following genomic markers were evaluated as being significant:

SEQ ID NOS:1-36, 38-43, 45-49, 51-58, and complements thereof.

<u>Aerodigestive cancer v. healthy aerodigestive tissues</u>

<u>In this classification the term 'aerodigestive' describes a sample set of tissues consisting</u> lung, liver, pancreas, bile duct, stomach, and colon. Figure 38 shows a ranked matrices of data obtained according to Examples 1 and 2 according to CpG methylation differences between the two classes of tissues, (Cancerous tissues on the left of the matrix were compared to normal tissues on the right of the matrix.) using an algorithim. The most significant CpG positions are at the bottom of the matrix with significance decreasing towards the top. Red indicates total methylation at a given CpG position, green represents no methylation at the particular position.. Each row represents one specific CpG position within a gene and each column shows the methylation profile for the different CpGs for one sample.The following genomic markers were considered significant SEQ ID NOS: 20, 41, 35, 3, 39 & 33.
The following examples describe the analysis of the methylation status of the genomic sequences SEQ ID NO:35, SEQ ID NO:34, SEQ ID NO:39, SEQ ID NO:29 in healthy and sick colorectal cell proliferative disorder samples. The initial link between said genes and colorectal cell proliferative disorders was initially carried by means of hybridisation analysis as described in EXAMPLE 1. The sequences were then selected from the larger set of genes analysed in said example, and the correlation between methylation status and colorectal cell proliferative disorder states was validated by analysis of samples using other methylation analysis techniques, namely the MSP- MethyLight™ and HeavyMethyl ™ MethyLight ™ assays. Please note that the term 'MethyLight™' is used to describe real time PCR analysis of bisulfite treated DNA using probes of both the Taqman™ (single probe) and Lightcycler™ (dual probe) technologies.

**Example 11:**

**Real time analysis of methylation sensitive restriction.**

DNA was prepared from from ten colon tumor samples and ten normal adjacent tissue samples. DNA isolation was performed using standard methods known to the expert and quantified by UV measurement.
A digestion reaction was performed with the following reagents:

1 $\mu$g genomic DNA;
25 U enzyme (10U/$\mu$l);
Y+/Tango Buffer
By incubation 37°C.

The following steps were performed for all samples in parallel. 1μg of DNA was dissolved in 190μl Y+/Tango Buffer (Fermentas) in 0,2ml PCR tubes. 95μl of the reaction solution (half of the overall volume) was pipetted into a clean PCR tube as a control. To the remaining reagent in the first tube 1,25μl HpaII and 1,25μl Hin6I restriction endunuclease (both from Fermentas)were added.

To the second tube 2,5μl of a mixture of glycerol and water (v:v =1:1) was added. After incubating all 40 tubes at 37°C in an Eppendorff Thermocycler for five hours. Then another aliquot of restriction enzyme, resp. water-glycerol mix, was added to each tube and the incubation was extended for ten hours. After this period of time the restriction enzymes were inactivated by heating at 65°C for 10 minutes.

Subsequently, dilutions of each tube were assembled in 96well plates by transferring 20μl from each tube into the plates and adding 80μl water.

Finally, both the restriction product and the control of each sample were analyzed by Quantitative Real Time PCR. In this example 12μl of a SybrGreen containing PCR mastermix containing one of the primers according to table 4 was pipetted into a 96well Taqman plate followed by 8μl of the corresponding dilutions of restricted sample and blank (each in duplicate).

Real Time PCR was performed on a ABI 7700 instrument applying the following temperature profile: 15 minutes at 95°C; 50 cycles of: 15 seconds at 95°C, 45 seconds at 65°C, 75 seconds at 72°C; 15 seconds at 95°C followed by 50°C for one hour.

Using the described primers and conditions amplification is observed only for methylated copies of the analysed sequences (according to table 4) when methylation sensitive restriction has been performed prior to PCR, since unmethylated copies are digested during this step. The amount of methylated DNA after restriction is normalized against the total amount of DNA present before the restriction as obtained by Real Time quantitation of the blank of the same sample. The equation

$$(1) \qquad \Delta Ct = Ct_{blank} - Ct_{restricted}$$

represents a measure for the relative amount of unmethylated DNA present in a DNA sample. A value of zero means that the DNA is completely methylated. A value of 0.5 corresponds to approximately 50% methylation in case of an optimised PCR reactions whereas larger values correspond to low methylation levels. The relative amount of methylated sequence present in a sample would be given by

$$(2) \qquad M = E^{-\Delta Ct}$$

where E is the PCR efficiency. The PCR efficiency is easily obtained by methods known to the expert.

Table 4

| Gene | Forward primer | Reverse Primer | Results Figure |
|---|---|---|---|
| | | | |
| SEQ ID NO: 5 | GCTAAGCCGGAGTCACCAAG (SEQ ID NO:277) | CGCTGAGACCGAGAAGAAG (SEQ ID NO:278) | 36 |

Results are shown in Figure 36. Each individual sample is annotated on the x-axis as 'n' denoting normal, our 't' denoting tumour. A bronchial tissue sample was accidentally analysed in some assays, these are annotated with an arrow, and are irrelevant in the context of the present invention. The Y-axis shows the level of methylation ('methylation rate') in the sample. The methylation rate {methylated/(methylated + unmethylated) are shown for the fragments corresponding to the primer pairs in Table 4. Formula (2) above was used for the calculation assuming a PCR efficiency of E = 2.

The data demonstrate that for several fragments the majority of the tumor samples show significantly higher methylation rates than the corresponding normal adjacent tissues.

**Example 12**

mRNA expression analysis

1. RNA samples

'Total RNA from normal colon (n=1) and colon cancer (adenocarcinoma; n=1) was obtained from Ambion (Huntingdon, United Kingdom). Each RNA sample was derived from a single donor. Total cellular RNA was prepared using the ToTALLY RNA Kit (Ambion, Huntingdon, United Kingdom) and Dnase-treatment was performed to minimize contamination. RNA integrity was assessed before and after 14 hours incubation at 37°C on an Agilent Bioanalyzer (Agilent Technologies, Inc, Palo Alto, CA).

2. Microarray Experiments

Target preparation and labeling, microarray hybridization, washing, and scanning was performed according to the manufacturer's protocol (Affymetrix, Inc., Santa Clara, CA). Briefly, 10 μg of total RNA were reverse transcribed using a T7-linked oligo-dT primer, followed by second strand synthesis. Biotin-labeled cRNA targets were prepared from double stranded cDNA using T7 RNA polymerase and biotinylated ribonucleotides (UTP, CTP). Fragmentation was performed at 94°C for 35 minutes. 15 μg of the fragmented biotinylated cRNA were mixed with MES buffer (2-(N-Morpholino)ethanesulfonic acid) containing 0.5 mg/ml acetylated bovine serum albumin and hybridised to Affymetrix GeneChip HG_U133A arrays (Santa Clara, CA) for 16 hours at 45°C. Microarrays were stained using streptavidin phycoerythrin conjugates (Molecular Probes, Inc., Eugene, OR). For signal amplification biotinylated anti-streptavidin antibodies (Vector Laboratories, Burlingame, CA) were used.

Arrays were scanned on an Affymetrix GeneChip Scanner. The expression values for each probe set (representing a gene) were calculated from the raw data using Bioconductor R packages (http://www.bioconductor.org, see below).

3. Data Analysis

The raw expression data obtained from the Affymetrix scanner (the so called cel-files) were background corrected and normalized using the default settings of the RMA normalization procedure (Irizarry RA, Hobbs B, Collin F, Beazer-Barclay YD, Antonellis KJ, Scherf U, Speed TP: Exploration, normalization, and summaries of high density oligonucleotide array probe level data. Biostatistics. 2003 Apr;4(2):249-64) as implemented in the Bioconductor R packages, release 1.4 (Gautier L, Cope L, Bolstad BM, Irizarry RA: affy--analysis of Affymetrix GeneChip data at the probe level. Bioinformatics. 2004 Feb 12;20(3):307-150). The raw data processing results in signal intensity values for each of the 22.293 probe sets available on the Affymetrix GeneChip HG_U133A chip.

To identify differentially expressed probe sets, the fold change of the normalized signal intensities between colon normal tissue and colon tumour tissue was calculated. Genes with a fold change bigger than 2 (or smaller than 0.5) were identified as differentially expressed. Differentially expressed genes are shown in Table 5, as can be seen hypermethylation does not correlate with underexpression in all cases.

**Table 5**

| Affymetrix Probe ID | Gene SEQ ID NO: | log-fold change | fold-change | up/down in cancer |
|---|---|---|---|---|
| 208944 at | 37 | -1,0 | 2,0 | down |
| 209945 s at | 27 | 1,2 | 2,3 | up |
| 214211 at | 54 | -1,3 | 2,5 | down |
| 203140 at | 16 | -1,8 | 3,4 | down |
| SEQ ID NO: 3497_at | 15 | -1,4 | 2,6 | down |

**Example 14**

MSP analysis of the genes according to Table 6 (below).

In the following analysis the methylation status of the ALX4 gene was analysed by means of methylation specific amplification using the primers according to Table 6 (below).

The study was run on approximately 140 samples from colon, breast and liver carcinoma, normal tissue (peripheral blood lymphocytes, healthy colon and healthy tissue adjacent to colon carcinoma), colon polyps and other colon diseases. The positive class consisted of Colon Cancer and Colon polyps. The negative class was PBL (peripheral blood lymphocytes), Colon-Normal, Colon-NAT and Colon-Inflammatory. For AUC values in brackets (see table 7, below) Breast-Cancer and Liver-Cancer were also included in the negative class.

Genomic DNA was analyzed using the MSP technique after bisulfite conversion. Total genomic DNA of all samples was bisulfite treated converting unmethylated cytosines to uracil. Methylated cytosines remained conserved. Bisulfite treatment was performed with minor modifications according to the protocol described in Olek et al. (1996).

The sequence of interest was then amplified by means of methylation specific primers, the amplificate is then detected by means of methylation specific Taqman probes.

//

//

//

**Table 6: Oligonucleotides for MSP Taqman.**

| Assay number | Gene/Genomic location | Forward primer sequence | Probe | Reverse primer sequence | Amplification curve |
|---|---|---|---|---|---|
| 11 | HOMEOBO X PROTEIN ARISTALES S-LIKE 4 | tacgtgtgagggg ttcgc | ccaatcgaaacta tccaaatacgata accga | ccaataaaattc gtaaaaaaaacg | Figure 37 |

Reaction Conditions for Taqman PCR program:

Denaturation at 95°C for 10 minutes.

50 cycles: Denaturation at 95°C for 15 seconds.
Annealing at 62°C for 1 minute..

Amplification curves for each assay are shown in figure 37 (according to Table 6).

The technical performance of a series of alternative assays was validated by testing each of the following using sperm DNA as the template:

Gene SEQ ID NO: 5 (HeavyMethyl™ assay)

| | | | | |
|---|---|---|---|---|
| **primer:** | | | | |
| Gagaggtttattaaggtaggg (SEQ ID NO: 14584) | | | | |
| Ctactaccaaaaataaaaaattattaac (SEQ ID NO: 14585) | | | | |
| **blocker:** | | | | |
| Aaaattattaacatccaacacatttatccaaacttcaaa (SEQ ID NO: 14586) | | | | |
| **probes:** | | | | |
| gtttaggggtttgtagagaagttcga-fluo (SEQ ID NO: 14587) | | | | |

(continued)

| | | | | |
|---|---|---|---|---|
| **probes:** | | | | |
| red640-tcggataaacgcgtcgg-pho (SEQ ID NO: 14588) | | | | |
| | | | | |
| **LightCycler program:** | | | | |
| activation: | 95°C | 10min | | |
| | | | | |
| 55 cycles: | 95°C | 10 sec | (20°C/s) | |
| | **56°C** | 30 sec | (20°C/s) | detection |
| | 72°C | 10 sec | (20°C/s) | |
| | | | | |
| melting curve: | 95°C | 10sec | 20 | |
| | 40°C | 10sec | 20 | |
| | 70°C | 0sec | 0,1 | |
| | | | | |
| cooling: | 40°C | 5 sec | | |

Results

Sensitivity and specificity were computed with SVM and 10 fold cross validation

Table 7

| Gene/Genomic location | AUC | Sensitivity | Specificity |
|---|---|---|---|
| HOMEOBOX PROTEIN ARISTALESS-LIKE 4 | 0.78 (0.75) | 0.74 | 0.95 |

**FURTHER TABLES**

TABLE 1.

| ***Gene:*** | ***Primer:*** | ***Amplificate Length:*** |
|---|---|---|
| 23_ (SEQ ID NO: 5) | CTCCTCCTTCCAACAAAAA (SEQ ID NO: 69) | 487 |
| | GTTTAGAGGTTTTGGGATGATT (SEQ ID NO: 70) | |
| 24_ (SEQ ID NO: 5) | TGAATAGGGTGATATTTTAGTTAGG (SEQ ID NO: 71) ATAAATCATCCCAAAACCTCTA (SEQ ID NO: 72) | 497 |

TABLE 2.

| ***No:*** | ***Gene*** | ***Oligo:*** |
|---|---|---|
| 17 | (SEQ ID NO: 5) | GACGTCGGTACGTAGT (SEQ ID NO: 73) |
| 18 | | GATGTTGGTATGTAGTAG |

(continued)

| No: | Gene | Oligo: |
|---|---|---|
| | (SEQ ID NO: 5) | (SEQ ID NO: 74) |
| 19 | (SEQ ID NO: 5) | TTCGGGGGAATTCGAGT (SEQ ID NO: 75) |
| 20 | (SEQ ID NO: 5) | TTTGGGGGAATTTGAGT (SEQ ID NO: 76) |
| 21 | (SEQ ID NO: 5) | TATTGCGAGGATTCGG (SEQ ID NO: 77) |
| 22 | (SEQ ID NO: 5) | ATTGTGAGGATTTGGT (SEQ ID NO: 78) |
| 23 | (SEQ ID NO: 5) | GTGCGTTCGTAGCGTA (SEQ ID NO: 79) |
| 24 | (SEQ ID NO: 5) | TGTGTTTGTAGTGTAGG (SEQ ID NO: 80) |
| 25 | (SEQ ID NO: 5) | GGACGTCGTTTGTTAG (SEQ ID NO: 81) |
| 26 | (SEQ ID NO: 5) | GGATGTTGTTTGTTAGG (SEQ ID NO: 82) |
| 27 | (SEQ ID NO: 5) | AGAGCGTCGTTTTGTA (SEQ ID NO: 83) |
| 28 | (SEQ ID NO: 5) | AGAGTGTTGTTTTGTAT (SEQ ID NO: 84) |
| 29 | (SEQ ID NO: 5) | TTTCGAGGGTAGGCGAG (SEQ ID NO: 85) |
| 30 | (SEQ ID NO: 5) | TTTTGAGGGTAGGTGAG (SEQ ID NO: 86) |
| 31 | (SEQ ID NO: 5) | TTTCGATTTTAATGCGAA (SEQ ID NO: 87) |
| 32 | (SEQ ID NO: 5) | TTTGATTTTAATGTGAAGT (SEQ ID NO: 88) |
| 33 | (SEQ ID NO: 5) | AGGAATTTCGTCGCGA (SEQ ID NO: 89) |
| 34 | (SEQ ID NO: 5) | AGGAATTTTGTTGTGAT (SEQ ID NO: 90) |
| 35 | (SEQ ID NO: 5) | TTTGAGTCGTACGCGT (SEQ ID NO: 91) |
| 36 | (SEQ ID NO: 5) | TTTTGAGTTGTATGTGT (SEQ ID NO: 92) |

Table 3: Genes or genomic locus associated with genomic sequences according to SEQ ID NO 1- SEQ ID NO 64.

| Genomic SEQ ID NO: | Bisulfite SEQ ID Nos: | Genbank Ref. ID No:/contig | Description |
|---|---|---|---|
| 1 | | AL355481.12.1.12Hypothetical 2435 | Hypothetical protein-leucine rich repeat (Vega gene ID OTTHUMG0O013001328) |
| 2 | | APC027601.25.1.1 92732 | 5 azacytidine induced (Ensembl gene ID 141577) |
| 3 | | AL831711.4.1.880 1 | No known gene, close to ATP/GTP-binding site motif A (P-loop) protein |
| 4 | | NM_002938 | RING FINGER PROTEIN 4; RNF4 |
| 5 | 65, 66, 67 & 68 | NM_021926 | HOMEOBOX PROTEIN ARISTALESS-LIKE 4;. ALX 4 |
| 6 | | NM_004852 | ONE CUT DOMAIN FAMILY MEMBER 2; ONC2 |
| 7 | | NM_002507 | TUMOR NECROSIS FACTOR RECEPTOR SUPERFAMILY MEMBER 16 PRECURSOR (LOW-AFFINITY NERVE GROWTH FACTOR RECEPTOR) (NGF RECEPTOR) (GP80-LNGFR) (P75 ICD) (LOW AFFINITY NEUROTROPHIN RECEPTOR P75NTR). NGFR |
| 8 | | NM_016192 | TRANSMEMBRANE PROTEIN WITH EGF-LIKE AND TWO FOLLISTATIN-LIKE DOMAINS 2; TRANSMEMBRANE PROTEIN TENB2; TOMOREGULIN; PUTATIVE TRANSMEMBRANE PROTEIN WITH EGF-LIKE AND TWO FOLLISTATIN-LIKE DOMAINS 2. |
| 9 | | NM_005221 | HOMEOBOX PROTEIN DLX-5 |
| 10 | | NM_080552 | VESICULAR INHIBITORY AMINO ACID TRANSPORTER (GABA AND GLYCINE TRANSPORTER) (VESICULAR GABA TRANSPORTER) (HVIAAT) |
| 11 | | NM_001208 | Transcription factor BTF3 Homolog 1 |
| 12 | | NM_005904 | MOTHERS AGAINST DECAPENTAPLEGIC HOMOLOG 7 (SMAD 7) (MOTHERS AGAINST DPP HOMOLOG 7) (SMAD7) (HSMAD7). |
| 13 | | NM_002146 | HOMEOBOX PROTEIN HOX-B3 (HOX-2G) (HOX-2.7) |
| 14 | | NM_025078 | Homo sapiens hypothetical protein FLJ22378 |
| 15 | | NM_001116 | ADENYLATE CYCLASE, TYPE IX (EC 4.6.1.1) (ATP PYROPHOSPHATE-LYASE) (ADENYLYL CYCLASE). ADCY9 |
| 16 | | NM_001706 | B-CELL LYMPHOMA 6 PROTEIN (BCL-6) (ZINC FINGER PROTEIN 51) (LAZ-3 PROTEIN) (BCL-5). |
| 17 | | NM_014068 | SEEK1 PROTEIN |
| 18 | | NM_017745 | BCL-6 INTERACTING COREPRESSOR ISOFORM 1 BCOR |
| 19 | | NM_005643 | TRANSCRIPTION INITIATION FACTOR TFIID 28 KDA SUBUNIT (TAFII-28) (TAFII28) (TFIID SUBUNIT P30-BETA) |

(continued)

| Genomic SEQ ID NO: | Bisulfite SEQ ID Nos: | Genbank Ref. ID No:/contig | Description |
|---|---|---|---|
| 20 | | NM_007374 | HOMEOBOX PROTEIN SIX6 (SINE HOMEOBOX HOMOLOG 6) (OPTIC HOMEOBOX 2) (HOMEODOMAIN PROTEIN OPTX2). |
| 21 | | AP003500.2.1.161 078 | Situated between Ensemble gene IDs ENSESTG00002308609 and ENSESTG00002308691 |
| 22 | | AC092385.4.1.97 218 | SPIR-2 PROTEIN (FRAGMENT) |
| 23 | | AC007223.5.1.15 9520 | Situated within Ensemble EST ID ENSESTG00001971415 |
| 24 | | NM_005229 | ETS-DOMAIN PROTEIN ELK-1 |
| 25 | | NM_000179 | DNA MISMATCH REPAIR PROTEIN MSH6 (MUTS-ALPHA 160 KDA SUBUNIT) (G/T MISMATCH BINDING PROTEIN) (GTBP) (GTMBP) (P160) |
| 26 | | NM_005427 | TUMOR PROTEIN P73 (P53-LIKE TRANSCRIPTION FACTOR) (P53-RELATED PROTEIN). |
| 27 | | NM_002093 | GLYCOGEN SYNTHASE KINASE-3 BETA (EC 2.7.1.37) (GSK-3 BETA). |
| 28 | | NM_006765 | N33 PROTEIN |
| 29 | | NM_016192 | TRANSMEMBRANE PROTEIN WITH EGF-LIKE AND TWO FOLLISTATIN-LIKE DOMAINS 2; TRANSMEMBRANE PROTEIN TENB2; TOMOREGULIN; PUTATIVE TRANSMEMBRANE PROTEIN WITH EGF-LIKE AND TWO FOLLISTATIN-LIKE DOMAINS 2. |
| 30 | | NM_004429 | EPHRIN-B1 PRECURSOR (EPH-RELATED RECEPTOR TYROSINE KINASE LIGAND 2) (LERK-2) (ELK LIGAND) (ELK-L). |
| 31 | | NM_018950 | HLA-F gene for human leukocyte antigen F |
| 32 | | NM_000038 | ADENOMATOUS POLYPOSIS COLI PROTEIN (APC PROTEIN) |
| 33 | | NM_000610 | CD44 ANTIGEN PRECURSOR (PHAGOCYTIC GLYCOPROTEIN I) (PGP-1) (HITCH-1) (EXTRACELLULAR MATRIX RECEPTOR-III) (ECMR-III) (GP90 LYMPHOCYTE HOMING/ADHESION RECEPTOR) (HERMES ANTIGEN) (HYALURONATE RECEPTOR) (HEPARAN SULFATE PROTEOGLYCAN) (EPICAN) (CDW44) |
| 34 | | NM_004385 | VERSICAN CORE PROTEIN PRECURSOR (LARGE FIBROBLAST PROTEOGLYCAN) (CHONDROITIN SULFATE PROTEOGLYCAN CORE PROTEIN 2) (PG-M) (GLIAL HYALURONATE-BINDING PROTEIN) (GHAP) CSPG2 |
| 35 | | | EYA 4 EnsEMBL ID ENSG00000112319 |
| 36 | | NM_000455 | SERINE/THREONINE-PROTEIN KINASE 11 (EC 2.7.1.-) (SERINE/THREONINE- PROTEIN KINASE LKB1) |

(continued)

| Genomic SEQ ID NO: | Bisulfite SEQ ID Nos: | Genbank Ref. ID No:/contig | Description |
|---|---|---|---|
| 37 | | NM_003242 | Transforming growth factor, beta receptor II (TGFBR2) |
| 38 | | NM_001753 | CAVEOLIN-1; CAV 1 |
| 39 | | NM_001257 | CADHERIN-13 PRECURSOR (TRUNCATED-CADHERIN) (T-CADHERIN) (T-CAD) (HEART-CADHERIN) (H-CADHERIN) (P105) |
| 40 | | NM_000044 | ANDROGEN RECEPTOR (DIHYDROTESTOSTERONE RECEPTOR) AR |
| 41 | | NM_032546 | RING FINGER PROTEIN 30, RNF 30, |
| 42 | | NM_033178 | DOUBLE HOMEOBOX, 4; DOUBLE HOMEOBOX PROTEIN 4. DUX4 |
| 43 | | NM_003573 | LATENT TRANSFORMING GROWTH FACTOR BETA BINDING PROTEIN 4.; LTBP4 |
| 44 | | | CGI-20 PROTEIN |
| 45 | | NM_014459 | PROTOCADHERIN 17; PROTOCADHERIN 68.; PCDH 17 |
| 46 | | NM_005285 | PROBABLE G PROTEIN-COUPLED RECEPTOR GPR7.; GPR7 |
| 47 | | NM_016269 | LYMPHOID ENHANCER BINDING FACTOR 1 (LEF-1) (T CELL-SPECIFIC TRANSCRIPTION FACTOR 1-ALPHA) (TCF1-ALPHA) |
| 48 | | AC139426.2.1.18 8448 | Situated between EnsEMBL ID ENSESTG000O3302072 and EnsEMBL ID ENSESTG000O3302068 |
| 49 | | NM_005904 | MOTHERS AGAINST DECAPENTAPLEGIC HOMOLOG 7 (SMAD 7) (MOTHERS AGAINST DPP HOMOLOG 7) (SMAD7) (HSMAD7). |
| 50 | | AL512590.2.1.165 432 | Homo sapiens mRNA for KIAA1529 protein EnsEMBL ID ENSG00000029402 |
| 51 | | NM_001866 | COX7B |
| 52 | | NM_001900 | CST5 |
| 53 | | NM_001990 | EYES ABSENT HOMOLOG 3. EYA3 |
| 54 | | NM_002032 | FERRITIN HEAVY CHAIN (FERRITIN H SUBUNIT). FTH1. |
| 55 | | NM_002938 | RING FINGER PROTEIN 4.; RNF4 |
| 56 | | | HPP1 EnsEMBL ID ENSG00000144339 |
| 57 | | NM_000852 | GLUTATHIONE S-TRANSFERASE P (EC 2.5.1.18) (GST CLASS-PI) (GSTP1-1) |
| 58 | | NM_007084 | TRANSCRIPTION FACTOR SOX-21 |
| 59 | | NM_003299 | |
| 60 | | NM_025218 | |
| 61 | | NM_001374 | |

(continued)

| Genomic SEQ ID NO: | Bisulfite SEQ ID Nos: | Genbank Ref. ID No:/contig | Description |
|---|---|---|---|
| 62 | | NM_003714 | |
| 63 | | NM_002196 | |
| 64 | | NM_000484 | |

## Claims

1. A method for detecting colon cancer in a subject comprising:

   i) contacting genomic DNA isolated from blood plasma, blood serum, whole blood, isolated blood cells, cells isolated from the blood obtained from the subject with at least one reagent, or series of reagents that distinguishes between methylated and non-methylated CpG dinucleotides within at least one target region of the genomic DNA, wherein said target region comprises, or hybridizes under stringent conditions to at least 16 contiguous nucleotides of the gene or the sequence of ALX4; and
   ii) detecting colon cancer in a subject based on the methylation state of at least one target CpG dinucleotide sequence, or an average, or a value reflecting an average methylation state of a plurality of target CpG dinucleotide sequences in said target region.

2. The method of claim 1, wherein the target region comprises, or hybridizes under stringent conditions to at least 16 contiguous nucleotides of the sequence SEQ ID NO: 5, and complements thereof.

3. A method for detecting colon cancer in a subject comprising determining the expression levels of ALX 4 in a biological sample isolated from said subject, wherein sais sample is blood plasma, blood serum, whole blood, isolated blood cells, cells isolated from the blood obtained from said subject.

4. The method according to claim 3, wherein said expression level is determined by detecting the presence, absence or level of mRNA transcribed from said gene or sequence, or wherein said expression level is determined by detecting the presence, absence or level of a polypeptide encoded by said gene or sequence, or wherein said expression is determined by detecting the presence or absence of CpG methylation within said gene or sequence, wherein hypermethylation indicates the presence of a colon cell proliferative disorder.

5. The method according to any of claims 1 or 2, comprising:

   a) extracting or otherwise isolating genomic DNA from said sample obtained from said subject,
   b) treating the genomic DNA of a), or a fragment thereof, with one or more reagents to convert cytosine bases that are unmethylated in the 5-position thereof to uracil or to another base that is detectably dissimilar to cytosine in terms of hybridization properties;
   c) contacting the treated genomic DNA, or the treated fragment thereof, with an amplification enzyme and at least two primers comprising, in each case a contiguous sequence of at least 9 nucleotides that is complementary to, or hybridizes under moderately stringent or stringent conditions to a sequence selected from the group consisting of SEQ ID NO: 65, 66,67, and 68, and complements thereof, wherein the treated genomic DNA or the fragment thereof is either amplified to produce at least one amplificate, or is not amplified; and
   d) determining, based on a presence or absence of, or on a property of said amplificate, the methylation state of at least one CpG dinucleotide, or an average, or a value reflecting an average methylation state of a plurality of CpG dinucleotides in the sequence according to SEQ ID NO: 5.

6. The method of claim 5, wherein treating the genomic DNA, or the fragment thereof in b), comprises use of a reagent selected from the group consisting of bisulfite, hydrogen sulfite, disulfite, and combinations thereof.

**7.** The method of claim 5 or 6, further comprising in step d) the use of at least one nucleic acid molecule or peptide nucleic acid molecule comprising in each case a contiguous sequence at least 9 nucleotides in length that is complementary to, or hybridizes under moderately stringent or stringent conditions to a sequence selected from the group consisting of SEQ ID NO: 65, 66, 67, and 68, and complements thereof, wherein said nucleic acid molecule or peptide nucleic acid molecule suppresses amplification of the nucleic acid to which it is hybridized.

**8.** The method of any of claims 5 to 7, wherein determining in d) comprises hybridization of at least one nucleic acid molecule or peptide nucleic acid molecule in each case comprising a contiguous sequence at least 9 nucleotides in length that is complementary to, or hybridizes under moderately stringent or stringent conditions to a sequence selected from the group consisting of SEQ ID NO: 65, 66, 67, and 68, and complements thereof.

**9.** The method of claim 8, wherein at least one such hybridizing nucleic acid molecule or peptide nucleic acid molecule is bound to a solid phase, and/or wherein at least one such hybridized nucleic acid molecule is extended by at least one nucleotide base.

**10.** The method of any of claims 5 to 9, wherein determining in d), comprises sequencing of the amplificate.

**11.** The method of any of claims 5 to 10, wherein contacting or amplifying in c), comprises use of methylation-specific primers.

**12.** A method for detecting colon cancer in a subject comprising:

a) providing, from a subject, blood plasma, blood serum, whole blood, isolated blood cells, cells isolated from the blood obtained from the subject having subject genomic DNA;
b) extracting, or otherwise isolating the genomic DNA;
c) contacting the genomic DNA of b), or a fragment thereof, comprising at least 16, contiguous nucleotides of a sequence consisting of SEQ ID NO: 5, and sequences that hybridize under stringent conditions thereto, with one or more methylation-sensitive restriction enzymes, wherein the genomic DNA is, with respect to each cleavage recognition motif thereof, either cleaved thereby to produce cleavage fragments, or not cleaved thereby; and
d) detecting colon cancer in a subject based on the methylation state of at least one CpG dinucleotide of a sequence consisting of SEQ ID NO: 5, or an average, or a value reflecting an average methylation state of a plurality of CpG dinucleotides of a sequence selected from the group consisting of SEQ ID NO: 5.

**13.** Use of a nucleic acid, obtainable by amplification of an isolated genomic nucleic acid having a sequence according to SEQ ID NO: 5, wherein said genomic DNA sequence is amplified following a treatment with a reagent selected from the group consisting of bisulfite, hydrogen sulfite, disulfite, and combinations thereof in order to convert at least one unmethylated cytosine base of the genomic DNA sequence to uracil or another base that is detectably dissimilar to cytosine in terms of hybridization for detecting colon cancer in a subject in blood plasma, blood serum, whole blood or isolated blood cells of the subject or cells isolated from the blood obtained from the subject.

**14.** Use of a kit for detecting according to a method of any of claims 1 to 12, comprising:

a) a bisulfite reagent; and
b) a set of primer oligonucleotides containing at least two oligonucleotides whose sequences in each case correspond, are complementary, or hybridize under stringent or highly stringent conditions to a 16-base long segment of a sequence selected from the group consisting of SEQ ID NOS: 65 and 66, SEQ ID NOS: 67 and 68, and complements thereof.

**15.** The use of claim 14, further comprising standard reagents for performing a methylation assay selected from the group consisting of SNuPE, MSP MethyLight, Heavy Methyl, COBRA, nucleic acid sequencing, and combinations thereof.

**Patentansprüche**

**1.** Ein Verfahren zur Detektion von Kolonkrebs in einem Subjekt umfassend:

i. In Kontakt bringen genomischer DNA, die aus Blutplasma, Blutserum, Voll-Blut, isolierten Blutzellen, Zellen, die aus dem Blut, das von einem Subjekt erhalten wurde, isoliert wurden, isoliert wurde, mit mindestens einem Reagenz, oder einer Serie von Reagenzien, die zwischen methylierten und unmethylierten CpG Dinukelotiden innerhalb mindestens einer Zielregion der genomischen DNA unterscheiden, wobei besagte Zielregion umfasst oder unter stringenten Bedingungen mit mindestens 16 kontinuierlichen Nukleotiden des Gens oder der Sequenz von ALX4 hybridisiert; und

ii. Detektion von Kolonkrebs in einem Subjekt basierend auf dem Methylierungsstatus von mindestens einer CpG Ziel-Dinucleotidsequenz, oder einem Mittelwert, oder einem Wert der einen mittleren Methylierungsstatus einer Vielzahl von Ziel-CpG Dinucleotidsequenzen in besagter Zielregion reflektiert.

**2.** Verfahren nach Anspruch 1, wobei die Zielregion mindestens 16 kontinuierlichen Nukleotiden der Sequenz SEQ ID NO: 5, und Komplementen dieser, umfasst oder mit diesen unter stringenten Bedingungen hybridisiert.

**3.** Ein Verfahren zur Detektion von Kolonkrebs in einem Subjekt umfassend das Bestimmen der Expressionniveaus von ALX4 in einer biologischen Probe, die aus besagtem Subjekt isoliert wurde, wobei besagte Probe Blutplasma, Blutserum, Voll-Blut, isolierte Blutzellen, oder Zellen, die aus Blut, das von besagten Subjekt erhalten wurde, isoliert wurde, ist.

**4.** Verfahren nach Anspruch 3, wobei besagtes Expressionsniveau durch Detektieren der Anwesenheit, Abwesenheit oder des Niveaus von mRNA, die von besagtem Gen oder besagter Sequenz transkribiert wird, bestimmt wird, oder wobei besagtes Expressionsniveau durch Detektieren der Anwesenheit, Abwesenheit oder des Niveaus eines Polypeptides, das von besagtem Gen oder besagter Sequenz kodiert wird, bestimmt wird, oder wobei besagte Expression durch Detektieren der Anwesenheit oder Abwesenheit der CpG Methylierung innerhalb des besagten Genes oder besagter Sequenz, bestimmt wird, wobei Hypermethylierung die Anwesenheit einer Kolon zellproliferativen Störung anzeigt.

**5.** Verfahren nach einem der Ansprüche 1 oder 2, umfassend:

a) Extrahieren oder in anderer Weise Isolieren genomischer DNA aus besagter Probe, die von besagtem Subjekt erhalten wurde,

b) Behandeln der DNA aus a) oder einem Fragment davon, mit einem oder mehreren Reagenzien um Cytosin-Basen, die in ihrer 5' Position unmethyliert sind, in Uracil zu verwandeln, oder in eine andere Base, die in Bezug auf ihre Hybridisierungseigenschaften detektierbar unähnlich zu Cytosin ist,

c) in Kontakt bringen der behandelten DNA, oder dem behandelten Fragment dieser, mit einem Amplifikationsenzym und mindestens zwei Primern, die in jedem Fall eine kontinuierliche Sequenz von mindestens 9 Nukleotiden umfassen, die komplementär ist zu, oder unter moderat stringenten Bedingungen oder stringenten Bedingungen mit einer Sequenz hybridisiert, die aus der Gruppe bestehend aus SEQ ID NO: 65, 66, 67, und 68 ausgewählt ist, und Komplementärsequenzen dieser, wobei die behandelte genomische DNA oder das Fragment dieser entweder amplifiziert wird um mindestens ein Amplifikat zu produzieren, oder nicht amplifiziert wird, und

d) Bestimmen, basierend auf der Anwesenheit oder Abwesenheit oder auf einer Eigenschaft von besagtem Amplifikat, des Methylierungsstatuses von mindestens einem CpG Dinucleotid, oder einem Mittelwert, oder einem Wert, der einen durchschnittlichen Methylierungsstatus einer Vielzahl an CpG Dinukleotiden in der Sequenz gemäß SEQ ID NO: 5 reflektiert.

**6.** Verfahren nach Anspruch 5, wobei behandeln der genomischen DNA, oder einem Fragment dieser in b) die Verwendung von Reagenzien umfasst, die aus der Gruppe bestehend aus Bisulfit, Hydrogensulfit, Disulfit, und Kombinationen dieser, ausgewählt sind.

**7.** Verfahren gemäß Anspruch 5 oder 6, des Weiteren umfassend in Schritt d) Verwendung mindestens eines Nukleinsäuremoleküls oder Peptid-Nucleinsäuremoleküls umfassend in jedem Fall eine kontinuierliche Sequenz von mindestens 9 Nukleotiden in Länge, die komplementär ist zu, oder unter moderat stringenten Bedingungen oder stringenten Bedingungen mit einer Sequenz hybridisiert, die aus der Gruppe bestehend aus SEQ ID NO: 65, 66, 67, und 68 ausgewählt ist, und Komplementärsequenzen dieser, wobei besagtes Nukleinsäuremolekül oder Peptid-Nucleinsäuremolekül die Amplifikation der Nukleinsäure, an die es hybridisiert ist, unterdrückt.

**8.** Verfahren nach einem der Ansprüche 5 bis 7, wobei bestimmen in d) Hybridisierung von mindestens einem Nukleinsäuremolekül oder Peptid-Nucleinsäuremolekül umfasst, wobei in jedem Fall eine kontinuierliche Sequenz von mindestens 9 Nukleotiden in Länge, die komplementär ist zu, oder unter moderat stringenten Bedingungen oder stringenten Bedingungen mit einer Sequenz hybridisiert, die aus der Gruppe bestehend aus SEQ ID NO: 65, 66, 67, und 68 ausgewählt ist, und Komplementärsequenzen dieser.

**9.** Verfahren nach Anspruch 8, wobei mindestens ein solches hybridisierendes Nukleinsäuremolekül oder Peptid-Nucleinsäuremolekülan an eine feste Phase gebunden ist, und/oder wobei mindestens ein solches hybridisierendes Nukleinsäuremolekül um mindestens eine Nukleotidbase verlängert wird.

**10.** Verfahren nach einem der Ansprüche 5 bis 9, wobei bestimmen in d) das Sequenzieren des Amplifikats umfasst.

**11.** Verfahren nach einem der Ansprüche 5 bis 10, wobei in Kontakt bringen oder Amplifizieren in c) die Verwendung von methylierungsspezifischen Primern umfasst.

**12.** Ein Verfahren zur Detektion von Kolonkrebs in einem Subjekt, umfassend:

a) Von einem Subjekt zur Verfügung stellen von Blutplasma, Blutserum, Voll-Blut, isolierten Blutzellen, Zellen, die aus Blut, das von dem Subjekt erhalten wurde, isoliert wurden und genomische DNA des Subjektes enthalten;
b) Extrahieren oder in anderer Weise Isolieren der genomischen DNA,
c) In Kontakt bringen der genomischen DNA aus b) oder einem Fragment dieser, welches mindestens 16 kontinuierliche Nukleotide einer Sequenz bestehend aus SEQ ID NO: 5 umfasst, und Sequenzen, die unter stringenten Bedingungen hiermit hybridisieren, mit einem oder mehreren methylierungssensitiven Restriktionsenzymen, wobei die genomische DNA mit Bezug auf jedes Schnitt-Erkennungsmotif entweder dadurch geschnitten wird, um ein geschnittenes Fragment zu produzieren, oder nicht geschnitten wird, und
d) Detektieren von Kolonkrebs in einem Subjekt basierend auf dem Methylierungsstatus von mindestens einem CpG Dinukleotid einer Sequenz bestehend aus SEQ ID NO: 5, oder einem Mittelwert, oder einem Wert, der einen durchschnittlichen Methylierungsstatus einer Vielzahl von CpG Dinukleotiden einer Sequenz ausgewählt aus der Gruppe bestehend aus SEQ ID NO: 5, reflektiert.

**13.** Verwendung einer Nukleinsäure, die durch Amplifikation einer isolierten genomischen Nukleinsäure erhältlich ist, die eine Sequenz gemäß SEQ ID NO: 5 hat, wobei besagte genomische DNA Sequenz nach einer Behandlung mit einem Reagenz amplifiziert wird, das aus der Gruppe bestehend aus Bisulfit, Hydrogensulfit, Disulfit, und Kombinationen dieser, ausgewählt ist, um mindestens eine unmethylierte Cytosinbase der genomischen DNA-Sequenz in Uracil umzuverwandeln, oder in eine andere Base, die in Bezug auf ihre Hybridisierungseigenschaften detektierbar unähnlich zu Cytosin ist,um in einem Subjekt Kolonkrebs im Blutplasma, Blutserum, Voll-Blut oder isolierten Blutzellen des Subjektes oder Zellen, die aus dem Blut, dass von dem Subjekt erhalten wurde, isoliert wurden, zu detektieren.

**14.** Verwendung von einem Kit zur Detektion gemäß einem der Verfahren nach einem der Ansprüche 1 bis 12, umfassend:

a) Ein Bisulfit Reagenz, und
b) Ein Set von Primer Oligonukleotiden, die mindestens zwei Oligonukleotide enthalten, deren Sequenz in jedem Fall korrespondieren, komplementär sind zu, oder unter stringenten oder hoch stringenten Bedingungen an ein 16-basen langes Segment einer Sequenz hybridisieren, die aus der Gruppe bestehend aus SEQ ID NO: 65 und 66, SEQ ID NO: 67 und 68, und Komplementärsequenzen dieser, ausgewählt ist.

**15.** Verwendung gemäß Anspruch 14, des Weiteren umfassend Standard Reagenzien zur Durchführung eines Methylierungs-Assays, der aus der Gruppe bestehend aus SNuPE, MSP, MthyLight, Heavy Methyl, COBRA, Nukleinsäuresequenzierung, und Kombination dieser, ausgewählt ist.

## Revendications

**1.** Procédé pour la détection d’un cancer du côlon chez un sujet, comprenant

i) la mise en contact d’ADN génomique isolé à partir de plasma sanguin, sérum sanguin, sang total, cellules sanguines isolées, cellules isolées du sang obtenu à partir du sujet avec au moins un réactif, ou des séries de réactifs qui font la distinction entre des dinucléotides CpG méthylés et non-méthylés dans au moins une région cible de l'ADN génomique, tandis que ladite région cible comprend, ou hybride dans des conditions stringentes avec au moins 16 nucléotides contigus du gène de séquence ALX4; et
ii) la détection d'un cancer du côlon chez un sujet fondée sur l'état de méthylation d'au moins une séquence dinucléotidique CpG cible, ou une moyenne, ou une valeur reflétant un état de méthylation moyen d'une pluralité de séquences dinucléotidiques CpG cibles dans ladite région cible.

**2.** Procédé selon la revendication 1, dans lequel la région cible comprend, ou hybride dans des conditions stringentes à au moins 16 nucléotides contigus de la séquence SEQ ID NO: 5, et ses compléments.

**3.** Procédé pour la détection d'un cancer du côlon chez un sujet, comprenant la détermination des niveaux d'expression de ALX 4 dans un échantillon biologique isolé à partir dudit sujet, tandis que ledit échantillon est du plasma sanguin, du sérum sanguin, du sang total, des cellules sanguines isolées, des cellules isolées du sang obtenu à partir dudit sujet

**4.** Procédé selon la revendication 3, dans lequel ledit niveau d'expression est déterminé par détection de la présence, l'absence ou le niveau d'ARNm transcrit à partir dudit gène ou de ladite séquence, ou dans lequel ledit niveau d'expression est déterminé par détection de la présence, l'absence ou le niveau d'un polypeptide codé par ledit gène ou ladite séquence, ou dans lequel ladite expression est déterminée par détection de la présence ou de l'absence de méthylation de CpG dans ledit gène ou ladite séquence, tandis que l'hyperméthylation indique la présente d'un trouble de prolifération cellulaire du côlon.

**5.** Procédé selon l'une quelconque des revendications 1 ou 2, comprenant:

a) l'extraction ou l'isolement d'une autre manière d'ADN génomique provenant dudit échantillon obtenu à partir dudit sujet,
b) le traitement de l'ADN génomique de a), ou d'un fragment de celui-ci, avec un ou plusieurs réactifs pour convertir les bases cytosine qui sont non-méthylées sur leur position 5 en uracile ou en une autre base qui à la détection n'est pas semblable à la cytosine en termes de propriétés d'hybridation;
c) la mise en contact de l'ADN génomique traité, ou du fragment traité de celui-ci, avec une enzyme d'amplification et au moins deux amorces comprenant, dans chaque cas une séquence contiguë d'au moins 9 nucléotides qui est complémentaire de, ou qui hybride dans des conditions modérément stringentes ou stringentes avec, une séquence choisie dans le groupe consistant en SEQ ID NO: 65, 66, 67, et 68, et leurs compléments, tandis que l'ADN génomique traité ou son fragment est soit amplifié pour donner au moins un produit d'amplification, ou n'est pas amplifié; et
d) la détermination, fondée sur une présence ou absence, ou sur une propriété dudit produit d'amplification, de l'état de méthylation d'au moins un dinucléotide CpG, ou d'une moyenne, ou d'une valeur reflétant un état de méthylation moyen d'une pluralité de dinucléotides CpG dans la séquence selon SEQ ID NO: 5.

**6.** Procédé selon la revendication 5, dans lequel le traitement de l'ADN génomique, ou du fragment de celui-ci selon b), comprend l'utilisation d'un réactif choisi dans le groupe consistant en bisulfite, hydrogéno sulfite, disulfite, et des combinaisons de ceux-ci.

**7.** Procédé selon la revendication 5 ou 6, comprenant en outre dans l'étape d) l'utilisation d'au moins une molécule d'acide nucléique ou d'une molécule d'acide nucléique peptidique comprenant dans chaque cas une séquence contiguë d'au moins 9 nucléotides de long qui est complémentaire, ou hybride dans des conditions modérément stringentes ou stringentes avec une séquence choisie dans le groupe consistant en SEQ ID NO: 65, 66, 67, et 68, et leurs compléments, tandis que ladite molécule d'acide nucléique ou ladite molécule d'acide nucléique peptidique supprime l'amplification de l'acide nucléique auquel elle est hybridée.

**8.** Procédé selon l'une quelconque des revendications 5 à 7, dans lequel la détermination sous d) comprend l'hybridation d'au moins une molécule d'acide nucléique ou d'au moins une molécule d'acide nucléique peptidique comprenant dans chaque cas une séquence contiguë ayant au moins 9 nucléotides de long, qui est complémentaire de, ou hybride dans des conditions modérément stringentes ou stringentes avec, une séquence choisie dans le groupe consistant en SEQ ID NO: 65, 66, 67, et 68, et leurs compléments.

**9.** Procédé selon la revendication 8, dans lequel au moins une telle molécule d'acide nucléique hybridante ou molécule d'acide nucléique peptidique hybridante est liée à une phase solide, et/ou dans lequel au moins une telle molécule d'acide nucléique hybridée est allongée d'au moins une base nucléotidique.

**10.** Procédé selon l'une quelconque des revendications 5 à 9, dans lequel la détermination sous d) comprend le séquençage du produit d'amplification.

**11.** Procédé selon l'une quelconque des revendications 5 à 10, dans lequel la mise en contact ou l'amplification selon c) comprend l'utilisation d'amorces spécifiques de la méthylation.

**12.** Procédé pour la détection d'un cancer du côlon chez un sujet, comprenant:

a) la fourniture, à partir d'un sujet, de plasma sanguin, de sérum sanguin, de sang total, de cellules sanguines isolées, de cellules isolées du sang obtenu à partir du sujet ayant l'ADN génomique du sujet;
b) l'extraction ou l'isolement d'une autre manière de l'ADN génomique;
c) la mise en contact de l'ADN génomique selon b), ou d'un fragment de celui-ci, comprenant au moins 16 nucléotides contigus d'une séquence consistant en SEQ ID NO: 5, et de séquences qui hybrident à celui-ci dans des conditions stringentes, avec une ou plusieurs enzymes de restriction sensibles à la méthylation, tandis que l'ADN génomique est, vis-à-vis de chaque motif de reconnaissance de clivage de celui-ci, soit clivé ainsi pour donner des fragments de clivage, soit pas clivé de la sorte; et
d) la détection d'un cancer du côlon chez un sujet fondée sur l'état de méthylation d'au moins un dinucléotide CpG d'une séquence consistant en SEQ ID NO: 5, ou une moyenne, ou une valeur reflétant un état de méthylation moyen d'une pluralité de dinucléotides CpG d'une séquence choisie dans le groupe consistant en SEQ ID NO: 5.

**13.** Utilisation d'un acide nucléique, pouvant être obtenu par amplification d'un acide nucléique génomique isolé ayant une séquence selon SEQ ID NO: 5, dans laquelle ladite séquence d'acide génomique est amplifiée à la suite d'un traitement avec un réactif choisi dans le groupe consistant en bisulfite, hydrogéno sulfite, disulfite et leurs combinaisons, afin de convertir au moins une base cytosine non-méthylée de la séquence d'ADN génomique en uracile ou en une autre base qui est détectable comme n'étant pas semblable à la cytosine en termes d'hybridation pour la détection d'un cancer du côlon chez un sujet dans du plasma sanguin, du sérum sanguin, du sang total ou des cellules sanguines isolées du sujet ou des cellules isolées du sang obtenu à partir du sujet.

**14.** Utilisation d'un kit pour la détection au moyen d'un procédé selon l'une quelconque des revendications 1 à 12, comprenant:

a) un réactif au bisulfite; et
b) un ensemble d'oligonucléotides d'amorce contenant au moins deux oligonucléotides dont les séquences, dans chaque cas, correspondent à, sont complémentaires de, ou hybrident à, dans des conditions stringentes ou fortement stringentes, un segment ayant 16 bases de long d'une séquence choisie dans le groupe consistant en SEQ ID NOS: 65 et 66, SEQ ID NOS: 67 et 68, et leurs compléments.

**15.** Utilisation selon la revendication 14, comprenant en outre des réactifs standards pour la mise en oeuvre d'un test de méthylation choisis dans groupe consistant en SNuPE, MSP MethyLight, Heavy Methyl, COBRA, séquençage d'acide nucléique, et leurs combinaisons.

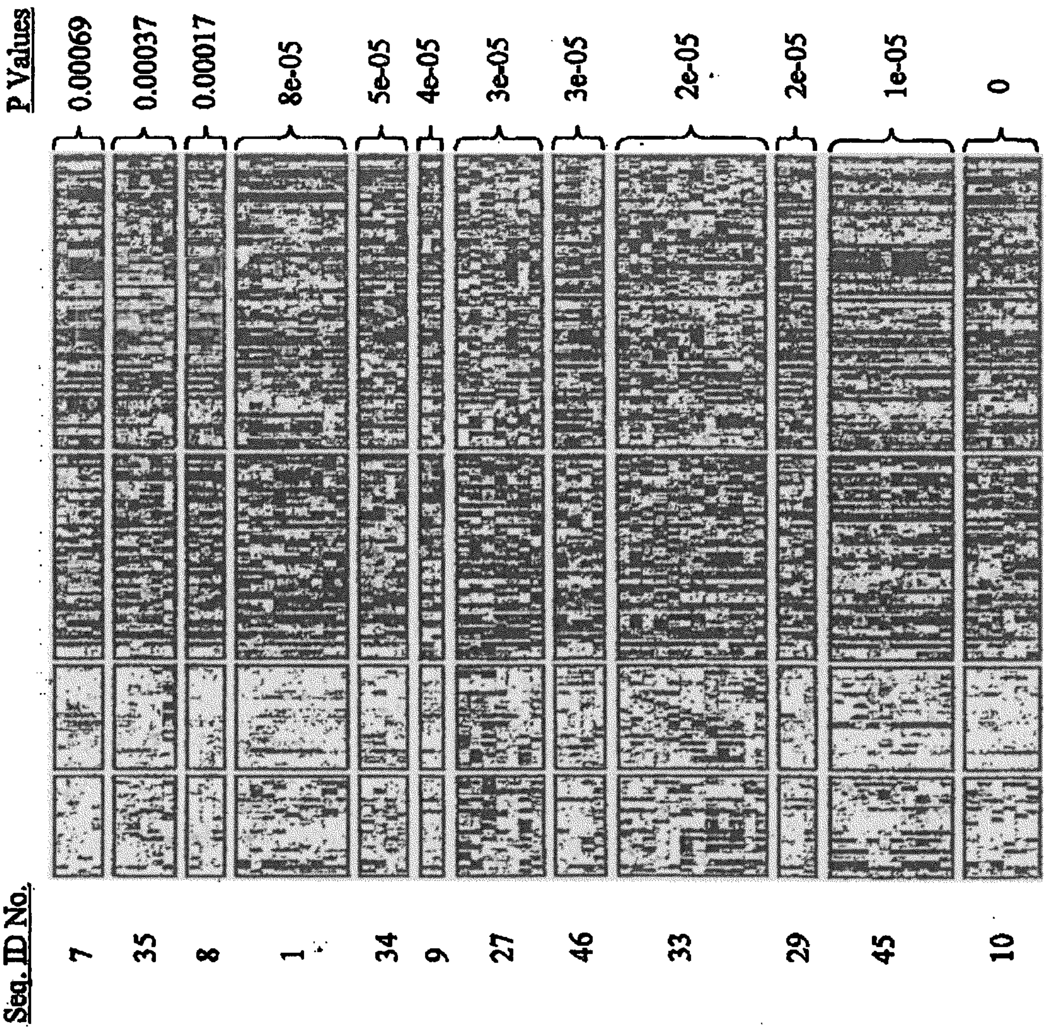
P Values
0.00069
0.00037
0.00017
8e-05
5e-05
4e-05
3e-05
3e-05
2e-05
2e-05
1e-05
0
Seq. ID No.
7
35
8
1
34
9
27
46
33
29
45
10

Figure 1

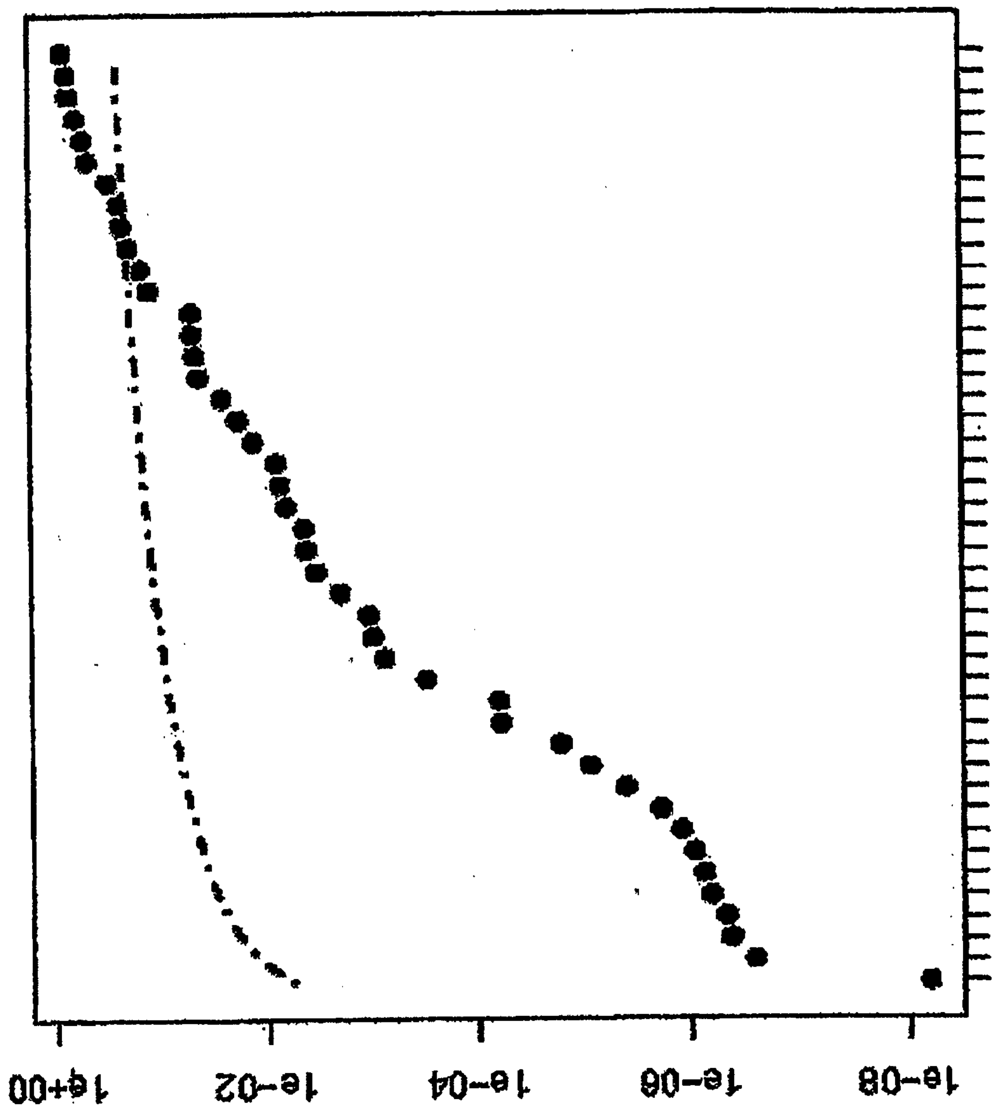


Figure 2

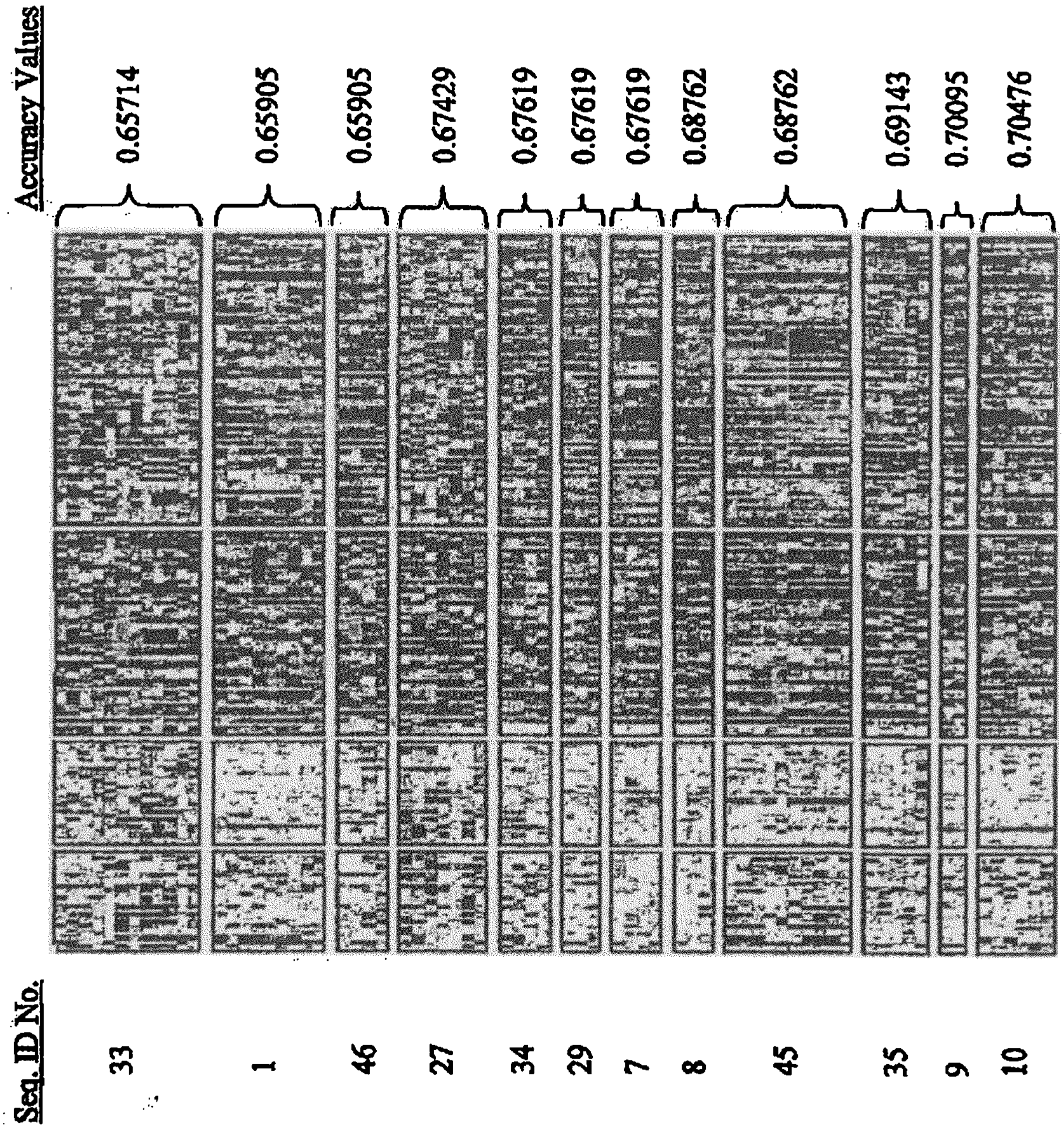
Accuracy Values
0.65714
0.65905
0.65905
0.67429
0.67619
0.67619
0.67619
0.68762
0.68762
0.69143
0.70095
0.70476
Seq. ID No.
33
1
46
27
34
29
7
8
45
35
9
10

Figure 3

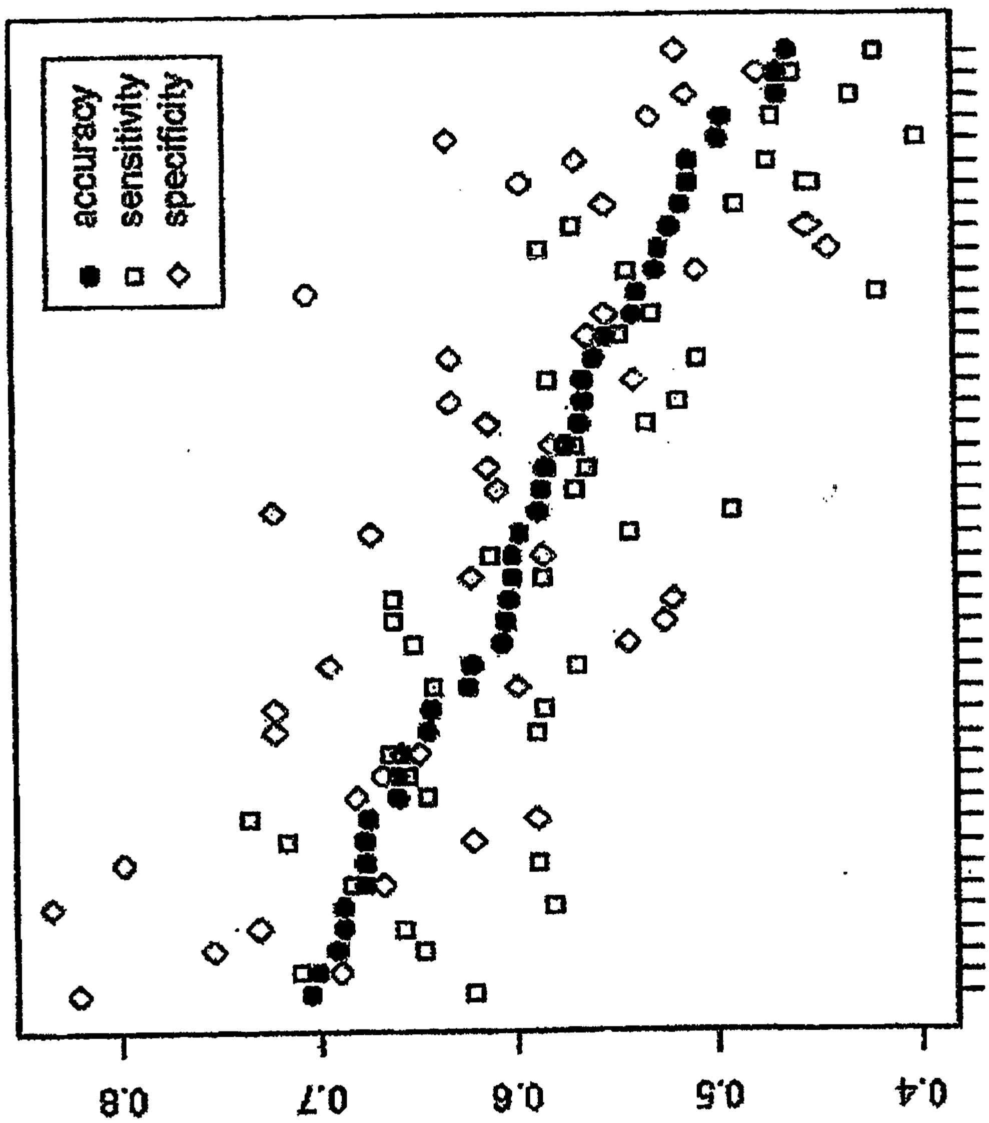


Figure 4

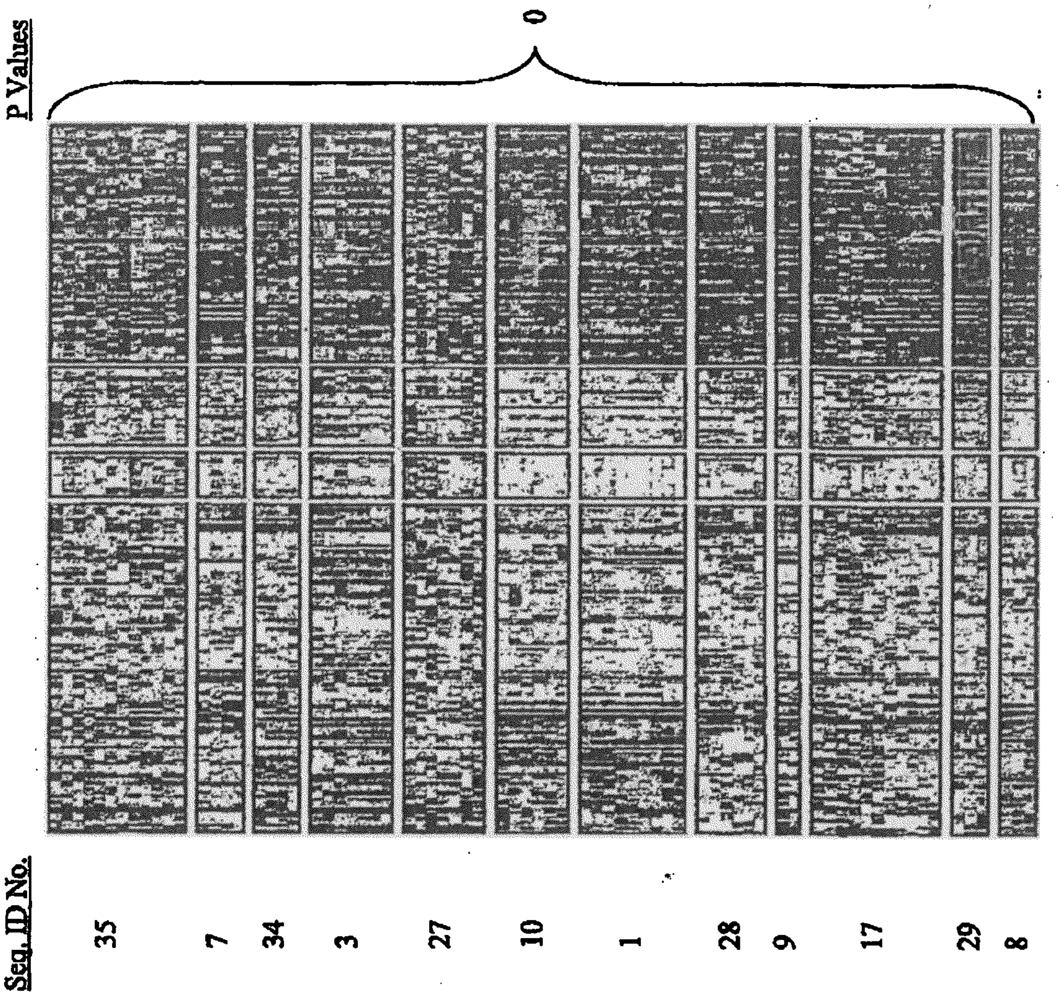


Figure 5

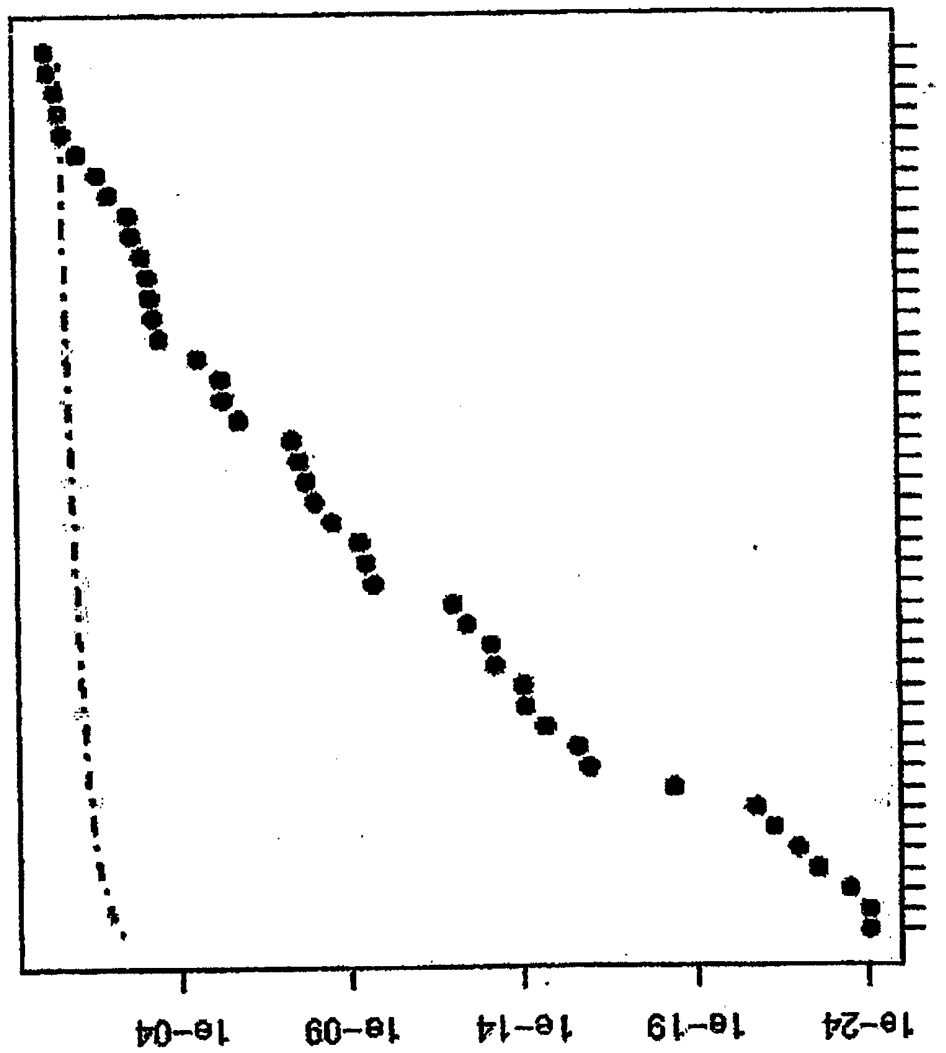


Figure 6

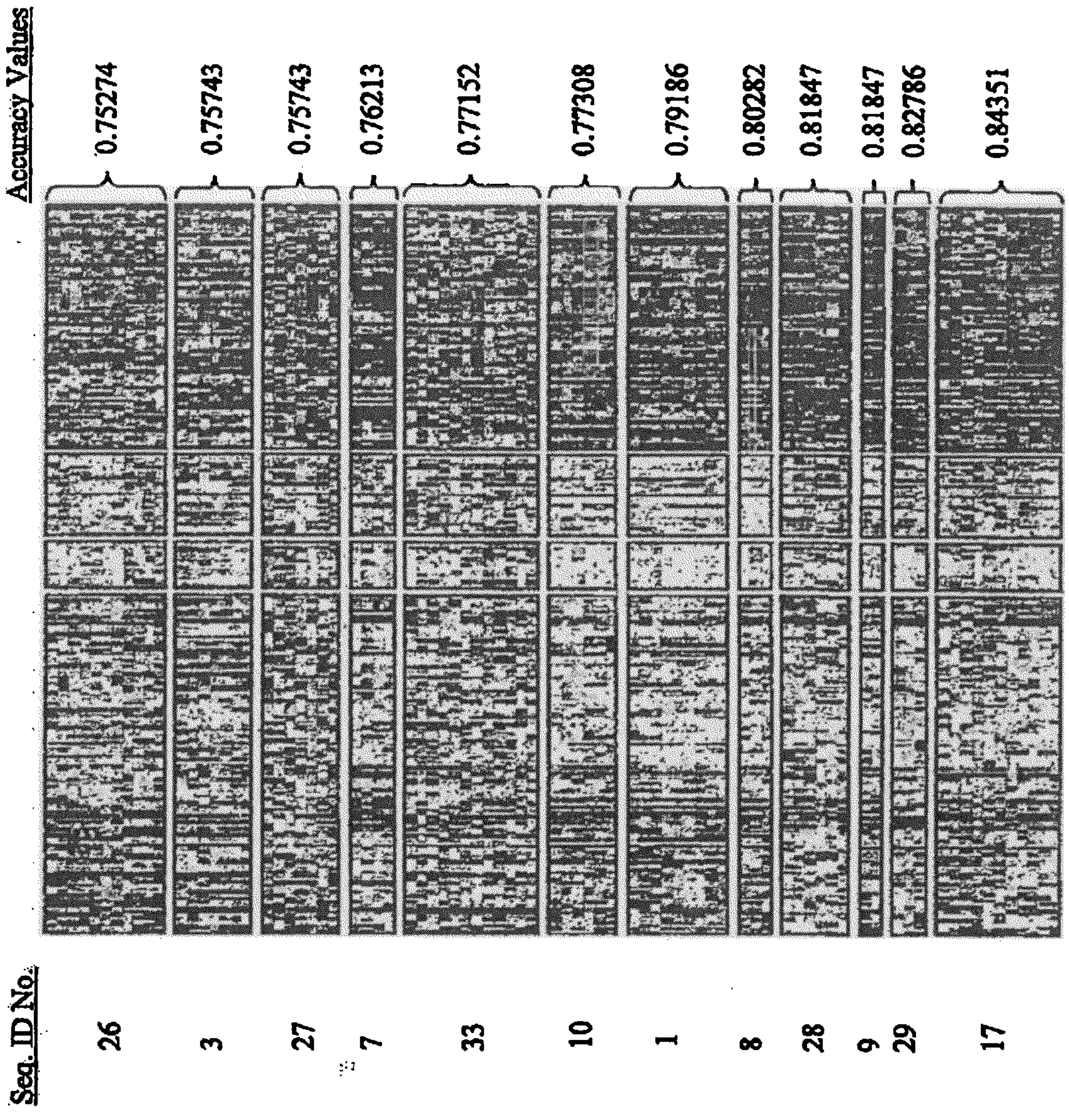
Seq. ID No.
Accuracy Values
26
0.75274
3
0.75743
27
0.75743
7
0.76213
33
0.77152
10
0.77308
1
0.79186
8
0.80282
28
0.81847
9
0.81847
29
0.82786
17
0.84351

Figure 7

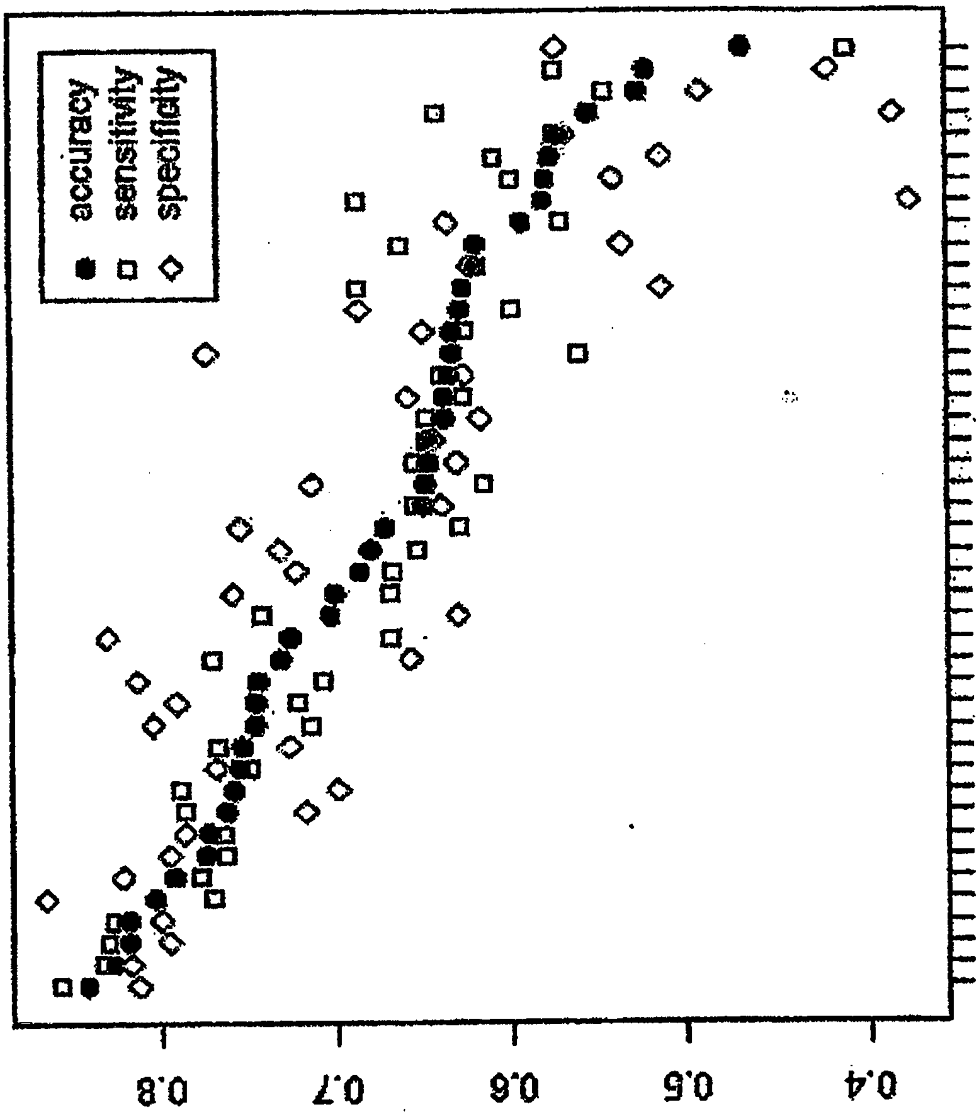


Figure 8

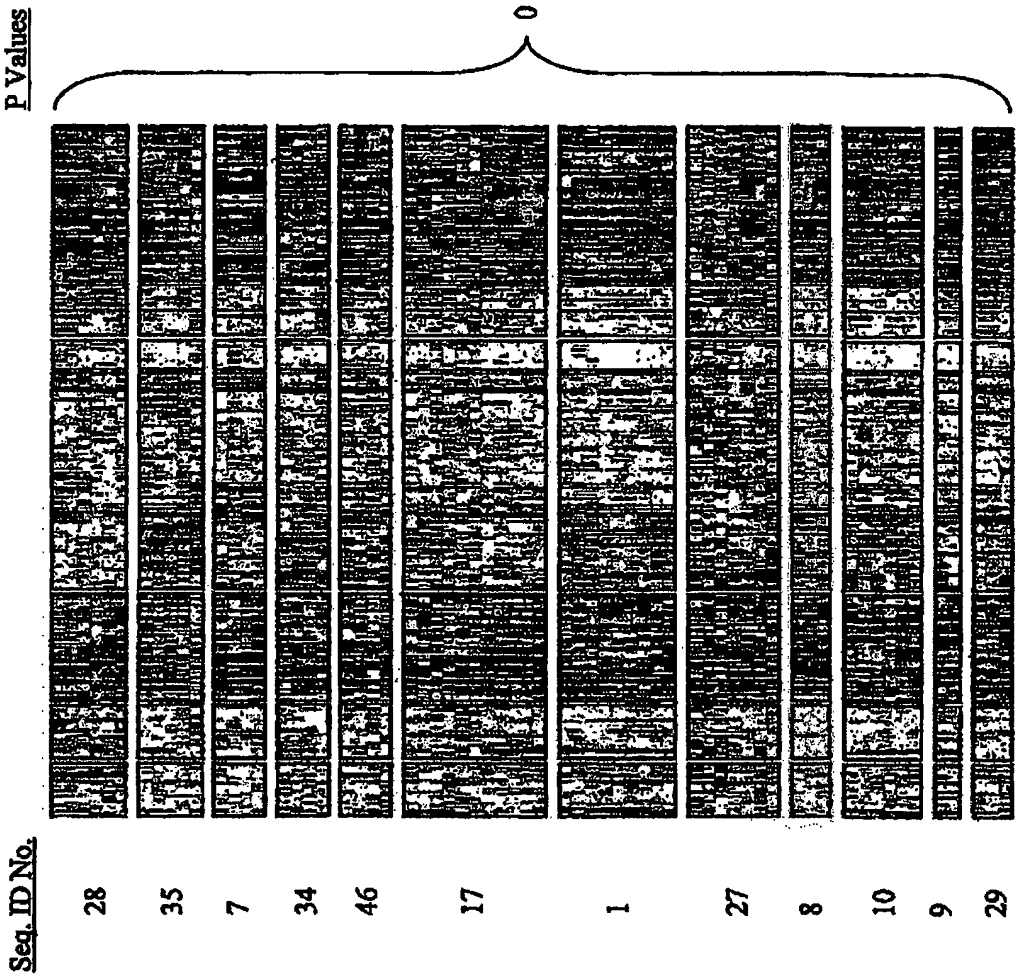


Figure 9

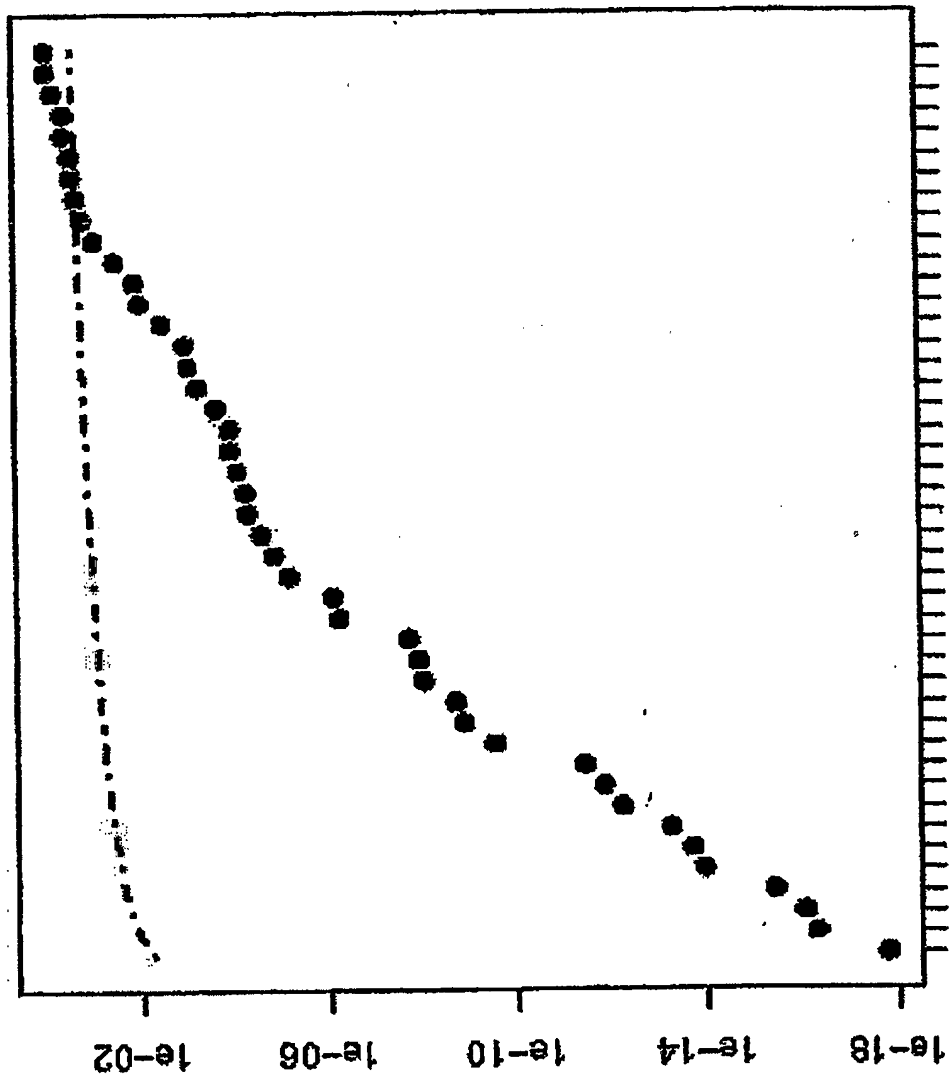


Figure 10

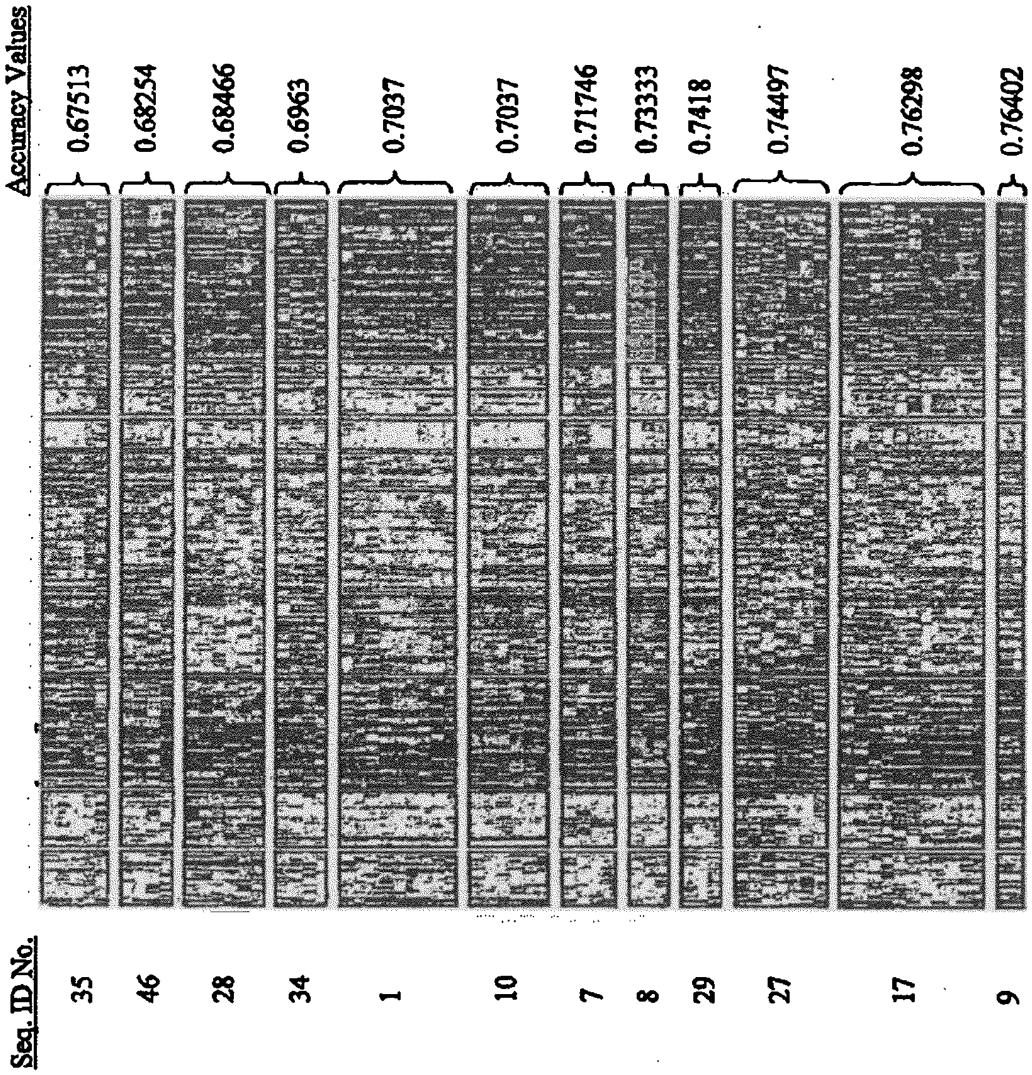
Accuracy Values
0.67513
0.68254
0.68466
0.6963
0.7037
0.7037
0.71746
0.73333
0.7418
0.74497
0.76298
0.76402
Seq. ID No.
35
46
28
34
1
10
7
8
29
27
17
9

Figure 11

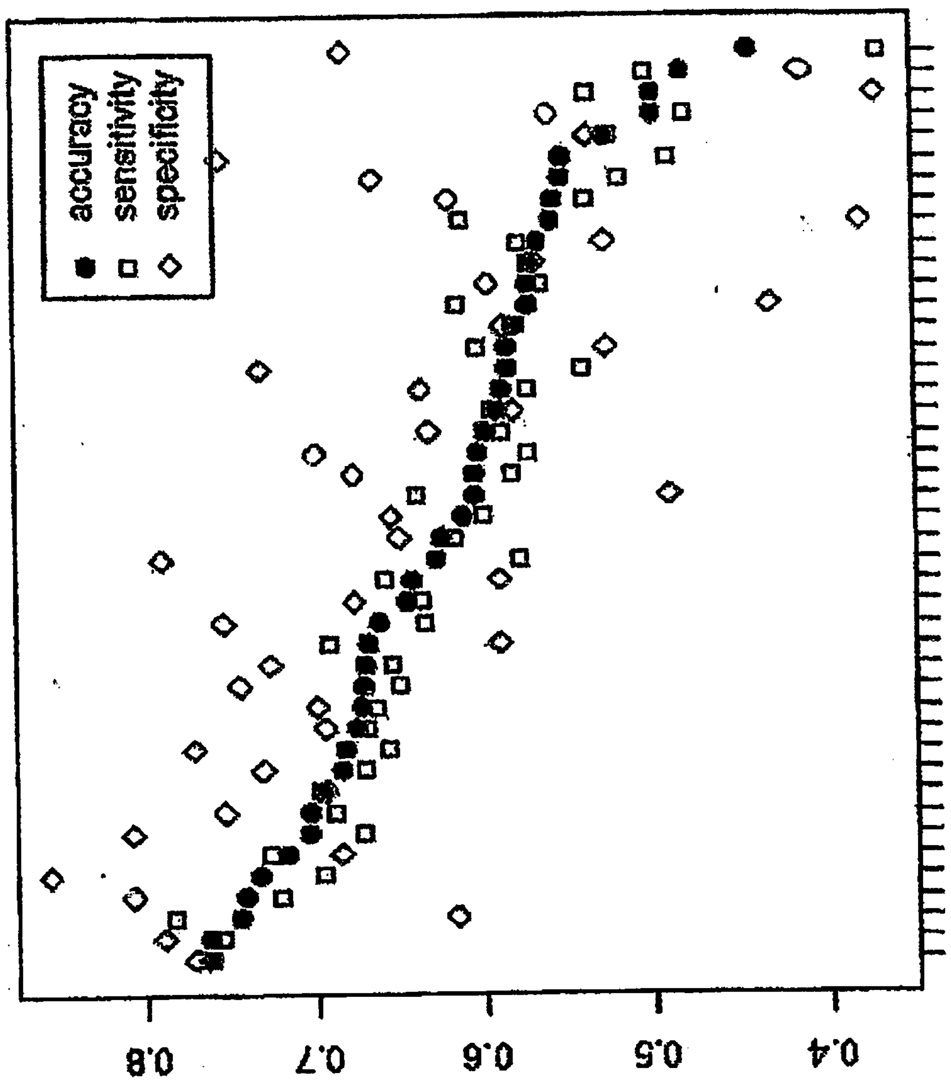


Figure 12

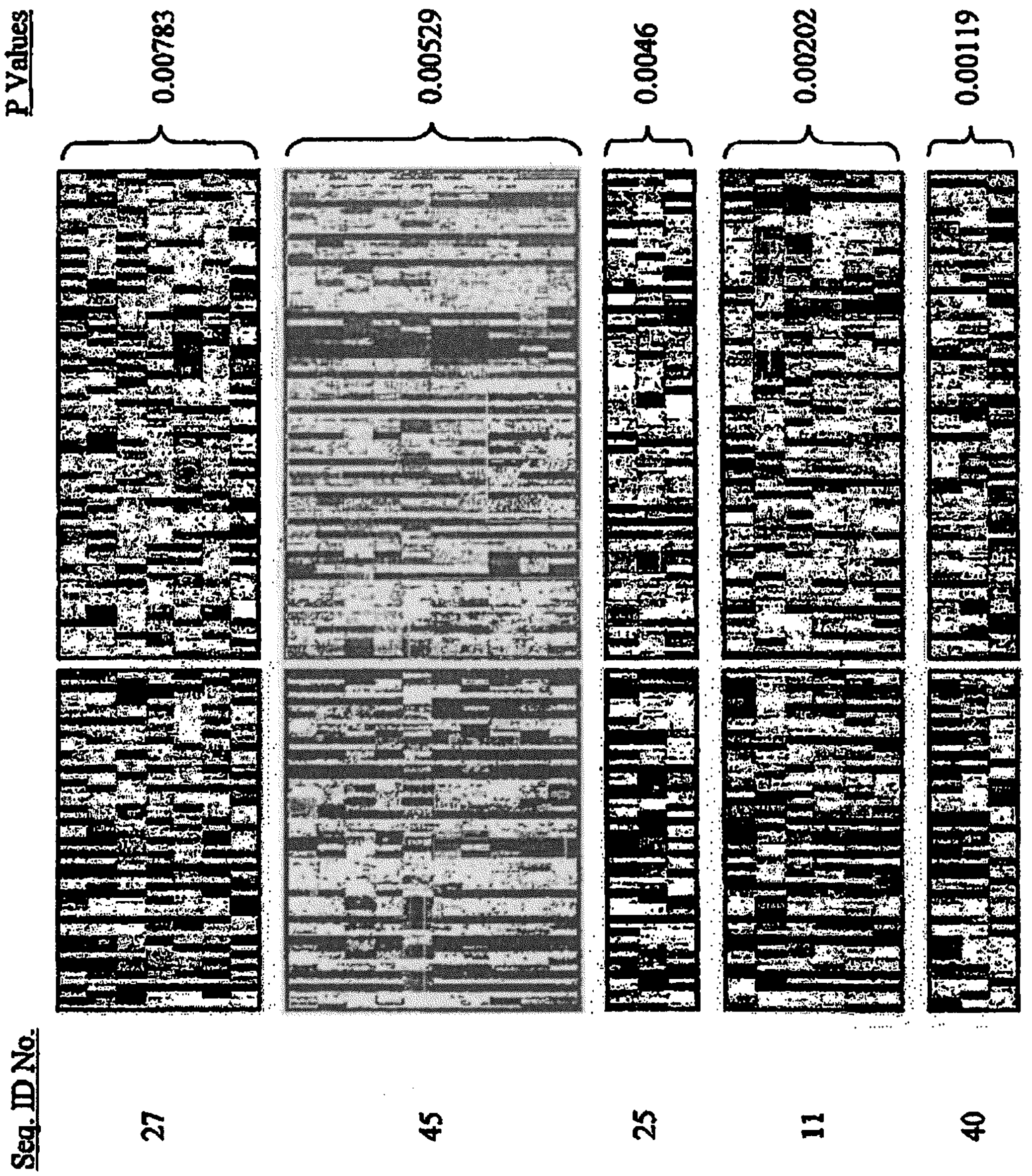
P Values
0.00783
0.00529
0.0046
0.00202
0.00119
Seq. ID No.
27
45
25
11
40

Figure 13

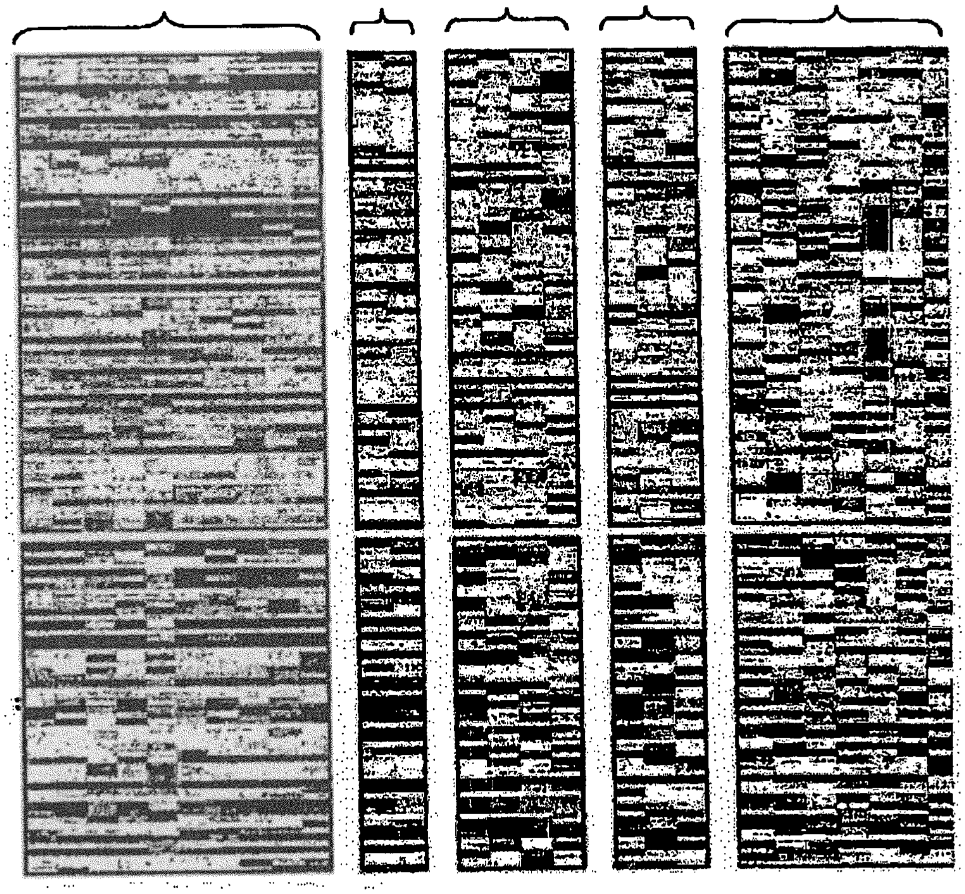
Accuracy Values
0.64247
0.64516
0.66054
0.67204
0.70161
Seq. ID No.
45
42
38
25
27

Figure 14

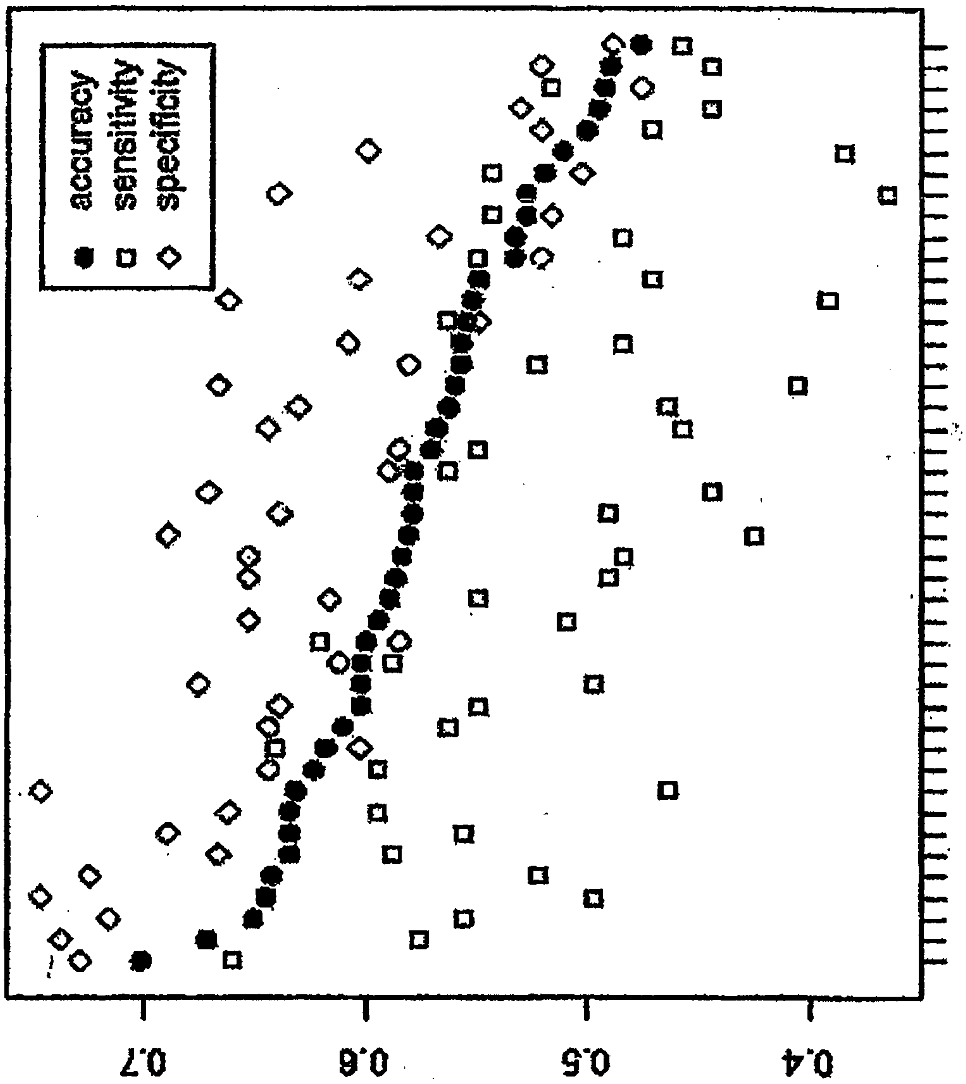


Figure 15

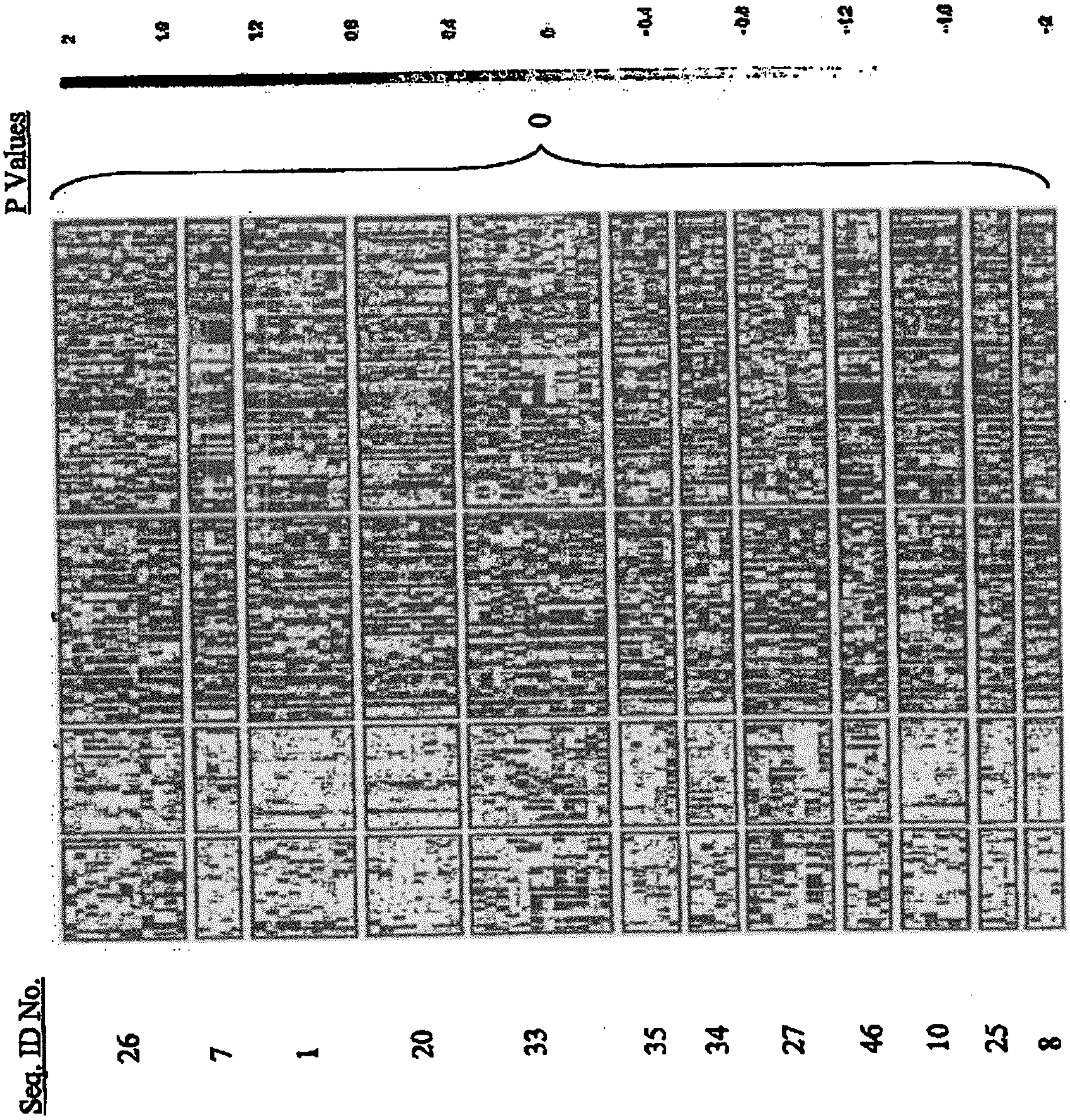


Figure 16

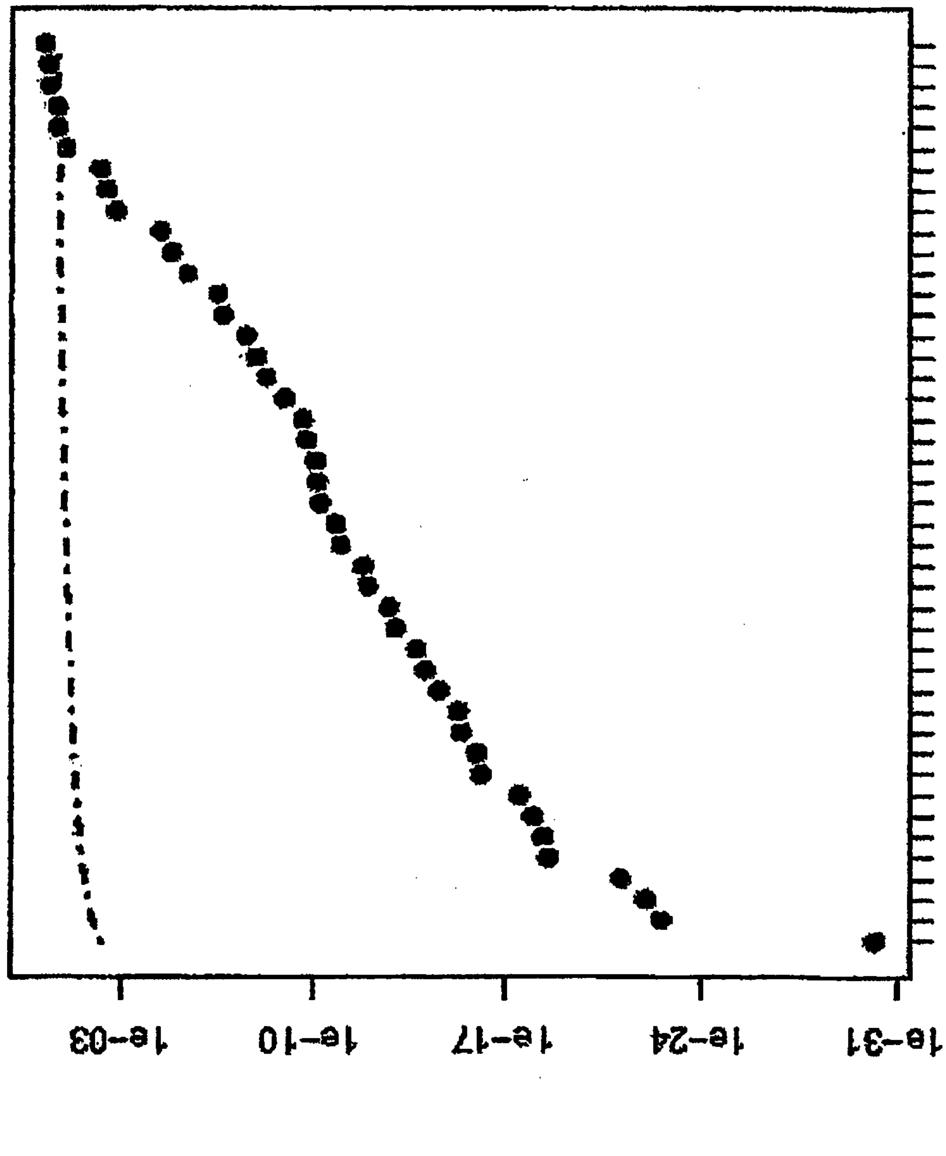


Figure 17

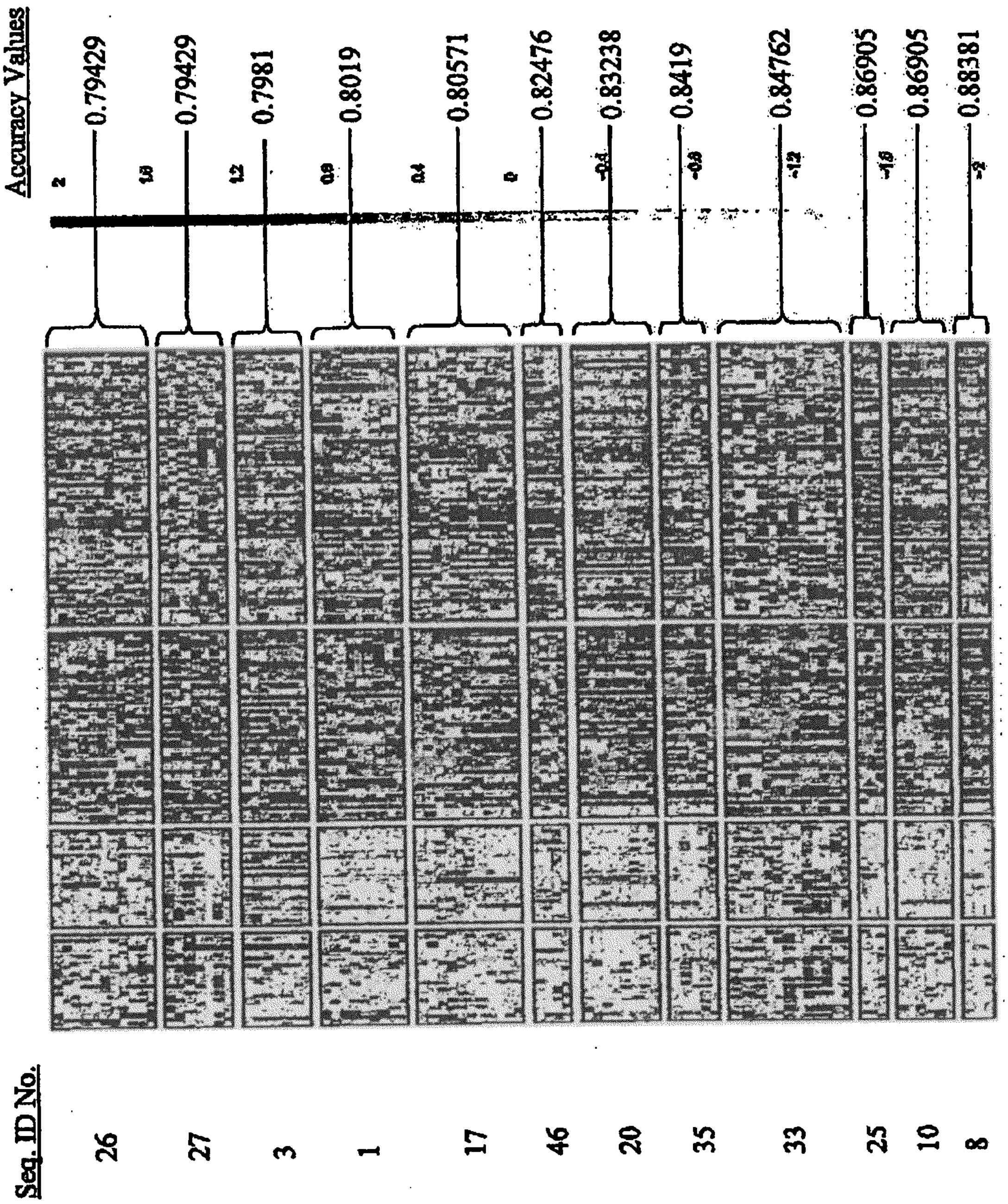
Accuracy Values
0.79429
0.79429
0.7981
0.8019
0.80571
0.82476
0.83238
0.8419
0.84762
0.86905
0.86905
0.88381
Seq. ID No.
26
27
3
1
17
46
20
35
33
25
10
8

Figure 18

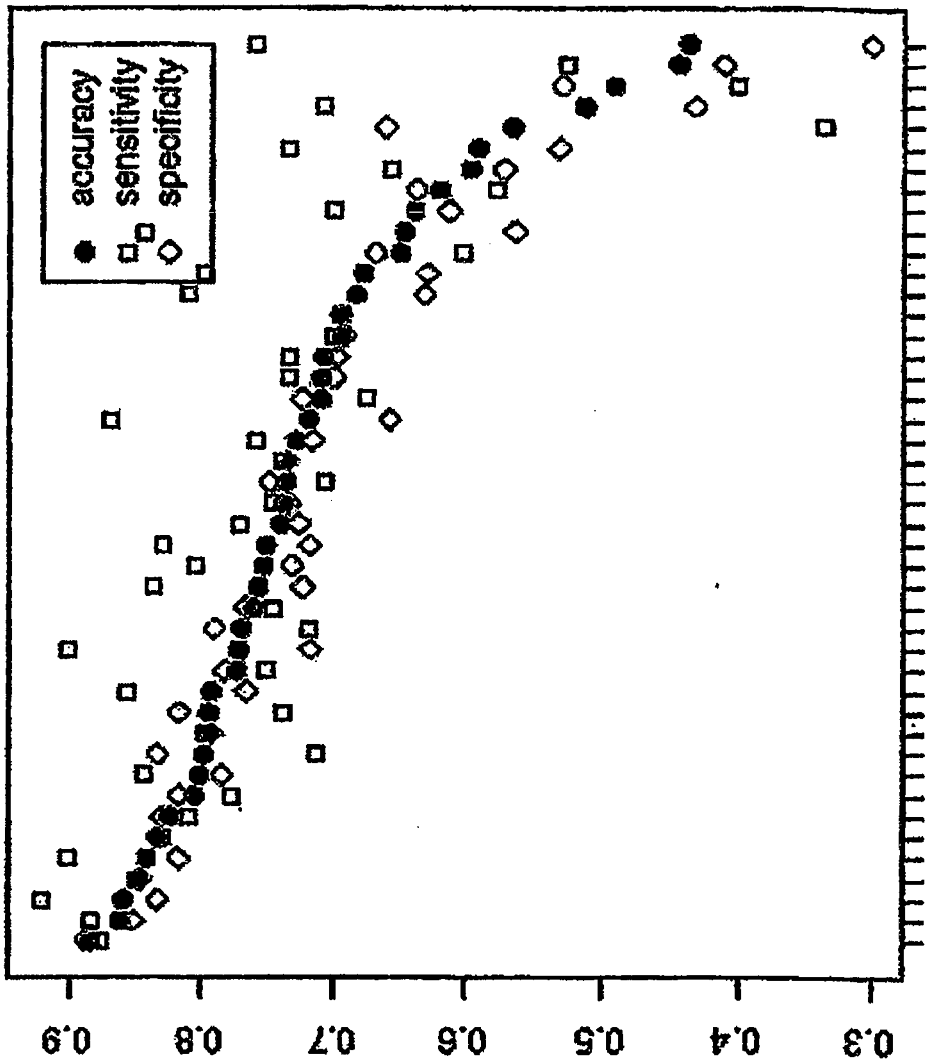


Figure 19

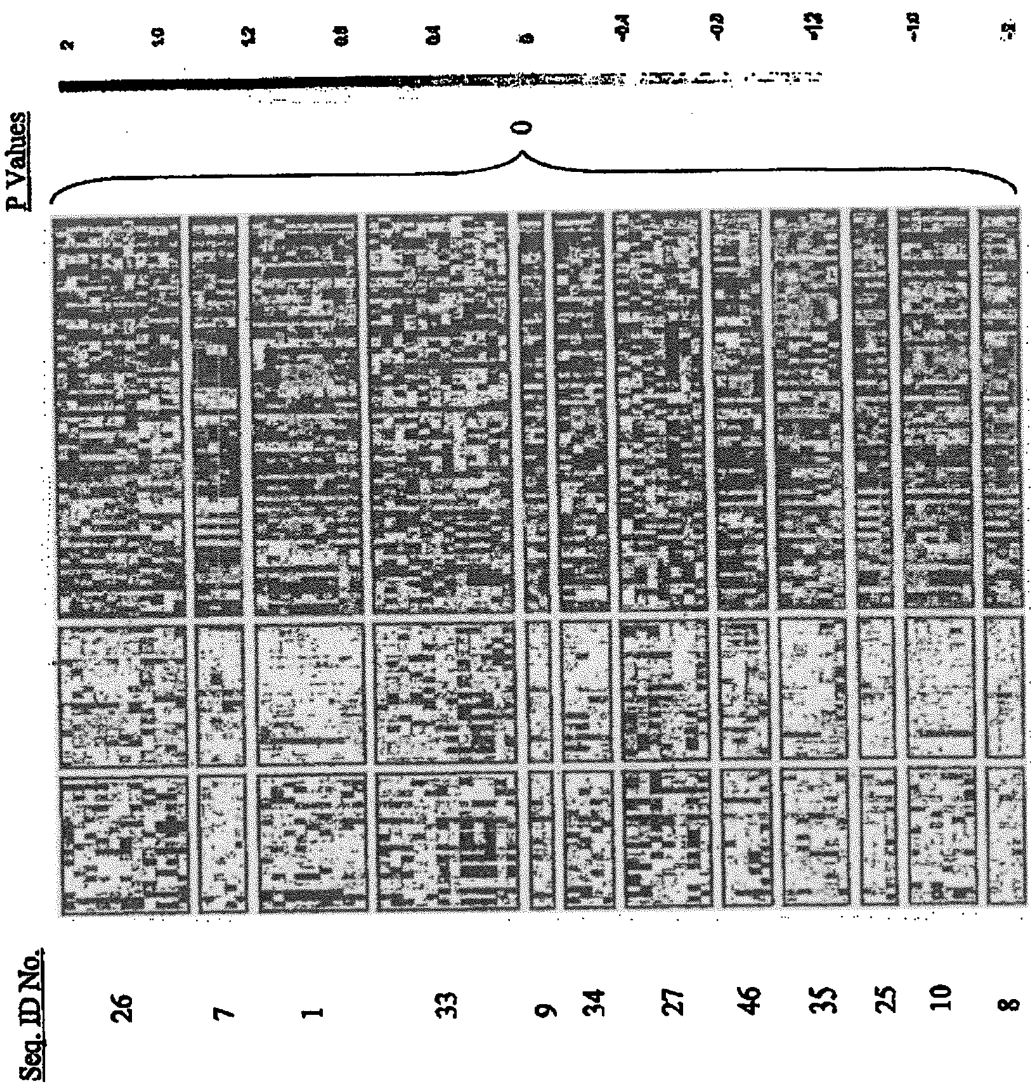


Figure 20

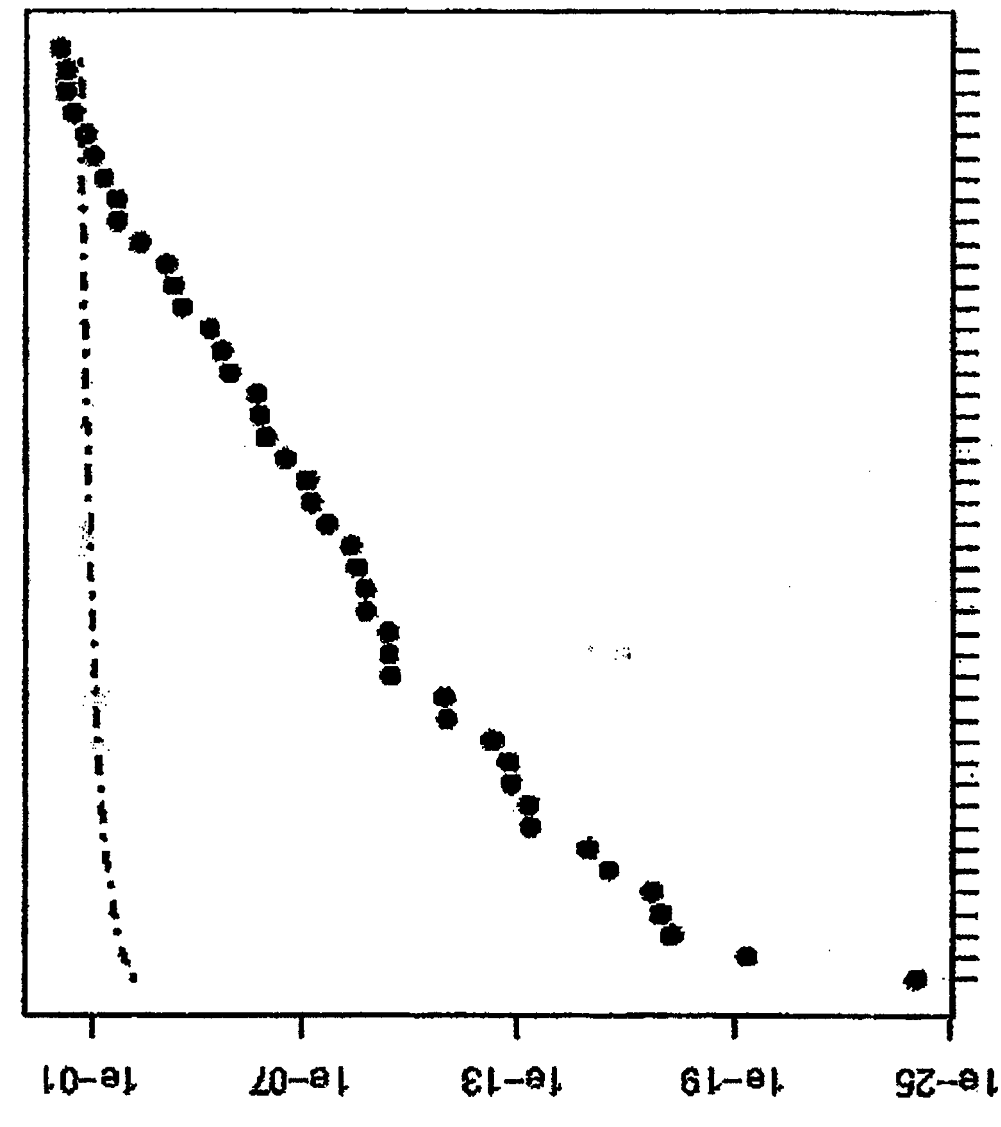


Figure 21

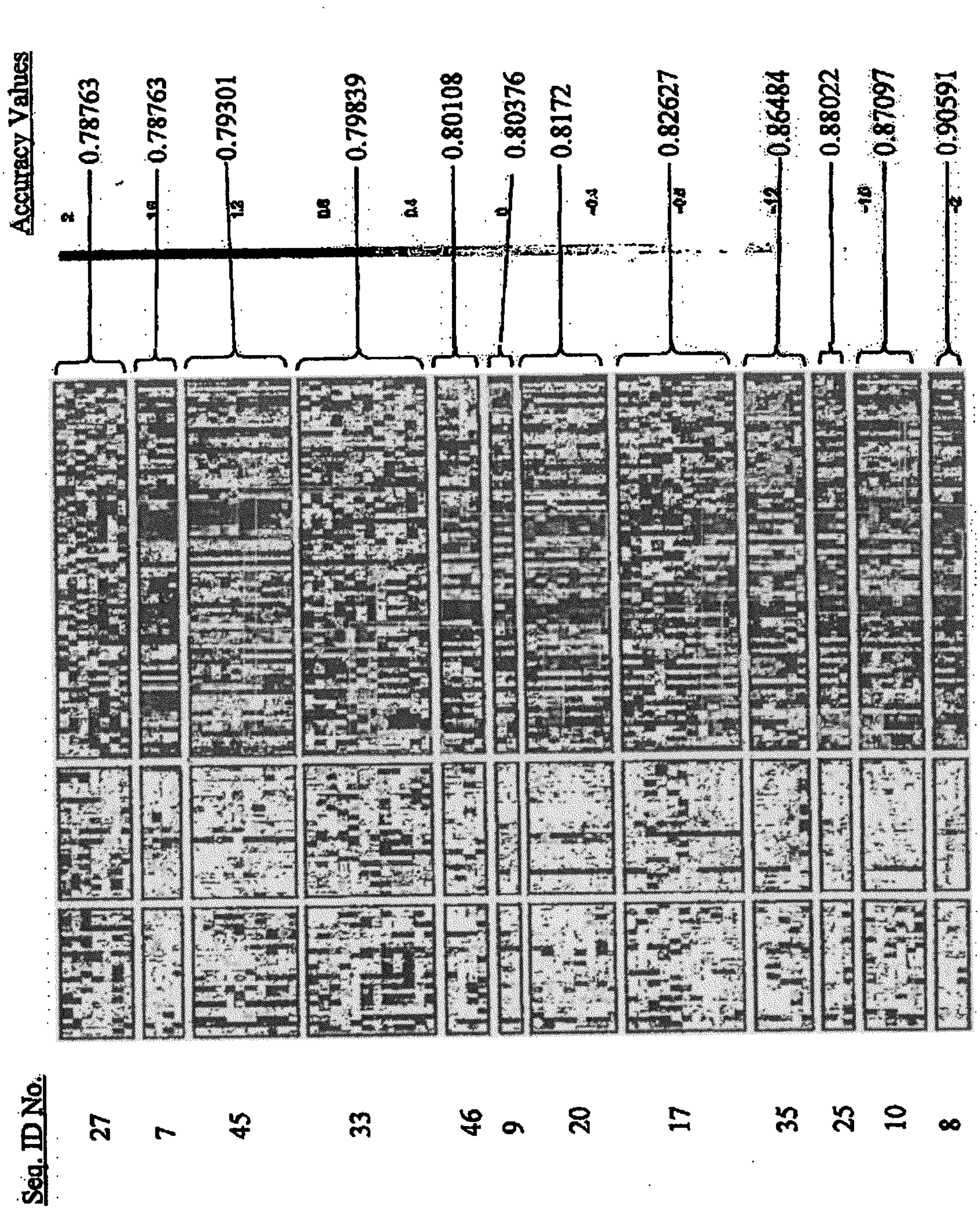
Seq. ID No.
27
7
45
33
46
9
20
17
35
25
10
8
Accuracy Values
0.78763
0.78763
0.79301
0.79839
0.80108
0.80376
0.8172
0.82627
0.86484
0.88022
0.87097
0.90591

Figure 22

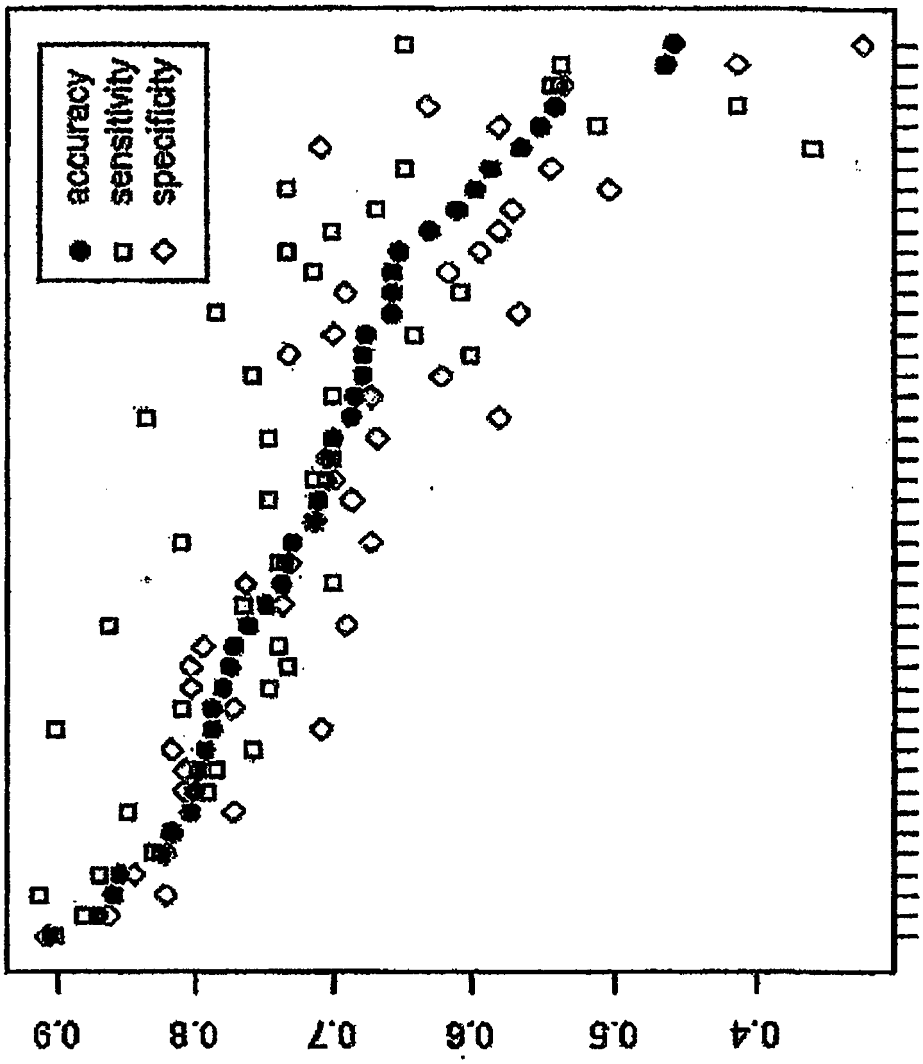


Figure 23

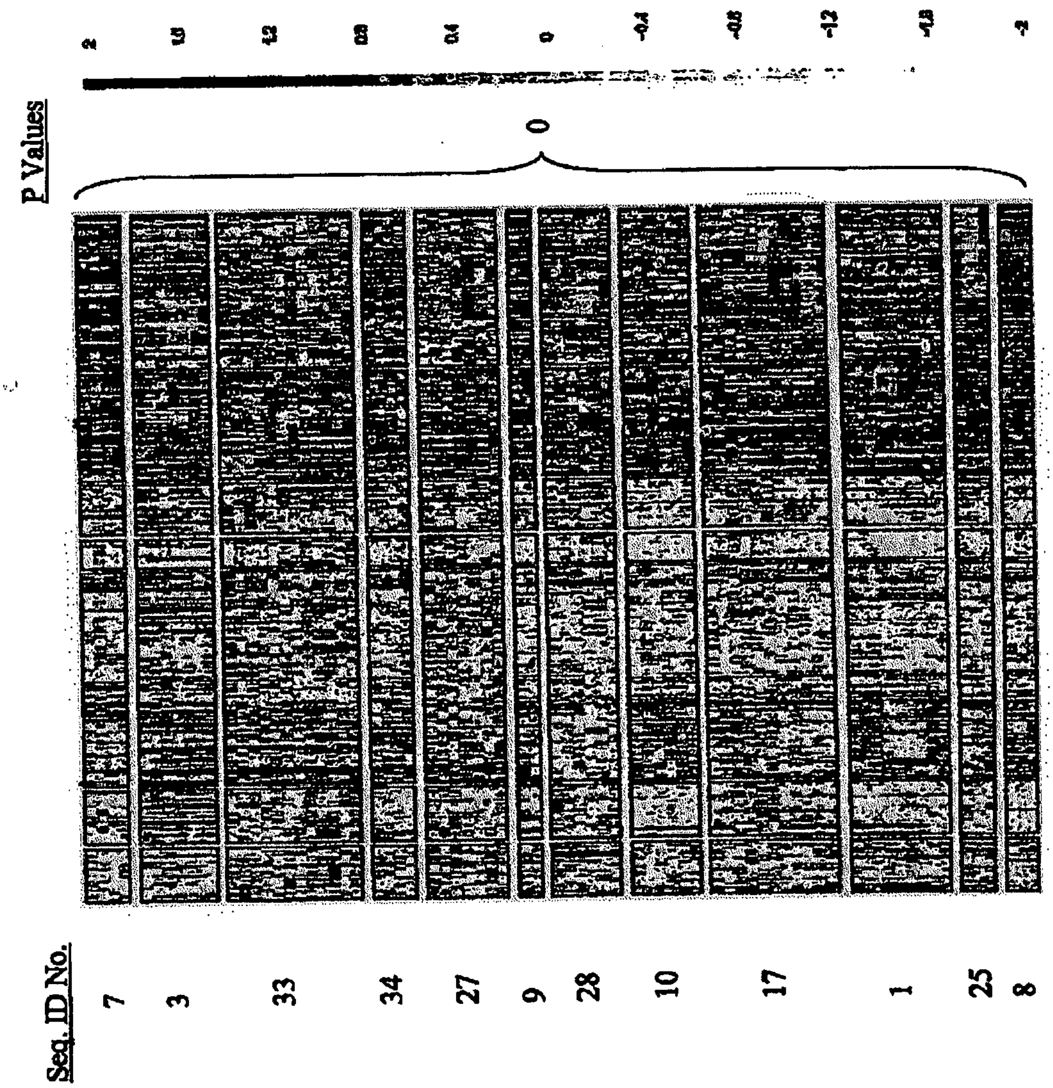


Figure 24

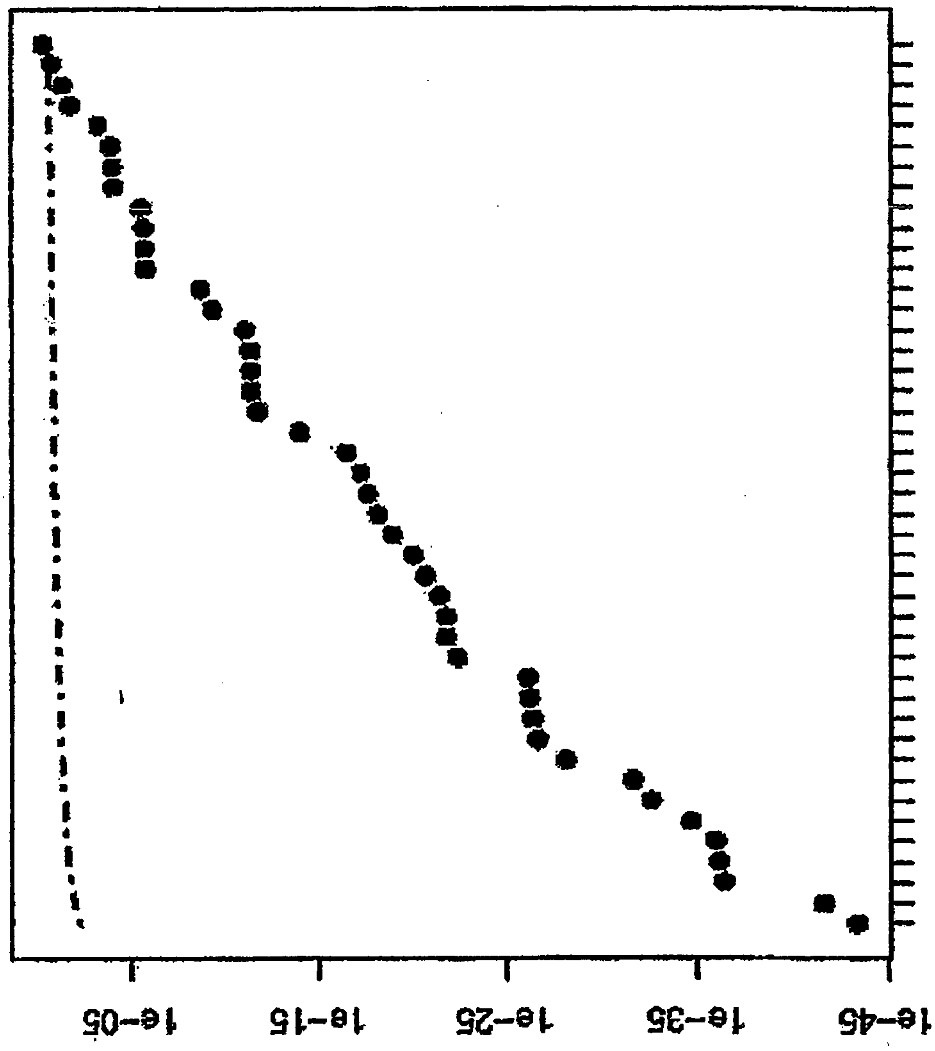


Figure 25

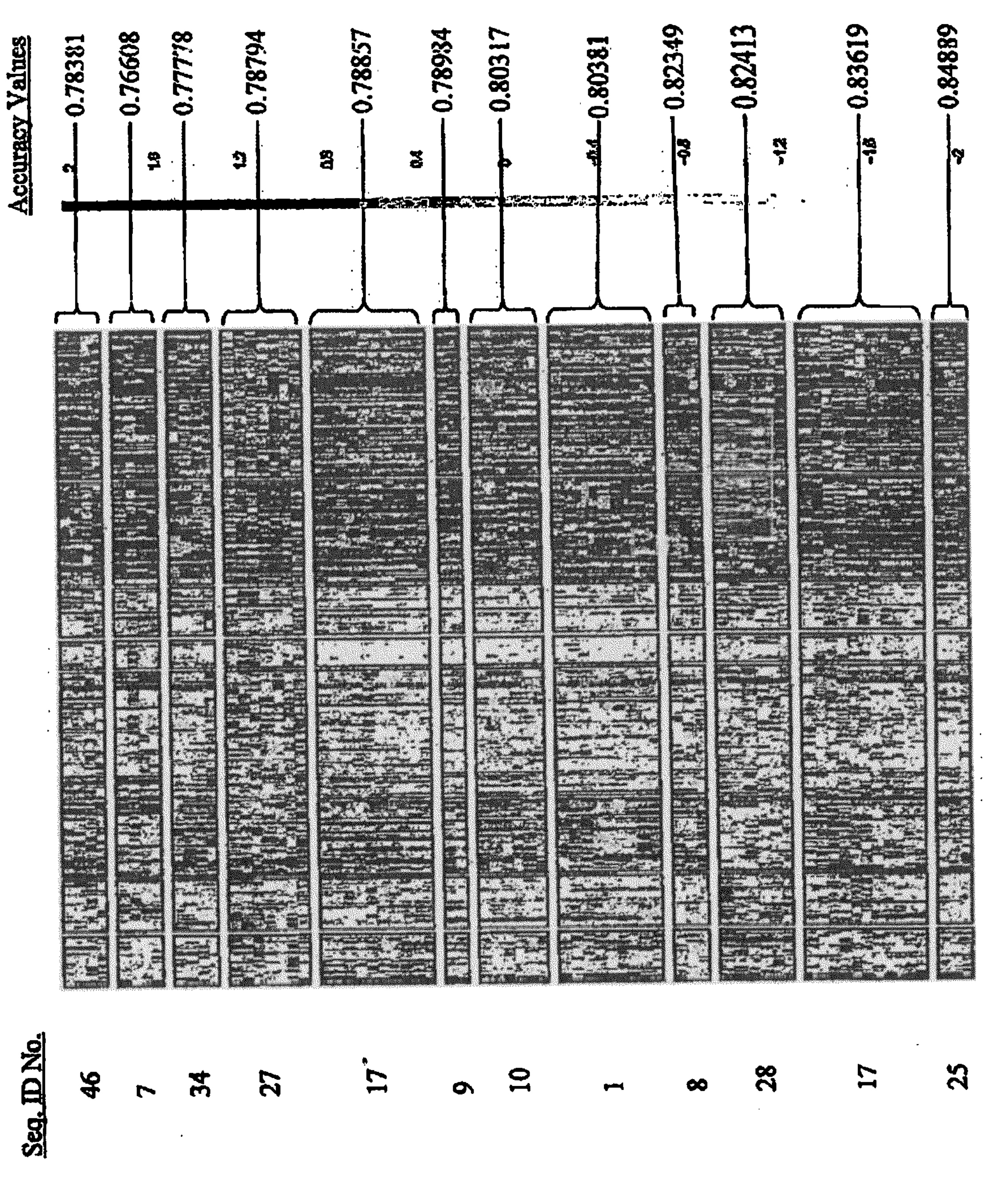
Accuracy Values
0.78381
0.76608
0.77778
0.78794
0.78857
0.78934
0.80317
0.80381
0.82349
0.82413
0.83619
0.84889
Seq. ID No.
46
7
34
27
17
9
10
1
8
28
17
25

Figure 26

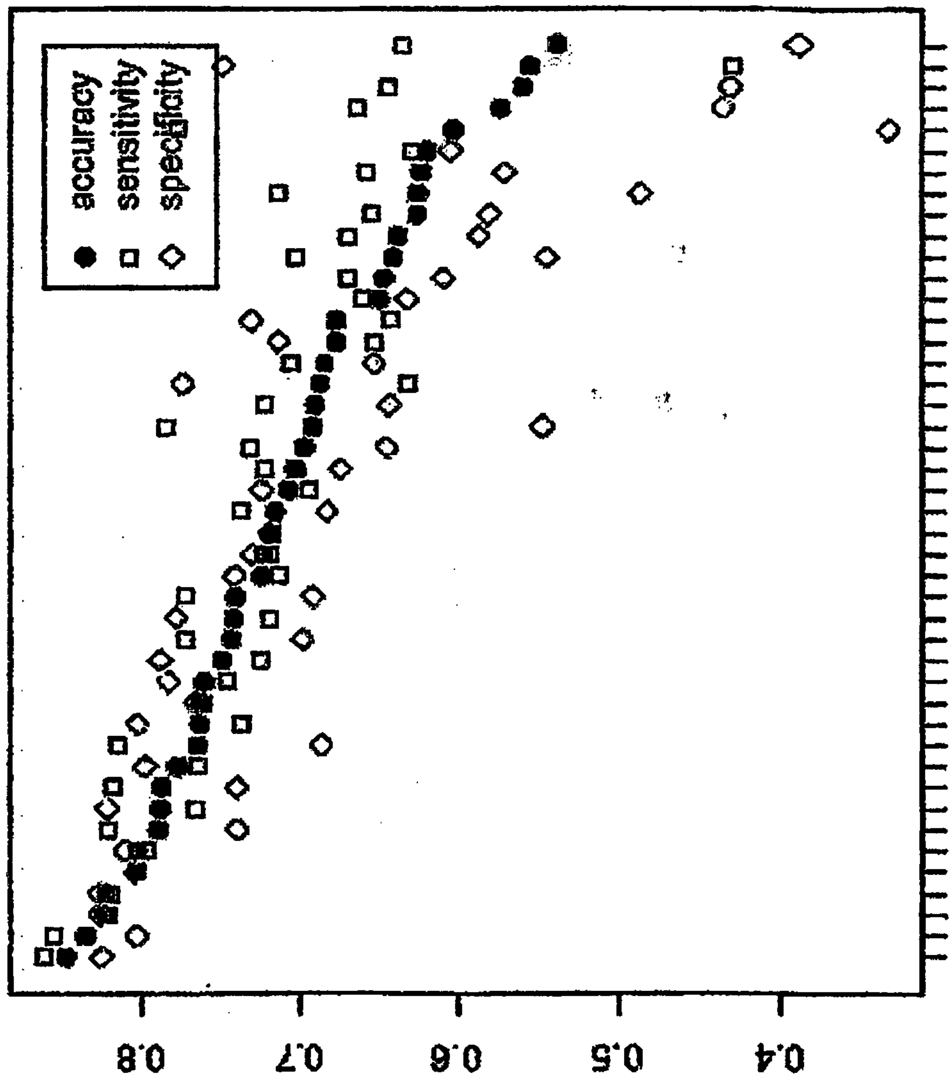


Figure 27

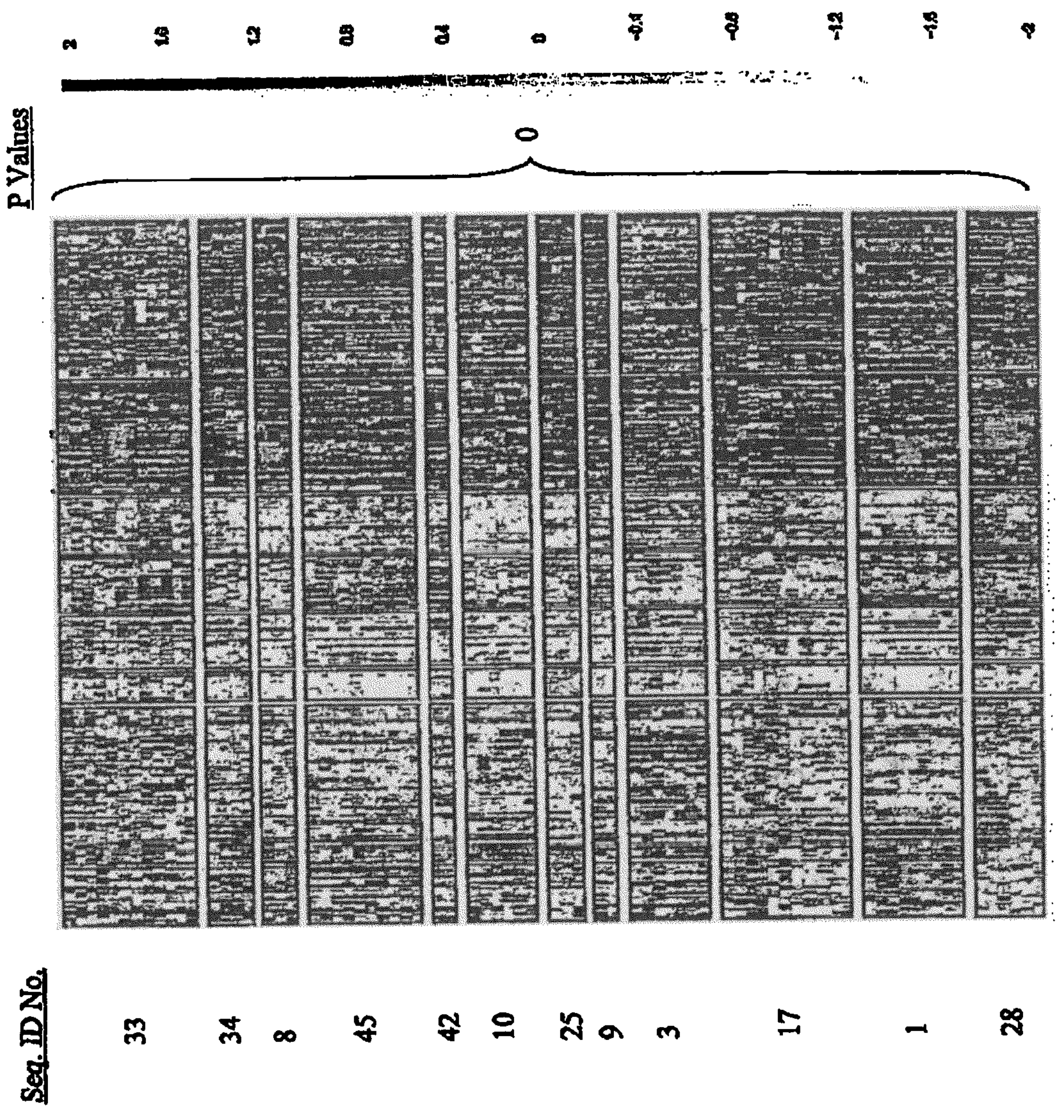
P Values
2
1.6
1.2
0.8
0.4
0
-0.4
-0.8
-1.2
-1.6
-2
0
Seq. ID No.
33
34
8
45
42
10
25
9
3
17
1
28

Figure 28

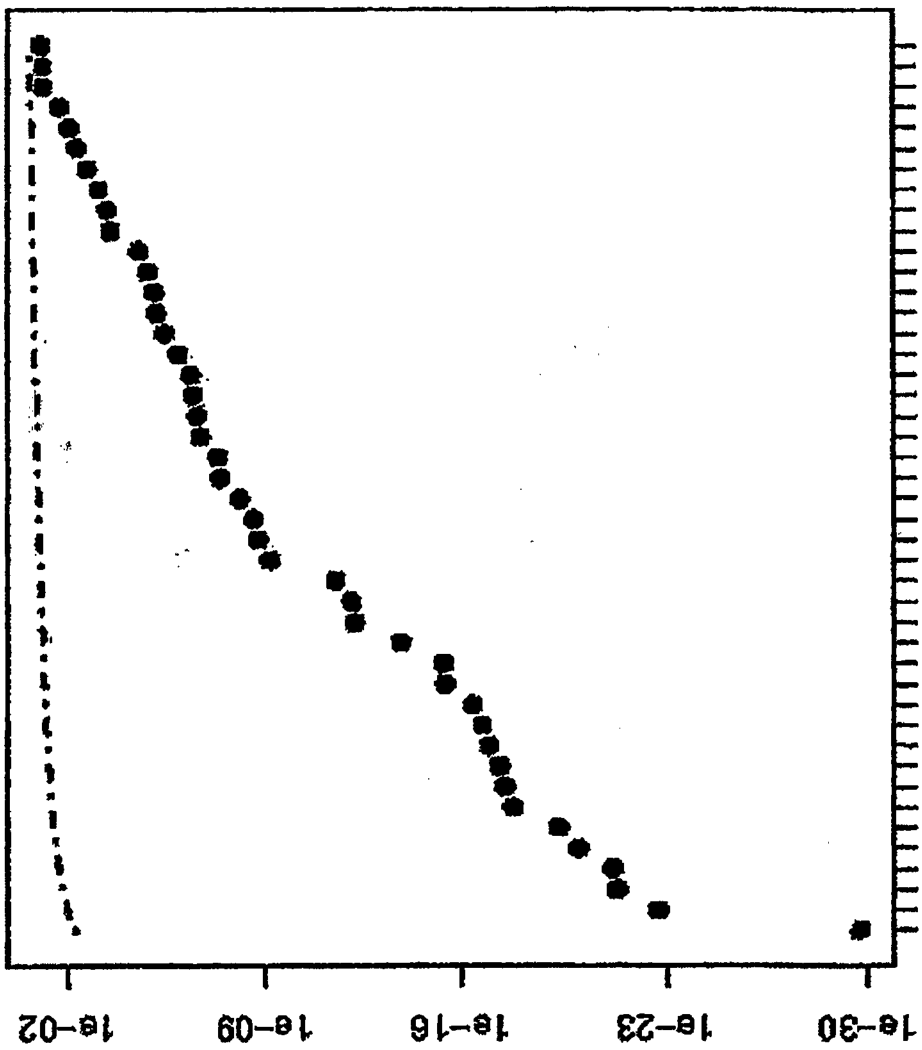


Figure 29

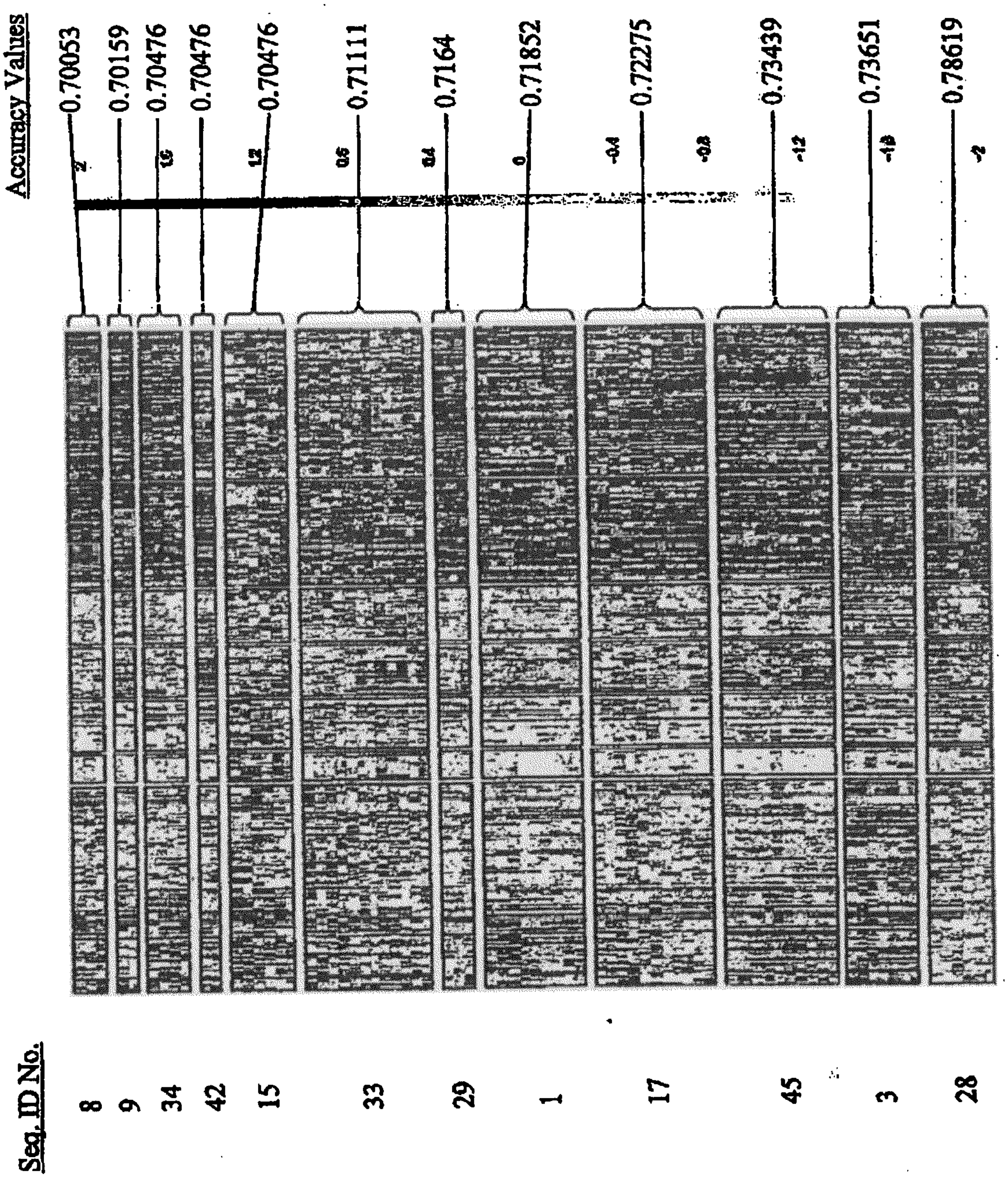
Accuracy Values
0.70053
0.70159
0.70476
0.70476
0.70476
0.71111
0.7164
0.71852
0.72275
0.73439
0.73651
0.78619
2
1.6
1.2
0.8
0.4
0
-0.4
-0.8
-1.2
-1.6
-2
Seq. ID No.
8
9
34
42
15
33
29
1
17
45
3
28

Figure 30

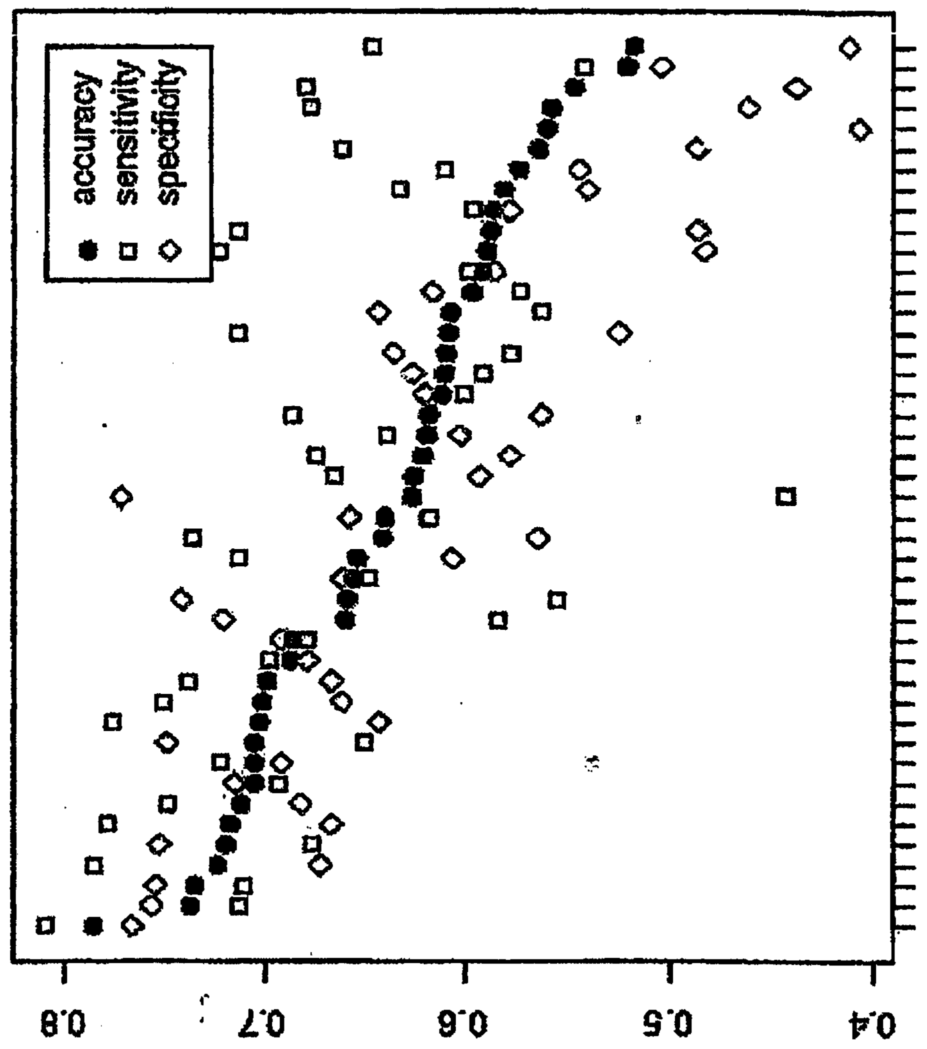


Figure 31

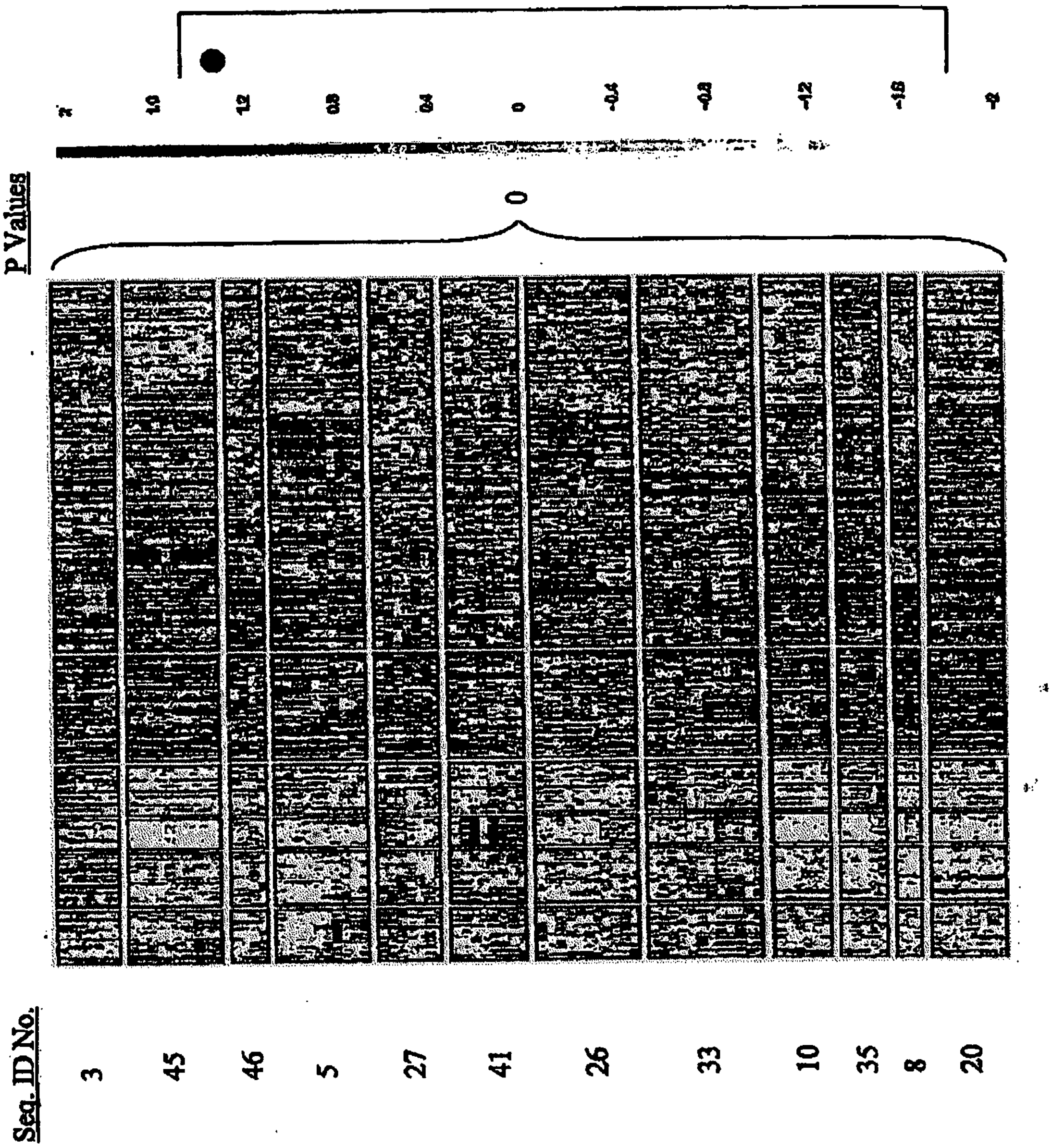
P Values
0
2
1.6
1.2
0.8
0.4
0
-0.4
-0.8
-1.2
-1.6
-2
Seq. ID No.
3
45
46
5
27
41
26
33
10
35
8
20

Figure 32

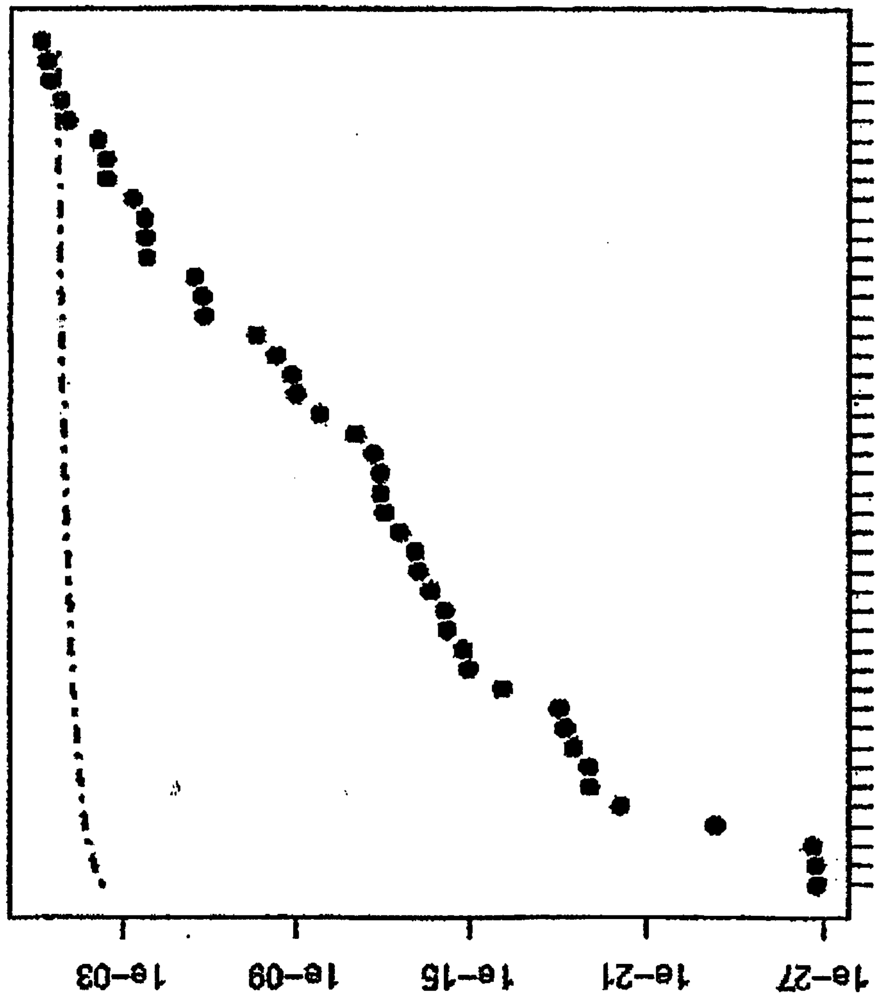


Figure 33

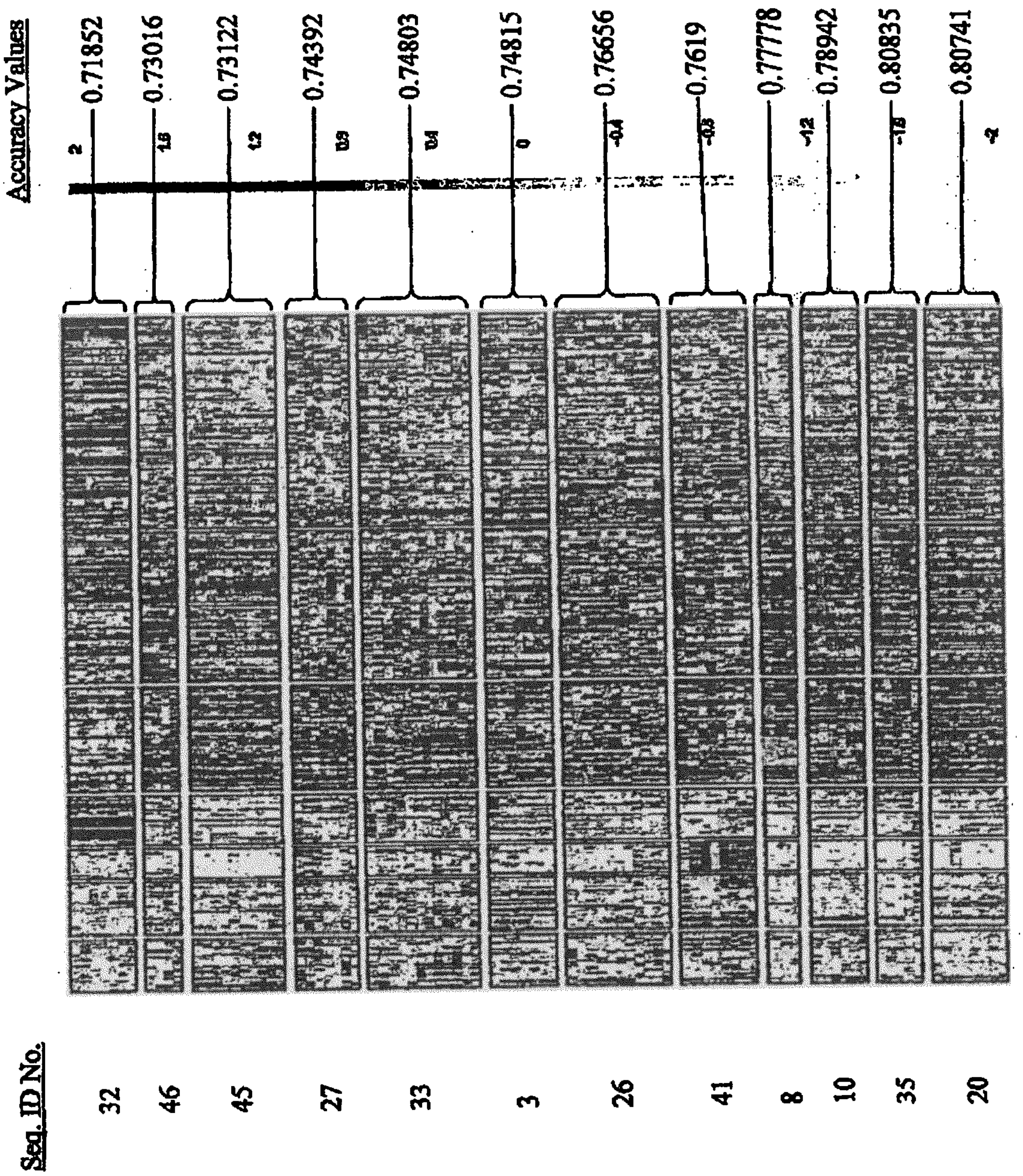
Accuracy Values
0.71852
0.73016
0.73122
0.74392
0.74803
0.74815
0.76656
0.7619
0.77778
0.78942
0.80835
0.80741
2
1.6
1.2
0.8
0.4
0
-0.4
-0.8
-1.2
-1.6
-2
Seq. ID No.
32
46
45
27
33
3
26
41
8
10
35
20

Figure 34

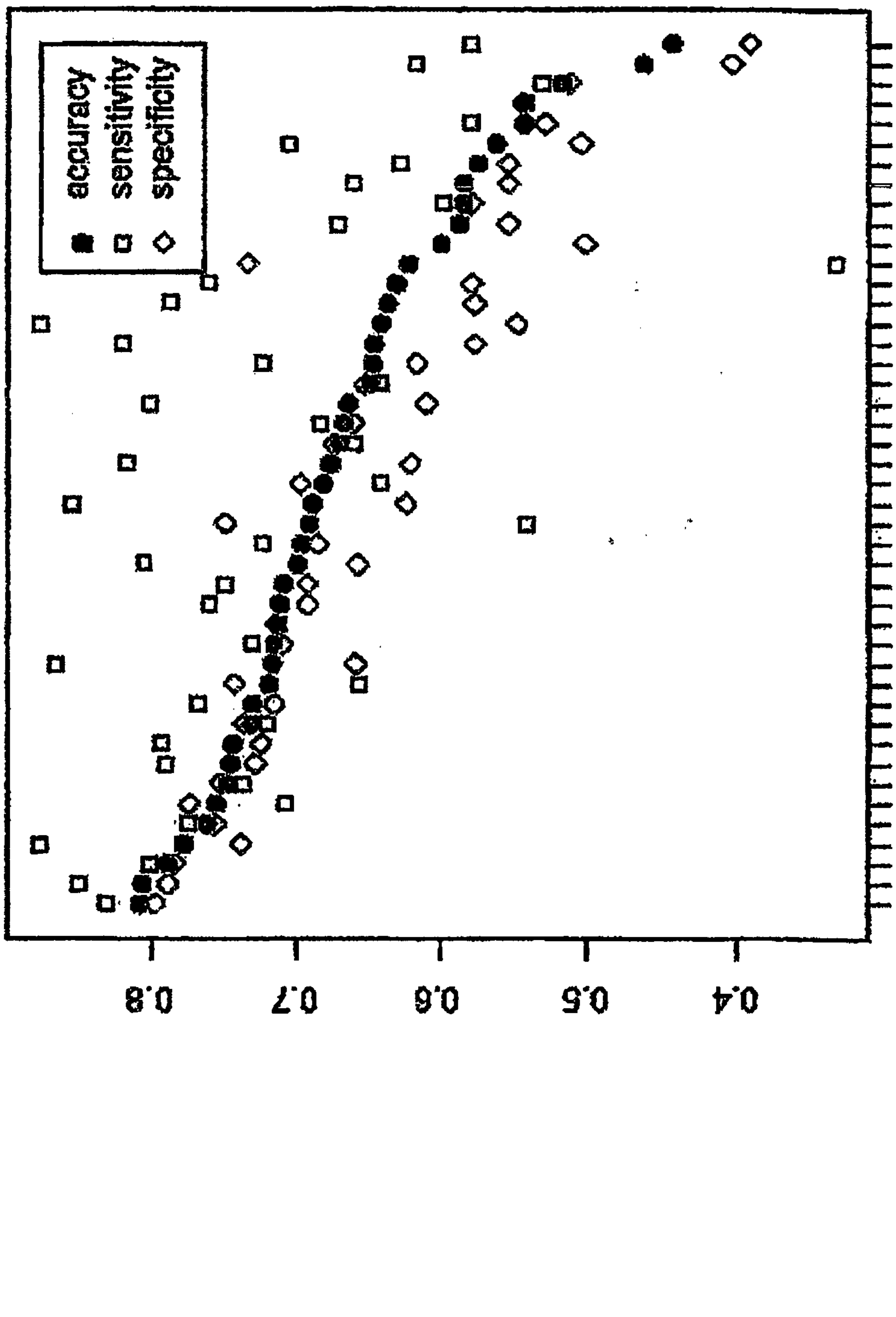


Figure 35

Figure 36

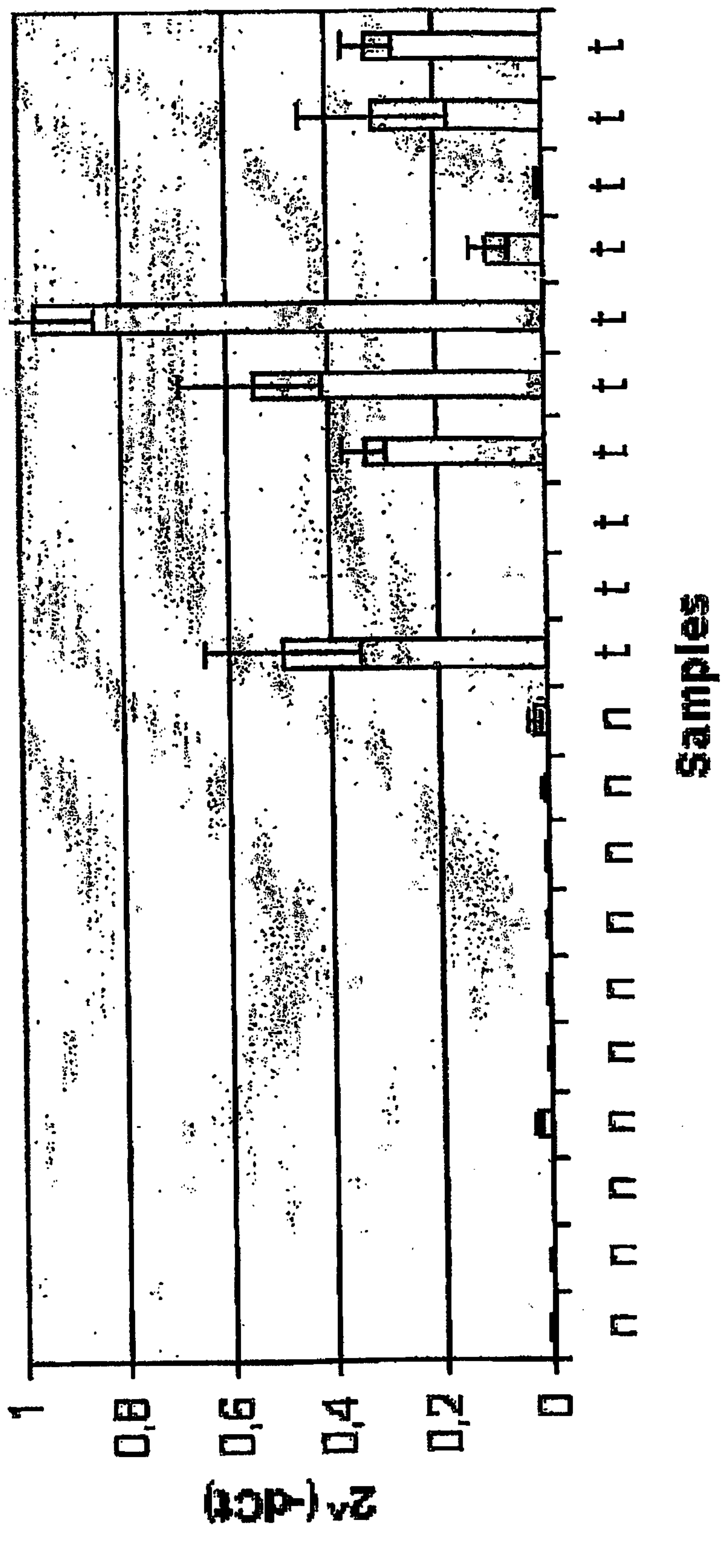

Figure 37

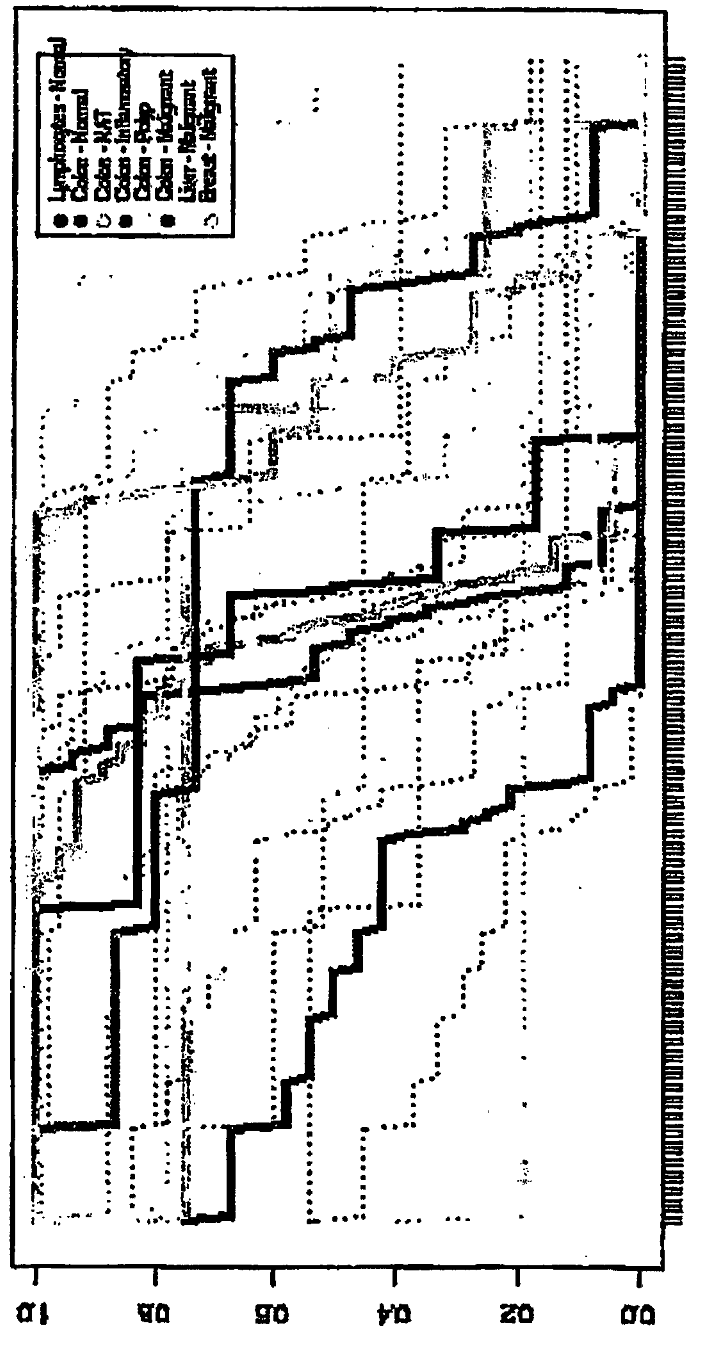
Lymphocytes - Normal
Colon - Normal
Colon - NAT
Colon - Inflammatory
Colon - Polyp
Colon - Malignant
Liver - Malignant
Breast - Malignant

Figure 38

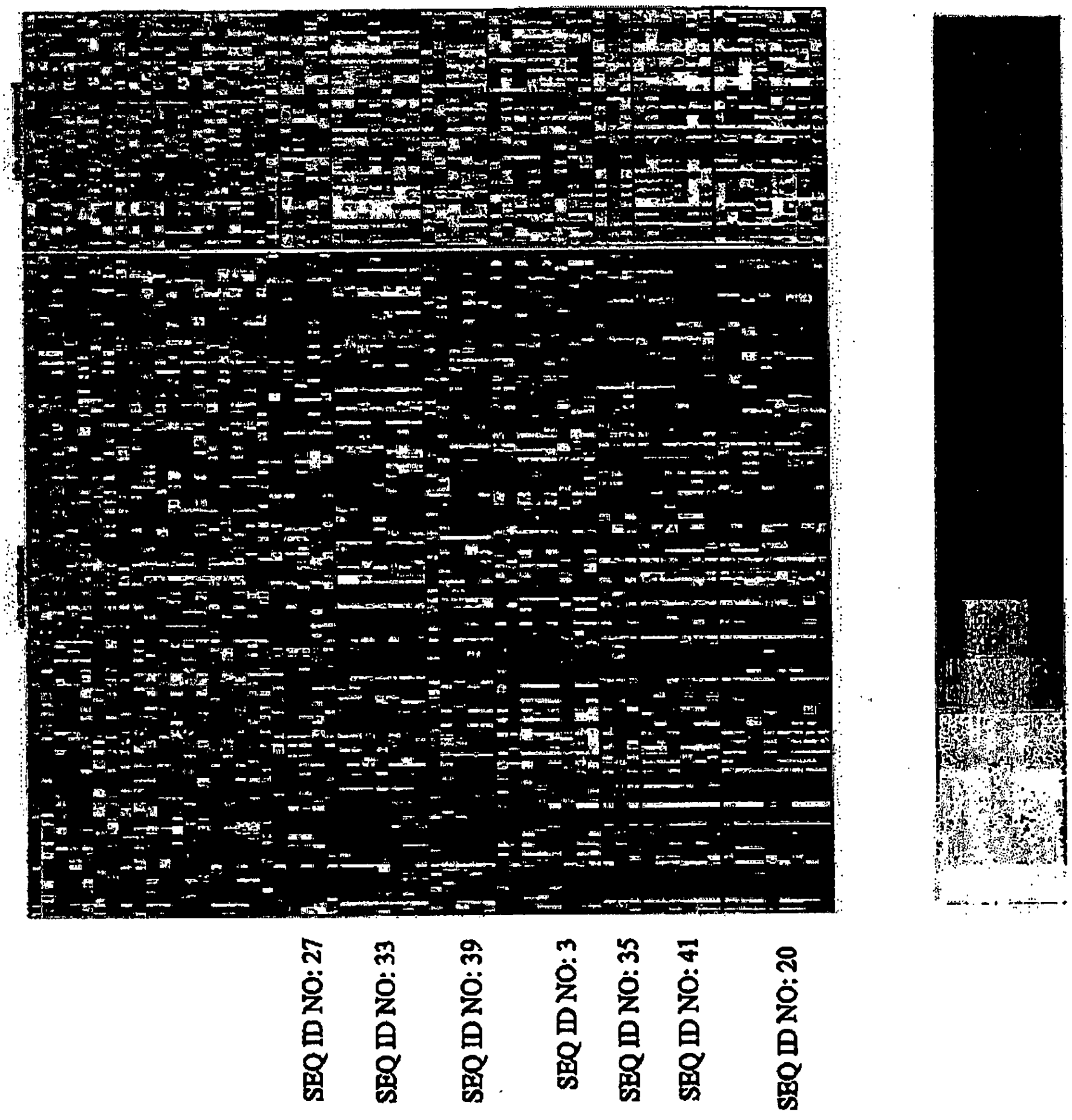
SEQ ID NO: 27
SEQ ID NO: 33
SEQ ID NO: 39
SEQ ID NO: 3
SEQ ID NO: 35
SEQ ID NO: 41
SEQ ID NO: 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5786146 A
- WO 0026401 A1
- WO 9500669 A
- US 6251594 B
- WO 9928498 A, Olek
- WO 9746705 A
- WO 9515373 A
- US 5514758 A
- US 5565552 A
- US 5567810 A
- US 5574142 A
- US 5585481 A
- US 5587371 A
- US 5597696AND5958773 A
- US 20030013091 A
- US 09898743 B
- US 6265171 B, Herman
- US 6331393 B
- US 10562383 A
- US 0420336 W
- US 10679062 B
- US 10603138 B
- US 10602494 B
- EP 04090175 A
- EP 04090072 A


### Non-patent literature cited in the description


- **LIPSHUTZ, R. J. et al.** *Nature Genetics,* 1999, vol. 21, 20-24
- **BOWTELL, D. D. L.** *Nature genetics,* 1999, vol. 21, 25-32
- **SABBIONI et al.** *Molecular Diagnosis,* 2003, vol. 7, 201-207
- **YOUNG J ; BIDEN KG ; SIMMS LA ; HUGGARD P ; KARAMATIC R ; EYRE HJ ; SUTHERLAND GR ; HERATH N ; BARKER M ; ANDERSON GJ.** HPP1: a transmembrane protein-encoding gene commonly methylated in colorectal polyps and cancers. *Proc Natl Acad Sci USA,* 2001, vol. 98, 265-270
- **J.P. EGAN.** Signal Detection Theory and ROC Analysis. Academic Press, 1975
- **GONZALGO et al.** *Cancer Research,* 1997, vol. 57, 594-599
- **EADS et al.** *Cancer Res.,* 1999, vol. 59, 2302-2306
- **GONZALGO ; JONES.** *Nucleic Acids Res.,* 1997, vol. 25, 2529-2531
- **HERMAN et al.** *Proc. Natl. Acad. Sci. USA,* 1996, vol. 93, 9821-9826
- **XIONG ; LAIRD.** *Nucleic Acids Res.,* 1997, vol. 25, 2532-2534
- **TOYOTA et al.** *Cancer Res.,* 1999, vol. 59, 2307-12
- **SAMBROOK et al.** Molecular Cloning, A Laboratory Manual. Cold Spring Harbor Press, 1989
- Current Protocols in Molecular Biology. John Wiley & Sons, 1995
- **OLEK A et al.** A modified and improved method for bisulfite based cytosine methylation analysis. *Nucleic Acids Res.,* 1996, vol. 24, 5064-6
- **REIN, T. et al.** *Nucleic Acids Res.,* 1998, vol. 26, 2255
- **ZESCHNIGK M et al.** *Eur J Hum Genet.,* 1997, vol. 5, 94-98
- **OLEK ; WALTER.** *Nat Genet.,* 1997, vol. 17, 275-6
- **GONZALGO ; JONES.** *Nucleic Acids Res.,* 1997, vol. 25, 2529-31
- **XIONG ; LAIRD.** *Nucleic Acids Res.,* 1997, vol. 25, 2532-4
- **GRIGG ; CLARK.** *Bioessays,* 1994, vol. 16, 431-6
- **ZESCHNIGK M et al.** *Hum Mol Genet.,* 1997, vol. 6, 387-95
- **FEIL R et al.** *Nucleic Acids Res.,* 1994, vol. 22, 695
- **MARTIN V et al.** *Gene,* 1995, vol. 157, 261-4
- **FROMMER et al.** *Proc. Natl. Acad. Sci. USA,* 1992, vol. 89, 1827-1831
- **SADRI ; HORNSBY.** *Nucl. Acids Res.,* 1996, vol. 24, 5058-5059
- **SAMBROOK et al.** Molecular Cloning: A Laboratory Manual. 1989
- **BELYAVSKY et al.** *Nucl Acid Res,* 1989, vol. 17, 2919-2932
- **KRUG ; BERGER.** Methods in Enzymology. Academic Press, 1987, vol. 152, 316-325
- Basic and Clinical Immunology. Appleton & Lange, 1991, 217-262

- **MILSTEIN ; KOHLER.** *Nature,* 1975, vol. 256, 495-497
- **GULFRE ; MILSTEIN.** Methods in Enzymology: Immunochemical Techniques. Academic Press, 1981, vol. 73, 1-46
- **KROL et al.** *BioTechniques,* 1988, vol. 6, 958-976
- **ZON.** *Pharm. Res.,* 1988, vol. 5, 539-549
- *Nature Genetics,* January 1999, vol. 21
- **YU et al.** *BioTechniques,* 1997, vol. 23, 714-720
- **KARAS ; HILLENKAMP.** *Anal Chem,* 1988, vol. 60, 2299-301
- **GUT ; BECK.** *Current Innovations and Future Trends,* 1995, vol. 1, 147-57
- **GUT ; BECK.** *Nucleic Acids Res.,* 1995, vol. 23, 1367-73
- **HEID et al.** *Genome Res.,* 1996, vol. 6, 986-994
- **SANGER F. et al.** *Proc Natl Acad Sci USA,* 1977, vol. 74, 5463-5467
- **IRIZARRY RA ; HOBBS B ; COLLIN F ; BEAZER-BARCLAY YD ; ANTONELLIS KJ ; SCHERF U ; SPEED TP.** Exploration, normalization, and summaries of high density oligonucleotide array probe level data. *Biostatistics,* April 2003, vol. 4 (2), 249-64
- **GAUTIER L ; COPE L ; BOLSTAD BM.** Irizarry RA: affy--analysis of Affymetrix GeneChip data at the probe level. *Bioinformatics,* 12 February 2004, vol. 20 (3), 307-150